(19) Europäisches Patentamt / European Patent Office / Office européen des brevets

(11) EP 4 135 842 B1

(12) **EUROPEAN PATENT SPECIFICATION**

(45) Date of publication and mention of the grant of the patent:
**23.10.2024 Bulletin 2024/43**

(21) Application number: **21717912.6**

(22) Date of filing: **15.04.2021**

(51) International Patent Classification (IPC):
***A61P 29/00*** *(2006.01)* ***C07D 471/04*** *(2006.01)*
***A61K 31/53*** *(2006.01)*

(52) Cooperative Patent Classification (CPC):
**C07D 471/04; A61P 29/00**

(86) International application number:
**PCT/EP2021/059755**

(87) International publication number:
**WO 2021/209539 (21.10.2021 Gazette 2021/42)**

(54) **PYRROLO[1,2-D][1,2,4]TRIAZINE-2-YL-ACETAMIDES AS INHIBITORS OF THE NLRP3 INFLAMMASOME PATHWAY**

PYRROLO[1,2-D][1,2,4]TRIAZIN-2-YL-ACETAMIDE ALS INHIBTOREN DES NLRP3 INFLAMMASOME STOFFWECHSELWEGS

PYRROLO[1,2-D][1,2,4]TRIAZINE-2-YL-ACETAMIDES EN TANT QU'INHIBITEURS DE LA VOIE INFLAMMASOME NLRP3

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.04.2020 EP 20382298**

(43) Date of publication of application:
**22.02.2023 Bulletin 2023/08**

(73) Proprietor: **JANSSEN Pharmaceutica NV**
**2340 Beerse (BE)**

(72) Inventors:
- **OEHLRICH, Daniel**
  **2340 Beerse (BE)**
- **VAN GOOL, Michiel Luc Maria**
  **28042 Madrid (ES)**
- **VAN OPDENBOSCH, Nina**
  **2340 Beerse (BE)**
- **LAMKANFI, Mohamed**
  **2340 Beerse (BE)**
- **LEENAERTS, Joseph, Elisabeth**
  **2340 Beerse (BE)**
- **MARTINEZ VITURRO, Carlos, Manuel**
  **28042 Madrid (ES)**
- **MURATORE, Michael, Eric**
  **2340 Beerse (BE)**

(74) Representative: **Carpmaels & Ransford LLP**
**One Southampton Row**
**London WC1B 5HA (GB)**

(56) References cited:
**WO-A1-2020/021447**

- **DATABASE REGISTRY [online] CHEMICAL ABSTRACTS SERVICE, COLUMBUS, OHIO, US; 10 July 2006 (2006-07-10), XP002800058, Database accession no. 891548-92-8**
- **DATABASE REGISTRY [online] CHEMICAL ABSTRACTS SERVICE, COLUMBUS, OHIO, US; 21 April 2011 (2011-04-21), XP002799887, Database accession no. 1283736-98-0**
- **DATABASE CA [online] CHEMICAL ABSTRACTS SERVICE, COLUMBUS, OHIO, US; 1980, LANCELOT, JEAN CHARLES ET AL: "Pyrrolo[1,2-d][1,2,4]triazines. II. Study of pyrrolo[1,2-d]triazin-1-ones and -4-ones", XP002799888, retrieved from STN Database accession no. 1981:3990**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- LANCELOT, JEAN CHARLES ET AL: "Pyrrolo[1,2-d][1,2,4]triazines. II. Study of pyrrolo[1,2-d]triazin-1-ones and -4-ones", JOURNAL OF HETEROCYCLIC CHEMISTRY , 17(4), 631-5 CODEN: JHTCAD; ISSN: 0022-152X, 1980
- DATABASE CA [online] CHEMICAL ABSTRACTS SERVICE, COLUMBUS, OHIO, US; 1973, ROBBA, M. ET AL: "New synthesis of the pyrrolo[1,2-d]-as-triazine ring", XP002799889, retrieved from STN Database accession no. 1973:546496
- ROBBA, M. ET AL: "New synthesis of the pyrrolo[1,2-d]-as-triazine ring", TETRAHEDRON LETTERS , (35), 3239-41 CODEN: TELEAY; ISSN: 0040-4039, 1973
- DATABASE CA [online] CHEMICAL ABSTRACTS SERVICE, COLUMBUS, OHIO, US; 1997, CHUNCHATPRASERT, LADDAWAN ET AL: "Antitumor heterocycles. Part 15. The synthesis of 2-substituted pyrroloindoles by acid-catalyzed condensations of pyrroles", XP002799890, retrieved from STN Database accession no. 1997:63272
- CHUNCHATPRASERT, LADDAWAN ET AL: "Antitumor heterocycles. Part 15. The synthesis of 2-substituted pyrroloindoles by acid-catalyzed condensations of pyrroles", JOURNAL OF CHEMICAL RESEARCH, SYNOPSES , (1), 2-3 CODEN: JRPSDC; ISSN: 0308-2342, 1997, DOI: 10.1039/A604709B 10.1039/A604709B
- DATABASE REGISTRY [online] CHEMICAL ABSTRACTS SERVICE, COLUMBUS, OHIO, US; 12 May 2006 (2006-05-12), XP002799891, Database accession no. 883948-73-0

## Description

## FIELD OF THE INVENTION

The present invention relates to novel pyrrolotriazinones that are useful as inhibitors of NOD-like receptor protein 3 (NLRP3) inflammasome pathway. The present invention also relates to processes for the preparation of said compounds, pharmaceutical compositions comprising said compounds, methods of using said compounds in the treatment of various diseases and disorders, and medicaments containing them, and their use in diseases and disorders mediated by NLRP3.

## BACKGROUND OF THE INVENTION

Inflammasomes, considered as central signalling hubs of the innate immune system, are multi-protein complexes that are assembled upon activation of a specific set of intracellular pattern recognition receptors (PRRs) by a wide variety of pathogen- or danger- associated molecular patterns (PAMPs or DAMPs). To date, it was shown that inflammasomes can be formed by nucleotide-binding oligomerization domain (NOD)-like receptors (NLRs) and Pyrin- and HIN200-domain-containing proteins (Van Opdenbosch N and Lamkanfi M. Immunity, 2019 Jun 18;50(6): 1352-1364). The NLRP3 inflammasome is assembled upon detection of environmental crystals, pollutants, host-derived DAMPs and protein aggregates (Tartey S and Kanneganti TD. Immunology, 2019 Apr; 156(4):329-338). Clinically relevant DAMPs that engage NLRP3 include uric acid and cholesterol crystals that cause gout and atherosclerosis, amyloid-β fibrils that are neurotoxic in Alzheimer's disease and asbestos particles that cause mesothelioma (Kelley et al., Int J Mol Sci, 2019 Jul 6;20(13)). Additionally, NLRP3 is activated by infectious agents such as *Vibrio cholerae;* fungal pathogens such as *Aspergillus fumigatus* and *Candida albicans;* adenoviruses, influenza A virus and SARS-CoV-2 (Tartey and Kanneganti, 2019 (see above); Fung et al. Emerg Microbes Infect, 2020 Mar 14;9(1):558-570).

Although the precise NLRP3 activation mechanism remains unclear, for human monocytes, it has been suggested that a one-step activation is sufficient while in mice a two-step mechanism is in place. Given the multitude in triggers, the NLRP3 inflammasome requires add-on regulation at both transcriptional and post-transcriptional level (Yang Y et al., Cell Death Dis, 2019 Feb 12; 10(2): 128).

The NLRP3 protein consists of an N-terminal pyrin domain, followed by a nucleotide-binding site domain (NBD) and a leucine-rich repeat (LRR) motif on C-terminal end (Sharif et al., Nature, 2019 Jun; 570(7761):338-343). Upon recognition of PAMP or DAMP, NLRP3 aggregates with the adaptor protein, apoptosis-associated speck-like protein (ASC), and with the protease caspase-1 to form a functional inflammasome. Upon activation, procaspase-1 undergoes autoproteolysis and consequently cleaves gasdermin D (Gsdmd) to produce the N-terminal Gsdmd molecule that will ultimately lead to pore-formation in the plasma membrane and a lytic form of cell death called pyroptosis. Alternatively, caspase-1 cleaves the pro-inflammatory cytokines pro-IL-1β and pro-IL-18 to allow release of its biological active form by pyroptosis (Kelley et al., 2019 - see above).

Dysregulation of the NLRP3 inflammasome or its downstream mediators are associated with numerous pathologies ranging from immune/inflammatory diseases, auto-immune/auto-inflammatory diseases (Cryopyrin-associated Periodic Syndrome (Miyamae T. Paediatr Drugs, 2012 Apr 1;14(2):109-17); sickle cell disease; systemic lupus erythematosus (SLE)) to hepatic disorders (eg. non-alcoholic steatohepatitis (NASH), chronic liver disease, viral hepatitis, alcoholic steatohepatitis, and alcoholic liver disease) (Szabo G and Petrasek J. Nat Rev Gastroenterol Hepatol, 2015 Jul;12(7):387-400) and inflammatory bowel diseases (eg. Crohn's disease, ulcerative colitis) (Zhen Y and Zhang H. Front Immunol, 2019 Feb 28;10:276). Also, inflammatory joint disorders (eg. gout, pseudogout (chondrocalcinosis), arthropathy, osteoarthritis, and rheumatoid arthritis (Vande Walle L et al., Nature, 2014 Aug 7;512(7512):69-73) were linked to NLRP3. Additionally, kidney related diseases (hyperoxaluria (Knauf et al., Kidney Int, 2013 Nov;84(5):895-901), lupus nephritis, hypertensive nephropathy (Krishnan et al., Br J Pharmacol, 2016 Feb;173(4):752-65), hemodialysis related inflammation and diabetic nephropathy which is a kidney-related complication of diabetes (Type 1, Type 2 and mellitus diabetes), also called diabetic kidney disease (Shahzad et al., Kidney Int, 2015 Jan;87(1):74-84) are associated to NLRP3 inflammasome activation. Reports link onset and progression of neuroinflammation-related disorders (eg. brain infection, acute injury, multiple sclerosis, Alzheimer's disease) and neurodegenerative diseases (Parkinsons disease) to NLRP3 inflammasome activation (Sarkar et al., NPJ Parkinsons Dis, 2017 Oct 17;3:30). In addition, cardiovascular or metabolic disorders (eg. cardiovascular risk reduction (CvRR), atherosclerosis, type I and type II diabetes and related complications (e.g. nephropathy, retinopathy), peripheral artery disease (PAD), acute heart failure and hypertension (Ridker et al., CANTOS Trial Group. N Engl J Med, 2017 Sep 21;377(12):1119-1131; and Toldo S and Abbate A. Nat Rev Cardiol, 2018 Apr;15(4):203-214) have recently been associated to NLRP3. Also, skin associated diseases were described (eg. wound healing and scar formation; inflammatory skin diseases, eg. acne, hidradenitis suppurativa (Kelly et al., Br J Dermatol, 2015 Dec;173(6)). In addition, respiratory conditions have been associated with NLRP3 inflammasome activity (eg. asthma, sarcoidosis, *Severe Acute Respiratory Syndrome* (SARS) (Nieto-Torres et al., Virology, 2015 Nov;485:330-9)) but also age-related macular degeneration (Doyle et al., Nat Med, 2012 May;18(5):791-8). Several cancer related diseases/disorders were described linked to NLRP3 (eg. myeloproliferative neoplasms, leukemias, myelodysplastic syndromes (MOS), myelofibrosis, lung cancer, colon cancer (Ridker et al., Lancet, 2017 Oct 21;390(10105):1833-1842; Derangere et al., Cell Death Differ. 2014 Dec;21(12):1914-24; Basiorka et al., Lancet Haematol, 2018 Sep;5(9): e393-e402, Zhang et al., Hum Immunol, 2018 Jan;79(1):57-62).

Several patent applications describe NLRP3 inhibitors, with recent ones including for instance international patent application WO 2020/018975, WO 2020/037116, WO 2020/021447, WO 2020/010143, WO 2019/079119, WO 2019/0166621 and WO 2019/121691, which disclose a range of specific compounds. Various specific compounds can be identified through the Chemical Abstracts Service, for instance compounds that may be available *via* a commercial source as that compound may be part of a library, but which has no ascribed use.

There is a need for inhibitors of the NLRP3 inflammasome pathway to provide new and/or alternative treatments for the diseases/disorders mentioned herein.

## SUMMARY OF THE INVENTION

The scope of the invention and thus of protection is defined by the appended claims. Subject-matter herein disclosed but not encompassed by the claims is not to be understood as forming part of the invention. In particular, any subject-matter relating to a therapeutic method carried out on the human or animal body (e.g. expressed by the wording "use of a compound according to the invention in therapy") shall not form part of the invention and is to be interpreted as meaning, in the context of the invention, the compound for such use (as in claims 12 or 13).

The invention provides compounds which inhibit the NLRP3 inflammasome pathway.

Thus, in an aspect of the invention, there is now provided a compound of formula (I),

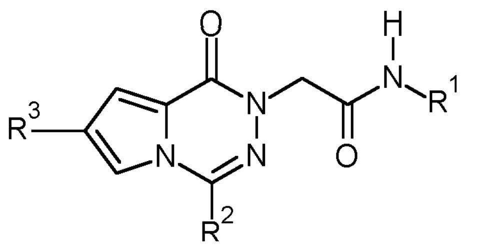

(I)

or a pharmaceutically acceptable salt thereof, wherein:

$R^1$ represents:

(i) $C_{3-6}$ cycloalkyl optionally substituted with one or more substituents independently selected from -OH and $-C_{1-3}$ alkyl;
(ii) aryl or heteroaryl, each of which is optionally substituted with 1 to 3 substituents independently selected from halo, -OH, $-O-C_{1-3}$ alkyl, $-C_{1-3}$ alkyl, halo$C_{1-3}$alkyl, hydroxy$C_{1-3}$ alkyl, halo$C_{1-3}$alkoxy; or
(iii) heterocyclyl, optionally substituted with 1 to 3 substituents independently selected from $C_{1-3}$ alkyl and $C_{3-6}$ cycloalkyl;

$R^2$ represents:

(i) $C_{1-3}$ alkyl optionally substituted with one or more substituents independently selected from halo, -OH, $-OC_{1-3}$alkyl and oxo;
(ii) $C_{3-6}$cycloalkyl;
(iii) $C_{2-4}$alkenyl optionally substituted with $-O-C_{1-3}$alkyl;
(iv) $-O-C_{1-3}$alkyl;
(v) -N(H)alkyl or $-N-(C_{1-3}alkyl)_2$, where each alkyl may be optionally substituted with $-OC_{1-3}$ alkyl; or
(vi) heterocyclyl;

$R^3$ represents:

(i) hydrogen;
(ii) halo;
(iii) $C_{1-4}$ alkyl optionally substituted with one or more substituents independently selected from halo, -OH, oxo,

-O-$C_{1-3}$alkyl, -C(O)OH, -C(O)N(H)heteroaryl, -C(O)N(H)aryl, -C(O)N(H)$C_{1-3}$ alkyl and -C(O)N($C_{1-3}$alkyl)$_2$;
(iv) $C_{2-4}$ alkenyl;
(v) $C_{3-6}$ cycloalkyl;
(vi) -O$C_{1-4}$ alkyl;
(vii) -N(H)$C_{1-3}$alkyl or - N($C_{1-3}$alkyl)$_2$;
(viii) -C(O)N(H)$C_{1-3}$alkyl or -C(O)N($C_{1-3}$alkyl)$_2$;
(ix) aryl or heteroaryl; or
(x) heterocyclyl,

which compounds may referred to herein as "compounds of the invention".

In an embodiment, compounds are of the invention that may be mentioned include those in which:

(i) when $R^2$ represents -$CH_3$, $R^3$ represents H, then $R^1$ does not represent cyclohexyl, 2,3-dimethylcyclohexyl, 2-methoxyphenyl, 2,3,5,6-tetrafluorophenyl, 2-bromo-4,6-difluorophenyl, 4-methylphenyl, 4-(diethylamino)-2-methylphenyl, 5-chloro-2-methoxyphenyl, 2,5-difluorophenyl, 2,3-dihydro-1,4-benzodioxin-6-yl, 4-methylcyclohexyl, 1,2,3,4-tetrahydro-1-naphthalenyl, 3-ethylphenyl, 4-isopropylphenyl, 2-ethyl-6-methylphenyl, 4-bromo-3-methylphenyl, 3,5-dimethylphenyl, 2,4,6-trimethylphenyl, 3,4-dimethylphenyl, 2-chloro-4-methylphenyl, 2,4-dimethylphenyl, 2,3,4,5,6-pentafluorophenyl, 2,5-dimethylphenyl, 3-chloro-2-methylphenyl, 2-methylphenyl, 4-bromo-2-fluorophenyl, 4-fluorophenyl, 2-fluorophenyl, 4-ethylphenyl, 2-ethylphenyl, 2,6-dimethylphenyl, 2,3-dimethylphenyl, 2,4-difluorophenyl, cyclopentyl, cycloheptyl, 5-chloro-2-methylphenyl, 5-chloro-2,4-dimethoxyphenyl, 4-chlorophenyl, 2-chlorophenyl, 2-bromophenyl, 3-methoxyphenyl, 3,4-dimethoxyphenyl, 3-(trifluoromethyl)phenyl or 2-(trifluoromethyl)phenyl;
(ii) when $R^2$ represents -$CH_2CH_3$, $R^3$ represents H, then $R^1$ does not represent cyclopentyl or cycloheptyl,

which we may refer to herein as "the provisos".

Hence, there is provided a compound of formula (I) as hereinbefore defined, or a pharmaceutically acceptable salt thereof, provided that it is not a compound of the provisos.

In another embodiment of the invention, there is provided compounds of the invention in which there is provided a compound of formula (I) as hereinbefore defined, or a pharmaceutically acceptable salt thereof, but in which:

$R^1$ represents:

(i) $C_{3-6}$ cycloalkyl optionally substituted with one or more substituents independently selected from -OH and -$C_{1-3}$ alkyl;
(ii) aryl or heteroaryl, each of which is optionally substituted with 1 to 3 substituents independently selected from halo, -OH, -O-$C_{1-3}$ alkyl, -$C_{1-3}$ alkyl, halo$C_{1-3}$alkyl, hydroxy$C_{1-3}$ alkyl, halo$C_{1-3}$alkoxy; or
(iii) heterocyclyl, optionally substituted with 1 to 3 substituents independently selected from $C_{1-3}$ alkyl and $C_{3-6}$ cycloalkyl;

$R^2$ represents:

(i) $C_{1-6}$ alkyl optionally substituted with one or more substituents independently selected from halo, -OH, -O$C_{1-3}$alkyl, -O$C_{3-6}$ cycloalkyl, -N(H)C(O)$C_{1-3}$alkyl and oxo;
(ii) $C_{3-6}$ cycloalkyl;
(iii) $C_{2-4}$alkenyl optionally substituted with -O-$C_{1-3}$alkyl;
(iv) -O-$C_{1-3}$alkyl;
(v) -C(O)$C_{1-3}$alkyl;
(vi)-N(H)$C_{1-3}$alkyl or -N-($C_{1-3}$alkyl)$_2$, where each alkyl may be optionally substituted with -O$C_{1-3}$ alkyl or $C_{3-6}$ cycloalkyl; or
(vii) heterocyclyl (optionally substituted by one or more substituents selected from halo, $C_{1-3}$ alkyl, -C(O)$C_{1-3}$alkyl and -C(O)O$C_{1-4}$alkyl);

$R^3$ represents:

(i) hydrogen;
(ii) halo or -CN;
(iii) $C_{1-4}$ alkyl optionally substituted with one or more substituents independently selected from halo, -OH, oxo, -O-$C_{1-3}$alkyl, -C(O)OH, -C(O)N(H)heteroaryl, -C(O)N(H)aryl, -C(O)N(H)$C_{1-3}$ alkyl, -C(O)N($C_{1-3}$-alkyl)$_2$, -N($C_{1-3}$

alkyl)-C(O)-$C_{1-3}$alkyl, -N(H)$C_{1-3}$ alkyl, heterocyclyl, aryl and heteroaryl, which latter three groups may themselves be optionally substituted by one or more substituents selected from halo, $C_{1-3}$ alkyl and, where applicable, =O;

(iv) $C_{2-4}$ alkenyl;

(v) $C_{3-6}$ cycloalkyl;

(vi) -O$C_{1-4}$ alkyl;

(vii) -C(O)H or -C(O)$C_{1-3}$ alkyl;

(viii) -N(H)$C_{1-3}$alkyl or - N($C_{1-3}$alkyl)$_2$;

(ix) -C(O)N(H)$C_{1-3}$alkyl or -C(O)N($C_{1-3}$alkyl)$_2$;

(x) aryl or heteroaryl, which groups may themselves be optionally substituted by one or more substituents selected from halo, $C_{1-3}$ alkyl, -C(O)O$C_{1-4}$alkyl and, where applicable, =O; or

(xi) Heterocyclyl, optionally substituted by one or more substituents selected from halo, $C_{1-3}$ alkyl, -C(O)O$C_{1-4}$alkyl and, where applicable, =O,

provided that it is not a compound of the provisos,

which compounds may also be referred to herein as "compounds of the invention".

In another aspect, there is provided compounds of the invention (without the provisos, where applicable) for use as a medicament. In another aspect, there is provided a pharmaceutical composition comprising a therapeutically effective amount of a compound of the invention (without the provisos, again where applicable).

In a further aspect, there is provided compounds of the invention (without the provisos) (and/or pharmaceutical compositions comprising such compounds) for use: in the treatment of a disease or disorder associated with NLRP3 activity (including inflammasome activity); in the treatment of a disease or disorder in which the NLRP3 signalling contributes to the pathology, and/or symptoms, and/or progression, of said disease/disorder; in inhibiting NLRP3 inflammasome activity (including in a subject in need thereof); and/or as an NLRP3 inhibitor. Specific diseases or disorders may be mentioned herein, and may for instance be selected from inflammasome-related diseases or disorders, immune diseases, inflammatory diseases, auto-immune diseases, or auto-inflmmatory diseases.

In another aspect, there is provided a use of compounds of the invention (without the provisos) (and/or pharmaceutical compositions comprising such compounds): in the treatment of a disease or disorder associated with NLRP3 activity (including inflammasome activity); in the treatment of a disease or disorder in which the NLRP3 signalling contributes to the pathology, and/or symptoms, and/or progression, of said disease/disorder; in inhibiting NLRP3 inflammasome activity (including in a subject in need thereof); and/or as an NLRP3 inhibitor.

In another aspect, there is provided use of compounds of the invention (without the provisos) (and/or pharmaceutical compositions comprising such compounds) in the manufacture of a medicament for: the treatment of a disease or disorder associated with NLRP3 activity (including inflammasome activity); the treatment of a disease or disorder in which the NLRP3 signalling contributes to the pathology, and/or symptoms, and/or progression, of said disease/disorder; and/or inhibiting NLRP3 inflammasome activity (including in a subject in need thereof).

In another aspect, there is provided a method of treating a disease or disorder in which the NLRP3 signalling contributes to the pathology, and/or symptoms, and/or progression, of said disease/disorder, comprising administering a therapeutically effective amount of a compound of the invention, for instance to a subject (in need thereof). In a further aspect there is provided a method of inhibiting the NLRP3 inflammasome activity in a subject (in need thereof), the method comprising administering to the subject in need thereof a therapeutically effective amount of a compound of the invention.

In further aspect, there is a provided a compound of the invention in combination (including a pharmaceutical combination) with one or more therapeutic agents (for instance as described herein). Such combination may also be provided for use as described herein in respect of compounds of the invention (without the provisos), or, a use of such combination as described herein in respect of compounds of the invention (without the provisos). There may also be provided methods as described herein in repsect of compounds of the invention (without the provisos), but wherein the method comprises administering a therapeutically effective amount of such combination.

## DETAILED DESCRIPTION OF THE INVENTION

The invention provides a compound of formula (I),

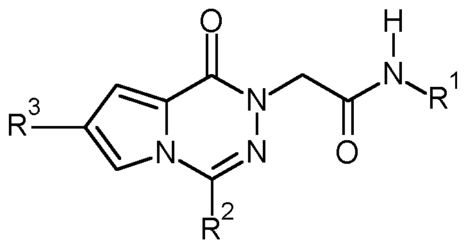

(I)

or a pharmaceutically acceptable salt thereof, wherein:

$R^1$ represents:

(i) $C_{3-6}$ cycloalkyl optionally substituted with one or more substituents independently selected from -OH and -$C_{1-3}$ alkyl;
(ii) aryl or heteroaryl, each of which is optionally substituted with 1 to 3 substituents independently selected from halo, -OH, -O-$C_{1-3}$ alkyl, -$C_{1-3}$ alkyl, halo$C_{1-3}$alkyl, hydroxy$C_{1-3}$ alkyl, halo$C_{1-3}$alkoxy; or
(iii) heterocyclyl, optionally substituted with 1 to 3 substituents independently selected from $C_{1-3}$ alkyl and $C_{3-6}$ cycloalkyl;

$R^2$ represents:

(i) $C_{1-3}$ alkyl optionally substituted with one or more substituents independently selected from halo, -OH, -O$C_{1-3}$alkyl and oxo;
(ii) $C_{3-6}$cycloalkyl;
(iii) $C_{2-4}$alkenyl optionally substituted with -O-$C_{1-3}$alkyl;
(iv) -O-$C_{1-3}$alkyl;
(v) -N(H)alkyl or -N-($C_{1-3}$alkyl)$_2$, where each alkyl may be optionally substituted with -O$C_{1-3}$ alkyl; or
(vi) heterocyclyl;

$R^3$ represents:

(i) hydrogen;
(ii) halo;
(iii) $C_{1-4}$ alkyl optionally substituted with one or more substituents independently selected from halo, -OH, oxo, -O-$C_{1-3}$alkyl, -C(O)OH, -C(O)N(H)heteroaryl, -C(O)N(H)aryl, -C(O)N(H)$C_{1-3}$ alkyl and -C(O)N($C_{1-3}$alkyl)$_2$;
(iv) $C_{2-4}$ alkenyl;
(v) $C_{3-6}$ cycloalkyl;
(vi) -O$C_{1-4}$ alkyl;
(vii) -N(H)$C_{1-3}$alkyl or - N($C_{1-3}$alkyl)$_2$;
(viii) -C(O)N(H)$C_{1-3}$alkyl or -C(O)N($C_{1-3}$alkyl)$_2$;
(ix) aryl or heteroaryl; or
(x) heterocyclyl.

As indicated above, such compounds may be referred to herein as "compounds of the invention".

Pharmaceutically-acceptable salts include acid addition salts and base addition salts. Such salts may be formed by conventional means, for example by reaction of a free acid or a free base form of a compound of the invention with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

Pharmaceutically acceptable acid addition salts can be formed with inorganic acids and organic acids.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like.

Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine, and tromethamine

For the purposes of this invention solvates, prodrugs, N-oxides and stereoisomers of compounds of the invention are also included within the scope of the invention.

The term "prodrug" of a relevant compound of the invention includes any compound that, following oral or parenteral administration, is metabolised *in vivo* to form that compound in an experimentally-detectable amount, and within a predetermined time (e.g. within a dosing interval of between 6 and 24 hours (i.e. once to four times daily)). For the avoidance of doubt, the term "parenteral" administration includes all forms of administration other than oral administration.

Prodrugs of compounds of the invention may be prepared by modifying functional groups present on the compound in such a way that the modifications are cleaved, *in vivo* when such prodrug is administered to a mammalian subject. The modifications typically are achieved by synthesising the parent compound with a prodrug substituent. Prodrugs include compounds of the invention wherein a hydroxyl, amino, sulfhydryl, carboxy or carbonyl group in a compound of the invention is bonded to any group that may be cleaved *in vivo* to regenerate the free hydroxyl, amino, sulfhydryl, carboxy or carbonyl group, respectively.

Examples of prodrugs include, but are not limited to, esters and carbamates of hydroxy functional groups, esters groups of carboxyl functional groups, N-acyl derivatives and N-Mannich bases. General information on prodrugs may be found e.g. in Bundegaard, H. "Design of Prodrugs" p. 1-92, Elesevier, New York-Oxford (1985).

Compounds of the invention may contain double bonds and may thus exist as E (*entgegen*) and *Z* (*zusammenl)* geometric isomers about each individual double bond. Positional isomers may also be embraced by the compounds of the invention. All such isomers (e.g. if a compound of the invention incorporates a double bond or a fused ring, the cis- and trans- forms, are embraced) and mixtures thereof are included within the scope of the invention (e.g. single positional isomers and mixtures of positional isomers may be included within the scope of the invention).

Compounds of the invention may also exhibit tautomerism. All tautomeric forms (or tautomers) and mixtures thereof are included within the scope of the invention. The term "tautomer" or "tautomeric form" refers to structural isomers of different energies which are interconvertible *via* a low energy barrier. For example, proton tautomers (also known as prototropic tautomers) include interconversions *via* migration of a proton, such as keto-enol and imine-enamine isomerisations. Valence tautomers include interconversions by reorganisation of some of the bonding electrons.

Compounds of the invention may also contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation (i.e. a 'chiral pool' method), by reaction of the appropriate starting material with a 'chiral auxiliary' which can subsequently be removed at a suitable stage, by derivatisation (i.e. a resolution, including a dynamic resolution), for example with a homochiral acid followed by separation of the diastereomeric derivatives by conventional means such as chromatography, or by reaction with an appropriate chiral reagent or chiral catalyst all under conditions known to the skilled person.

All stereoisomers (including but not limited to diastereoisomers, enantiomers and atropisomers) and mixtures thereof (e.g. racemic mixtures) are included within the scope of the invention.

In the structures shown herein, where the stereochemistry of any particular chiral atom is not specified, then all stereoisomers are contemplated and included as the compounds of the invention. Where stereochemistry is specified by a solid wedge or dashed line representing a particular configuration, then that stereoisomer is so specified and defined.

When an absolute configuration is specified, it is according to the Cahn-Ingold-Prelog system. The configuration at an asymmetric atom is specified by either R or S. Resolved compounds whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other isomers. Thus, when a compound of formula (I) is for instance specified as (R), this means that the compound is substantially free of the (S) isomer.

The compounds of the present invention may exist in unsolvated as well as solvated forms with pharmaceutically acceptable solvents such as water, ethanol, and the like, and it is intended that the invention embrace both solvated

and unsolvated forms.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Exemplary isotopes that can be incorporated into compounds of the invention include isotopes of hydrogen, carbon, nitrogen, oxygen, phosphorus, sulfur, fluorine, chlorine and iodine, such as $^{2}H$, $^{3}H$, $^{11}C$, $^{13}C$, $^{14}C$ , $^{13}N$, $^{15}O$, $^{17}O$, $^{18}O$, $^{32}P$, $^{33}P$, $^{35}S$, $^{18}F$, $^{36}Cl$, $^{123}I$, and $^{125}I$. Certain isotopically-labeled compounds of the present invention (e.g., those labeled with $^{3}H$ and $^{14}C$) are useful in compound and for substrate tissue distribution assays. Tritiated ($^{3}H$) and carbon-l4 ($^{14}C$) isotopes are useful for their ease of preparation and detectability. Further, substitution with heavier isotopes such as deuterium (i.e., $^{2}H$ may afford certain therapeutic advantages resulting from greater metabolic stability (e.g., increased *in vivo* half-life or reduced dosage requirements) and hence may be preferred in some circumstances. Positron emitting isotopes such as $^{15}O$, $^{13}N$, $^{11}C$ and $^{18}F$ are useful for positron emission tomography (PET) studies to examine substrate receptor occupancy. Isotopically labeled compounds of the present invention can generally be prepared by following procedures analogous to those disclosed in the description/Examples hereinbelow, by substituting an isotopically labeled reagent for a non-isotopically labeled reagent.

Unless otherwise specified, $C_{1-q}$ alkyl groups (where q is the upper limit of the range) defined herein may be straight-chain or, when there is a sufficient number (i.e. a minimum of two or three, as appropriate) of carbon atoms, be branched-chain. Such a group is attached to the rest of the molecule by a single bond.

$C_{2-q}$ alkenyl when used herein (again where q is the upper limit of the range) refers to an alkyl group that contains unsaturation, i.e. at least one double bond.

$C_{3-q}$ cycloalkyl (where q is the upper limit of the range) refers to an alkyl group that is cyclic, for instance cycloalkyl groups may be monocyclic or, if there are sufficient atoms, bicyclic. In an embodiment, such cycloalkyl groups are monocyclic. Such cycloalkyl groups are unsaturated. Substituents may be attached at any point on the cycloalkyl group.

The term "halo", when used herein, preferably includes fluoro, chloro, bromo and iodo.

$C_{1-q}$ alkoxy groups (where q is the upper limit of the range) refers to the radical of formula $-OR^a$, where $R^a$ is a $C_{1-q}$ alkyl group as defined herein.

Halo$C_{1-q}$ alkyl (where q is the upper limit of the range) goups refer to $C_{1-q}$ alkyl groups, as defined herein, where such group is substituted by one or more halo. Hydroxy$C_{1-q}$ alkyl (where q is the upper limit of the range) refers to $C_{1-q}$ alkyl groups, as defined herein, where such group is substituted by one or more (e.g. one) hydroxy (-OH) groups (or one or more, e.g. one, of the hydrogen atoms is replaced with -OH). Similarly, halo$C_{1-q}$ alkoxy and hydroxy$C_{1-q}$ alkoxy represent corresponding $-OC_{1-q}$ alkyl groups that are substituted by one or more halo, or, substituted by one or more (e.g. one) hydroxy, respectively.

Heterocyclyl groups that may be mentioned include non-aromatic monocyclic and bicyclic heterocyclyl groups in which at least one (e.g. one to four) of the atoms in the ring system is other than carbon (i.e. a heteroatom), and in which the total number of atoms in the ring system is between 3 and 20 (e.g. between three and ten, e.g between 3 and 8, such as 5- to 8-). Such heterocyclyl groups may also be bridged. Such heterocyclyl groups are saturated. $C_{2-q}$ heterocyclyl groups that may be mentioned include 7-azabicyclo[2.2.1]heptanyl, 6-azabicyclo[3.1.1]heptanyl, 6-azabicyclo[3.2.1]-octanyl, 8-azabicyclo-[3.2.1]octanyl, aziridinyl, azetidinyl, dihydropyranyl, dihydropyridyl, dihydropyrrolyl (including 2,5-dihydropyrrolyl), dioxolanyl (including 1,3-dioxolanyl), dioxanyl (including 1,3-dioxanyl and 1,4-dioxanyl), dithianyl (including 1,4-dithianyl), dithiolanyl (including 1,3-dithiolanyl), imidazolidinyl, imidazolinyl, morpholinyl, 7-oxabicyclo[2.2.1]heptanyl, 6-oxabicyclo-[3.2.1]octanyl, oxetanyl, oxiranyl, piperazinyl, piperidinyl, non-aromatic pyranyl, pyrazolidinyl, pyrrolidinonyl, pyrrolidinyl, pyrrolinyl, quinuclidinyl, sulfolanyl, 3-sulfolenyl, tetrahydropyranyl, tetrahydrofuranyl, tetrahydropyridyl (such as 1,2,3,4-tetrahydropyridyl and 1,2,3,6-tetrahydropyridyl), thietanyl, thiiranyl, thiolanyl, thiomorpholinyl, trithianyl (including 1,3,5-trithianyl), tropanyl and the like. Substituents on heterocyclyl groups may, where appropriate, be located on any atom in the ring system including a heteroatom. The point of attachment of heterocyclyl groups may be *via* any atom in the ring system including (where appropriate) a heteroatom (such as a nitrogen atom), or an atom on any fused carbocyclic ring that may be present as part of the ring system. Heterocyclyl groups may also be in the *N*- or *S*- oxidised form. In an embodiment, heterocyclyl groups mentioned herein are monocyclic.

Aryl groups that may be mentioned include $C_{6-20}$, such as $C_{6-12}$ (e.g. $C_{6-10}$) aryl groups. Such groups may be monocyclic, bicyclic or tricyclic and have between 6 and 12 (e.g. 6 and 10) ring carbon atoms, in which at least one ring is aromatic. $C_{6-10}$ aryl groups include phenyl, naphthyl and the like, such as 1,2,3,4-tetrahydronaphthyl. The point of attachment of aryl groups may be *via* any atom of the ring system. For example, when the aryl group is polycyclic the point of attachment may be *via* atom including an atom of a non-aromatic ring. However, when aryl groups are polycyclic (e.g. bicyclic or tricyclic), they are preferably linked to the rest of the molecule *via* an aromatic ring. When aryl groups are polycyclic, in an embodiment, each ring is aromatic. In an embodiment, aryl groups mentioned herein are monocyclic or bicyclic. In a further embodiment, aryl groups mentioned herein are monocyclic.

"Heteroaryl" when used herein refers to an aromatic group containing one or more heteroatom(s) (e.g. one to four heteroatoms) preferably selected from N, O and S. Heteroaryl groups include those which have between 5 and 20 members (e.g. between 5 and 10) and may be monocyclic, bicyclic or tricyclic, provided that at least one of the rings is aromatic (so forming, for example, a mono-, bi-, or tricyclic heteroaromatic group). When the heteroaryl group is polycyclic the point of attachment may be *via* any atom including an atom of a non-aromatic ring. However, when heteroaryl groups are polycyclic (e.g. bicyclic or tricyclic), they are preferably linked to the rest of the molecule *via* an aromatic ring. In an embodiment, when heteroaryl groups are polycyclic, then each ring is aromatic. Heteroaryl groups that may be mentioned include 3,4-dihydro-1*H*-isoquinolinyl, 1,3-dihydroisoindolyl, 1,3-dihydroisoindolyl (e.g. 3,4-dihydro-1*H*-isoquinolin-2-yl, 1,3-dihydroisoindol-2-yl, 1,3-dihydroisoindol-2-yl; i.e. heteroaryl groups that are linked *via* a non-aromatic ring), or, preferably, acridinyl, benzimidazolyl, benzodioxanyl, benzodioxepinyl, benzodioxolyl (including 1,3-benzodioxolyl), benzofuranyl, benzofurazanyl, benzothiadiazolyl (including 2,1,3-benzothiadiazolyl), benzothiazolyl, benzoxadiazolyl (including 2,1,3-benzoxadiazolyl), benzoxazinyl (including 3,4-dihydro-2*H*-1,4-benzoxazinyl), benzoxazolyl, benzomorpholinyl, benzoselenadiazolyl (including 2,1,3-benzoselenadiazolyl), benzothienyl, carbazolyl, chromanyl, cinnolinyl, furanyl, imidazolyl, imidazo[1,2-*a*]pyridyl, indazolyl, indolinyl, indolyl, isobenzofuranyl, isochromanyl, isoindolinyl, isoindolyl, isoquinolinyl, isothiaziolyl, isothiochromanyl, isoxazolyl, naphthyridinyl (including 1,6-naphthyridinyl or, preferably, 1,5-naphthyridinyl and 1,8-naphthyridinyl), oxadiazolyl (including 1,2,3-oxadiazolyl, 1,2,4-oxadiazolyl and 1,3,4-oxadiazolyl), oxazolyl, phenazinyl, phenothiazinyl, phthalazinyl, pteridinyl, purinyl, pyranyl, pyrazinyl, pyrazolyl, pyridazinyl, pyridyl, pyrimidinyl, pyrrolyl, quinazolinyl, quinolinyl, quinolizinyl, quinoxalinyl, tetrahydroisoquinolinyl (including 1,2,3,4-tetrahydroisoquinolinyl and 5,6,7,8-tetrahydroisoquinolinyl), tetrahydroquinolinyl (including 1,2,3,4-tetrahydroquinolinyl and 5,6,7,8-tetrahydroquinolinyl), tetrazolyl, thiadiazolyl (including 1,2,3-thiadiazolyl, 1,2,4-thiadiazolyl and 1,3,4-thiadiazolyl), thiazolyl, thiochromanyl, thiophenetyl, thienyl, triazolyl (including 1,2,3-triazolyl, 1,2,4-triazolyl and 1,3,4-triazolyl) and the like. Substituents on heteroaryl groups may, where appropriate, be located on any atom in the ring system including a heteroatom. The point of attachment of heteroaryl groups may be *via* any atom in the ring system including (where appropriate) a heteroatom (such as a nitrogen atom), or an atom on any fused carbocyclic ring that may be present as part of the ring system. Heteroaryl groups may also be in the *N*- or *S*- oxidised form. When heteroaryl groups are polycyclic in which there is a non-aromatic ring present, then that non-aromatic ring may be substituted by one or more =O group. In an embodiment, heteroaryl groups mentioned herein may be monocyclic or bicyclic. In a further embodiment, heteroaryl groups mentioned herein are monocyclic.

Heteroatoms that may be mentioned include phosphorus, silicon, boron and, preferably, oxygen, nitrogen and sulfur.

For the avoidance of doubt, where it is stated herein that a group may be substituted by one or more substituents (e.g. selected from $C_{1-6}$ alkyl), then those substituents (e.g. alkyl groups) are independent of one another. That is, such groups may be substituted with the same substituent (e.g. same alkyl substituent) or different (e.g. alkyl) substituents.

All individual features (e.g. preferred features) mentioned herein may be taken in isolation or in combination with any other feature (including preferred feature) mentioned herein (hence, preferred features may be taken in conjunction with other preferred features, or independently of them).

The skilled person will appreciate that compounds of the invention that are the subject of this invention include those that are stable. That is, compounds of the invention include those that are sufficiently robust to survive isolation from e.g. a reaction mixture to a useful degree of purity.

Various embodiments of the invention will now be described, including embodiments of the compounds of the invention.

In an embodiment of the invention, $R^2$ does not represent unsubstituted methyl or ethyl. In an alternative embodiment, when $R^2$ represents $C_{1-3}$ alkyl, then it represents $C_3$ alkyl optionally substituted as defined herein or $C_{1-2}$ alkyl substituted as defined herein.

In an embodiment, compounds of the invention include those in which $R^1$ represents: (i) $C_{3-6}$ cycloalkyl; (ii) aryl or heteroaryl; or (iii) or heterocyclyl, all of which are optionally substituted as herein defined.

In an embodiment when $R^1$ represents optionally substituted $C_{3-6}$ cycloalkyl, then it represents $C_{3-4}$ cycloalkyl optionally substituted by one or two substituents selected from $C_{1-3}$ alkyl (e.g. methyl) and -OH. In a further embodiment, $R^1$ represents cyclopropyl (e.g. unsubstituted) or cyclobutyl. In yet a further embodiment, $R^1$ represents unsubstituted cyclopropyl or cyclobutyl substituted by -OH and methyl (e.g. at the same carbon atom). In an embodiment therefore, $R^1$ represents:

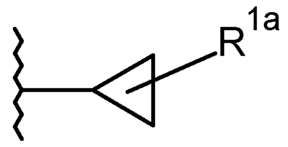

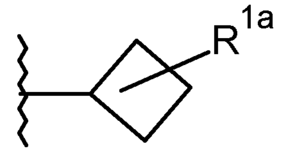

where each $R^{1a}$ represents one or two optional substituents selected from -OH and $C_{1-3}$ alkyl (e.g. methyl). In a particular embodiment of this aspect, $R^1$ represents $C_{3-6}$ cyclolkyl, such as substituted cyclobutyl or unsubstituted cyclopropyl, for instance:

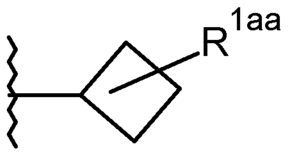

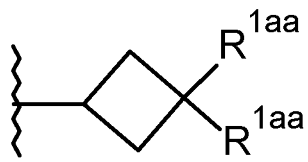

where each $R^{1aa}$ represents one or two optional substituents selected from those defined by $R^{1a}$, and in an embodiment represents two substituents, methyl and -OH; or

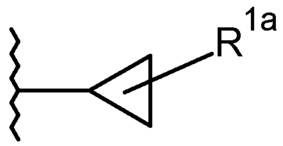

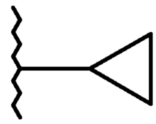

where $R^{1a}$ is as defined above, but where, in a particular embodiment, it is not present.

In an embodiment where $R^1$ represents aryl or heteroaryl, optionally substituted as defined herein, then it may represent: (i) phenyl; (ii) a 5- or 6-membered mono-cyclic heteroaryl group; or (iii) a 9- or 10-membered bicyclic heteroaryl group, all of which are optionally substituted by one to three substituents as defined herein. In an embodiment, the aforementioned aryl and heteroaryl groups are optionally substituted with one or two (e.g. one) substituent(s) selected from halo (e.g. fluoro), -OH and $-OC_{1-3}$ alkyl. In a further embodiment, such optional substituents are selected from fluoro and methoxy. In one embodiment, $R^1$ represents phenyl or a mono-cyclic 6-membered heteroaryl group and in another embodiment it may represent a 9- or 10-membered (e.g. 9-membered) bicyclic heteroaryl group. Hence, in an embodiment, $R^1$ may represent:

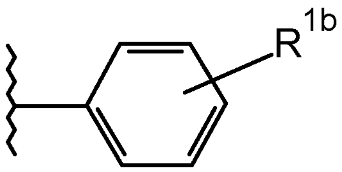

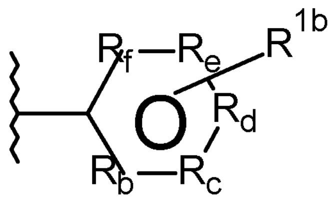

wherein $R^{1b}$ represents one or two optional substituents selected from halo, -OH and $-OCH_3$ (and in a further embodiment, such optional substituents are selected from fluoro and methoxy), and at least one of $R_b$, $R_c$, $R_d$, $R_e$ and $R_f$ represents a nitrogen heteroatom (and the others represent CH). In an embodiment, either one or two of $R_b$, $R_c$, $R_d$, $R_e$ and $R_f$ represent(s) a nitrogen heteroatom, for instance, $R_d$ represents nitrogen and, optionally, $R_b$ represents nitogen, or, $R_c$ represents nitogen. In an aspect: (i) $R_b$ and $R_d$ represent nitrogen; (ii) $R_d$ represents nitrogen; or (iii) $R_c$ represents nitrogen. Hence, $R^1$ may represent 3-pyridyl, 4-pyridyl or 4-pyrimidinyl, all of which are optionally substituted as herein defined, for instance with one substituent selected from fluoro and methoxy (and in a further embodiment in this aspect, $R^1$ represents unsubstituted 4-pyrimidinyl, unsubstituted 4-pyridyl, unsubstituted 3-pyridyl, 3-fluoro-4-pyridyl or 3-methoxy-4-pyridyl). In another embodiment, $R^1$ may represent:

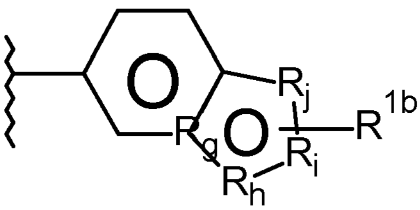

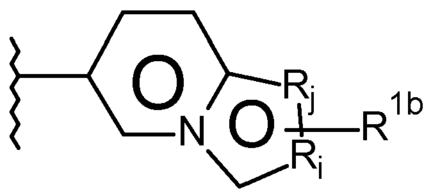

wherein $R^{1b}$ is as defined above (i.e. represents one or two optional substituent as defined above), each ring of the bicyclic system is aromatic, $R_g$ represents a N or C atom and any one or two of $R_h$, $R_i$ and $R_j$ (for instance, one or two

of $R_i$ and $R_j$) represents N and the other(s) represent(s) C (provided that, as the skilled person would understand, the rules of valency are adhered to).

In an embodiment $R^1$ represents:

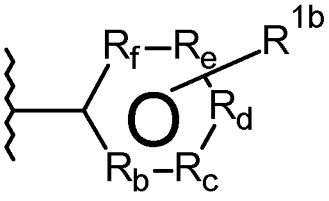

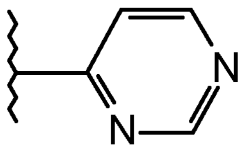

in which $R_b$ and $R_d$ represent a nitrogen atom, and, in an embodiment, there is no $R^{1b}$ substituent present.

In another embodiment, $R^1$ represents:

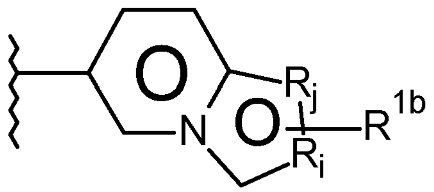

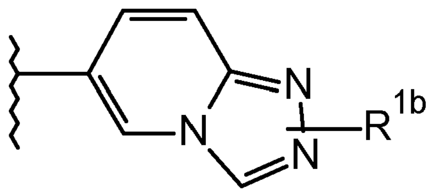

in which one of $R_i$ and $R_j$ represents N and the other represents C, or, both $R_i$ and $R_j$ represent N, and, in an embodiment, there is no $R^{1b}$ substituent present.

In an embodiment where $R^1$ represents heterocyclyl, optionally substituted as defined herein, such goup is in a further aspect a 5- or 6-membered heterocyclyl group, for instance containing at least one nitrogen heteroatom; for instance, in a particular embodiment, in this instance $R^1$ may represent a 6-membered nitrogen-containing heterocyclyl group optionally substituted by one substituent selected from $C_{1-3}$ alkyl and $C_{3-6}$ cycloalkyl. In an aspect of this embodiment, the 6-membered heterocyclyl group may be piperidinyl (e.g. 3-piperidinyl) optionally substituted by $C_{3-4}$ cycloalkyl (e.g. cyclobutyl).

In an embodiment where $R^1$ represents aryl, specific groups that may be mentioned include phenyl and methoxy-phenyl (such as 2-methoxy-phenyl). In an embodiment where $R^1$ represents heteroaryl, it is preferably a mono-cyclic 6-membered ring, for instance containing at least one nitrogen heteroatom and thereby forming a pyridyl or pyrimidinyl group. Specific groups that $R^1$ may represent include 4-pyridyl, 3-pyridyl and 4-pyrimidinyl (all of which are optionally substituted as defined herein). In view of the optionaly substitution mentioned herein, such groups may represent an unsubstituted 4-pyrimidinyl, unsubstituted 3-pyridyl, 3-fluoro-4-pyridyl and 3-methoxy-pyridyl.

In a particular embodiment, $R^1$ represents cyclopropyl or a mono-cyclic heteraryl group optionally substituted as defined herein. In an aspect, $R^1$ represents a mono-cyclic heteroaryl group, for instance a 6-membered mono-cyclic heteroaryl group containing one or two nitrogen heteroatoms, and which groups is optionally substituted by one or more substituents selected from fluoro and methoxy.

In an embodiment $R^2$ represents: (i) $C_{1-3}$ alkyl optionally substituted as defined herein (e.g. with one or more substituents independently selected from -OH, -$OC_{1-3}$ alkyl) and oxo); (ii) $C_{2-4}$alkenyl optionally substituted with -O-$C_{1-3}$alkyl; (iii) -N-($C_{1-3}$alkyl)$_2$; (iv) heterocyclyl. In a further embodiment, $R^2$ represents (i) $C_{1-3}$ alkyl optionally substituted by one or two (e.g. one) substituent(s) selected from -OH, methoxy, ethoxy and oxo ; (ii) $C_{2-4}$alkenyl optionally substituted with methoxy or ethoxy; (iii) -N-($C_{1-3}$alkyl)$_2$, where the alkyl moieties are unsubstituted; (iv) a 5- or 6-membered heterocyclyl group, for example a 6-membered heterocyclyl group (e.g. in which there is at least one nitrogen heteroatom and optionally an oxygen heteroatom, so forming for example a morpholinyl group).

In an embodiment when $R^2$ represents $C_{1-3}$ alkyl, then it represents isopropyl, ethyl, $-(CH_2)_2-OCH_3$, $-C(H)(CH_3)-OCH_3$, $-C(H)(CH_3)-OCH_2CH_3$, $-C(O)-CH_3$, $-C(H)(OH)-CH_3$ or $-C(OH)(CH_3)-CH_3$.

In an embodiment when $R^2$ represents $C_{2-4}$ alkenyl, then it represents $-C(=CH_2)-OCH_3$ or $-C(=CH_2)-OCH_2CH_3$.

In an embodiment when $R^2$ represent -N-($C_{1-3}$alkyl)$_2$, then it represents $-N(CH_3)(CH_2CH_3)$.

In an embodiment when $R^2$ represents heterocyclyl, then it represents morpholinyl, for instance 4-morpholinyl.

In an embodiment, $R^2$ represents $C_{1-3}$ alkyl optionally substituted as defined herein (for instance, it may be unsubsituted or substituted by one subsituent selected from -OH and -O-$C_{1-2}$ alkyl). In an embodiment, $R^2$ represents unsubstituted $C_{1-3}$ alkyl.

In an embodiment, $R^3$ represents (i) hydrogen; (ii) halo (bromo, chloro, iodo, fluoro); (iii) $C_{1-4}$ alkyl optionally substituted by one or more substituent selected from fluoro, -OH and -O-$C_{1-2}$ alkyl; (iv) $C_{2-4}$ alkenyl (which is, in an

embodiment, unsubstituted); or (v) $C_{3-6}$ cycloalkyl (which is, in an embodiment, $C_{3-4}$ cycloalkyl). In a further embodiment, $R^3$ may, additionally or alternatively, represent (vi) -N(H)$C_{1-2}$ alkyl or -N($C_{1-2}$ alkyl)$_2$; or (vii) heterocyclyl (e.g. in a nitogen containing 3- to 6-membered heterocyclyl group, which, in an embodiment, is attached *via* the nitrogen atom).

In a particular embodiment, $R^3$ represents hydrogen, halo (bromo, iodo, fluoro, chloro), methyl, ethyl, isopropyl, isobutyl ($-CH_2C(H)(CH_3)_2$), $-CHF_2$, $-CF_3$, $-CH_2OH$, $-CH_2OCH_3$, $-C(F)(H)CH_3$, $-(CH_2)_2CF_3$, $-C(CH_3)=CH_2$, $-C(H)=CH_2$, cyclopropyl, cycylobutyl. In a further embodiment, additional or alternative $R^3$ groups that may be mentioned include $-N(H)CH_3$, $-N(CH_3)_2$ and azetidinyl (attached *via* the nitrogen atom).

The names of the compounds of the present invention were generated according to the nomenclature rules agreed upon by the Chemical Abstracts Service (CAS) using Advanced Chemical Development, Inc., software (ACD/Name product version 10.01; Build 15494, 1 Dec 2006) or according to the nomenclature rules agreed upon by the International Union of Pure and Applied Chemistry (IUPAC) using Advanced Chemical Development, Inc., software (ACD/Name product version 10.01.0.14105, October 2006). In case of tautomeric forms, the name of the depicted tautomeric form of the structure was generated. The other non-depicted tautomeric form is also included within the scope of the present invention.

**Preparation of the compounds**

In an aspect of the invention, there is provided a process for the preparation of compounds of the invention, where reference here is made to compounds of formula (I) as defined herein.

Compounds of formula (I) may be prepared by:

(i) reaction of a compound of formula (II),

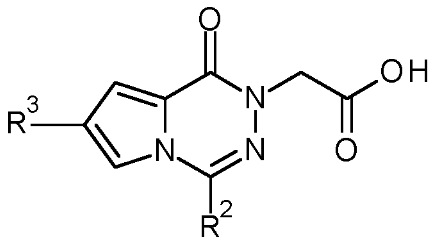

(II)

or a derivative thereof (e.g. a salt), wherein $R^2$ and $R^3$ are as hereinbefore defined, with a compound of formula (III),

$H_2N$-$R^1$ (III)

or a derivative thereof, wherein $R^1$ is as hereinbefore defined, under amide-forming reaction conditions (also referred to as amidation), for example in the presence of a suitable coupling reagent (e.g. 1-[bis(dimethylamino)methylene]-1H-1,2,3-triazolo[4,5-b]pyridinium 3-oxide hexafluorophosphate (*O*-(7-azabenzotriazol-1-yl)-*N*,*N*,*N*',*N*'-tetramethyluronium hexafluorophosphate), 1,1'-carbonyldiimidazole, *N*,*N*'-dicyclohexylcarbodiimide, 1-(3-dimethylaminopropyl)-3-ethylcarbodiimide (or hydrochloride thereof), *N*,*N*'-disuccinimidyl carbonate, benzotriazol-1-yl-oxytris(dimethylamino)phosphonium hexafluoro-phosphate, 2-(1*H*-benzotriazol-1-yl)-1,1,3,3-tetramethyluronium hexa-fluorophosphate (i.e. *O*-(1H-benzotriazol-1-yl)-*N*,*N*,*N*',*N*'-tetramethyluronium hexafluorophosphate), benzotriazol-1-yloxytris-pyrrolidinophosphonium hexa-fluorophosphate, bromo-tris-pyrrolidinophosponium hexafluorophosphate, 2-(1*H*-benzotriazol-1 -yl)-1,1,3,3 -tetramethyluronium tetra-fluorocarbonate, 1-cyclohexylcarbodiimide-3-propyloxymethyl polystyrene, *O*-benzotriazol-1-yl-*N*,*N*,*N*',*N*'-tetramethyluronium tetrafluoroborate), optionally in the presence of a suitable base (e.g. sodium hydride, sodium bicarbonate, potassium carbonate, pyridine, triethylamine, dimethylaminopyridine, diisopropylamine, sodium hydroxide, potassium *tert*-butoxide and/or lithium diisopropylamide (or variants thereof) and an appropriate solvent (e.g. tetrahydrofuran, pyridine, toluene, dichloromethane, chloroform, acetonitrile, dimethylformamide, trifluoromethylbenzene, dioxane or triethylamine). Such reactions may be performed in the presence of a further additive such as 1-hydroxybenzotriazole hydrate. Alternatively, a carboxylic acid group may be converted under standard conditions to the corresponding acyl chloride (e.g. in the presence of $SOCl_2$ or oxalyl chloride), which acyl chloride is then reacted with a compound of formula (II), for example under similar conditions to those mentioned above;

(ii) reaction of a compound of formula (IV),

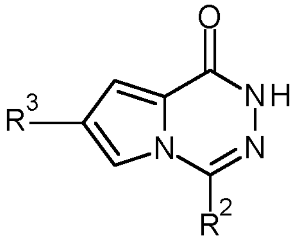

(IV)

wherein $R^2$ and $R^3$ are as hereinbefore defined, with a compound of formula (V),

$LG^a-CH_2-C(O)-N(H)R^1$ (V)

wherein $LG^a$ represents a suitable leaving group (e.g. halo, such as chloro) and $R^1$ is as defined herein, under suitable reaction conditions, e.g. in the presence of an appropriate base, e.g. $Cs_2CO_3$ or LiHMDS, or the like, or alternative alkylation reaction conditions;

(iii) by transformation (such transformation steps may also take place on intermediates) of a certain compound of formula (I) into another, for example:

- for compounds of formula (I) in which $R^3$ represents $-N(H)C_{1-6}$ alkyl or $-N(C_{1-6}$ alkyl$)_2$, reaction of a corresponding compound of formula (I) in which $R^3$ represents halo, with an appropriate amine $H_2NC_{1-6}$ alkyl or $HN(C_{1-6}$ alkyl$)_2$, in an amination reaction under appropriate conditions, e.g. using under standard coupling conditions, in the presence of a catalyst, *e.g.* CuI, a ligand, *e.g.* D/L-proline and a base, *e.g.* $K_2CO_3$; similar transformations may be performed on compounds in which $R^2$ represents halo, and an amine (or heterocyclyl group attached *via* a nitrogen atom) is desired at $R^2$;

- for compounds of formula (I) containing an alkene, reduction to a corresponding compound of formula (I) containing an alkane, under reduction conditions, e.g. with hydrogen in the presence of a suitable

catalyst such as, for example, palladium on carbon, in a suitable reaction-inert solvent, such as, for example, ethyl acetate or methanol;

- coupling to convert a halo group to e.g. an alkyl, alkenyl or aryl/heteroaryl group, for example in the presence of a suitable coupling reagent, e.g. where the reagent comprises the appropriate alkyl, alkenyl or aryl/heteroaryl group attached to a suitable group such as $-B(OH)_2$,$-B(OR^{wx})_2$, zincates (e.g. including $-Zn(R^{wx})_2$,
- $ZnBrR^{wx}$) or $-Sn(R^{wx})_3$, in which each $R^{wx}$ independently represents a $C_{1-6}$ alkyl group, or, in the case of $-B(OR^{wx})_2$, the respective $R^{wx}$ groups may be linked together to form a 4- to 6-membered cyclic group (such as a 4,4,5,5-tetramethyl-1,3,2-dioxaborolan-2-yl group), thereby forming e.g. a pinacolato boronate ester group. The reaction may be performed in the presence of a suitable catalyst system, e.g. a metal (or a salt or complex thereof) such as Pd, CuI, Pd/C, $PdCl_2$, $Pd(OAc)_2$, $Pd(Ph_3P)_2Cl_2$, $Pd(Ph_3P)_4$ (i.e. palladium tetrakistriphenylphosphine), $Pd_2(dba)_3$ and/or $NiCl_2$ (preferred cataysts include RuPhos Pd G3, XPhos Pd and bis(tri-*tert*-butylphosphine)palladium(0)) and optionally a ligand such as $PdCl_2$(dppf).DCM, *t*-$Bu_3P$, $(C_6H_{11})_3P$, $Ph_3P$, $AsPh_3$, P(*o*-Tol$)_3$, 1,2-bis(diphenylphosphino)ethane, 2,2'-bis(di-*tert*-butylphosphino)-1,1'-biphenyl, 2,2'-bis(diphenylphosphino)-1,1'-bi-naphthyl, 1,1'-bis(diphenyl-phosphino-ferrocene), 1,3-bis(diphenylphosphino)propane, xantphos, or a mixture thereof, together with a suitable base, such as $Na_2CO_3$, $K_3PO_4$, $Cs_2CO_3$, NaOH, KOH, $K_2CO_3$, CsF, $Et_3N$, (*i*-$Pr)_2NEt$, *t*-BuONa or *t*-BuOK (or mixtures thereof; preferred bases include $Na_2CO_3$ and $K_2CO_3$) in a suitable solvent such as dioxane, toluene, ethanol, dimethylformamide, dimethoxyethane, ethylene glycol dimethyl ether, water, dimethylsulfoxide, acetonitrile, dimethylacetamide, N-methylpyrrolidinone, tetrahydrofuran or mixtures thereof (preferred solvents include dimethylformamide and dimethoxyethane);
- reduction of a ketone to an alcohol, in the presence of suitable reducing conditions, e.g. $NaBH_4$ or the like;
- conversion of $-C(CH_2)-OCH_2CH_3$ to $-C(O)CH_3$, by reaction in the presence of HCl, e.g. also in a suitable solvent such as THF;
- conversion of a -C(O)alkyl moiety to a -C(OH)(alkyl)(alkyl) moiety by reaction of an appropriate Grignard reagent, e.g. alkylMgBr;
- transformation of a alkene $=CH_2$ moiety to a carbonyl =O moiety, for instance, in the presence of AD-mix-Alpha and methane-sulfonamide;
- transformation of a ketone to an alcohol -OH moiety;

- alkylation of a -OH moiety (to -O-alkyl), under appropriate reaction conditions.

The compound of formula (II) may be prepared by hydrolysis of the corresponding carboxylic acid ester (for example under standard hydrolysis conditions, e.g. base hydrolysis in the presence of an alkai metal hydroxide (such as lithium hydroxide)), which in turn is prepared by reaction of a compound of formula (IV),

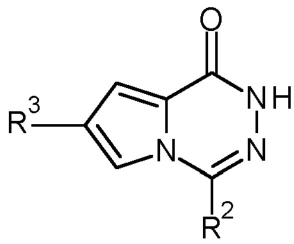

(IV)

wherein $R^2$ and $R^3$ are as hereinbefore defined, with a compound of formula (VI),

$$LG\text{-}CH_2\text{-}C(O)O\text{-}R^{aa} \qquad (VI)$$

wherein $R^{aa}$ represents $C_{1-6}$ alkyl (e.g ethyl) and LG represents a suitable leaving group, such as halo (e.g. chloro), for instance under reaction conditions and using reagent such as those described herein.

In general the compounds of the invention can therefore be made with reference to the procedures above. However, in the interests of versatility, further schemes are provided below in order to provide intermediate and final compounds of the invention. Further details are provided in the schemes below (as well as in the specific details of the experimental described hereinafter).

In this respect, Scheme 1 outlines a typical synthesis:

**Scheme 1**

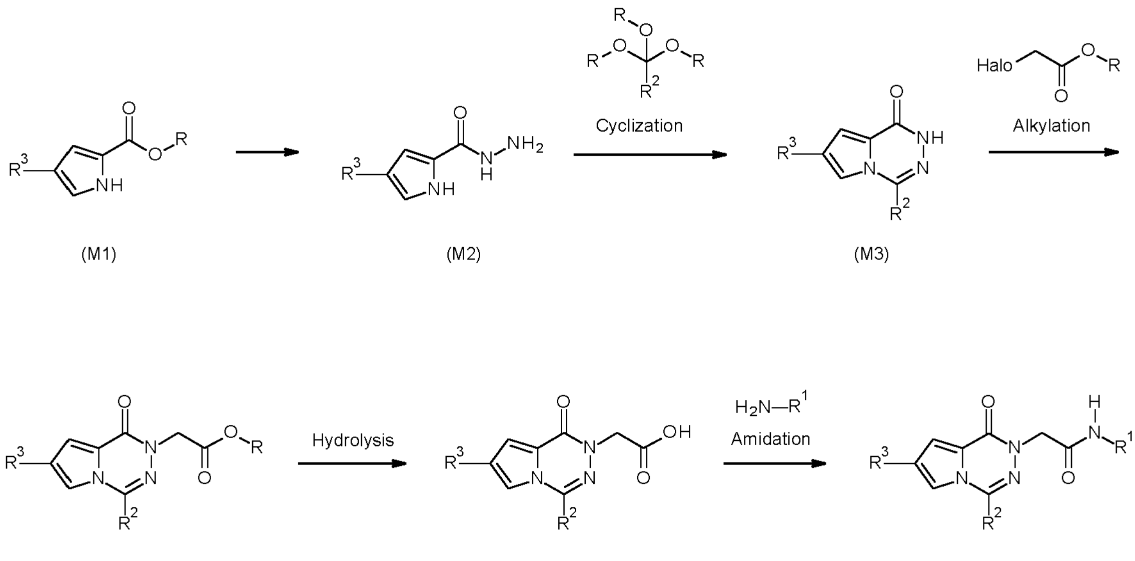


(M1) (M2) (M3)

(M4) (M5) (I)

Compounds of the invention, as described herein, can be prepared by a reaction sequence shown in Scheme 1 (above), whereby an appropriately substituted pyrrole (M1), wherein R is $C_{1-4}$ alkyl (and $R^3$ is as defined herein), is reacted with hydrazine to give hydrazide (M2), which is then cyclized by reaction with an appropriate orthoester, wherein R is $C_{1-4}$ alkyl (and $R^2$ is as defined herein), in the presence of a Lewis acid, *e.g.* aluminum isopropoxide, to the triazinone (M3) (also referred to herein as compound of formula (IV)) which is then alkylated with an appropriate alkyl haloacetate, wherein R is $C_{1-4}$ alkyl, in the presence of a base, *e.g.* $K_2CO_3$, a nucleophilic catalyst, *e.g.* KI and a crown ether, *e.g.* 18-crown-6, to provide ester (M4) which is typically cleaved *e.g.* under basic conditions, *e.g.* aqueous LiOH in THF or

NaOH in MeOH to yield the acid intermediate (M5) (also referred to herein as compound of formula (II)), followed by amidation with $R^1$-$NH_2$ (wherein if $R^1$ has a functional group such as OH, $NH_2$, $CO_2H$, such group is optionally protected) using standard coupling conditions, *e.g.* HATU and a base, *e.g.* Hünig's base, optionally followed by an additional deprotection step to provide a compound of Formula (I), or a pharmaceutically acceptable salt thereof.

Modifications and transformations may also be done on intermediates, and in this respect, the processes described above may also be applied to intermediates, as depicted for instance in the following Scheme 2:

**Scheme 2**

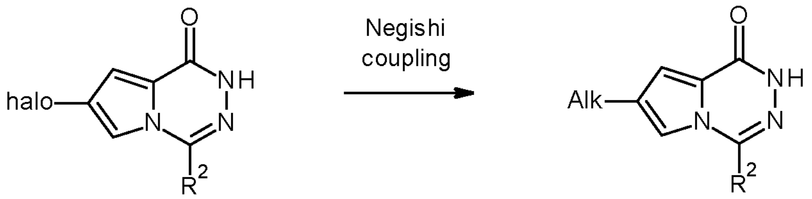


(M6) (M3)

For instance, as per Scheme 2 above, the triazinone (M3) wherein $R^3$ is $C_{1-4}$ alkyl, as defined herein, may be prepared by a reaction of a halo-triazinone (M6) (i.e. where $R^3$ is halo) with a zincate, *e.g.* diethylzinc, using standard Negishi cross-coupling conditions, in the presence of a catalyst, *e.g.* XPhos Pd G3 and a base, *e.g.* $K_2CO_3$, to provide the alkyl-triazinone (M3).

Further the following transformations, depicted in Scheme 3 below, show versatility in allowing introduction of other substituents at the $R^2$ position of such intermediates too (as well as for final compounds):

**Scheme 3**

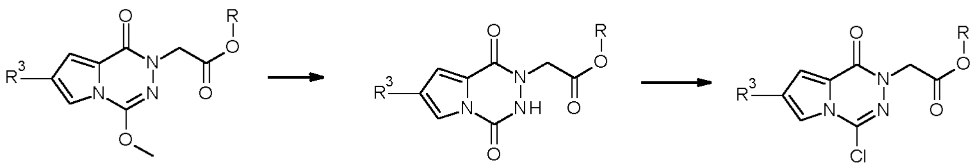

In Scheme 3 (where R represents $C_{1-6}$ alkyl, and $R^3$ is as defined herein, e.g. an alkyl group, such as isopropyl), the methoxy moiety is coverted to a carbonyl group by reaction with sodium iodide and trimethylsilyl chloride, then the carbonyl group is converted to a chloro group by reaction with a chlorinating reagent such as $POCl_3$; such intermediate is then very versatile as the chloro moiety may be replaced in a variety of coupling reactions, for instance as described above in respect of the final compound transformations, e.g. coupling with an amine, or with an alkyl/alkenyl group, etc.

Certain intermediate compounds may be commercially available, may be known in the literature, or may be obtained either by analogy with the processes described herein, or by conventional synthetic procedures, in accordance with standard techniques, from available starting materials using appropriate reagents and reaction conditions.

Certain substituents on/in final compounds of the invention or relevant intermediates may be modified one or more times, after or during the processes described above by way of methods that are well known to those skilled in the art. Examples of such methods include substitutions, reductions, oxidations, alkylations, acylations, hydrolyses, esterifications, etherifications, halogenations, nitrations or couplings.

Compounds of the invention may be isolated from their reaction mixtures using conventional techniques (e.g. recrystallisations, where possible under standard conditions).

It will be appreciated by those skilled in the art that, in the processes described above and hereinafter, the functional groups of intermediate compounds may need to be protected by protecting groups.

The need for such protection will vary depending on the nature of the remote functionality and the conditions of the preparation methods (and the need can be readily determined by one skilled in the art). Suitable amino-protecting groups include acetyl, trifluoroacetyl, t-butoxycarbonyl (BOC), benzyloxycarbonyl (CBz), 9-fluorenyl-methyleneoxycarbonyl (Fmoc) and 2,4,4-trimethylpentan-2-yl (which may be deprotected by reaction in the presence of an acid, e.g. HCl in water/alcohol (e.g. MeOH)) or the like. The need for such protection is readily determined by one skilled in the art. For example the a -C(O)O-*tert*-butyl ester moiety may serve as a protecting group for a -C(O)OH moiety, and hence the former may be converted to the latter for instance by reaction in the presence of a mild acid (e.g. TFA, or the like).

The protection and deprotection of functional groups may take place before or after a reaction in the above-mentioned schemes.

Protecting groups may be removed in accordance with techniques that are well known to those skilled in the art and as described hereinafter. For example, protected compounds/intermediates described herein may be converted chemically to unprotected compounds using standard deprotection techniques.

The type of chemistry involved will dictate the need, and type, of protecting groups as well as the sequence for accomplishing the synthesis.

The use of protecting groups is fully described in "Protective Groups in Organic Synthesis", 3rd edition, T.W. Greene & P.G.M. Wutz, Wiley-Interscience (1999).

The compounds of the invention as prepared in the hereinabove described processes may be synthesized in the form of racemic mixtures of enantiomers which can be separated from one another following art-known resolution procedures. Those compounds of the invention that are obtained in racemic form may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of the invention involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

## PHARMACOLOGY

There is evidence for a role of NLRP3-induced IL-1 and IL-18 in the inflammatory responses occurring in connection with, or as a result of, a multitude of different disorders (Menu et al., Clinical and Experimental Immunology, 2011, 166, 1-15; Strowig et al., Nature, 2012, 481, 278-286). NLRP3 mutations have been found to be responsible for a set of rare autoinflammatory diseases known as CAPS (Ozaki et al., J. Inflammation Research, 2015, 8,15-27; Schroder et al., Cell, 2010, 140: 821-832; Menu et al., Clinical and Experimental Immunology, 2011, 166, 1-15). CAPS are heritable diseases characterized by recurrent fever and inflammation and are comprised of three autoinflammatory disorders that form a clinical continuum. These diseases, in order of increasing severity, are familial cold autoinflammatory syndrome (FCAS), Muckle-Wells syndrome (MWS), and chronic infantile cutaneous neurological articular syndrome (CINCA; also called neonatal- onset multisystem inflammatory disease, NOMID), and all have been shown to result from gain-of-function mutations in the NLRP3 gene, which leads to increased secretion of IL-1 beta. NLRP3 has also been implicated in a number of autoinflammatory diseases, including pyogenic arthritis, pyoderma gangrenosum and acne (PAPA), Sweet's syndrome, chronic nonbacterial osteomyelitis (CNO), and acne vulgaris (Cook et al., Eur. J. Immunol., 2010, 40, 595-653).

A number of autoimmune diseases have been shown to involve NLRP3 including, in particular, multiple sclerosis, type-1 diabetes (T1D), psoriasis, rheumatoid arthritis (RA), Behcet's disease, Schnitzler syndrome, macrophage activation syndrome (Braddock et al., Nat. Rev. Drug Disc. 2004, 3, 1-10; Inoue et al., Immunology, 2013, 139, 11-18; Coll et al., Nat. Med. 2015, 21(3), 248-55; Scott et al., Clin. Exp. Rheumatol. 2016, 34(1), 88-93), systemic lupus erythematosus and its complications such as lupus nephritis (Lu et al., J. Immunol. , 2017, 198(3), 1119-29), and systemic sclerosis (Artlett et al., Arthritis Rheum. 2011, 63(11), 3563-74). NLRP3 has also been shown to play a role in a number of lung diseases including chronic obstructive pulmonary disorder (COPD), asthma (including steroid-resistant asthma), asbestosis, and silicosis (De Nardo et al., Am. J. Pathol., 2014, 184: 42-54; Kim et al., Am. J. Respir. Crit. Care Med, 2017, 196(3), 283-97). NLRP3 has also been suggested to have a role in a number of central nervous system conditions, including Multiple Sclerosis (MS), Parkinson's disease (PD), Alzheimer's disease (AD), dementia, Huntington's disease, cerebral malaria, brain injury from pneumococcal meningitis (Walsh et al., Nature Reviews, 2014, 15, 84-97; and Dempsey et al., Brain. Behav. Immun. 2017, 61, 306-16), intracranial aneurysms (Zhang et al., J. Stroke and Cerebrovascular Dis., 2015, 24, 5, 972-9), and traumatic brain injury (Ismael et al., J. Neurotrauma., 2018, 35(11), 1294-1303). NLRP3 activity has also been shown to be involved in various metabolic diseases including type 2 diabetes (T2D) and its organ-specific complications, atherosclerosis, obesity, gout, pseudo-gout, metabolic syndrome (Wen et al., Nature Immunology, 2012, 13, 352-357; Duewell et al., Nature, 2010, 464, 1357-1361; Strowig et al., Nature, 2014, 481, 278- 286), and non-alcoholic steatohepatitis (Mridha et al., J. Hepatol. 2017, 66(5), 1037-46). A role for NLRP3 via IL-1 beta has also been suggested in atherosclerosis, myocardial infarction (van Hout et al., Eur. Heart J. 2017, 38(11), 828-36), heart failure (Sano et al., J. Am. Coll. Cardiol. 2018, 71(8), 875-66), aortic aneurysm and dissection (Wu et al., Arteriosc/er. Thromb. Vase. Biol., 2017,37(4), 694-706), and other cardiovascular events (Ridker et al., N. Engl. J. Med., 2017, 377(12), 1119-31).

Other diseases in which NLRP3 has been shown to be involved include: ocular diseases such as both wet and dry age-related macular degeneration (Doyle et al., Nature Medicine, 2012, 18, 791-798; Tarallo et al., Cell2012, 149(4), 847-59), diabetic retinopathy (Loukovaara et al., Acta Ophthalmol., 2017, 95(8), 803-8), non-infectious uveitis and optic nerve damage (Puyang et al., Sci. Rep. 2016, 6, 20998); liver diseases including non-alcoholic steatohepatitis (NASH)

and acute alcoholic hepatitis (Henao-Meija et al., Nature, 2012, 482, 179-185); inflammatory reactions in the lung and skin (Primiano et al., J. Immunol. 2016, 197(6), 2421-33) including contact hypersensitivity (such as bullous pemphigoid (Fang et al., J Dermatol Sci. 2016, 83(2), 116-23)), atopic dermatitis (Niebuhr et al., Allergy, 2014, 69(8), 1058-67), Hidradenitis suppurativa (Alikhan et al., J. Am. Acad. Dermatol., 2009 ,60(4), 539-61), and sarcoidosis (Jager et al., Am. J. Respir. Crit. Care Med., 2015, 191, A5816); inflammatory reactions in the joints (Braddock et al., Nat. Rev. Drug Disc, 2004, 3, 1-10); amyotrophic lateral sclerosis (Gugliandolo et al., Int. J. Mol. Sci., 2018, 19(7), E1992); cystic fibrosis (lannitti et al., Nat. Commun., 2016, 7, 10791); stroke (Walsh et al., Nature Reviews, 2014, 15, 84-97); chronic kidney disease (Granata et al., PLoS One 2015, 10(3), eoi22272); and inflammatory bowel diseases including ulcerative colitis and Crohn's disease (Braddock et al., Nat. Rev. Drug Disc, 2004, 3, 1-10; Neudecker et *a*/., J. Exp. Med. 2017, 214(6), 1737-52; Lazaridis et al., Dig. Dis. Sci. 2017, 62(9), 2348-56). The NLRP3 inflammasome has been found to be activated in response to oxidative stress. NLRP3 has also been shown to be involved in inflammatory hyperalgesia (Dolunay et al., Inflammation, 2017, 40, 366-86).

Activation of the NLRP3 inflammasome has been shown to potentiate some pathogenic infections such as influenza and Leishmaniasis (Tate et al., Sci Rep., 2016, 10(6), 27912-20; Novias et al., PLOS Pathogens 2017, 13(2), e1006196).

NLRP3 has also been implicated in the pathogenesis of many cancers (Menu et al., Clinical and Experimental Immunology, 2011, 166, 1-15). For example, several previous studies have suggested a role for IL-1 beta in cancer invasiveness, growth and metastasis, and inhibition of IL-1 beta with canakinumab has been shown to reduce the incidence of lung cancer and total cancer mortality in a randomised, double-blind, placebo-controlled trial (Ridker et al., Lancet., 2017, 390(10105), 1833-42). Inhibition of the NLRP3 inflammasome or IL-1 beta has also been shown to inhibit the proliferation and migration of lung cancer cells in vitro (Wang et al., Onco/Rep., 2016, 35(4), 2053-64). A role for the NLRP3 inflammasome has been suggested in myelodysplastic syndromes, myelofibrosis and other myeloproliferative neoplasms, and acute myeloid leukemia (AML) (Basiorka et al., Blood, 2016, 128(25), 2960-75.) and also in the carcinogenesis of various other cancers including glioma (Li et al., Am. J. Cancer Res. 2015, 5(1), 442-9), inflammation-induced tumors (Allen et al., J. Exp. Med. 2010, 207(5), 1045-56; Hu et al., PNAS., 2010, 107(50), 21635-40), multiple myeloma (Li et al., Hematology, 2016 21(3), 144-51), and squamous cell carcinoma of the head and neck (Huang et al., J. Exp. Clin. Cancer Res., 2017, 36(1), 116). Activation of the NLRP3 inflammasome has also been shown to mediate chemoresistance of tumor cells to 5-Fluorouracil (Feng et al., J. Exp. Clin. Cancer Res., 2017, 36(1), 81), and activation of NLRP3 inflammasome in peripheral nerve contributes to chemotherapy-induced neuropathic pain (Jia et al., Mol. Pain., 2017, 13, 1-11). NLRP3 has also been shown to be required for the efficient control of viruses, bacteria, and fungi.

The activation of NLRP3 leads to cell pyroptosis and this feature plays an important part in the manifestation of clinical disease (Yan-gang et al., Cell Death and Disease, 2017, 8(2), 2579; Alexander et al., Hepatology, 2014, 59(3), 898-910; Baldwin et al., J. Med. Chem., 2016, 59(5), 1691- 1710; Ozaki et al., J. Inflammation Research, 2015, 8, 15-27; Zhen et al., Neuroimmunology Neuroinflammation, 2014, 1(2), 60-65; Mattia et al., J. Med. Chem., 2014, 57(24), 10366-82; Satoh et al., Cell Death and Disease, 2013, 4, 644). Therefore, it is anticipated that inhibitors of NLRP3 will block pyroptosis, as well as the release of pro-inflammatory cytokines (e.g. IL-1 beta) from the cell.

Hence, the compounds of the invention (and, where applicable, without the provisos), as described herein (e.g. in any of the embodiments described herein, including by the examples, and/or in any of the forms described herein, e.g. in a salt form or free form, etc) exhibit valuable pharmacological properties, e.g. NLRP3 inhibiting properties on the NLRP3 inflammasome pathway e.g. as indicated *in vitro* tests as provided herein, and are therefore indicated for therapy or for use as research chemicals, e.g. as tool compounds. Compounds of the invention (and, where applicable, without the provisos) may be useful in the treatment of an indication selected from: inflammasome-related diseases/disorders, immune diseases, inflammatory diseases, auto-immune diseases, or auto-inflammatory diseases, for example, of diseases, disorders or conditions in which NLRP3 signaling contributes to the pathology, and/or symptoms, and/or progression, and which may be responsive to NLRP3 inhibition and which may be treated or prevented, according to any of the methods/uses described herein, e.g. by use or administration of a compound of the invention, and, hence, in an embodiment, such indications may include:

I. Inflammation, including inflammation occurring as a result of an inflammatory disorder, *e.g.* an autoinflammatory disease, inflammation occurring as a symptom of a non- inflammatory disorder, inflammation occurring as a result of infection, or inflammation secondary to trauma, injury or autoimmunity. Examples of inflammation that may be treated or prevented include inflammatory responses occurring in connection with, or as a result of:

a. a skin condition such as contact hypersensitivity, bullous pemphigoid, sunburn, psoriasis, atopical dermatitis, contact dermatitis, allergic contact dermatitis, seborrhoetic dermatitis, lichen planus, scleroderma, pemphigus, epidermolysis bullosa, urticaria, erythemas, or alopecia;

b. a joint condition such as osteoarthritis, systemic juvenile idiopathic arthritis, adult-onset Still's disease, relapsing polychondritis, rheumatoid arthritis, juvenile chronic arthritis, crystal induced arthropathy (e.g. pseudo-gout, gout), or a seronegative spondyloarthropathy (e.g. ankylosing spondylitis, psoriatic arthritis or Reiter's disease);

c. a muscular condition such as polymyositis or myasthenia gravis;

d. a gastrointestinal tract condition such as inflammatory bowel disease (including Crohn's disease and ulcerative colitis), gastric ulcer, coeliac disease, proctitis, pancreatitis, eosinopilic gastro- enteritis, mastocytosis, antiphospholipid syndrome, or a food-related allergy which may have effects remote from the gut (e.g., migraine, rhinitis or eczema);

e. a respiratory system condition such as chronic obstructive pulmonary disease (COPD), asthma (including bronchial, allergic, intrinsic, extrinsic or dust asthma, and particularly chronic or inveterate asthma, such as late asthma and airways hyper- responsiveness), bronchitis, rhinitis (including acute rhinitis, allergic rhinitis, atrophic rhinitis, chronic rhinitis, rhinitis caseosa, hypertrophic rhinitis, rhinitis pumlenta, rhinitis sicca, rhinitis medicamentosa, membranous rhinitis, seasonal rhinitis e.g. hay fever, and vasomotor rhinitis), sinusitis, idiopathic pulmonary fibrosis (IPF), sarcoidosis, farmer's lung, silicosis, asbestosis, adult respiratory distress syndrome, hypersensitivity pneumonitis, or idiopathic interstitial pneumonia;

f. a vascular condition such as atherosclerosis, Behcet's disease, vasculitides, or Wegener's granulomatosis;

g. an immune condition, e.g. autoimmune condition, such as systemic lupus erythematosus (SLE), Sjogren's syndrome, systemic sclerosis, Hashimoto's thyroiditis, type I diabetes, idiopathic thrombocytopenia purpura, or Graves disease;

h. an ocular condition such as uveitis, allergic conjunctivitis, or vernal conjunctivitis;

i. a nervous condition such as multiple sclerosis or encephalomyelitis;

j. an infection or infection-related condition, such as Acquired Immunodeficiency Syndrome (AIDS), acute or chronic bacterial infection, acute or chronic parasitic infection, acute or chronic viral infection, acute or chronic fungal infection, meningitis, hepatitis (A, B or C, or other viral hepatitis), peritonitis, pneumonia, epiglottitis, malaria, dengue hemorrhagic fever, leishmaniasis, streptococcal myositis, mycobacterium tuberculosis, mycobacterium avium intracellulare, Pneumocystis carinii pneumonia, orchitis/epidydimitis, legionella, Lyme disease, influenza A, epstein-barr virus, viral encephalitis/aseptic meningitis, or pelvic inflammatory disease;

k. a renal condition such as mesangial proliferative glomerulonephritis, nephrotic syndrome, nephritis, glomerular nephritis, acute renal failure, uremia, or nephritic syndrome;

l. a lymphatic condition such as Castleman's disease;

m. a condition of, or involving, the immune system, such as hyper IgE syndrome, lepromatous leprosy, familial hemophagocytic lymphohistiocytosis, or graft versus host disease;

n. a hepatic condition such as chronic active hepatitis, non-alcoholic steatohepatitis (NASH), alcohol-induced hepatitis, non-alcoholic fatty liver disease (NAFLD), alcoholic fatty liver disease (AFLD), alcoholic steatohepatitis (ASH) or primary biliary cirrhosis;

o. a cancer, including those cancers listed herein below;

p. a burn, wound, trauma, haemorrhage or stroke;

q. radiation exposure;

r. obesity; and/or

s. pain such as inflammatory hyperalgesia;

II. Inflammatory disease, including inflammation occurring as a result of an inflammatory disorder, e.g. an autoinflammatory disease, such as cryopyrin-associated periodic syndromes (CAPS), Muckle-Wells syndrome (MWS), familial cold autoinflammatory syndrome (FCAS), familial Mediterranean fever (FMF), neonatal onset multisystem inflammatory disease (NOMID), Majeed syndrome, pyogenic arthritis, pyoderma gangrenosum and acne syndrome (PAPA), adult-onset Still's disease (AOSD), haploinsufficiency of A20 (HA20), pediatric granulomatous arthritis (PGA), PLCG2-associated antibody deficiency and immune dysregulation (PLAID), PLCG2- associated autoinflammatory, antibody deficiency and immune dysregulation (APLAID), or sideroblastic anaemia with B-cell immunodeficiency, periodic fevers and developmental delay (SIFD);

III. Immune diseases, e.g. auto-immune diseases, such as acute disseminated encephalitis, Addison's disease, ankylosing spondylitis, antiphospholipid antibody syndrome (APS), anti-synthetase syndrome, aplastic anemia, autoimmune adrenalitis, autoimmune hepatitis, autoimmune oophoritis, autoimmune polyglandular failure, autoimmune thyroiditis, Coeliac disease, Crohn's disease, type 1 diabetes (T1D), Goodpasture's syndrome, Graves' disease, Guillain-Barre syndrome (GBS), Hashimoto's disease, idiopathic thrombocytopenic purpura, Kawasaki's disease, lupus erythematosus including systemic lupus erythematosus (SLE), multiple sclerosis (MS) including primary progressive multiple sclerosis (PPMS), secondary progressive multiple sclerosis (SPMS) and relapsing remitting multiple sclerosis (RRMS), myasthenia gravis, opsoclonus myoclonus syndrome (OMS), optic neuritis, Ord's thyroiditis, pemphigus, pernicious anaemia, polyarthritis, primary biliary cirrhosis, rheumatoid arthritis (RA), psoriatic arthritis, juvenile idiopathic arthritis or Still's disease, refractory gouty arthritis, Reiter's syndrome, Sjogren's syndrome, systemic sclerosis a systemic connective tissue disorder, Takayasu's arteritis, temporal arteritis, warm autoimmune hemolytic anemia, Wegener's granulomatosis, alopecia universalis, Beliefs disease, Chagas' disease, dysautonomia, endometriosis, hidradenitis suppurativa (HS), interstitial cystitis, neuromyotonia, psoriasis, sarcoidosis, scleroderma, ulcerative colitis, Schnitzler syndrome, macrophage activation syndrome, Blau syndrome, giant cell arteritis, vitiligo or vulvodynia;

IV. Cancer including lung cancer, renal cell carcinoma, non-small cell lung carcinoma (NSCLC), Langerhans cell histiocytosis (LCH), myeloproliferative neoplams (MPN), pancreatic cancer, gastric cancer, myelodysplastic syndrome (MOS), leukaemia including acute lymphocytic leukaemia (ALL) and acute myeloid leukaemia (AML), promyelocytic leukemia (APML, or APL), adrenal cancer, anal cancer, basal and squamous cell skin cancer, bile duct cancer, bladder cancer, bone cancer, brain and spinal cord tumours, breast cancer, cervical cancer, chronic lymphocytic leukaemia (CLL), chronic myeloid leukaemia (CML), chronic myelomonocytic leukaemia (CMML), colorectal cancer, endometrial cancer, oesophagus cancer, Ewing family of tumours, eye cancer, gallbladder cancer, gastrointestinal carcinoid tumours, gastrointestinal stromal tumour (GIST), gestational trophoblastic disease, glioma, Hodgkin lymphoma, Kaposi sarcoma, kidney cancer, laryngeal and hypopharyngeal cancer, liver cancer, lung carcinoid tumour, lymphoma including cutaneous T cell lymphoma, malignant mesothelioma, melanoma skin cancer, Merkel cell skin cancer, multiple myeloma, nasal cavity and paranasal sinuses cancer, nasopharyngeal cancer, neuroblastoma, non-Hodgkin lymphoma, non-small cell lung cancer, oral cavity and oropharyngeal cancer, osteosarcoma, ovarian cancer, penile cancer, pituitary tumours, prostate cancer, retinoblastoma, rhabdomyosarcoma, salivary gland cancer, skin cancer, small cell lung cancer, small intestine cancer, soft tissue sarcoma, stomach cancer, testicular cancer, thymus cancer, thyroid cancer including anaplastic thyroid cancer, uterine sarcoma, vaginal cancer, vulvar cancer, Waldenstrom macroglobulinemia, and Wilms tumour;

V. Infections including viral infections (e.g. from influenza virus, human immunodeficiency virus (HIV), alphavirus (such as Chikungunya and Ross River virus), flaviviruses (such as Dengue virus and Zika virus), herpes viruses (such as Epstein Barr Virus, cytomegalovirus, Varicella-zoster virus, and KSHV), poxviruses (such as vaccinia virus (Modified vaccinia virus Ankara) and Myxoma virus), adenoviruses (such as Adenovirus 5), papillomavirus, or SARS-CoV-2) bacterial infections (e.g. from Staphylococcus aureus, Helicobacter pylori, Bacillus anthracis, Bordatella pertussis, Burkholderia pseudomallei, Corynebacterium diptheriae, Clostridium tetani, Clostridium botulinum, Streptococcus pneumoniae, Streptococcus pyogenes, Listeria monocytogenes, Hemophilus influenzae, Pasteurella multicida, Shigella dysenteriae, Mycobacterium tuberculosis, Mycobacterium leprae, Mycoplasma pneumoniae, Mycoplasma hominis, Neisseria meningitidis, Neisseria gonorrhoeae, Rickettsia rickettsii, Legionella pneumophila, Klebsiella pneumoniae, Pseudomonas aeruginosa, Propionibacterium acnes, Treponema pallidum, Chlamydia trachomatis, Vibrio cholerae, Salmonella typhimurium, Salmonella typhi, Borrelia burgdorferi or Yersinia pestis), fungal infections (e.g. from Candida or Aspergillus species), protozoan infections (e.g. from Plasmodium, Babesia, Giardia, Entamoeba, Leishmania or Trypanosomes), helminth infections (e.g. from schistosoma, roundworms, tapeworms or flukes), and prion infections;

VI. Central nervous system diseases such as Parkinson's disease, Alzheimer's disease, dementia, motor neuron

disease, Huntington's disease, cerebral malaria, brain injury from pneumococcal meningitis, intracranial aneurysms, traumatic brain injury, multiple sclerosis, and amyotrophic lateral sclerosis;

VII. Metabolic diseases such as type 2 diabetes (T2D), atherosclerosis, obesity, gout, and pseudo-gout;

VIII. Cardiovascular diseases such as hypertension, ischaemia, reperfusion injury including post-MI ischemic reperfusion injury, stroke including ischemic stroke, transient ischemic attack, myocardial infarction including recurrent myocardial infarction, heart failure including congestive heart failure and heart failure with preserved ejection fraction, embolism, aneurysms including abdominal aortic aneurysm, cardiovascular risk reduction (CvRR), and pericarditis including Dressler's syndrome;

IX. Respiratory diseases including chronic obstructive pulmonary disorder (COPD), asthma such as allergic asthma and steroid-resistant asthma, asbestosis, silicosis, nanoparticle induced inflammation, cystic fibrosis, and idiopathic pulmonary fibrosis;

X. Liver diseases including non-alcoholic fatty liver disease (NAFLD) and nonalcoholic steatohepatitis (NASH) including advanced fibrosis stages F3 and F4, alcoholic fatty liver disease (AFLD), and alcoholic steatohepatitis (ASH);

XI. Renal diseases including acute kidney disease, hyperoxaluria, chronic kidney disease, oxalate nephropathy, nephrocalcinosis, glomerulonephritis, and diabetic nephropathy;

XII. Ocular diseases including those of the ocular epithelium, age-related macular degeneration (AMO) (dry and wet), uveitis, corneal infection, diabetic retinopathy, optic nerve damage, dry eye, and glaucoma;

XIII. Skin diseases including dermatitis such as contact dermatitis and atopic dermatitis, contact hypersensitivity, sunburn, skin lesions, hidradenitis suppurativa (HS), other cyst-causing skin diseases, and acne conglobata;

XIV. Lymphatic conditions such as lymphangitis, and Castleman's disease;

XV. Psychological disorders such as depression, and psychological stress;

XVI. Graft versus host disease;

XVII. Bone diseases including osteoporosis, osteopetrosis;

XVIII. Blood disease including sickle cell disease;

XIX. Allodynia including mechanical allodynia; and

XX. Any disease where an individual has been determined to carry a germline or somatic non-silent mutation in NLRP3.

More specifically the compounds of the invention (without the provisos) may be useful in the treatment of an indication selected from: inflammasome-related diseases/disorders, immune diseases, inflammatory diseases, autoimmune diseases, or auto-inflammatory diseases, for example, autoinflammatory fever syndromes (e.g., cryopyrin-associated periodic syndrome), sickle cell disease, systemic lupus erythematosus (SLE), liver related diseases/disorders (e.g. chronic liver disease, viral hepatitis, non-alcoholic steatohepatitis (NASH), alcoholic steatohepatitis, and alcoholic liver disease), inflammatory arthritis related disorders (e.g. gout, pseudogout (chondrocalcinosis), osteoarthritis, rheumatoid arthritis, arthropathy *e.g* acute, chronic), kidney related diseases (e.g. hyperoxaluria, lupus nephritis, Type I/Type II diabetes and related complications (e.g. nephropathy, retinopathy), hypertensive nephropathy, hemodialysis related inflammation), neuroinflammation-related diseases (e.g. multiple sclerosis, brain infection, acute injury, neurodegenerative diseases, Alzheimer's disease), cardiovascular/metabolic diseases/disorders (e.g. cardiovascular risk reduction (CvRR), hypertension, atherosclerosis, Type I and Type II diabetes and related complications, peripheral artery disease (PAD), acute heart failure), inflammatory skin diseases (e.g. hidradenitis suppurativa, acne), wound healing and scar formation, asthma, sarcoidosis, age-related macular degeneration, and cancer related diseases/disorders (e.g. colon cancer, lung cancer, myeloproliferative neoplasms, leukemias, myelodysplastic syndromes (MOS), myelofibrosis). In particular, autoinflammatory fever syndromes (e.g. CAPS), sickle cell disease, Type I/Type II diabetes and related complications (e.g. nephropathy, retinopathy), hyperoxaluria, gout, pseudogout (chondrocalcinosis), chronic liver disease, NASH, neuroinflammation-related disorders (e.g. multiple sclerosis, brain infection, acute injury, neurodegenerative diseases, Alzheimer's disease), atherosclerosis and cardiovascular risk (e.g. cardiovascular risk reduction (CvRR), hypertension), hidradenitis suppurativa, wound healing and scar formation, and cancer (e.g. colon cancer, lung cancer, myeloproliferative neoplasms, leukemias, myelodysplastic syndromes (MOS), myelofibrosis).

In particular, compounds of the invention (without the provisos), may be useful in the treatment of a disease or disorder selected from autoinflammatory fever syndromes (e.g. CAPS), sickle cell disease, Type I/ Type II diabetes and related complications (e.g. nephropathy, retinopathy), hyperoxaluria, gout, pseudogout (chondrocalcinosis), chronic liver disease, NASH, neuroinflammation-related disorders (e.g. multiple sclerosis, brain infection, acute injury, neurodegenerative diseases, Alzheimer's disease), atherosclerosis and cardiovascular risk (e.g. cardiovascular risk reduction (CvRR), hypertension), hidradenitis suppurativa, wound healing and scar formation, and cancer (e.g. colon cancer, lung cancer, myeloproliferative neoplasms, leukemias, myelodysplastic syndromes (MOS), myelofibrosis). Thus, as a further aspect, the present invention provides the use of a compound of the invention (without the provisos) (hence, including a compound as defined by any of the embodiments/forms/examples herein) in therapy. In a further embodiment, the therapy is selected from a disease, which may be treated by inhibition of NLRP3 inflammasome. In another embodiment, the disease is as defined in any of the lists herein. Hence, there is provided any one of the compounds of the invention (without the provisos) described herein (including any of the embodiments/forms/examples) for use in the treatment of any of the diseases or disorders described herein (e.g. as described in the aforementioned lists).

## PHARMACEUTICAL COMPOSITIONS AND COMBINATIONS

In an embodiment, the invention also relates to a composition comprising a pharmaceutically acceptable carrier and, as active ingredient, a therapeutically effective amount of a compound of the invention (without the provisos). The compounds of the invention may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations.

In an embodiment, and depending on the mode of administration, the pharmaceutical composition will preferably comprise from 0.05 to 99 % by weight, more preferably from 0.1 to 70 % by weight, even more preferably from 0.1 to 50 % by weight of the active ingredient(s), and, from 1 to 99.95 % by weight, more preferably from 30 to 99.9 % by weight, even more preferably from 50 to 99.9 % by weight of a pharmaceutically acceptable carrier, all percentages being based on the total weight of the composition.

The pharmaceutical composition may additionally contain various other ingredients known in the art, for example, a lubricant, stabilising agent, buffering agent, emulsifying agent, viscosity-regulating agent, surfactant, preservative, flavouring or colorant.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

The daily dosage of the compound according to the invention will, of course, vary with the compound employed, the mode of administration, the treatment desired and the mycobacterial disease indicated. However, in general, satisfactory results will be obtained when the compound according to the invention is administered at a daily dosage not exceeding 1 gram, e.g. in the range from 10 to 50 mg/kg body weight.

In an embodiment, there is provided a combination comprising a therapeutically effective amount of a compound

of the invention (without the provisos), according to any one of the embodiments described herein, and another therapeutic agent (including one or more therapeutic agents). In a further embodiment, there is provided such a combination wherein the other therapeutic agent is selected from (and where there is more than one therapeutic agent, each is independently selected from): farnesoid X receptor (FXR) agonists; anti-steatotics; anti-fibrotics; JAK inhibitors; checkpoint inhibitors including anti-PD1 inhibitors, anti-LAG-3 inhibitors, anti-TIM-3 inhibitors, or anti-POL1 inhibitors; chemotherapy, radiation therapy and surgical procedures; urate-lowering therapies; anabolics and cartilage regenerative therapy; blockade of IL-17; complement inhibitors; Bruton's tyrosine Kinase inhibitors (BTK inhibitors); Toll Like receptor inhibitors (TLR7/8 inhibitors); CAR-T therapy; antihypertensive agents; cholesterol lowering agents; leukotriene A4 hydrolase (LTAH4) inhibitors; SGLT2 inhibitors; 132-agonists; anti-inflammatory agents; nonsteroidal anti-inflammatory drugs ("NSAIDs"); acetylsalicylic acid drugs (ASA) including aspirin; paracetamol; regenerative therapy treatments; cystic fibrosis treatments; or atherosclerotic treatment. In a further embodiment, there is also provided such (a) combination(s) for use as described herein in respect of compounds of the invention (without the provisos), e.g. for use in the treatment of a disease or disorder in which the NLRP3 signalling contributes to the pathology, and/or symptoms, and/or progression, of said disease/disorder, or, a disease or disorder associated with NLRP3 activity (including NLRP3 inflammasome activity), including inhibiting NLRP3 inflammasome activity, and in this respect the specific disease/disorder mentioned herein apply equally here. There may also be provided methods as described herein in repsect of compounds of the invention (without the provisos), but wherein the method comprises administering a therapeutically effective amount of such combination (and, in an embodiment, such method may be to treat a disease or disorder mentioned herein in the context of inhibiting NLRP3 inflammasome activity). The combinations mentioned herein may be in a single preparation or they may be formulated in separate preparations so that they can be administered simultaneously, separately or sequentially. Thus, in an embodiment, the present invention also relates to a combination product containing (a) a compound according to the invention, according to any one of the embodiments described herein, and (b) one or more other therapeutic agents (where such therapeutic agents are as described herein), as a combined preparation for simultaneous, separate or sequential use in the treatment of a disease or disorder associated with inhibiting NLRP3 inflammasome activity (and where the disease or disorder may be any one of those described herein), for instance, in an embodiment, the combination may be a kit of parts. Such combinations may be referred to as "pharmaceutical combinations". The route of administration for a compound of the invention (without the provisos) as a component of a combination may be the same or different to the one or more other therapeutic agent(s) with which it is combined. The other therapeutic agent is, for example, a chemical compound, peptide, antibody, antibody fragment or nucleic acid, which is therapeutically active or enhances the therapeutic activity when administered to a patient in combination with a compound of the invention (without the provisos).

The weight ratio of (a) the compound according to the invention and (b) the other therapeutic agent(s) when given as a combination may be determined by the person skilled in the art. Said ratio and the exact dosage and frequency of administration depends on the particular compound according to the invention and the other antibacterial agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention. A particular weight ratio for the present compound of the invention and another antibacterial agent may range from 1/10 to 10/1, more in particular from 1/5 to 5/1, even more in particular from 1/3 to 3/1.

The pharmaceutical composition or combination of the present invention can be in unit dosage of about 1-1000 mg of active ingredient(s) for a subject of about 50 - 70 kg, or about 1 - 500 mg, or about 1 - 250 mg, or about 1 - 150 mg, or about 1- 100 mg, or about 1-50 mg of active ingredients. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable *in vitro* and *in vivo* tests using advantageously mammals, *e.g.,* mice, rats, dogs, monkeys or isolated organs, tissues and preparations thereof. The compounds of the present invention can be applied *in vitro* in the form of solutions, *e.g.,* aqueous solutions, and *in vivo* either enterally, parenterally, advantageously intravenously, *e.g.,* as a suspension or in aqueous solution. The dosage *in vitro* may range between about $10^{-3}$ molar and $10^{-9}$ molar concentrations. A therapeutically effective amount *in vivo* may range depending on the route of administration, between about 0.1 - 500 mg/kg, or between about 1 - 100 mg/kg.

As used herein, term "pharmaceutical composition" refers to a compound of the invention, or a pharmaceutically acceptable salt thereof, together with at least one pharmaceutically acceptable carrier, in a form suitable for oral or parenteral administration.

As used herein, the term "pharmaceutically acceptable carrier" refers to a substance useful in the preparation

or use of a pharmaceutical composition and includes, for example, suitable diluents, solvents, dispersion media, surfactants, antioxidants, preservatives, isotonic agents, buffering agents, emulsifiers, absorption delaying agents, salts, drug stabilizers, binders, excipients, disintegration agents, lubricants, wetting agents, sweetening agents, flavoring agents, dyes, and combinations thereof, as would be known to those skilled in the art (see, for example, Remington The Science and Practice of Pharmacy, 22nd Ed. Pharmaceutical Press, 2013, pp. 1049-1070).

The term "subject" as used herein, refers to an animal, preferably a mammal, most preferably a human, for example who is or has been the object of treatment, observation or experiment.

The term "therapeutically effective amount" as used herein, means that amount of compound of the invention (including, where applicable, form, composition, combination comprising such compound of the invention) elicits the biological or medicinal response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the compound of the present invention that, when administered to a subject, is effective to (1) at least partially alleviate, inhibit, prevent and/or ameliorate a condition, or a disorder or a disease (i) mediated by NLRP3, or (ii) associated with NLRP3 activity, or (iii) characterised by activity (normal or abnormal) of NLRP3; or (2) reduce or inhibit the activity of NLRP3; or (3) reduce or inhibit the expression of NLRP3. In another non-limiting embodiment, the term "a therapeutically effective amount" refers to the amount of the compound of the present invention that, when administered to a cell, or a tissue, or a non-cellular biological material, or a medium, is effective to at least partially reduce or inhibit the activity of NLRP3; or at least partially reduce or inhibit the expression of NLRP3.

As used herein, the term "inhibit", "inhibition" or "inhibiting" refers to the reduction or suppression of a given condition, symptom, or disorder, or disease, or a significant decrease in the baseline activity of a biological activity or process. Specifically, inhibiting NLRP3 or inhibiting NLRP3 inflammasome pathway comprises reducing the ability of NLRP3 or NLRP3 inflammasome pathway to induce the production of IL-1 and/or IL-18. This can be achieved by mechanisms including, but not limited to, inactivating, destabilizing, and/or altering distribution of NLRP3.

As used herein, the term "NLRP3" is meant to include, without limitation, nucleic acids, polynucleotides, oligonucleotides, sense and anti-sense polynucleotide strands, complementary sequences, peptides, polypeptides, proteins, homologous and/or orthologous NLRP molecules, isoforms, precursors, mutants, variants, derivatives, splice variants, alleles, different species, and active fragments thereof.

As used herein, the term "treat", "treating" or "treatment" of any disease or disorder refers to alleviating or ameliorating the disease or disorder (i.e., slowing or arresting the development of the disease or at least one of the clinical symptoms thereof); or alleviating or ameliorating at least one physical parameter or biomarker associated with the disease or disorder, including those which may not be discernible to the patient.

As used herein, the term "prevent", "preventing" or "prevention" of any disease or disorder refers to the prophylactic treatment of the disease or disorder; or delaying the onset or progression of the disease or disorder.

As used herein, a subject is "in need of" a treatment if such subject would benefit biologically, medically or in quality of life from such treatment.

"Combination" refers to either a fixed combination in one dosage unit form, or a combined administration where a compound of the present invention and a combination partner (e.g. another drug as explained below, also referred to as "therapeutic agent" or "co-agent") may be administered independently at the same time or separately within time intervals. The single components may be packaged in a kit or separately. One or both of the components (e.g. powders or liquids) may be reconstituted or diluted to a desired dose prior to administration. The terms "co-administration" or "combined administration" or the like as utilized herein are meant to encompass administration of the selected combination partner to a single subject in need thereof (e.g. a patient), and are intended to include treatment regimens in which the agents are not necessarily administered by the same route of administration or at the same time.

The term "pharmaceutical combination" as used herein means a product that results from the mixing or combining of more than one therapeutic agent and includes both fixed and non-fixed combinations of the therapeutic agents. The term "pharmaceutical combination" as used herein refers to either a fixed combination in one dosage unit form, or non-fixed combination or a kit of parts for the combined administration where two or more therapeutic agents may be administered independently at the same time or separately within time intervals. The term "fixed combination" means that the therapeutic agents, *e.g.* a compound of the present invention and a combination partner, are both administered to a patient simultaneously in the form of a single entity or dosage. The term "non-fixed combination" means that the therapeutic agents, *e.g.* a compound of the present invention and a combination partner, are both administered to a patient as separate entities either simultaneously, concurrently or sequentially with no specific time limits, wherein such administration provides therapeutically effective levels of the two compounds in the body of the patient. The latter also applies to cocktail therapy, e.g. the administration of three or more therapeutic agents.

The term "combination therapy" refers to the administration of two or more therapeutic agents to treat a therapeutic condition or disorder described in the present disclosure. Such administration encompasses co-administration of these therapeutic agents in a substantially simultaneous manner, such as in a single capsule having a fixed ratio of

active ingredients. Alternatively, such administration encompasses co-administration in multiple, or in separate containers (e.g. tablets, capsules, powders, and liquids) for each active ingredient. Powders and/or liquids may be reconstituted or diluted to a desired dose prior to administration. In addition, such administration also encompasses use of each type of therapeutic agent in a sequential manner, either at approximately the same time or at different times. In either case, the treatment regimen will provide beneficial effects of the drug combination in treating the conditions or disorders described herein.

Summary of pharmacology, uses, compositions and combinations

In an embodiment, there is provided a pharmaceutical composition comprising a therapeutically effective amount of a compound of the invention (without the provisos), according to any one of the embodiments described herein, and a pharmaceutically acceptable carrier (including one or more pharmaceutically acceptale carriers).

In an embodiment, there is provided a compound of the invention (without the provisos), according to any one of the embodiments described herein, for use as a medicament.

In an embodiment, there is provided a compound of the invention (without the provisos), according to any one of the embodiments described herein (and/or pharmaceutical compositions comprising such compound of the invention (without the provisos), according to any one of the embodiment described herein) for use: in the treatment of a disease or disorder associated with NLRP3 activity (including inflammasome activity); in the treatment of a disease or disorder in which the NLRP3 signalling contributes to the pathology, and/or symptoms, and/or progression, of said disease/disorder; in inhibiting NLRP3 inflammasome activity (including in a subject in need thereof); and/or as an NLRP3 inhibitor.

In an embodiment, there is provided a use of compounds of the invention (without the provisos), according to any one of the embodiments described herein (and/or pharmaceutical compositions comprising such compound of the invention (without the provisos), according to any one of the embodiment described herein): in the treatment of a disease or disorder associated with NLRP3 activity (including inflammasome activity); in the treatment of a disease or disorder in which the NLRP3 signalling contributes to the pathology, and/or symptoms, and/or progression, of said disease/disorder; in inhibiting NLRP3 inflammasome activity (including in a subject in need thereof); and/or as an NLRP3 inhibitor.

In an embodiment, there is provided use of compounds of the invention (without the provisos), according to any one of the embodiments described herein (and/or pharmaceutical compositions comprising such compound of the invention (without the provisos), according to any one of the embodiment described herein), in the manufacture of a medicament for: the treatment of a disease or disorder associated with NLRP3 activity (including inflammasome activity); the treatment of a disease or disorder in which the NLRP3 signalling contributes to the pathology, and/or symptoms, and/or progression, of said disease/disorder; and/or inhibiting NLRP3 inflammasome activity (including in a subject in need thereof).

In an embodiment, there is provided a method of treating a disease or disorder in which the NLRP3 signalling contributes to the pathology, and/or symptoms, and/or progression, of said disease/disorder, comprising administering a therapeutically effective amount of a compound of the invention (without the provisos), according to any one of the embodiments described herein (and/or pharmaceutical compositions comprising such compound of the invention (without the provisos), according to any one of the embodiment described herein), for instance to a subject (in need thereof). In a further embodiment, there is provided a method of inhibiting the NLRP3 inflammasome activity in a subject (in need thereof), the method comprising administering to the subject in need thereof a therapeutically effective amount of a compound of the invention (without the provisos), according to any one of the embodiments described herein (and/or pharmaceutical compositions comprising such compound of the invention (without the provisos), according to any one of the embodiment described herein).

In all relevant embodiment of the invention, where a disease or disorder is mentioned (e.g. hereinabove), for instance a disease or disorder in which the NLRP3 signalling contributes to the pathology, and/or symptoms, and/or progression, of said disease/disorder, or, a disease or disorder associated with NLRP3 activity (including NLRP3 inflammasome activity), including inhibiting NLRP3 inflammasome activity, then such disease may include inflammasome-related diseases or disorders, immune diseases, inflammatory diseases, auto-immune diseases, or auto-inflammatory diseases. In a further embodiment, such disease or disorder may include autoinflammatory fever syndromes (e.g cryopyrin-associated periodic syndrome), liver related diseases/disorders (e.g. chronic liver disease, viral hepatitis, non-alcoholic steatohepatitis (NASH), alcoholic steatohepatitis, and alcoholic liver disease), inflammatory arthritis related disorders (e.g. gout, pseudogout (chondrocalcinosis), osteoarthritis, rheumatoid arthritis, arthropathy e.g acute, chronic), kidney related diseases (e.g. hyperoxaluria, lupus nephritis, Type I/Type II diabetes and related complications (e.g. nephropathy, retinopathy), hypertensive nephropathy, hemodialysis related inflammation), neuroinflammation-related diseases (e.g. multiple sclerosis, brain infection, acute injury, neurodegenerative diseases, Alzheimer's disease), cardiovascular/metabolic diseases/ disorders (e.g. cardiovascular risk reduction (CvRR), hypertension, atherosclerosis, Type I and Type II diabetes and related complications, peripheral artery disease (PAD), acute heart failure), inflammatory skin diseases (e.g. hidradenitis suppurativa, acne), wound healing and scar formation, asthma, sarcoidosis, age-related

macular degeneration, and cancer related diseases/disorders (e.g. colon cancer, lung cancer, myeloproliferative neoplasms, leukaemia, myelodysplastic syndromes (MOS), myelofibrosis). In a particular aspect, such disease or disorder is selected from autoinflammatory fever syndromes (e.g. CAPS), sickle cell disease, Type I/Type II diabetes and related complications (e.g. nephropathy, retinopathy), hyperoxaluria, gout, pseudogout (chondrocalcinosis), chronic liver disease, NASH, neuroinflammation-related disorders (e.g. multiple sclerosis, brain infection, acute injury, neurodegenerative diseases, Alzheimer's disease), atherosclerosis and cardiovascular risk (e.g. cardiovascular risk reduction (CvRR), hypertension), hidradenitis suppurativa, wound healing and scar formation, and cancer (e.g. colon cancer, lung cancer, myeloproliferative neoplasms, leukemias, myelodysplastic syndromes (MOS), myelofibrosis). In a particular embodiment, the disease or disorder associated with inhibition of NLRP3 inflammasome activity is selected from inflammasome related diseases and disorders, immune diseases, inflammatory diseases, auto-immune diseases, auto-inflammatory fever syndromes, cryopyrin-associated periodic syndrome, chronic liver disease, viral hepatitis, non-alcoholic steatohepatitis, alcoholic steatohepatitis, alcoholic liver disease, inflammatory arthritis related disorders, gout, chondrocalcinosis, osteoarthritis, rheumatoid arthritis, chronic arthropathy, acute arthropathy, kidney related disease, hyperoxaluria, lupus nephritis, Type I and Type II diabetes, nephropathy, retinopathy, hypertensive nephropathy, hemodialysis related inflammation, neuroinflammation-related diseases, multiple sclerosis, brain infection, acute injury, neurodegenerative diseases, Alzheimer's disease, cardiovascular diseases, metabolic diseases, cardiovascular risk reduction, hypertension, atherosclerosis, peripheral artery disease, acute heart failure, inflammatory skin diseases, acne, wound healing and scar formation, asthma, sarcoidosis, age-related macular degeneration, colon cancer, lung cancer, myeloproliferative neoplasms, leukemias, myelodysplastic syndromes and myelofibrosis.

In an embodiment, there is provided a combination comprising a therapeutically effective amount of a compound of the invention (without the provisos), according to any one of the embodiments described herein, and another therapeutic agent (including one or more therapeutic agents). In a further embodiment, there is provided such a combination wherein the other therapeutic agent is selected from (and where there is more than one therapeutic agent, each is independently selected from): farnesoid X receptor (FXR) agonists; anti-steatotics; anti-fibrotics; JAK inhibitors; checkpoint inhibitors including anti-PD1 inhibitors, anti-LAG-3 inhibitors, anti-TIM-3 inhibitors, or anti-POL 1 inhibitors; chemotherapy, radiation therapy and surgical procedures; urate-lowering therapies; anabolics and cartilage regenerative therapy; blockade of IL-17; complement inhibitors; Bruton's tyrosine Kinase inhibitors (BTK inhibitors); Toll Like receptor inhibitors (TLR7/8 inhibitors); CAR-T therapy; antihypertensive agents; cholesterol lowering agents; leukotriene A4 hydrolase (LTAH4) inhibitors; SGLT2 inhibitors; 132-agonists; anti-inflammatory agents; nonsteroidal anti-inflammatory drugs ("NSAIDs"); acetylsalicylic acid drugs (ASA) including aspirin; paracetamol; regenerative therapy treatments; cystic fibrosis treatments; or atherosclerotic treatment. In a further embodiment, there is also provided such (a) combination(s) for use as described herein in respect of compounds of the invention (without the provisos), e.g. for use in the treatment of a disease or disorder in which the NLRP3 signalling contributes to the pathology, and/or symptoms, and/or progression, of said disease/disorder, or, a disease or disorder associated with NLRP3 activity (including NLRP3 inflammasome activity), including inhibiting NLRP3 inflammasome activity, and in this respect the specific disease/disorder mentioned herein apply equally here. There may also be provided methods as described herein in repsect of compounds of the invention (without the provisos), but wherein the method comprises administering a therapeutically effective amount of such combination (and, in an embodiment, such method may be to treat a disease or disorder mentioned herein in the context of inhibiting NLRP3 inflammasome activity). The combinations mentioned herein may be in a single preparation or they may be formulated in separate preparations so that they can be administered simultaneously, separately or sequentially. Thus, in an embodiment, the present invention also relates to a combination product containing (a) a compound according to the invention, according to any one of the embodiments described herein, and (b) one or more other therapeutic agents (where such therapeutic agents are as described herein), as a combined preparation for simultaneous, separate or sequential use in the treatment of a disease or disorder associated with inhibiting NLRP3 inflammasome activity (and where the disease or disorder may be any one of those described herein).

Compounds of the invention (including forms and compositions/combinations comprising compounds of the invention) may have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise.

For instance, compounds of the invention may have the advantage that they have a good or an improved thermodynamic solubility (e.g. compared to compounds known in the prior art; and for instance as determined by a known method and/or a method described herein). Compounds of the invention may have the advantage that they will block pyroptosis, as well as the release of pro-inflammatory cytokines (e.g. IL-1$\beta$) from the cell. Compounds of the invention may also have the advantage that they avoid side-effects, for instance as compared to compounds of the prior art, which may be due to selectivity of NLRP3 inhibition. Compounds of the invention may also have the advantage that they have good or improved *in vivo* pharmacokinetics and oral bioavailabilty. They may also have the advantage that

they have good or improved *in vivo* efficacy. Specifically, compounds of the invention may also have advantages over prior art compounds when compared in the tests outlined hereinafter (e.g. in Examples C and D).

## GENERAL PREPARATION AND ANALYTICAL PROCESSES

The compounds according to the invention can generally be prepared by a succession of steps, each of which may be known to the skilled person or described herein.

It is evident that in the foregoing and in the following reactions, the reaction products may be isolated from the reaction medium and, if necessary, further purified according to methodologies generally known in the art, such as extraction, crystallization and chromatography. It is further evident that reaction products that exist in more than one enantiomeric form, may be isolated from their mixture by known techniques, in particular preparative chromatography, such as preparative HPLC, chiral chromatography. Individual diastereoisomers or individual enantiomers can also be obtained by Supercritical Fluid Chromatography (SFC).

The starting materials and the intermediates are compounds that are either commercially available or may be prepared according to conventional reaction procedures generally known in the art.

### Analytical Part

LC-MS (LIQUID CHROMATOGRAPHY/MASS SPECTROMETRY)

*General procedure*

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software. Compounds are described by their experimental retention times (Rt) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc...). For molecules with multiple isotopic patterns (Br, Cl..), the reported value is the one obtained for the lowest isotope mass. All results were obtained with experimental uncertainties that are commonly associated with the method used.

Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

Table: LCMS Method codes (Flow expressed in mL/min; column temperature (T) in °C; Run time in minutes).

| Method code | Instrument | Column | mobile phase | gradient | Flow<br>------<br>Col T | Run time |
|---|---|---|---|---|---|---|
| Method A | Waters: Acquity® IClassUPLC®-DAD and Xevo G2-S QTOF | Waters: BEH C18 (1.7μm, 2.1x50mm) | A: 95% $CH_3COONH_4$ 6.5mM + 5% $CH_3CN$, B: $CH_3CN$ | From 95% A to 5% A in 4.6min, held for 0.4min | 1<br>----<br>50 | 5 |
| Method B | Waters: Acquity® IClassUPLC®-DAD and SQD | Agilent: RRHD (1.8μm, 2.1x50mm) | A: 95% $CH_3COONH_4$ 6.5mM + 5% $CH_3CN$, B: $CH_3CN$ | From 95% A to 5% A in 2.0min, held for 0.5min | 0.8<br>-------<br>50 | 2.5 |

(continued)

| Method code | Instrument | Column | mobile phase | gradient | Flow ------ Col T | Run time |
|---|---|---|---|---|---|---|
| Method C | Agilent: 1290 Infinity II - DAD and MSD/XT | Waters: XBridgeC18 (2.5 µm, 2.1x50mm) | A: $HCO_3NH_4$ 2.5g/L (32 mM) B: $CH_3CN$ | From 90% A to 0% A in 3.0min, held for 0.5 min, to 90% A in 0.7 min, held for 0.8 min | 0.8 ---- 25 | 5 |
| Method D | Waters: Acquity® UPLC®-DAD and SQD | Waters: BEH C18 (1.7µm, 2.1x50mm) | A: 95% $CH_3COONH_4$ 6.5mM + 5% $CH_3CN$, B: $CH_3CN$ | From 95% A to 5% A in 2.0 min, held for 0.5 min | 0.8 ------- 50 | 2.5 |
| Method E | Waters: Acquity® UPLC®-DAD and SQD | Waters: BEH C18 (1.7µm, 2.1x50mm) | A: 95% $CH_3COONH_4$ 6.5mM + 5% $CH_3CN$, B: $CH_3CN$ | From 95% A to 5% A in 4.5min, held for 0.5 min | 0.8 ------- 50 | 5 |
| Method F | Waters: Acquity® HClass UPLC® - DAD and QDa | Waters: XBridgeC18 (2.5 µm, 2.1x50mm) | A: $HCO_3NH_4$ 2.5g/L (32 mM) B: $CH_3CN$ | From 90% A to 0% A in 2.0min, held for 0.5 min, to 90% A, held for 0.5 min | 0.8 ------- 25 | 3.0 |
| Method G | Waters: Acquity® UPLC® - DAD and SQD | Waters :BEH (1.8µm, 2.1 * 100mm) | A: 0.1% $NH_4HCO3$ in 95% H2O + 5% $CH_3CN$ B: CH3CN | From 100% A to 5% A in 2.10min, to 0% A in 0.9min, to 5% A in 0.5min | 0.6 ------- 55 | 3.5 |
| Method H | Waters: Acquity® UPLC® - DAD and SQD | Waters :BEH (1.8µm, 2.1 * 100mm) | A: 0.1% $NH_4HCO3$ in 95% H2O + 5% $CH_3CN$ B: MeOH | From 100% A to 5% A in 2.10min, to 0% A in 0.9min, to 5% A in 0.5min | 0.6 ------- 55 | 3.5 |
| Method I | Waters: Acquity® UPLC® - DAD and SQD | Waters :BEH (1.8µm, 2.1*50mm) | A: 0.1% $NH_4HCO3$ in 95% H2O + 5% $CH_3CN$ B: CH3CN | From 100% A to 5% A in 1.3 min, hold 0.7min | 0.8 ------- 55 | 2.0 |
| Method J | Waters: Acquity® UPLC® - DAD and SQD2 | Waters :BEH (1.8µm, 2.1 * 100mm) | A: 0.1% $NH_4HCO3$ in 95% H2O + 5% $CH_3CN$ B: CH3CN | From 100% A to 5% A in 2.10min, to 0% A in 1.4min | 0.6 ------- 55 | 3.5 |
| Method K | Waters: Acquity® UPLC® - DAD and SQD | Waters :BEH (1.8µm, 2.1 * 100mm) | A: 10mM $CH_3COONH_4$ in 95% H2O + 5% $CH_3CN$ B: CH3CN | From 100% A to 5% A in 2.00min, to 0% A in 0.9min, to 5% A in 0.5min | 0.6 ------- 55 | 3.5 |

(continued)

| Method code | Instrument | Column | mobile phase | gradient | Flow ------ Col T | Run time |
|---|---|---|---|---|---|---|
| Method L | Waters: Acquity® UPLC® - DAD and SQD | Waters :BEH (1.8μm, 2.1 * 100mm) | A: 0.1% $NH_4HCO3$ in 95% H2O + 5% $CH_3CN$ B: CH3CN | From 100% A to 5% A in 2.10min, to 0% A in 0.9min, to 5% A in 0.4min | 0.6 ------- 55 | 3.5 |
| Method M | Agilent 1100 HPLC DAD LC/MS G1956A | YMC-pack ODS-AQ C18 (50 x 4.6 mm, 3 μm) | A: 0.1% HCOOH in H2O B: CH3CN | From 95% A to 5% A in 4.8 min, held for 1.0 min, to 95% A in 0.2 min | 2.6 ---- 35 | 6.2 |
| Method N | Waters: Acquity® UPLC®-DAD and SQD | Waters :BEH (1.8μm, 2.1 * 100mm) | A: 10mM $CH_3COONH_4$ in 95% H2O + 5% $CH_3CN$ B: CH3CN | From 100% A to 5% A in 2.10min, to 0% A in 0.9min, to 5% A in 0.5min | 0.6 ------- 55 | 3.5 |

NMR

For a number of compounds, $^1H$ NMR spectra were recorded on a Bruker Avance III spectrometer operating at 400 MHz, on a Bruker Avance III-HD operating at 400 MHz, on a Bruker Avance NEO spectrometer operating at 400 MHz, on a Bruker Avance Neo spectrometer operating at 500 MHz, or on a Bruker Avance 600 spectrometer operating at 600 MHz, using CHLOROFORM-*d* (deuterated chloroform, $CDCl_3$), DMSO-$d_6$ (deuterated DMSO, dimethyl-d6 sulfoxide), METHANOL-$d_4$ (deuterated methanol), BENZENE-$d_6$ (deuterated benzene, $C_6D_6$) or ACETONE-$d_6$ (deuterated acetone, $(CD_3)_2CO$) as solvents. Chemical shifts (δ) are reported in parts per million (ppm) relative to tetramethylsilane (TMS), which was used as internal standard.

Melting Points

Values are either peak values or melt ranges, and are obtained with experimental uncertainties that are commonly associated with this analytical method. For a number of compounds, melting points were determined with a DSC823e (Mettler-Toledo) apparatus. Melting points were measured with a temperature gradient of 10°C/minute. Standard maximum temperature was 300°C.

**EXPERIMENTAL PART**

Hereinafter, the term "m.p." means melting point, "aq." means aqueous, "r.m." means reaction mixture, "rt" means room temperature, 'DIPEA' means *N,N*-diisopropylethylamine, "DIPE" means diisopropylether, 'THF' means tetrahydrofuran, 'DMF' means dimethylformamide, 'DCM' means dichloromethane, "EtOH" means ethanol 'EtOAc' means ethyl acetate, "AcOH" means acetic acid, "iPrOH" means isopropanol, "$iPrNH_2$" means isopropylamine, "MeCN" or "ACN" means acetonitrile, "MeOH" means methanol, "$Pd(OAc)_2$" means palladium(II)diacetate, "rac" means racemic, 'sat.' means saturated, 'SFC' means supercritical fluid chromatography, 'SFC-MS' means supercritical fluid chromatography/mass spectrometry, "LC-MS" means liquid chromatography/mass spectrometry, "GCMS" means gas chromatography/mass spectrometry, "HPLC" means high-performance liquid chromatography, "RP" means reversed phase, "UPLC" means ultra-performance liquid chromatography, "$R_t$" (or "RT") means retention time (in minutes), "$[M+H]^+$" means the protonated mass of the free base of the compound, "DAST" means diethylaminosulfur trifluoride, "DMTMM" means 4-(4,6-dimethoxy-1,3,5-triazin-2-yl)-4-methylmorpholinium chloride, "HATU" means *O*-(7-azabenzotriazol-1-yl)-*N,N,N',N'*-tetramethyluronium hexafluorophosphate (1-[bis(dimethylamino)methylene]-1H-1,2,3-triazolo[4,5-b]pyridinium 3-oxide hexafluorophosphate), "Xantphos" means (9,9-dimethyl-9H-xanthene-4,5-diyl)bis[diphenylphosphine], "TBAT" means tetrabutyl ammonium triphenyldifluorosilicate, "TFA" means trifuoroacetic acid, "$Et_2O$" means diethylether,

"DMSO" means dimethylsulfoxide, "$SiO_2$" means silica, "XPhos Pd G3" means (2-dicyclohexylphosphino-2',4',6'-triisopropyl-1,1'-biphenyl)[2-(2'-amino-1,1'-biphenyl)]palladium(II) ethanesulfonate.

For key intermediates, as well as some final compounds, the absolute configuration of chiral centers (indicated as *R* and/or *S*) were established via comparison with samples of known configuration, or the use of analytical techniques suitable for the determination of absolute configuration, such as VCD (vibrational cicular dichroism) or X-ray crystallography. When the absolute configuration at a chiral center is unknown, it is arbitrarily designated R*.

**Example A - Intermediates and Final Compounds of the Invention**

Synthesis of ethyl 4-acetyl-1H-pyrrole-2-carboxylate **1B**

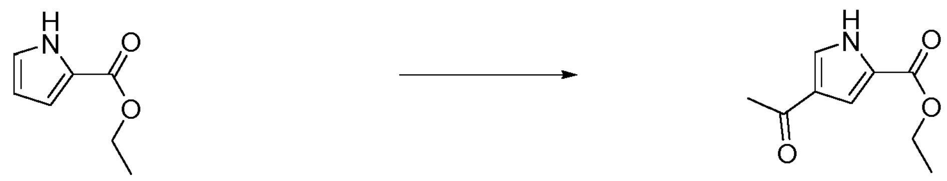

Aluminium chloride [7446-70-0] (20.12 g, 150.91 mmol) was added to a mixture of ethyl 1H-pyrrole-2-carboxylate [2199-43-1] (7 g, 50.3 mmol) and acetyl chloride [75-36-5] (4.85 mL, 65.4 mmol) in DCM (100 mL) at 0°C. The mixture was stirred at rt overnight. The reaction mixture was poured into ice water and extracted with DCM. The organic layer was dried over MgSO4, filtered and concentrated under vacuum to yield ethyl 4-acetyl-1H-pyrrole-2-carboxylate **1B** (9290 mg, 98% yield) as a black solid. The crude was used such as in the next step.

Additional analogs were synthesized according to the above procedure substituting the reagents as appropriate.

| Reagent | Intermediate XX | Intermediate XX |
|---|---|---|
| 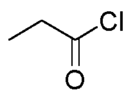 | 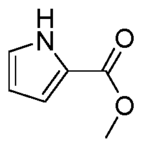 [1193-62-0] | 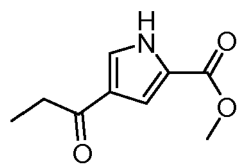 **2B** |

Synthesis of ethyl (E)-4-(1-(2-tosylhydrazineylidene)ethyl)-1H-pyrrole-2-carboxylate 3B

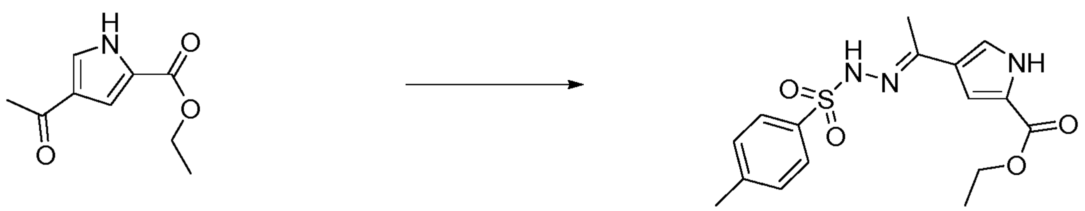

Ethyl 4-acetyl-1H-pyrrole-2-carboxylate 1B (9290 mg, 51.27 mmol) and p-toluenesulfonyl hydrazide [1576-35-8] (11.51 g, 61.81 mmol) was dissolved in EtOH (90 mL). The mixture was stirred and refluxed overnight and at rt over weekend. The resulting precipitate was collected by vacuum filtration to yield ethyl (E)-4-(1-(2-tosylhydrazineylidene)ethyl)-1H-pyrrole-2-carboxylate 3B (16060 mg, 88% yield) as a pale brown solid. The crude was used such as in the next step.

Additional analogs were synthesized according to the above procedure substituting the reagents as appropriate.

| Intermediate XX | Intermediate XX |
|---|---|
| 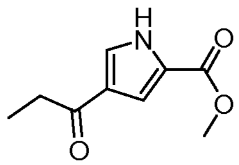 2B | 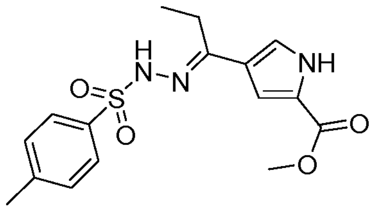 4B |

Synthesis of ethyl 4-ethyl-1H-pyrrole-2-carboxylate **5B**

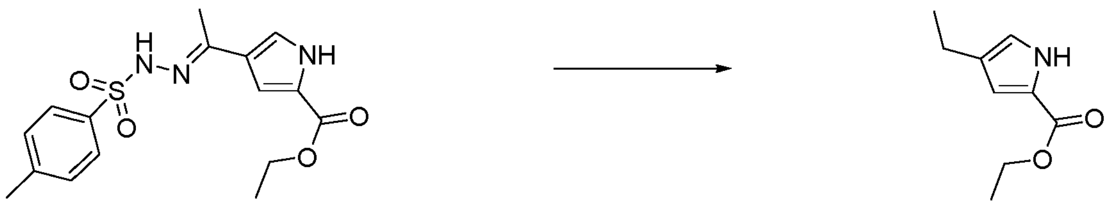

Sodium cyanoborohydride [25895-60-7] (5.76 g, 91.58 mmol) was added portionwise to a stirred solution of ethyl (E)-4-(1-(2-tosylhydrazineylidene)ethyl)-1H-pyrrole-2-carboxylate **3B** (16 g, 45.79 mmol) in glacial AcOH (185 mL). The mixture was stirred for 2h at 40°C. Then it was carefully poured into ice cold water. The mixture was extracted with diethyl ether. The combined extracts were washed carefully with sat. aq. sol. NaHCO3, dried (MgSO4 anh.), filtered and concentrated under vacuum. The crude product was purified by flash column chromatography (silica (120 g); AcOEt in heptane 0/100 to 25/75). The desired fractions were collected and concentrated to yield of ethyl 4-ethyl-1H-pyrrole-2-carboxylate **5B** (3700 mg, 47% yield) as a colourless oil.

Additional analogs were synthesized according to the above procedure substituting the reagents as appropriate.

| Intermediate XX | Intermediate XX |
|---|---|
| 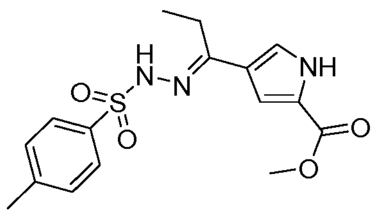 4B | 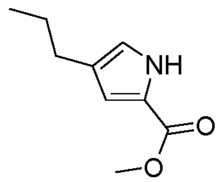 6B |

Synthesis of methyl 4-(prop-1-en-2-yl)-1H-pyrrole-2-carboxylate and methyl (E,Z)-4-(prop-1-en-1-yl)-1H-pyrrole-2-carboxylate **7B** (mixture of cis/trans)

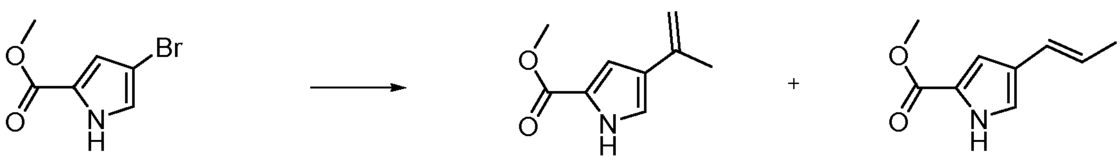

4,4,5,5-Tetramethyl-2-(prop-1-en-2-yl)-1,3,2-dioxaborolane [126726-62-3] (5.35 g, 31.86 mmol) was added to a stirred mixture of methyl 4-bromo-1H-pyrrole-2-carboxylate [934-05-4] (5 g, 24.51 mmol) in 1,4-dioxane (60 mL) and water (20 mL) under N2, followed by the addition of cesium carbonate [534-17-8] (15.97 g, 49.01 mmol) and Pd(dppf)Cl2

[95464-05-4] (1.62 g, 1.96 mmol). The reaction was stirred at 100°C for 18h. The reaction was cooled down to 0°C, then filtered over celite, and washed with water and extracted with EtOAc.The organic layes was separated, dried over sodium sulfate, filtered and concentrated in vacuo, the crude was purified by flash column chromatography (silica; EtOAc in Heptane 0/100 to 10/90). The desired fractions were collected and evaporated in vacuo to yield of methyl 4-(prop-1-en-2-yl)-1H-pyrrole-2-carboxylate and methyl (E,Z)-4-(prop-1-en-1-yl)-1H-pyrrole-2-carboxylate **7B** (2g, 49% yield; mixture of cis/trans) as a solid. The crude was used in the next step without purification.

Synthesis of 4-isopropyl-1H-pyrrole-2-carbohydrazide and 4-propyl-1H-pyrrole-2-carbohydrazide **8B**

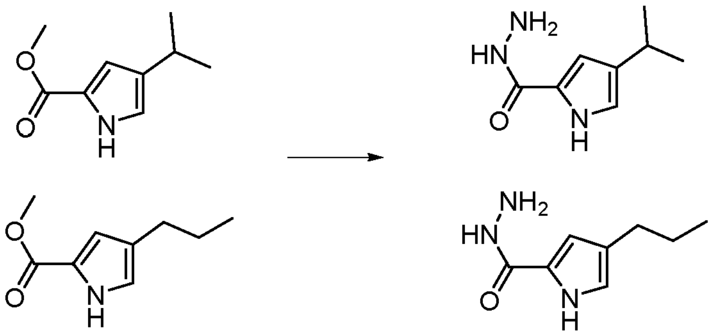

Hydrazine hydrate [7803-57-8] (5.23 mL, 107.73 mmol) was added dropwise to a stirred suspension of methyl 4-isopropyl-1H-pyrrole-2-carboxylate and methyl 4-propyl-1H-pyrrole-2-carboxylate **9B** (1.8 g, 10.77 mmol) in EtOH (20 mL). The mixture was stirred at 90°C for 18 hours. The reaction mixture was cooled to 0°C, water was added to the mixture and the precipitated solid was filtered off, washed with water and EtOH and then dried under vacuum at 50°C overnight, yielding 4-isopropyl-1H-pyrrole-2-carbohydrazide and 4-propyl-1H-pyrrole-2-carbohydrazide **8B** (1 g, 28% yield) as a white solid that was used in the next step reaction without any further purification.

Additional analogs were synthesized according to the above procedure substituting the reagents as appropriate.

| Intermediate XX | Intermediate XX |
|---|---|
| 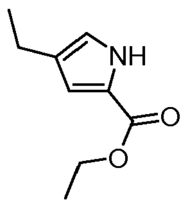 **5B** | 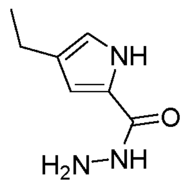 **10B** |
| 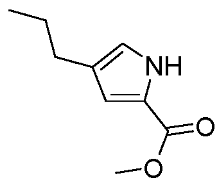 **6B** | 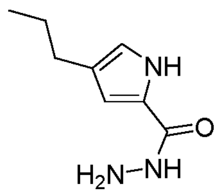 **11B** |

Synthesis of 7-bromo-4-isopropyl-2H-pyrrolo[1,2-d][1,2,4]triazin-1-one **1C**

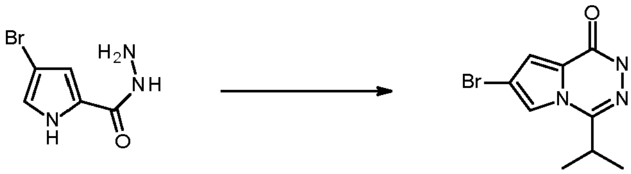

To a solution of 4-bromo-1H-pyrrole-2-carbohydrazide [259792-69-3] (300 mg, 1.47 mmol) in MeCN [75-05-8] (7.68 mL, 0.786 g/mL, 147.04 mmol) was added aluminum isopropoxide [555-31-7] (30.032 mg, 0.147 mmol). To the suspension was added 1,1,1-trimethyoxy-2-propane [52698-46-1] (0.281 mL, 0.93 g/mL, 1.764 mmol). The reaction was heated at 120 °C, while being vigorous stirred for 14h. The reaction mixture was concentrated in vacuo. The residue was suspended in DCM and filtered through a pad of Celite and the filtrate concentrated and purified by column chromatography eluting with an eluent of 85% heptane and 15% ethyl acetate. Combination and evaporation of pure fractions yielded 7-bromo-4-isopropyl-2H-pyrrolo[1,2-d][1,2,4]triazin-1-one **1C** (870 mg, 70 %, 1.02 mmol).

Structure analogs were synthesized using a similar procedure, replacing the pyrroloheterocycle for an appropriate analog.

| | Reagent | Intermediate |
|---|---|---|
| 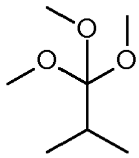 | 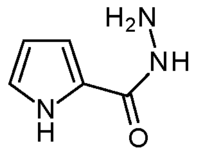 | 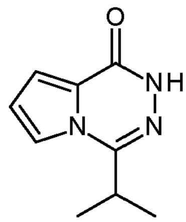 2C |
| 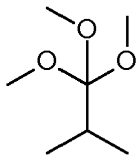 | 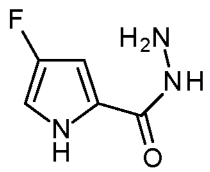 | 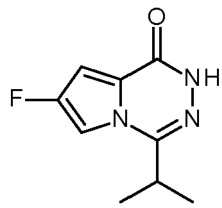 3C |
| 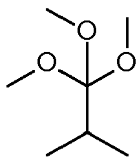 | 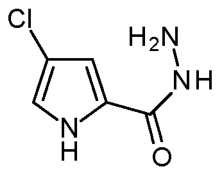 | 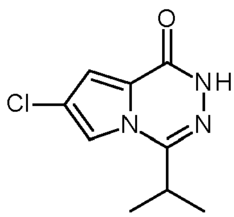 4C |
| 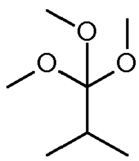 | 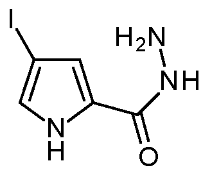 | 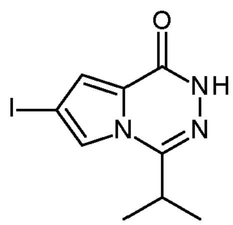 5C |

(continued)

| | Reagent | Intermediate |
|---|---|---|
| 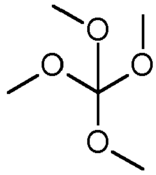 | 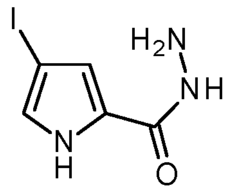 | 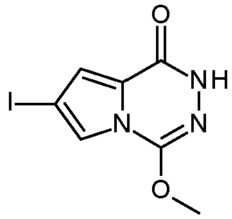 6C |
| 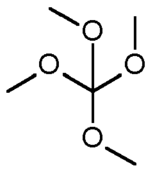 | 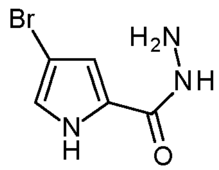 | 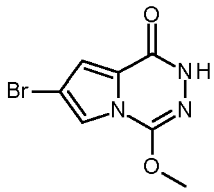 7C |
| 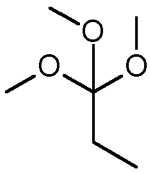 | 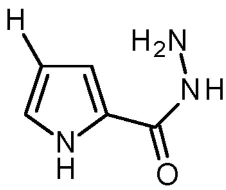 | 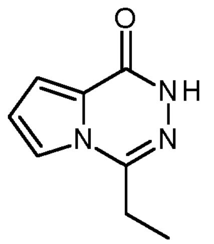 8C |
| 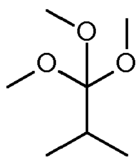 | 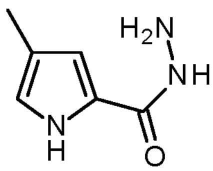 | 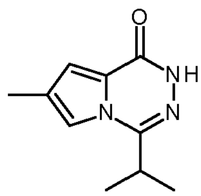 9C |
| 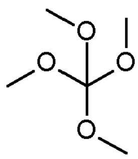 | 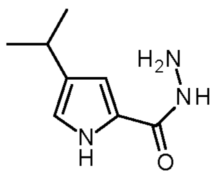 | 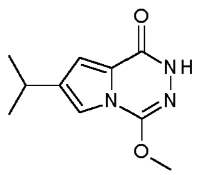 10C |
| 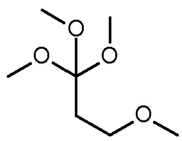 | 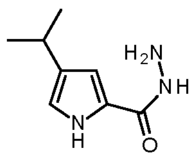 | 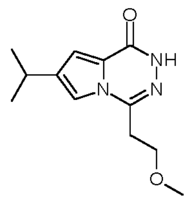 11C |
| 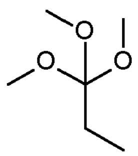 | 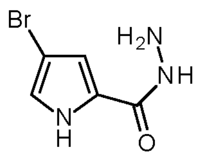 | 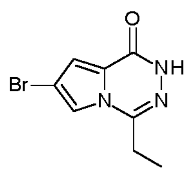 19C |

(continued)

| | Reagent | Intermediate |
|---|---|---|
| 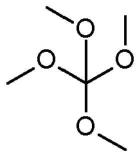 | 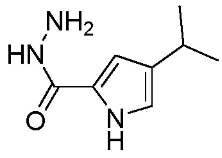 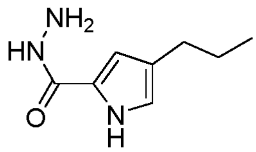 8B | 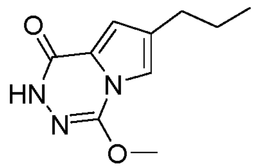 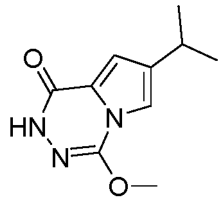 20C |
| 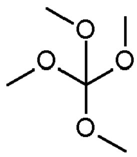 | 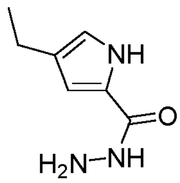 10B | 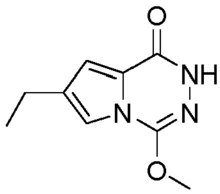 21C |
| 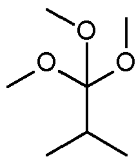 | 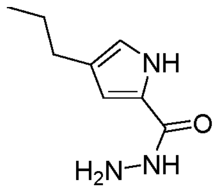 11B | 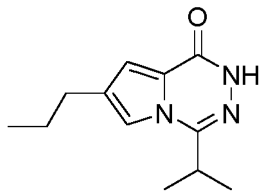 22C |
| 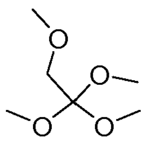 | 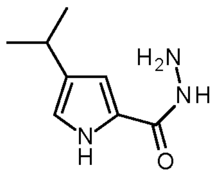 | 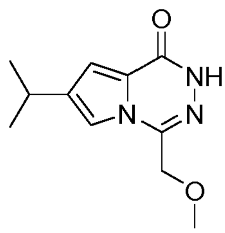 23C |

Synthesis of 4-isopropyl-7-vinyl-2H-pyrrolo[1,2-d][1,2,4]triazin-1-one **12C.**

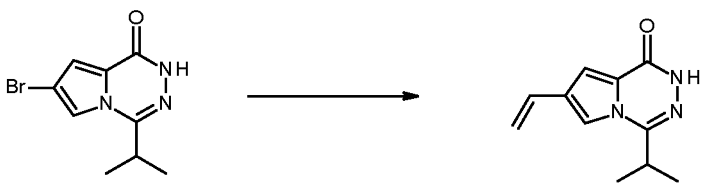

To a solution of Intermediate **1C** (630 mg, 2.46 mmol) in dioxane (15 mL) and water(5 mL) was added potassium phosphate [7778-53-2] (1488.174 mg, 7.011 mmol), RUPHOS Pd G3 [1445085-77-7] (88.471 mg, 0.106 mmol), and vinyl boronic acid pinacol ester [75927-49-0] (0.626 mL, 0.908 g/mL, 3.69 mmol). The reaction was heated at 90 °C for

3h. The mixture was partitioned between EtOAc (50 mL) and saturated brine solution (50 mL). The aqueous layer was extracted with EtOAc (50 mL) and the combined organics were dried over MgSO4 and concentrated in vacuo. The obtained brownish residue was purified by column chromatography eluting with gradient elution between Hept/EtOAc 9:1 to 55:45 to give pure 4-isopropyl-7-vinyl-2H-pyrrolo[1,2-d][1,2,4]triazin-1-one as a colorless solid **12C** (474 mg, 95%).

1HNMR (400 MHz, CHLOROFORM-d) 9.94 - 9.35 (m, 1 H), 7.33 (d, J=1.4 Hz, 1 H), 7.23 (d, J=1.5 Hz, 1 H), 6.67 (dd, J=17.6, 10.9 Hz, 1 H), 5.70 (dd, J=17.6, 0.9 Hz, 1 H), 5.29 (d, J=11.0 Hz, 1 H), 3.16 (spt, J=6.8 Hz, 1 H), 1.38 (d, J=6.8 Hz, 6 H).

Additional analogs were synthesized according to the above procedure substituting the reagents as appropriate.

| Intermediate XX | Reagent | Intermediate XX |
|---|---|---|
| **1C** | 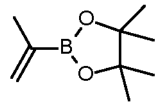 | 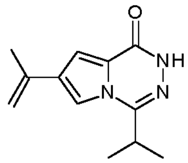 13C |
| **1C** | 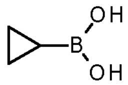 | 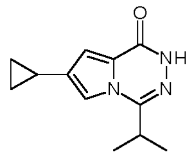 14C |
| **7C** | 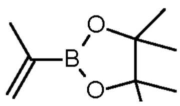 | 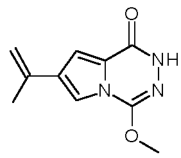 15C |
| **19C** | 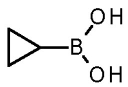 | 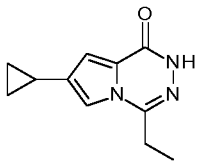 16C |

Synthesis of 7-ethyl-4-isopropyl-2H-pyrrolo[1,2-d]-[1,2,4]triazin-1-one, **17C**

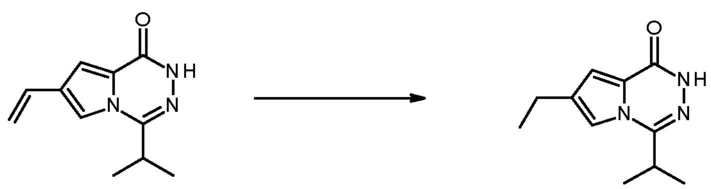

To a solution of 4-isopropyl-7-vinyl-2H-pyrrolo[1,2-d][1,2,4]triazin-1-one **12C** (150 mg, 0.738 mmol) in EtOH [64-17-5] (6.033 mL, 0.789 g/mL, 103.324 mmol) was added Pd/C (10%) (54.979 mg, 0.0517 mmol). The suspension was stirred under an atmosphere of hydrogen for 2.5 hours. The mixture filtered over Decalite, washing thoroughly with EtOH (ca. 60 mL), the solvent was concentrated in vacuo to give the crude product as a colorless solid 7-ethyl-4-isopropyl-2H-pyrrolo[1,2-d]-[1,2,4]triazin-1-one **17C** (150 mg, 0.74 mmol) that was used without further purification.

Additional analogs were synthesized according to the above procedure substituting the reagents as appropriate.

| Intermediate XX | Intermediate XX |
|---|---|
| 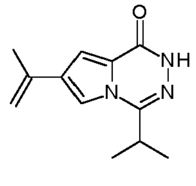 13C | 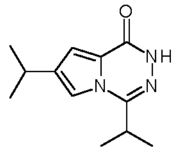 18C |
| 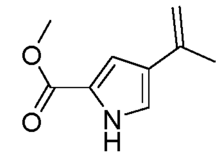 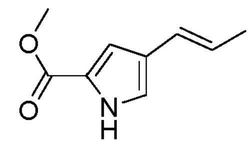 7B | 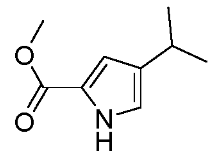 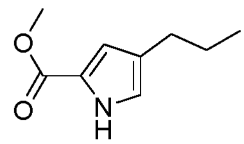 9B |

Synthesis of 4-isopropyl-1-oxo-1,2-dihydropyrrolo[1,2-d][1,2,4]triazine-7-carbonitrile **25C**

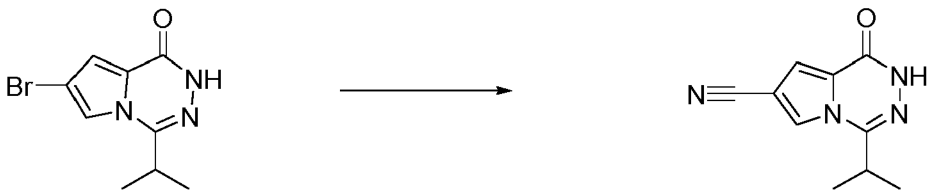

A 0.5-2 mL MW vial was charged with tBuXPhos Pd G3 (155.092 mg, 195.235 μmol), zinc cyanide [557-21-1] (137.553 mg, 1.171 mmol) and 7-bromo-4-isopropylpyrrolo[1,2-d][1,2,4]triazin-1(2H)-one **(1C)** (200 mg, 0.781 mmol). The vial was sealed and placed under nitrogen (3 cycles vacuum/nitrogen) and 1.5 mL of degassed THF and 3.5 mL of degassed DI water were added. The vial was stirred vigorously at 55°C for 18h. The crude mixture was partitioned between DCM (150 mL) and DI water (75 mL). The organic layer was collected and the aqueous re-extracted with DCM (30 mL). The combined organic layers were dried over sodium sulfate, filtered and concentrated in vacuo. The obtained residue was purified by FCC on Biotage (SNAP 100g, 75 mL/min, Hept/EtOAc 9:1 to 1:1 over 30 CV). The relevant fractions were combined to give 4-isopropyl-1-oxo-1,2-dihydropyrrolo[1,2-d][1,2,4]triazine-7-carbonitrile **25C** as a colorless solid (61 mg, 39%).

Synthesis of 2-chloro-N-(pyrimidin-4-yl)acetamide **1D**

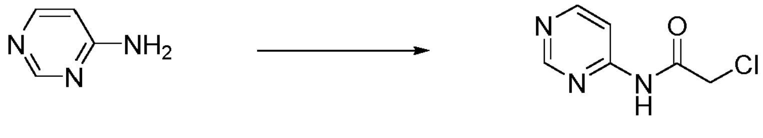

A solution of chloroacetyl chloride [79-04-9] (6.53 g, 57.78 mmol) in anhydrous CHCl3 (24 mL) was added slowly to a mixture of 4-aminopyrimidine [591-54-8] (5 g, 52.57 mmol) and TEA [121-44-8] (10.9 mL, 0.73 g/mL, 78.62 mmol) in anhydrous CHCl3 (800 mL) at room temperature. After 4 hr the reaction was quenched with H2O (100 mL). After stirring for 15 min the OL was separated, dried on MgSO4 and evaporated till 100 ml. The solids were filtered and washed with DCM. The solids were dried to yield 2-chloro-N-(pyrimidin-4-yl)acetamide **1D** (4.5 g, yield 83%) as a white

solid.

Synthesis of ethyl 2-(7-bromo-4-isopropyl-1-oxo-pyrrolo[1,2-d][1,2,4]triazin-2-yl)acetate, **1E**

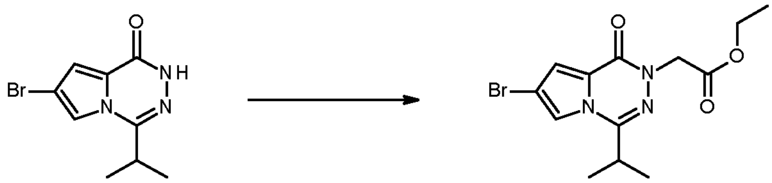

To a solution of 7-bromo-4-isopropyl-2H-pyrrolo[1,2-d][1,2,4]triazin-1-one **1C** (100 mg, 0.39 mmol) in MeCN (6 mL) was added 18-crown-6 [17455-13-9] (5.16 mg, 0.0195 mmol), potassium iodide [7681-11-0] (7.778 mg, 0.0469 mmol), $K_2CO_3$ [584-08-7] (64.758 mg, 0.469 mmol) and ethyl 2-chloroacetate (0.0499 mL, 1.15 g/mL, 0.469 mmol). The suspension was stirred vigorously and heated at 90 °C for 14h. The reaction mixture was diluted with 5 mL of water and extracted with DCM (4 x 3 mL). The combined organic extracts were concentrated and purified by column chromatography eluting with a gradient elution of heptane/EtOAc; 9:1 to 65:35 to give the pure product ethyl 2-(7-bromo-4-isopropyl-1-oxo-pyrrolo[1,2-d][1,2,4]triazin-2-yl)acetate **1E** as a colorless solid (125 mg, 94%, 0.366 mmol). 1H NMR (400 MHz, CHLOROFORM-d) 7.25 (d, J=1.5 Hz, 1 H), 7.18 (d, J=1.5 Hz, 1 H), 4.76 (s, 2 H), 4.23 (q, J=7.2 Hz, 2 H), 3.10 (spt, J=6.7 Hz, 1 H), 1.36 (d, J=6.8 Hz, 6 H), 1.28 (t, J=7.1 Hz, 3 H).

Additional analogs were synthesized according to the above procedure substituting the reagents as appropriate.

| Reagent | Intermediate XX | Intermediate |
|---|---|---|
| 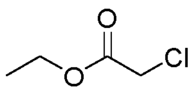 | **2C** | 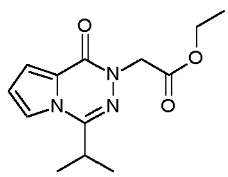 **2E** |
| 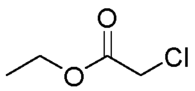 | **3C** | 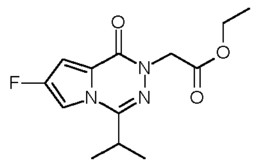 **3E** |
| 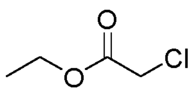 | **4C** | 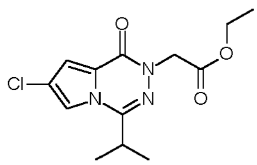 **4E** |
| 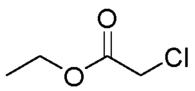 | **5C** | 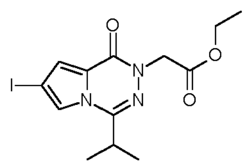 **5E** |

(continued)

| Reagent | Intermediate XX | Intermediate |
|---|---|---|
| 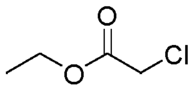 | **6C** | 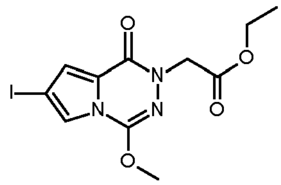 **6E** |
| 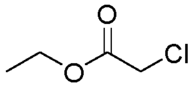 | **7C** | 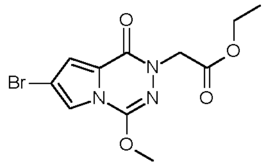 **7E** |
| 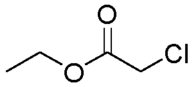 | **8C** | 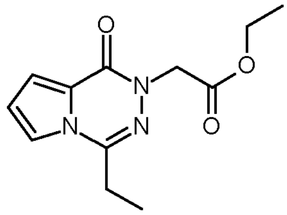 **8E** |
| 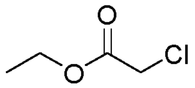 | **9C** | 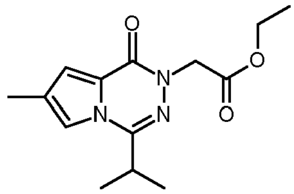 **9E** |
| 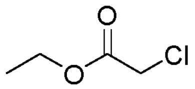 | **10C** | 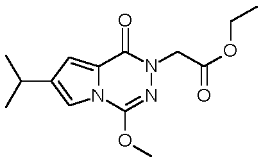 **10E** |
| 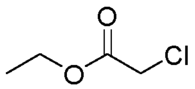 | **11C** | 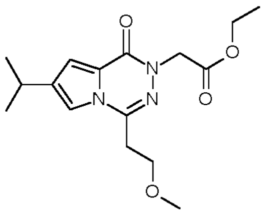 **11E** |
| 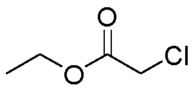 | **14C** | 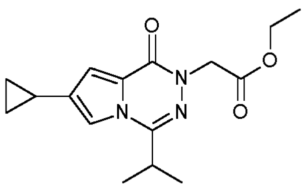 **12E** |

(continued)

| Reagent | Intermediate XX | Intermediate |
|---|---|---|
| 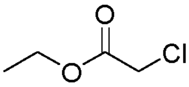 | **16C** | 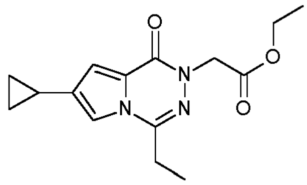 **13E** |
| 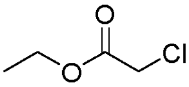 | **18C** | 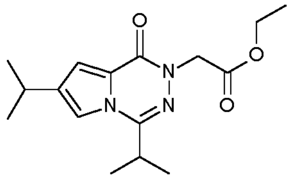 **14E** |
| 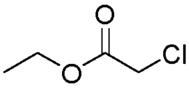 | **2C** | 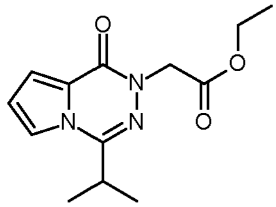 **22E** |
| 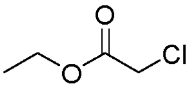 | **13C** | 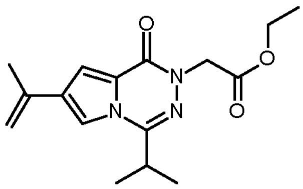 **23E** |
| 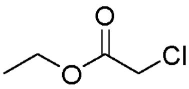 | **12C** | 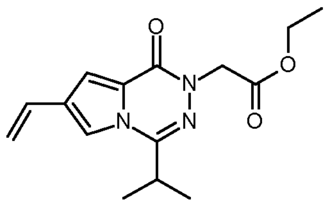 **24E** |
| 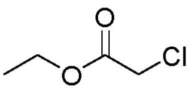 | **17C** | 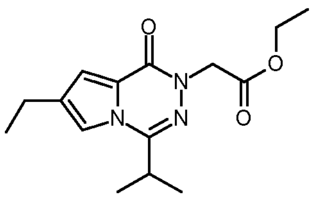 **25E** |
| 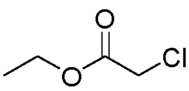 | **24C** | 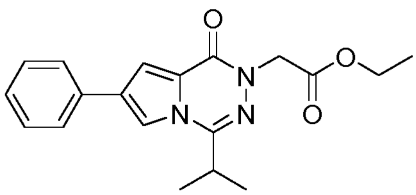 **29E** |

(continued)

| Reagent | Intermediate XX | Intermediate |
|---|---|---|
| 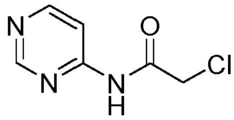 1D | 25C | 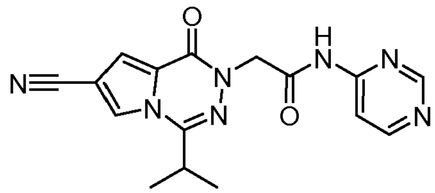 42F |
| 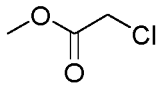 | 20C | 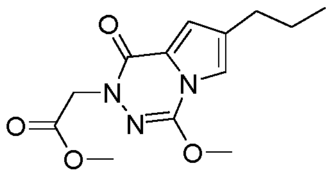 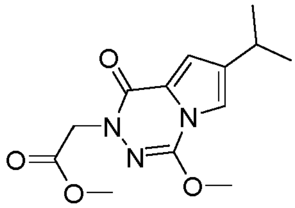 30E |
| 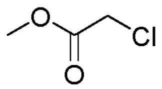 | 12C | 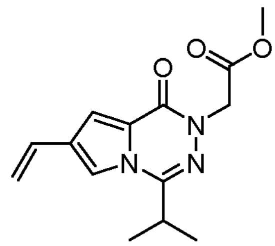 31E |
| 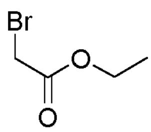 , Cs2CO3 | 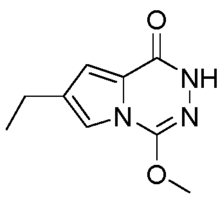 21C | 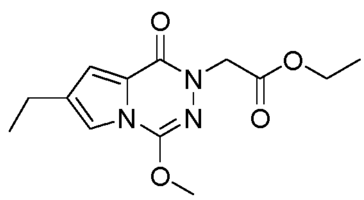 32E |
| 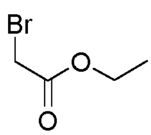 , Cs2CO3 | 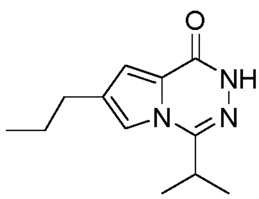 22C | 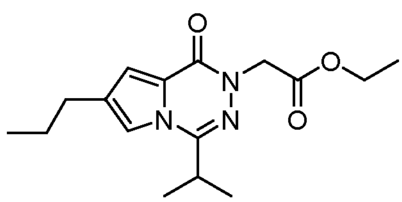 33E |

(continued)

| Reagent | Intermediate XX | Intermediate |
|---|---|---|
| 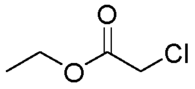 | 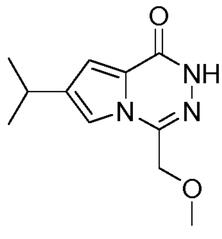 23C | 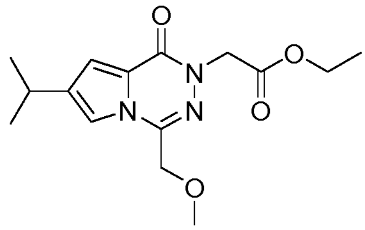 34E |

Synthesis of ethyl 2-[7-(difluoromethyl)-4-isopropyl-1-oxo-pyrrolo[1,2-d][1,2,4]triazin-2-yl]acetate **15E**

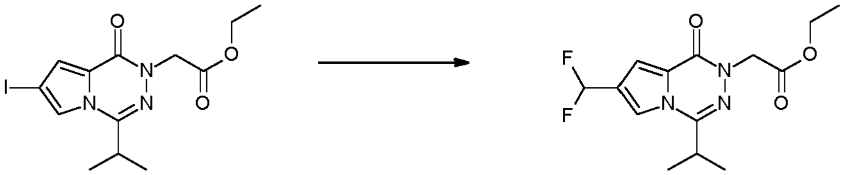

To a solution of ethyl 2-(7-iodo-4-isopropyl-1-oxo-pyrrolo[1,2-d][1,2,4]triazin-2-yl)acetate **5E** (80 mg, 0.19 mmol) in toluene (4 mL) was added silver [1,2-bis[2,6-bis(1-methylethyl)phenyl]-2-imidazolidinylidine(difluoromethyl) (145 mg, 0.26 mmol), XPhos Pd G3 (12 mg, 0.014 mmol), 2-dicyclohexylphosphino-2,4,6-triisopropylbiphenyl (8 mg, 0.0168 mmol). The resulting solution was stirred vigorously and heated at 100 °C for 5h. The crude mixture was cooled and filtered, the residue was washed with DCM, the filtrate was concentrated in vacuo. The residue was purified by column chromatography eluting with gradient elution of Hept/EtOAc from 93:7 to 7:3. ethyl 2-[7-(difluoromethyl)-4-isopropyl-1-oxo-pyrrolo[1,2-d][1,2,4]triazin-2-yl]acetate **15E** as a colorless oil (75 mg, 100%, 0.19 mmol).

Synthesis of ethyl 2-(7-isopropyl-1,4-dioxo-3H-pyrrolo[1,2-d][1,2,4]triazin-2-yl)acetate, **16E**

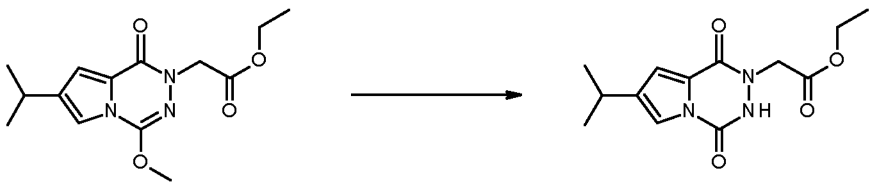

To a solution of ethyl 2-(7-isopropyl-4-methoxy-1-oxo-pyrrolo[1,2-d][1,2,4]triazin-2-yl)acetate **10E** (6.7 g, 22.8 mmol) in ACN (105 mL) was added sodium iodide (7.53 g, 50.25 mmol) and trimethylsilyl chloride (6.34 mL, 50.3 mmol). The resulting reaction was heated at 82°C for 90 minutes. At which point the reaction was quenched by the addition of water (450 mL), the solid that precipitated was collected by filtration. It was further washed with CAN/water and dried in vacuo to afford ethyl 2-(7-isopropyl-1,4-dioxo-3H-pyrrolo[1,2-d][1,2,4]triazin-2-yl)acetate **16E** as a white solid (5.7 g, 89% yield, 20.4 mmol).

Additional analogs were synthesized according to the above procedure substituting the reagents as appropriate.

| Intermediate | Product |
|---|---|
| 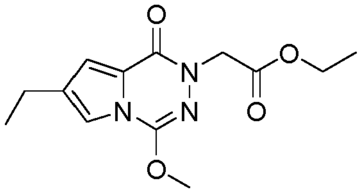 32E | 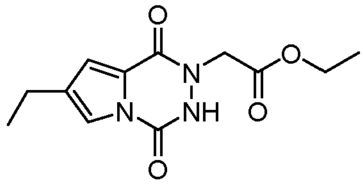 35E |

Synthesis of ethyl 2-(4-chloro-7-isopropyl-1-oxo-pyrrolo[1,2-d][1,2,4]triazin-2-yl)acetate, **17E**

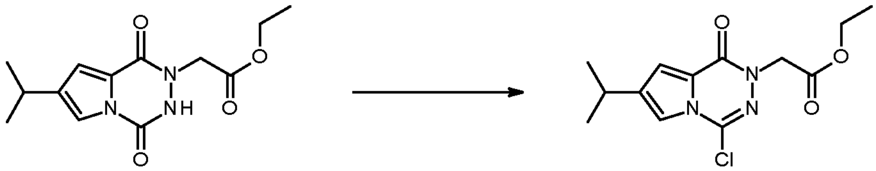

A solution of ethyl 2-(7-isopropyl-1,4-dioxo-3H-pyrrolo[1,2-d][1,2,4]triazin-2-yl)acetate **16E** (5 g, 17.9 mmol) in POCl3 (40 mL, 450 mmol) was heated at 125 °C for 18 hours. The reaction mixture was concentrated in vacuo and the residue was treated with a saturated solution of NaHCO3 and then extracted with DCM. The combined organic layers were dried over MgSO4 and concentrated in vacuo to afford ethyl 2-(4-chloro-7-isopropyl-1-oxo-pyrrolo[1,2-d][1,2,4]triazin-2-yl)acetate **17E** (5.1 g, 95% yield, 17.2 mmol) as brown solid.

Additional analogs were synthesized according to the above procedure substituting the reagents as appropriate.

| Intermediate | Product |
|---|---|
| 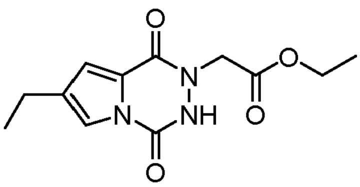 35E | 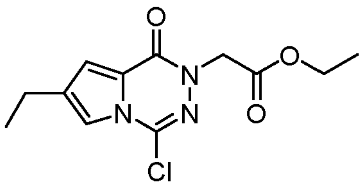 36E |

Synthesis of ethyl 2-(7-isopropyl-4-morpholino-1-oxo-pyrrolo[1,2-d][1,2,4]triazin-2-yl)acetate, **18E**

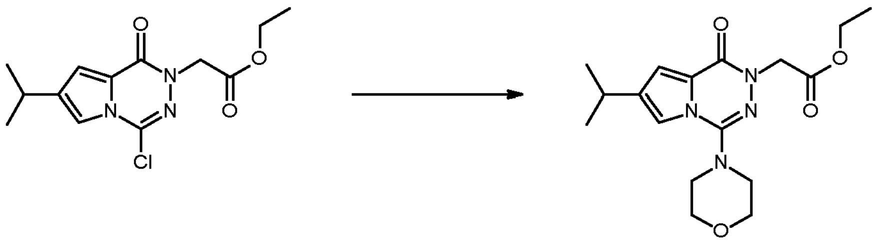

To a solution of ethyl 2-(4-chloro-7-isopropyl-1-oxo-pyrrolo[1,2-d][1,2,4]triazin-2-yl)acetate **17E** (200 mg, 0.67 mmol) in THF (4mL) was added morpholine (292 mg, 3.35 mmol). The resulting solution was heated at 90°C for 16 hours. The reaction mixture was concentrated in vacuo and the residue was purified using reverse phase chromatography, eluting with NH4HCO3/H2O/ACN to afford ethyl 2-(7-isopropyl-4-morpholino-1-oxo-pyrrolo[1,2-d][1,2,4]triazin-2-yl)acetate **18E** (155 mg, 66% yield, 0.445 mmol).

Additional analogs were synthesized according to the above procedure substituting the reagents as appropriate.

| Intermediate | Reagent | Product |
|---|---|---|
| **17E** | 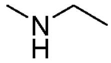 | 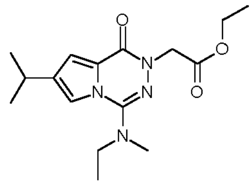 19E |
| **17E** | 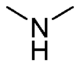 2 M in THF | 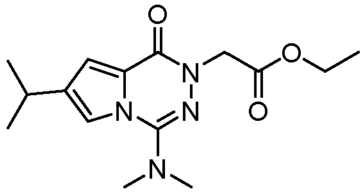 37E |
| **17 E** | 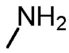 2 M in THF | 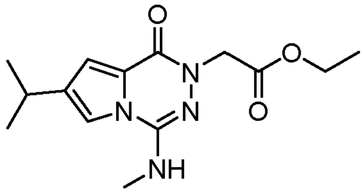 38E |
| **17E** | 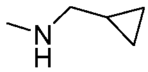 | 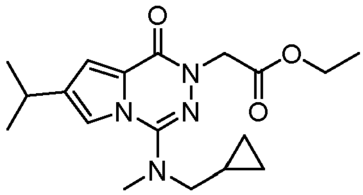 39E |

Synthesis of ethyl 2-(4-(dimethylamino)-7-ethyl-1-oxopyrrolo[1,2-d][1,2,4]triazin-2(1H)-yl)acetate **40E**

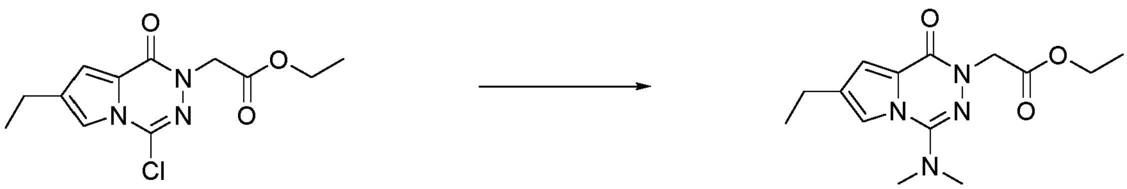

Dimethylamine solution 2M in THF [124-40-3] (238 μL, 0.48 mmol) was added to a stirred solution of ethyl 2-(4-chloro-7-ethyl-1-oxopyrrolo[1,2-d][1,2,4]triazin-2(1H)-yl)acetate **36E** (90 mg, 0.32 mmol) and N,N-diisopropylethylamine [7087-68-5] (83 μL, 0.48 mmol) in 1,4-dioxane (2 mL) in a sealed tube. The mixture was stirred at 90°C for 16 h. Then, dimethylamine solution 2M in THF[124-40-3] (476 μL, 0.95 mmol) was added and the mixture was stirred at 90°C for 3 days. The mixture was diluted with sat NaHCO3 and extracted with AcOEt. The organic layer was separated, dried (Na2SO4), filtered and the solvents evaporated in vacuo. The crude was purified by reverse phase (Phenomenex Gemini C18 30x100mm 5μm Column; from 72% [25mM NH4HCO3] - 28% MeCN to 36% [25mM NH4HCO3] - 64% MeCN). The desired fractions were collected and concentrated in vacuo to yield ethyl 2-(4-(dimethylamino)-7-ethyl-1-oxopyrrolo[1,2-d][1,2,4]triazin-2(1H)-yl)acetate **40E** (54 mg, 55% yield) as a pale yellow solid.

Additional analogs were synthesized according to the above procedure substituting the reagents as appropriate.

| Intermediate | Reagent | Product |
|---|---|---|
| 36E | 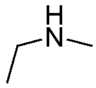 | 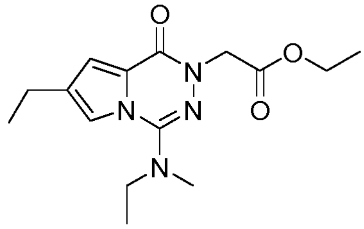 41E |

Synthesis of ethyl 2-[4-(1-ethoxyvinyl)-7-isopropyl-1-oxo-pyrrolo[1,2-d][1,2,4]triazin-2-yl]acetate, **20E**

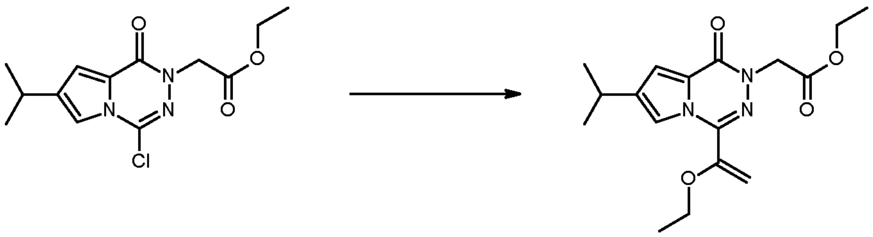

To a solution of ethyl 2-(4-chloro-7-isopropyl-1-oxo-pyrrolo[1,2-d][1,2,4]triazin-2-yl)acetate **17E** (1.5 g, 5.04 mmol) in dry THF[109-99-9] (61.23 mL, 0.89 g/mL, 755.69 mmol), was added bis(tri-tert-butylphosphine)palladium (0) [53199-31-8] (0.51 g, 1.01 mmol) was added followed by tributyl(1-ethoxyvinyl)tin [97674-02-7] (3.64 g, 10.08 mmol). The mixture was stirred for 5 hours at 90°C. The crude was evaporated in vacuo and was purified by column chromatography (Silica; MeOH in DCM 0/100 to 2/98). The desired fractions were collected and concentrated in vacuo to afford ethyl 2-[4-(1-ethoxyvinyl)-7-isopropyl-1-oxo-pyrrolo[1,2-d][1,2,4]triazin-2-yl]acetate **20E** (1.34 g, yield 79.78%) as a brown oil.

Additional analogs were synthesized according to the above procedure substituting the reagents as appropriate.

| Intermediate | |
|---|---|
| 17E | 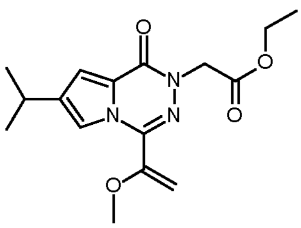 21E |

Synthesis of ethyl 2-(7-acetyl-4-isopropyl-1-oxo-pyrrolo[1,2-d][1,2,4]triazin-2-yl)acetate, **26E**

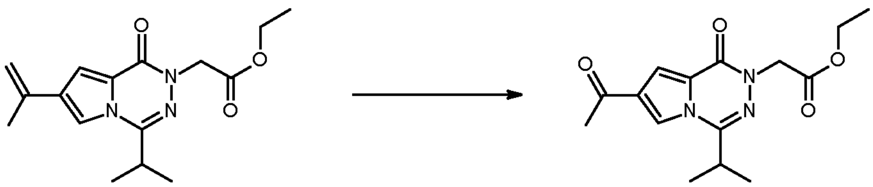

To a solution of ethyl 2-(7-isopropenyl-4-isopropyl-1-oxo-pyrrolo[1,2-d][1,2,4]triazin-2-yl)acetate **23E** (1600mg,

5.3 mmol) in tert-butyl alcohol (35 mL, 363 mmol), was added AD-mix-Alpha (7.35 g) and methane-sulfonamide (501 mg, 5.27 mmol) and water (35 mL). The resulting reaction mixture was stirred for 18 hours at RT. After 18 hrs, sodium periodate (2.48 g, 11.6 mmol) was added and the resulting solution was stirred for an additional 18 hrs. The reaction mixture was then poured onto brine (150 mL) and extracted with EtOAc (3 x 150 mL). The organic extracts were washed with brine (2 x 50 mL), concentrated and purified by column chromatography eluting with gradient elution of Hept/EtOAc (7:3 to 0:1) to give the ethyl 2-(7-acetyl-4-isopropyl-1-oxo-pyrrolo[1,2-d][1,2,4]triazin-2-yl)acetate, **26E** as a colorless solid (880 mg, 55%, 2.88 mmol).

Additional analogs were synthesized according to the above procedure substituting the reagents as appropriate.

| Intermediate | |
|---|---|
| **17F** | 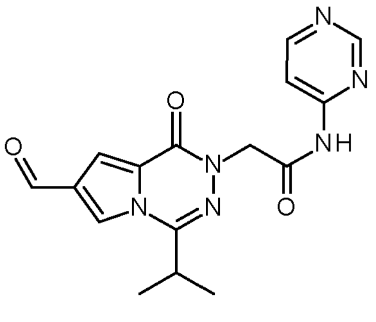 **41F** |

Synthesis of methyl 2-(7-formyl-4-isopropyl-1-oxopyrrolo[1,2-d][1,2,4]triazin-2(1H)-yl)acetate **42E**

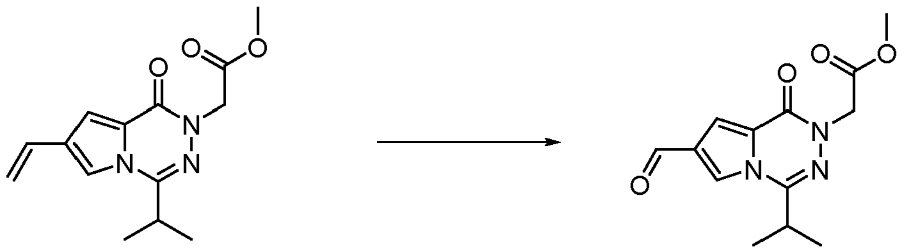

Water, distilled (3.5 mL) followed by sodium periodate [7790-28-5] (812 mg, 3.8 mmol) and osmium tetroxide (2.5% in tBuOH) [20816-12-0] (200 μL, 0.015 mmol) were added to a stirred solution of methyl 2-(4-isopropyl-1-oxo-7-vinylpyrrolo[1,2-d][1,2,4]triazin-2(1H)-yl)acetate **31E** (311 mg, 1.13 mmol) in 1,4-dioxane (8 mL) in a round-bottom flask and under N2. The mixture was stirred at rt for 16 h. The mixture was treated with 10% NaSO3H, basified with 10% Na2CO3 and extracted with DCM. The organic layer was separated, dried (MgSO4), filtered and the solvents evaporated in vacuo. The crude product was purified by flash column chromatography (SiO2, EtOAc in DCM 5/95 to 100/0). The desired fractions were collected and concentrated in vacuo to yield methyl 2-(7-formyl-4-isopropyl-1-oxopyrrolo[1,2-d][1,2,4]triazin-2(1H)-yl)acetate **42E** (195 mg, 62% yield) as a white solid.

Additional analogs were synthesized according to the above procedure substituting the reagents as appropriate.

| Intermediate | Product |
|---|---|
| **17F** | 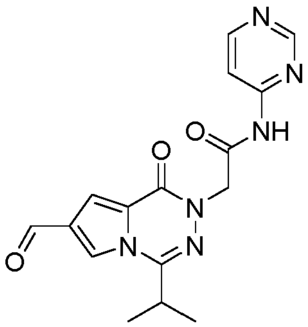 43E |

Synthesis of ethyl 2-[7-(1-hydroxyethyl)-4-isopropyl-1-oxo-pyrrolo[1,2-d][1,2,4]triazin-2-yl]acetate, **27E**

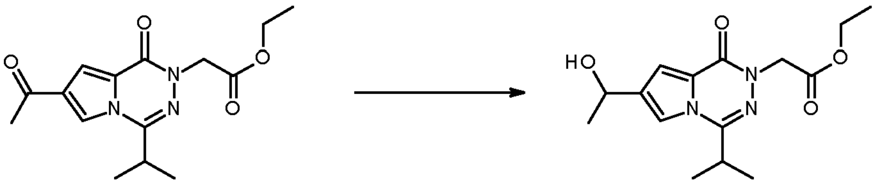

To a solution of ethyl 2-(7-acetyl-4-isopropyl-1-oxo-pyrrolo[1,2-d][1,2,4]tri azin-2-yl)acetate, **26E** (100 mg, 0.328 mmol) in methanol (1.5 mL), was added sodium borohydride (18.5 mg, 0.49 mmol). The resulting reaction mixture was stirred for 1 hour at 0°C. The mixture was quenched by addition of aq. sat. $NH_4Cl$ (2 mL), diluted with brine (30 mL) and extracted with DCM (3 x 15 mL) and the organic solutions were combined, dried over $Na_2SO_4$, filtered and concentrated to afford crude alcohol ethyl 2-[7-(1 -hydroxyethyl)-4-isopropyl-1-oxo-pyrrolo[1,2-d][1,2,4]triazin-2-yl]acetate **27E** (100 mg, 0.33 mmol, quant.) which was used without further purification.

Additional analogs were synthesized according to the above procedure substituting the reagents as appropriate.

| Intermediate | Final compound |
|---|---|
| **41F** | 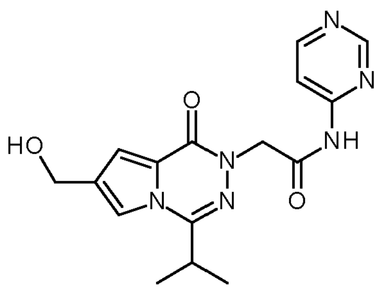 12F |
| **44E** | 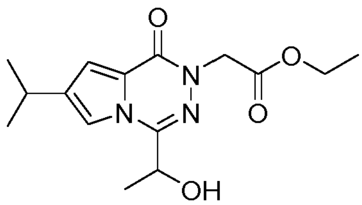 45E |

Synthesis of ethyl 2-(7-isopropyl-4-(1-((methylsulfonyl)oxy)ethyl)-1-oxopyrrolo[1,2-d][1,2,4]triazin-2(1H)-yl)acetate and methyl 2-(7-isopropyl-4-(1-((methylsulfonyl)oxy)ethyl)-1-oxopyrrolo[1,2-d][1,2,4]triazin-2(1H)-yl)acetate **46E**

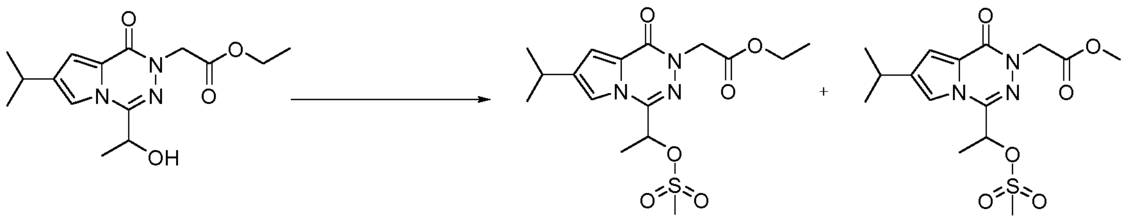

MsCl [124-63-0] (56.81 μL, 1.48 g/mL, 0.73 mmol) was added dropwise to a mixture of ethyl 2-(4-(1-hydroxyethyl)-7-isopropyl-1-oxopyrrolo[1,2-d][1,2,4]triazin-2(1H)-yl)acetate **45E** (188 mg, 0.61 mmol) in DCM, dry (4 mL) and TEA [121-44-8] (127.19 μL, 0.73 g/mL, 0.92 mmol) while stirring at 0°C. After 30 min the RM was allowed to reach rt and stirred at rt for 1 h. The mixture was poured out in NaHCO3 solution and the Ol was separated, dried on MgSO4 and evaporated, yielding ethyl 2-(7-isopropyl-4-(1-((methylsulfonyl)oxy)ethyl)-1-oxopyrrolo[1,2-d][1,2,4]triazin-2(1H)-yl)acetate and methyl 2-(7-isopropyl-4-(1-((methylsulfonyl)oxy)ethyl)-1-oxopyrrolo[1,2-d][1,2,4]triazin-2(1H)-yl)acetate **46E** (220 mg).

Synthesis of **47E**

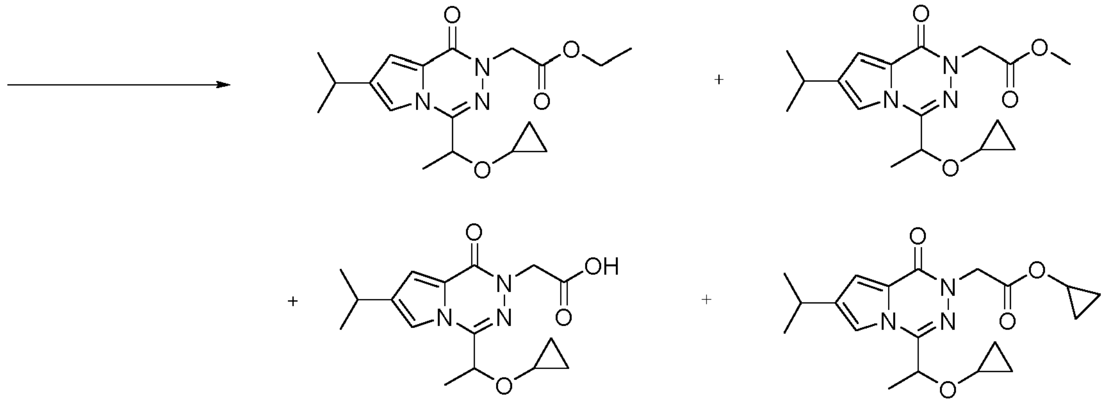

NaH (60% dispersion in mineral oil) [7646-69-7] (26.84 mg, 0.67 mmol) was added to a solution of cyclopropanol [16545-68-9] (38.97 mg, 0.67 mmol) in DMF PA, dry at rt under a nitrogen atmosphere. The mixture was stirred at rt for 10 min and then a solution of ethyl 2-(7-isopropyl-4-(1-((methylsulfonyl)oxy)ethyl)-1-oxopyrrolo[1,2-d][1,2,4]triazin-2(1H)-yl)acetate and methyl 2-(7-isopropyl-4-(1-((methylsulfonyl)oxy)ethyl)-1-oxopyrrolo[1,2-d][1,2,4]triazin-2(1H)-yl)acetate **46E** (199 mg) in 2 ml DMF was added and the mixture was stirred at rt for 1 h. The solvent was evaporated and the mixture **47E** was used as a crude for saponification.

Synthesis of ethyl 2-(4-isopropyl-7-(1-methoxyethyl)-1-oxopyrrolo[1,2-d][1,2,4]triazin-2(1H)-yl)acetate **48E**

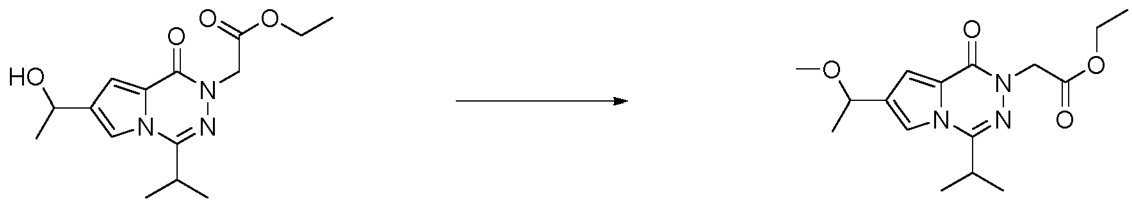

In a 20-mL MW vial, 2,6-di-tert-butyl-4-methylpyridine [38222-83-2] (231.033 mg, 1.125 mmol) and trimethyloxonium tetrafluoroborate [420-37-1] (124.809 mg, 0.844 mmol) were charged and placed under nitrogen (3 vacuum/nitrogen cycles). Then ethyl 2-[7-(1-hydroxyethyl)-4-isopropyl-1-oxo-pyrrolo[1,2-d][1,2,4]triazin-2-yl]acetate **27E** (95 mg, 0.281 mmol) was added as a solution in dry DCM (11 mL) and the resulting mixture was stirred at r.t. for 1.5 hours. The reaction was quenched by addition of aq. sat. NaHCO3 (8 mL), the organic layer collected and the aqueous layer re-extracted with DCM (2 x 8 mL). The combined organic extracts were dried over Na2SO4, filtered and concentrated in vacuo. The obtained residue was purified by FCC on Biotage (Sfar 25g, 75 mL/min, Hept/EtOAc 93:7 to 3:2 over 30 CV) to give ethyl 2-(4-isopropyl-7-(1-methoxyethyl)-1-oxopyrrolo[1,2-d][1,2,4]triazin-2(1H)-yl)acetate **48E** as a colorless oil (67 mg, 74%).

Synthesis of 2-(7-((dimethylamino)methyl)-4-isopropyl-1-oxopyrrolo[1,2-d][1,2,4]triazin-2(1H)-yl)-N-(pyrimidin-4-yl)acetamide **43F**

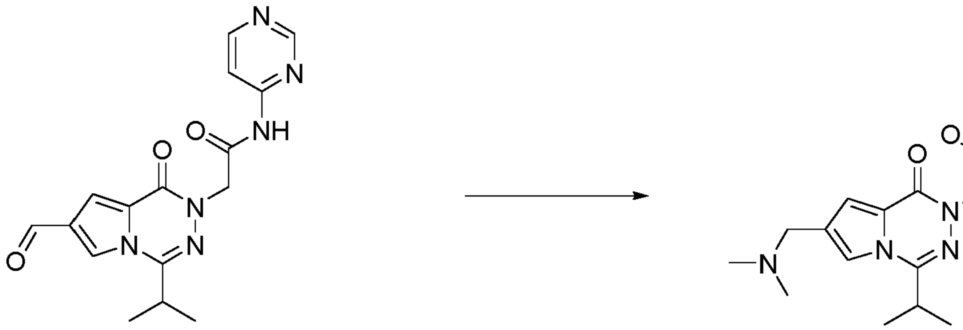

Sodium triacetoxyborohydride [56553-60-7] (67 mg, 0.32 mmol) was added to a stirred suspension of 2-(7-formyl-4-isopropyl-1-oxopyrrolo[1,2-d][1,2,4]triazin-2(1H)-yl)-N-(pyrimidin-4-yl)acetamide **41F** (59 mg, 0.17 mmol) and dimethylamine 2 M in THF [124-40-3] (0.3 mL, 0.6 mmol) in DCM (0.9 mL) in a tube and under N2. The mixture was stirred at rt for 16 h. The mixture was treated with 10% Na2CO3 and extracted with DCM. The organic layer was separated, dried (MgSO4), filtered and the solvents evaporated in vacuo. The crude product was purified by RP flash chromatography (C18, ACN in NH4HCO3 0.25% solution in Water 5/95 to 100/0). The desired fractions were collected and extracted with DCM. The organic layer was separated, dried (MgSO4), filtered and the solvents evaporated in vacuo to yield 2-(7-((dimethylamino)methyl)-4-isopropyl-1-oxopyrrolo[1,2-d][1,2,4]triazin-2(1H)-yl)-N-(pyrimidin-4-yl)acetamide **43F** (22 mg, 34% yield) as a white solid.

Additional analogs were synthesized according to the above procedure substituting the reagents as appropriate.

| Reagent | Intermediate | Product |
|---|---|---|
| 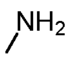 | 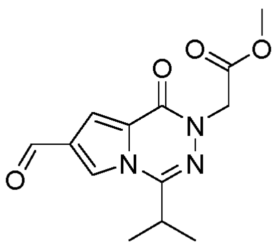 **42E** | 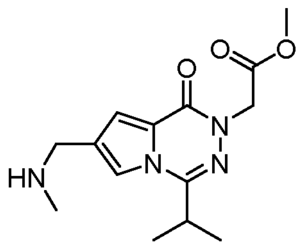 **49E** |

(continued)

| Reagent | Intermediate | Product |
|---|---|---|
| 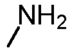 | 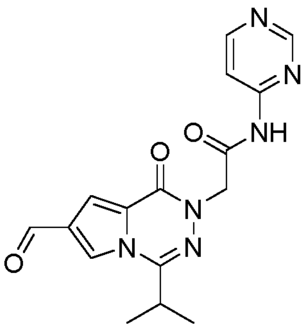 43E | 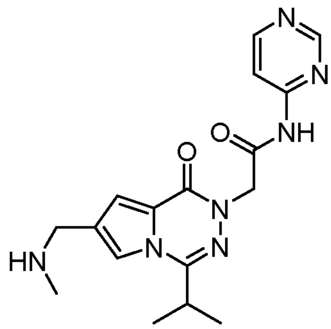 44F |

Synthesis of methyl 2-(4-isopropyl-7-((N-methylacetamido)methyl)-1-oxopyrrolo[1,2-d][1,2,4]triazin-2(1H)-yl)acetate **50E**

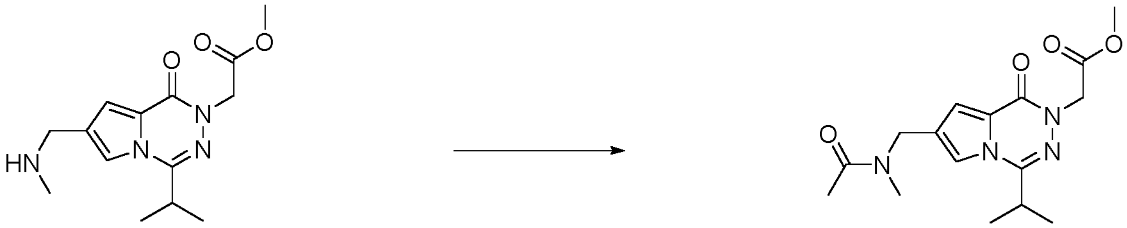

AcCl [75-36-5] (30 μL, 0.42 mmol) was added to a stirred solution of methyl 2-(4-isopropyl-7-((methylamino)methyl)-1-oxopyrrolo[1,2-d][1,2,4]triazin-2(1H)-yl)acetate **49E** (88 mg, 0.3 mmol) and DIPEA [7087-68-5] (83 μL, 0.48 mmol) in DCM (1.5 mL) at 0°C in a tube and under N2. The mixture was stirred at 0°C for 5 min and at rt for 90 min. The mixture was treated with 10% Na2CO3 and extracted with DCM. The organic layer was separated, dried (MgSO4), filtered and the solvent evaporated in vacuo to yield methyl 2-(4-isopropyl-7-((N-methylacetamido)methyl)-1-oxopyrrolo[1,2-d][1,2,4]triazin-2(1H)-yl)acetate **50E** (93 mg, 92% yield) as an orange oil.

Synthesis of ethyl 2-[7-(1-fluoroethyl)-4-isopropyl-1-oxo-pyrrolo[1,2-d][1,2,4]triazin-2-yl]acetate, **28E**

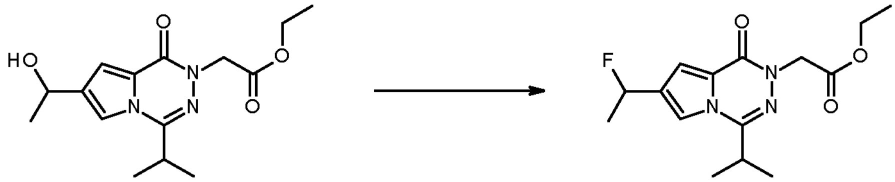

To a solution of ethyl 2-[7-(1-hydroxyethyl)-4-isopropyl-1-oxo-pyrrolo[1,2-d][1,2,4]triazin-2-yl]acetate, **27E** (20 mg, 0.06mmol) in DCM (1.5 mL), was added bis(2-methoxyethyl)aminosulfur trifluoride (0.03 mL, 2.7M, 0.08 mmol). The resulting solution was stirred at RT for 45 minutes. The mixture was quenched by addition of sat. aq. NaHCO3 (3 drops) and diluted with DCM (5 mL) and the solution filtered through a pad of Na2SO4, washing the reaction tube and filtration pad with additional DCM (5 mL). The filtrate was concentrated in vacuo and used without further purification ethyl 2-[7-(1-fluoroethyl)-4-isopropyl-1-oxo-pyrrolo[1,2-d][1,2,4]triazin-2-yl]acetate, **28E** (20mg, 0.06 mmol, quant).

Synthesis of isopropyl 2-(4-(1-(hydroxyimino)ethyl)-7-isopropyl-1-oxopyrrolo[1,2-d][1,2,4]triazin-2(1H)-yl)acetate and ethyl 2-(4-(1-(hydroxyimino)ethyl)-7-isopropyl-1-oxopyrrolo[1,2-d][1,2,4]triazin-2(1H)-yl)acetate **51E** (E/Z mixture)

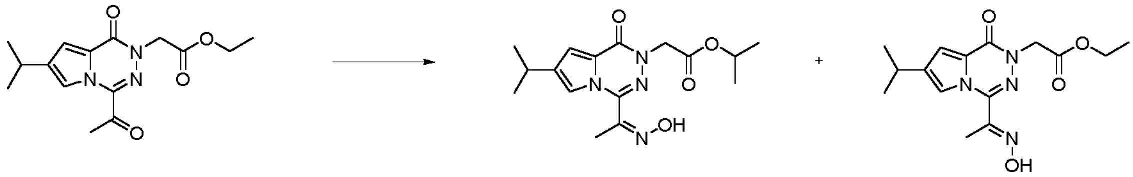

Ethyl 2-(4-acetyl-7-isopropyl-1-oxopyrrolo[1,2-d][1,2,4]triazin-2(1H)-yl)acetate **44E** (670 mg, 2.19 mmol) was stirred in THF (17.86 mL), hydroxylamine hydrochloride [5470-11-1] (167.73 mg, 2.41 mmol) was added, titanium(IV) isopropoxide [546-68-9] (1.3 mL, 0.96 g/mL, 4.39 mmol) was added, the reaction mixture was stirred for 3 h at 60°C and then allowed to reach room temperature. 1ml water and 10 ml MeOH and 10 ml DCM were added and the mixture was stirred at room temp for 5 min. The solids were filtered and the filtrate was evaporated. The residue was purified on silica, eluent MeOH in DCM, from 0 to 2 %. The pure fractions were evaporated, yielding isopropyl 2-(4-(1-(hydroxyimino)ethyl)-7-isopropyl-1-oxopyrrolo[1,2-d][1,2,4]triazin-2(1H)-yl)acetate and ethyl 2-(4-(1-(hydroxyimino)ethyl)-7-isopropyl-1-oxopyrrolo[1,2-d][1,2,4]triazin-2(1H)-yl)acetate **51E** (320 mg) as a white solid.

Synthesis of isopropyl 2-(4-(1-aminoethyl)-7-isopropyl-1-oxopyrrolo[1,2-d][1,2,4]triazin-2(1H)-yl)acetate and ethyl 2-(4-(1-aminoethyl)-7-isopropyl-1-oxopyrrolo[1,2-d][1,2,4]triazin-2(1H)-yl)acetate **52E**

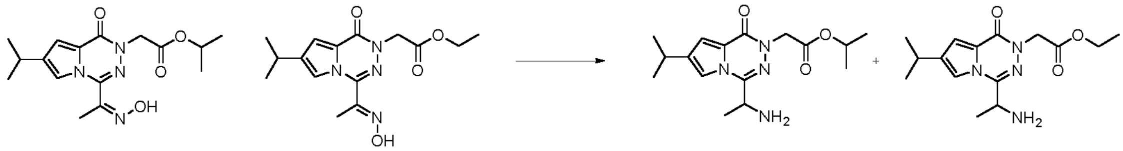

A mixture of isopropyl 2-(4-(1-(hydroxyimino)ethyl)-7-isopropyl-1-oxopyrrolo[1,2-d][1,2,4]triazin-2(1H)-yl)acetate and ethyl 2-(4-(1-(hydroxyimino)ethyl)-7-isopropyl-1-oxopyrrolo[1,2-d][1,2,4]triazin-2(1H)-yl)acetate **51E** (mixture E/Z) (300 mg, 0.9 mmol) and RaNi [7440-02-0] (2.76 mg, 0.047 mmol) in EtOH (25.02 mL) was hydrogenated at rt with hydrogen (1.81 mg, 0.9 mmol) for 48 h. The catalyst was filtered and the filtrate was evaporated. The residue was purified on a column with silica, eluent : MeOH in DCM, from 0 to 4 % to obtain isopropyl 2-(4-(1-aminoethyl)-7-isopropyl-1-oxopyrrolo[1,2-d][1,2,4]triazin-2(1H)-yl)acetate and ethyl 2-(4-(1-aminoethyl)-7-isopropyl-1-oxopyrrolo[1,2-d][1,2,4]triazin-2(1H)-yl)acetate **52E.**

Synthesis of isopropyl 2-(4-(1-acetamidoethyl)-7-isopropyl-1-oxopyrrolo[1,2-d][1,2,4]triazin-2(1H)-yl)acetate and ethyl 2-(4-(1-acetamidoethyl)-7-isopropyl-1-oxopyrrolo[1,2-d][1,2,4]triazin-2(1H)-yl)acetate **53E**

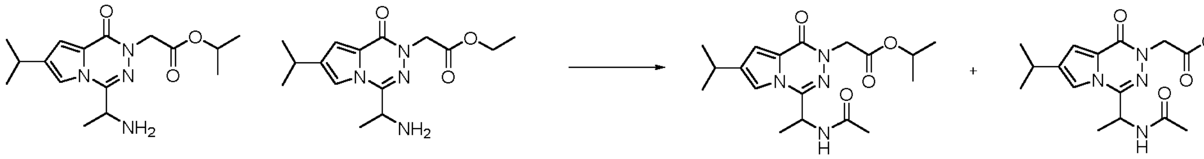

Acetic anhydride [108-24-7] (44.26 µL, 1.08 g/mL, 0.46 mmol) was added to a stirred solution of isopropyl 2-(4-(1-aminoethyl)-7-isopropyl-1-oxopyrrolo[1,2-d][1,2,4]triazin-2(1H)-yl)acetate and ethyl 2-(4-(1-aminoethyl)-7-isopropyl-1-oxopyrrolo[1,2-d][1,2,4]triazin-2(1H)-yl)acetate **52E** (99 mg, 0.31 mmol) and triethylamine [121-44-8] (108.76 µL, 0.73 g/mL, 0.77 mmol) in DCM anhydrous (6 mL) at rt. The mixture was stirred at rt for 4 h. The mixture was diluted with sat. NaHCO3 and extracted with DCM. The organic layer was separated, dried (MgSO4), filtered and the solvents evaporated in vacuo. The crude product was used without any further purification as isopropyl 2-(4-(1-acetamidoethyl)-7-isopropyl-1-oxopyrrolo[1,2-d][1,2,4]triazin-2(1H)-yl)acetate and ethyl 2-(4-(1-acetamidoethyl)-7-isopropyl-1-oxopyrrolo[1,2-d][1,2,4]triazin-2(1H)-yl)acetate **53E** (100 mg, yield 89%).

Additional analogs were synthesized according to the above procedure substituting the reagents as appropriate.

| Intermediate E- X | Intermediate A-X |
|---|---|
| 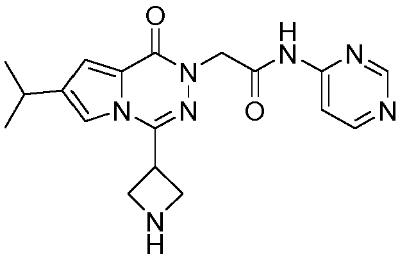 **45F** | 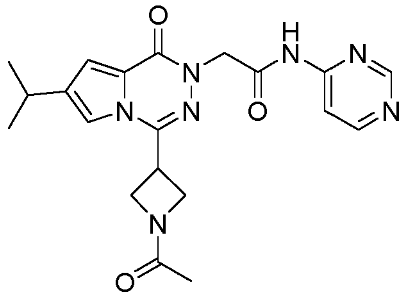 **46F** |

Synthesis of 2-(7-bromo-4-isopropyl-1-oxo-pyrrolo[1,2-d][1,2,4]triazin-2-yl)acetic acid, **1A**

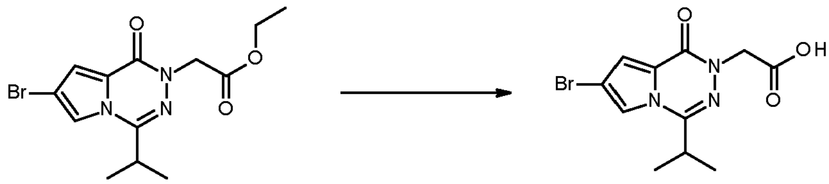

To a solution of ethyl 2-(7-bromo-4-isopropyl-1-oxo-pyrrolo[1,2-d][1,2,4]triazin-2-yl)acetate **1E** (1.82 g, 5.56 mmol) in THF (20 mL) and water (6.2 mL) was added LiOH (265 mg, 11 mmol). The resulting solution was stirred at room temperature for 18 hours, after which the mixture was diluted with water and acidified with a solution of 1N HCl, which resulted in the formation of a white precipitate, which was filtered and washed with water to afford 2-(7-bromo-4-isopropyl-1-oxo-pyrrolo[1,2-d][1,2,4]triazin-2-yl)acetic acid **1A** (1.74 g, 92%, 5.12 mmol).

Additional analogs were synthesized according to the above procedure substituting the reagents as appropriate.

| Intermediate E- X | Intermediate A-X |
|---|---|
| **2E** | 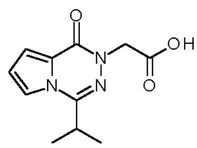 **2A** |
| **3E** | 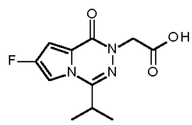 **3A** |
| **4E** | 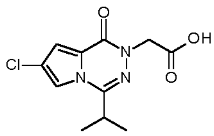 **4A** |

(continued)

| Intermediate E- X | Intermediate A-X |
|---|---|
| **5E** | 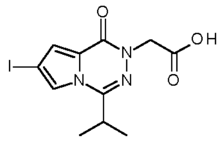 5A |
| **8E** | 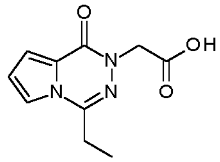 6A |
| **12E** | 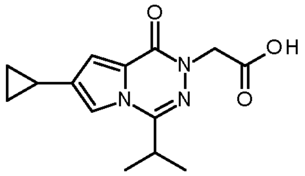 7A |
| **11E** | 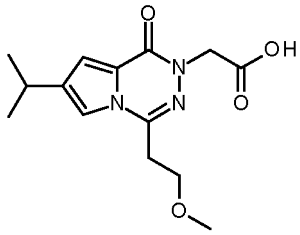 8A |
| **23E** | 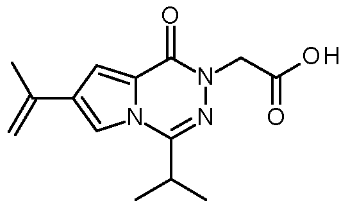 9A |
| **24E** | 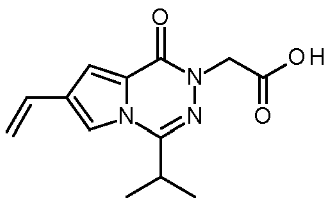 10A |
| **14E** | 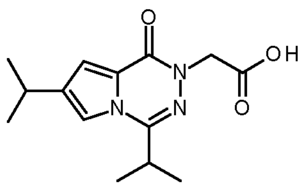 11A |

(continued)

| Intermediate E- X | Intermediate A-X |
|---|---|
| **13E** | 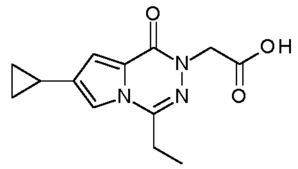 **12A** |
| **18E** | 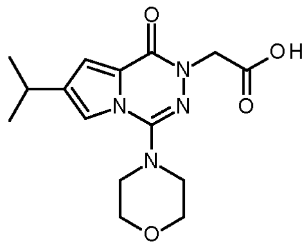 **13A** |
| **19E** | 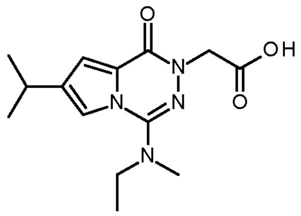 **14A** |
| **21E** | 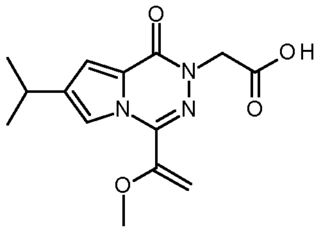 **15A** |
| **10E** | 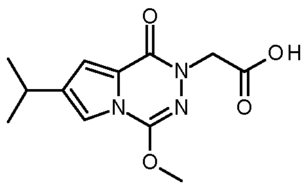 **16A** |
| **20E** | 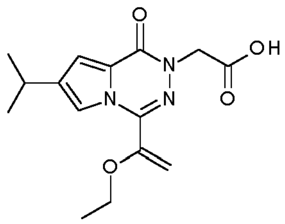 **17A** |

(continued)

| Intermediate E- X | Intermediate A-X |
|---|---|
| **15E** | 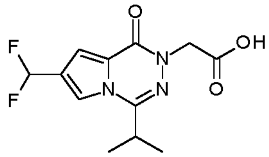 **18A** |
| **25E** | 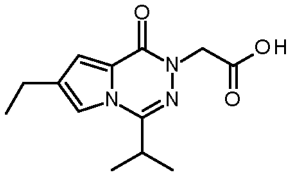 **19A** |
| **27E** | 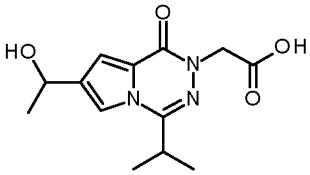 **20A** |
| **29E** | 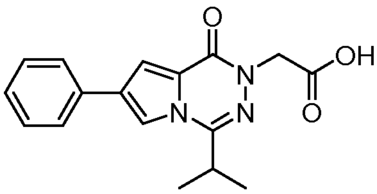 **22A** |
| 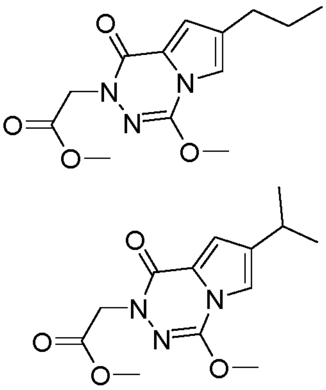 **30E** | 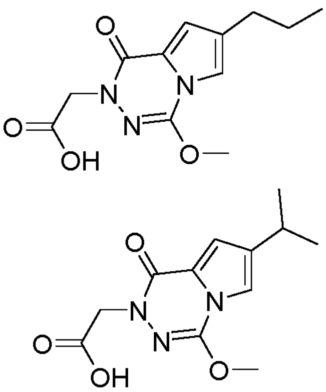 **23A** |
| 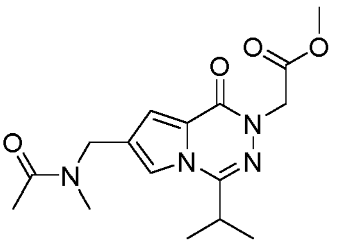 **50E** | 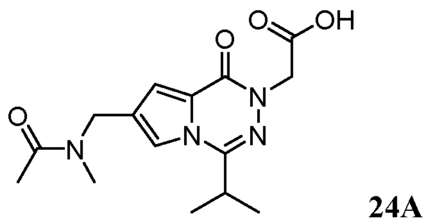 **24A** |

(continued)

| Intermediate E- X | Intermediate A-X |
|---|---|
| 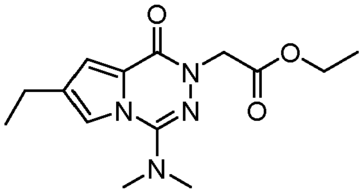 40E | 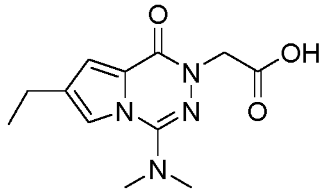 25A |

Synthesis of 2-(4-(dimethylamino)-7-isopropyl-1-oxopyrrolo[1,2-d] [1,2,4]triazin-2(1H)-yl)acetic acid **26A**

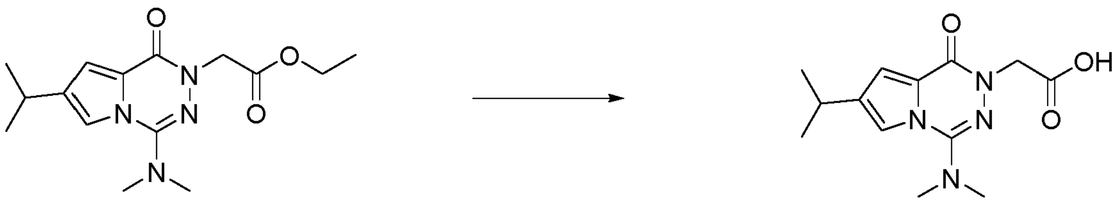

A mixture of ethyl 2-(4-(dimethylamino)-7-isopropyl-1-oxopyrrolo[1,2-d][1,2,4]triazin-2(1H)-yl)acetate **37E** (160 mg, 0.52 mmol), NaOH (1M in H2O) [1310-73-2] (783.38 μL, 1 M, 0.78 mmol) in THF (1976.17 μL) and water, distilled (1.98 mL) was stirred at rt for 2 h. HCl 1 N (0.783 ml) was added and the mixture was evaporated till water. The residue was dried in the oven under vacuum to yield 2-(4-(dimethylamino)-7-isopropyl-1-oxopyrrolo[1,2-d][1,2,4]triazin-2(1H)-yl)acetic acid **26A** (150 mg, quantitative) as a white solid.

Additional analogs were synthesized according to the above procedure substituting the reagents as appropriate.

| Intermediate E- X | Intermediate A-X |
|---|---|
| 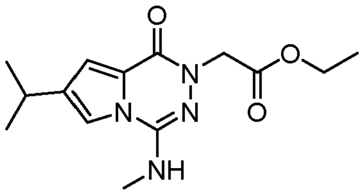 38E | 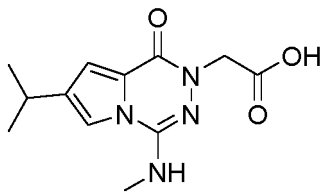 27A |
| 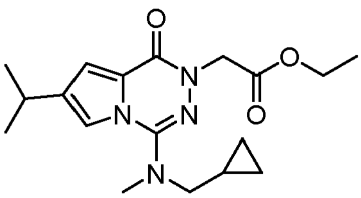 39E | 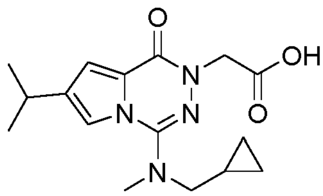 28A |

(continued)

| Intermediate E- X | Intermediate A-X |
| --- | --- |
| 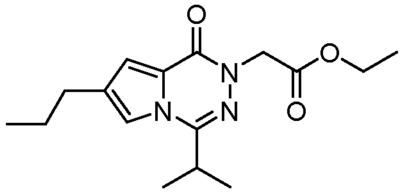 33E | 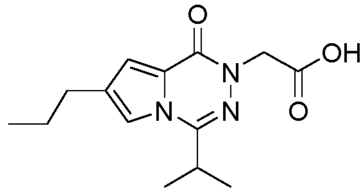 Solvent: MeOH 29A |
| 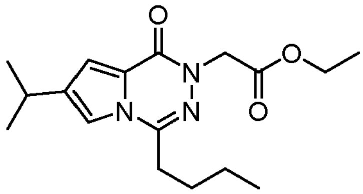 54E | 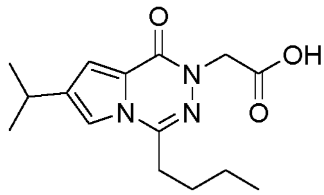 30A |
| 47E | 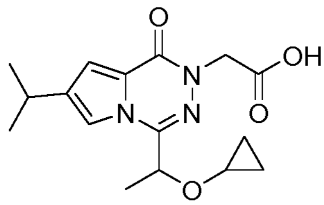 31A |
| 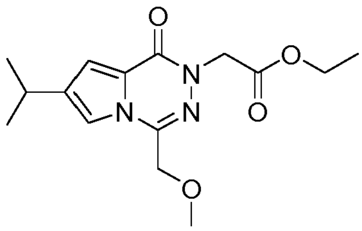 34E | 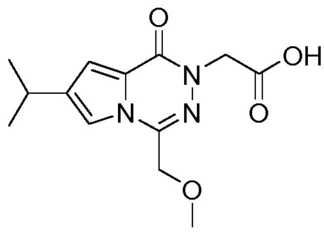 32A |
| 51E | 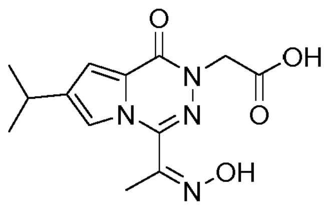 33A |

(continued)

| Intermediate E- X | Intermediate A-X |
|---|---|
| **53E** | 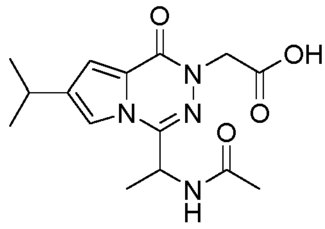 34A |
| **55E** | 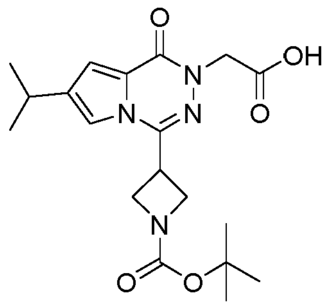 35A |

Synthesis of 2-[4-isopropyl-7-(methoxymethyl)-1-oxo-pyrrolo[1,2-d][1,2,4]triazin-2-yl]acetic acid **21A**

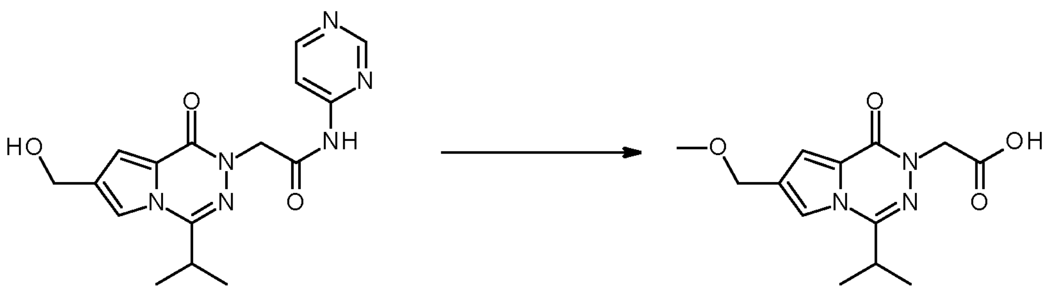

To a stirred solution of 2-[7-(hydroxymethyl)-4-isopropyl-1-oxo-pyrrolo[1,2-d][1,2,4]triazin-2-yl]-N-pyrimidin-4-yl-acetamide **38F** (93 mg, 0.27 mmol) in DCM (2mL) was added thionyl chloride (72 μL, 099 mmol). The reaction mixture was stirred at RT for 30 minutes after which a solution of sodium methoxide (1mL, 5.4M, 5.4mmol) was added. The resulting solution was stirred at rt for 16 h. The solvent was evaporated in vacuo. The mixture was carefully acidified with 1N HCl (pH=2) and extracted with DCM/iPrOH (9/1). The organic layer was separated, dried (MgSO4), filtered and the solvents evaporated in vacuo to yield 2-[4-isopropyl-7-(methoxymethyl)-1-oxo-pyrrolo[1,2-d][1,2,4]triazin-2-yl]acetic acid **21A** (75mg, 0.269, 98% yield) as a yellow oil that was used without further purification.

Synthesis of (1-(tert-butoxycarbonyl)piperidin-4-yl)zinc(II) **2D**

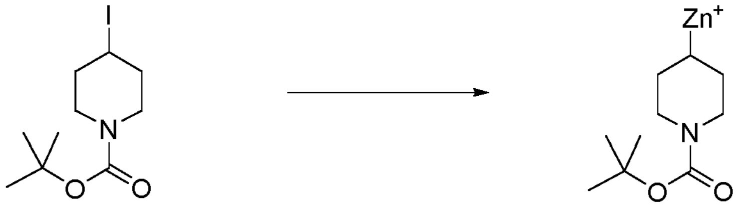

A solution of tert-butyl 4-iodopiperidine-1-carboxylate [301673-14-3] (1000 mg, 3.21 mmol) in THF (6.4 mL) was pumped using syringe pump through a column containing Zn at 0.5 ml/min and at 40°C. The final solution **2D** was collected and used in without further purification. (8.5 ml; 0.31 M).

Synthesis of (1-(tert-butoxycarbonyl)azetidin-3-yl)zinc(II) **3D**

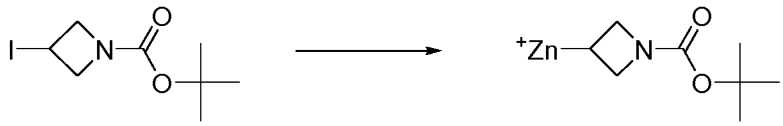

A solution of tert-butyl 3-iodoazetidine-1-carboxylate [254454-54-1] (1902.49 mg, 6.72 mmol) in LiCl 0.5 M in THF [7447-41-8] (19.02 mL, 0.5 M, 9.51 mmol) was pumped through a column containing activated Zn (29.32 g, 448.44 mmol) at 0.5 ml/min at 40°C. The solution was collected under nitrogen atmosphere and this solution **3D** was used in the next step without further purification.

Synthesis of tert-butyl (R)-(1-cyclobutylpiperidin-3-yl)carbamate **4D**

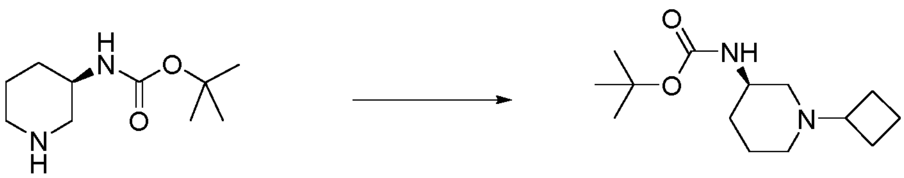

Sodium triacetoxyborohydride [56553-60-7] (19.84 g, 93.62 mmol) was added portionwise to a mixture of (R)-3-(boc-amino)piperidine [309956-78-3] (12.5 g, 62.41 mmol) and cyclobutanone [1191-95-3] (5.25 g, 74.89 mmol). The mixture was stirred at rt for 4h. Water and NaHCO3 were added and the mixture mixture was extracted with DCM. The combined organic extracts were dried over MgSO4, filtered and concetrated in vacuum. The crude was purified by flash column chromatography (silica; MeOH-NH3 in DCM, 0 to 4 %). The desired fractions were collected and concentrated in vacuo to yield tert-butyl (R)-(1-cyclobutylpiperidin-3-yl)carbamate **4D** (12.9 g, yield 81%) as a white solid.

Synthesis of (R)-1-cyclobutylpiperidin-3-amine **5D**

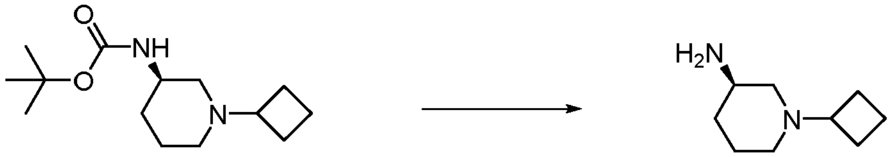

A mixture of tert-butyl (R)-(1-cyclobutylpiperidin-3-yl)carbamate **4D** (12.9 g, 50.71 mmol), HCl/dioxane, 4 N (154.8 mL, 4 M, 619.2 mmol) and 1,4-dioxane (154.8 mL) was stirred at rt overnight. The solvent was evaporated and the residue stirred in sat K2CO3 solution and DCM. The Ol was separated, dried on MgSO4 and evaporated, yielding

(R)-1-cyclobutylpiperidin-3-amine **5D** (6.7 g, yield 86%) as a orange oil.

Synthesis of 2-(7-bromo-4-isopropyl-1-oxo-pyrrolo[1,2-d][1,2,4]triazin-2-yl)-N-pyrimidin-4-yl-acetamide **1F**

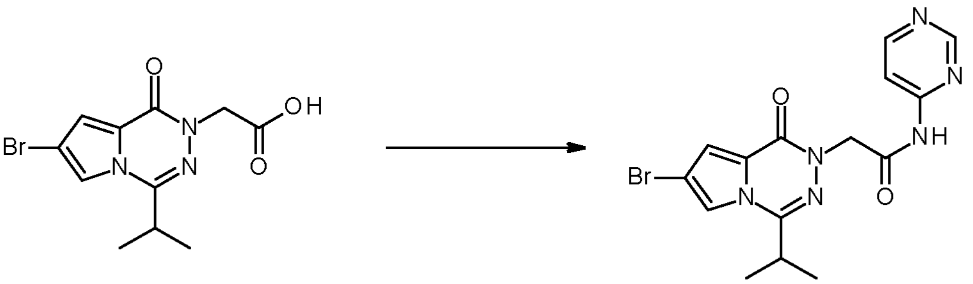

To a solution of 2-(7-bromo-4-isopropyl-1-oxo-pyrrolo[1,2-d][1,2,4]triazin-2-yl)acetic acid **1A** (1.6 g, 5.12 mmol) in DCM (33 mL) and triethylamine (2.85 mL, 20.5 mmol) was added 1-propanephosponic anhydride (7.67 mL, 12.8 mmol) followed by 4-aminopyrimidine (633 mg, 6.6 mmol). The resulting reaction mixture was stirred at room temperature for 3 hours, after which the reaction mixture was quenched by addition of ammonium chloride aqueous solution. The layers were separated and the aqueous was extracted with DCM, combined organic fractions were dried over $MgSO_4$ and concentrated in vacuo. The residue was purified by column chromatography eluting with gradient elution of heptane: EtOAc (from 85:15 to 0:100) to afford 2-(7-bromo-4-isopropyl-1-oxo-pyrrolo[1,2-d][1,2,4]triazin-2-yl)-N-pyrimidin-4-yl-acetamide **1F** (2.05 g, 4.35 mmol, 85%) as a white solid.

Additional analogs were synthesized according to the above procedure substituting the reagents as appropriate (where reagent represents the amine, and intermediate XX represents **1A** or analogous intermediate).

| Reagent | Intermediate XX | Final compound |
|---|---|---|
| 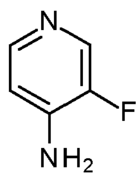 | **1A** | 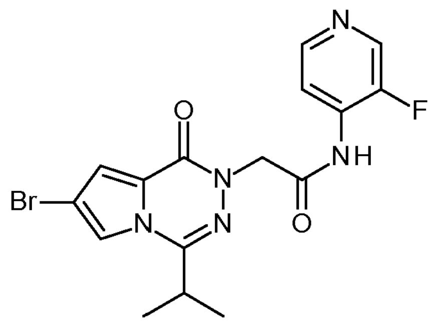 **2F** |
| 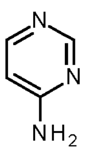 | **21A** | 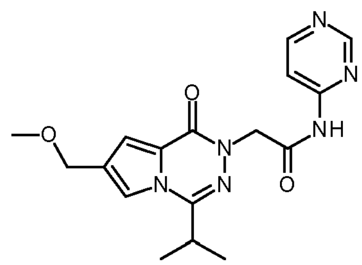 **3F** |

(continued)

| Reagent | Intermediate XX | Final compound |
|---|---|---|
| 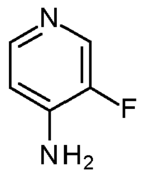 | **5A** | 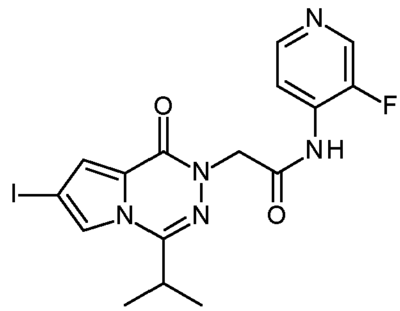 **4F** |
| 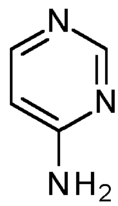 | **5A** | 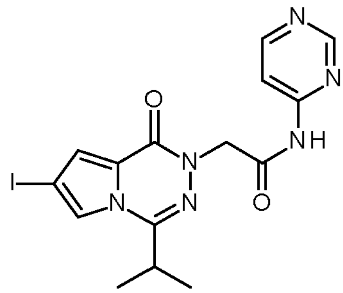 **5F** |
| 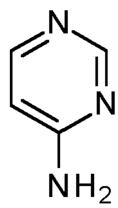 | **4A** | 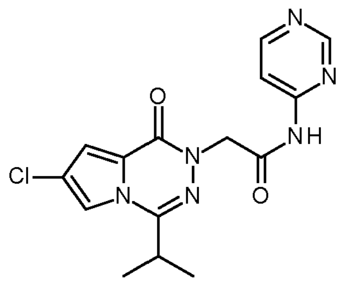 **6F** |
| 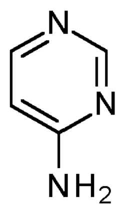 | **3A** | 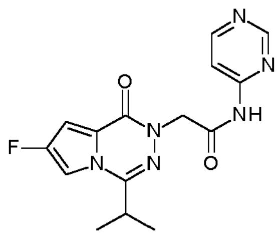 **7F** |
| 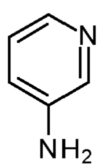 | **1A** | 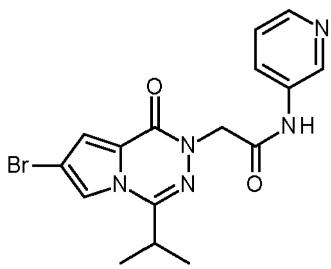 **8F** |

(continued)

| Reagent | Intermediate XX | Final compound |
|---|---|---|
| 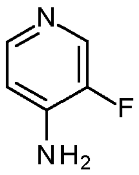 | **4A** | 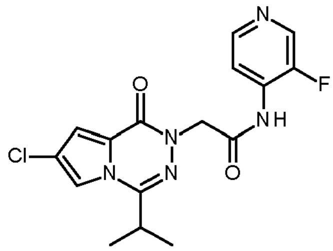 **9F** |
| 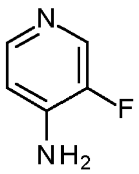 | **1A** | 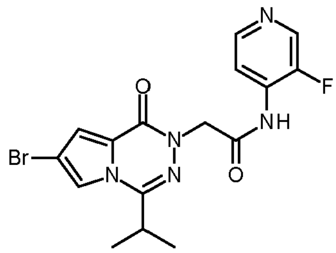 **10F** |
| 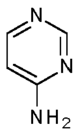 | **2A** | 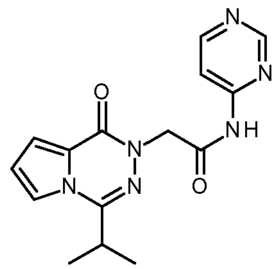 **11F** |
| 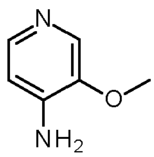 | **6A** | 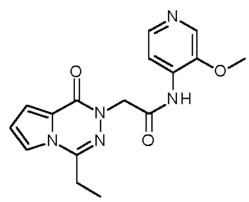 **13F** |
| 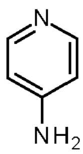 | **6A** | 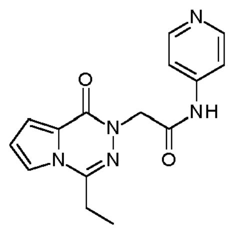 **14F** |
| 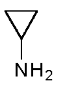 | **6A** | 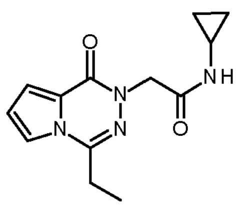 **15F** |

(continued)

| Reagent | Intermediate XX | Final compound |
|---|---|---|
| 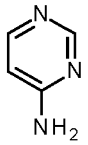 | **9A** | 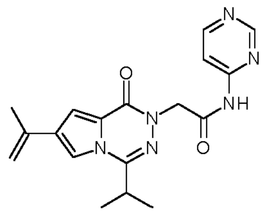 16F |
| 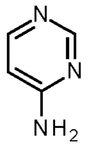 | **10A** | 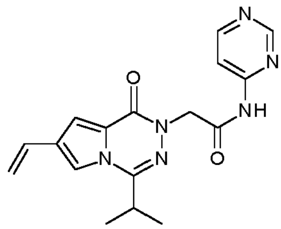 17F |
| 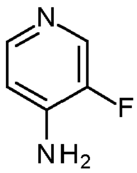 | **11A** | 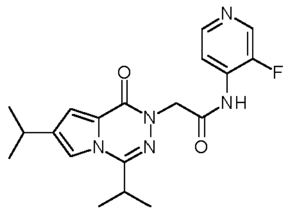 18F |
| 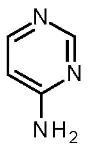 | **11A** | 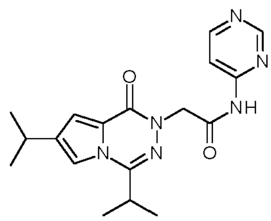 19F |
| 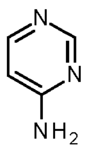 | **12A** | 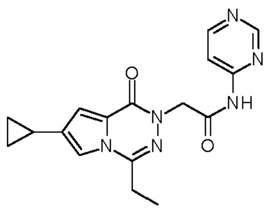 20F |
| 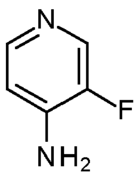 | **12A** | 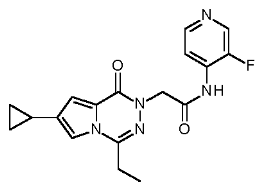 21F |

(continued)

| Reagent | Intermediate XX | Final compound |
|---|---|---|
| 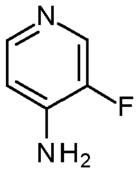 | **19A** | 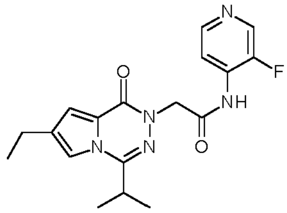 22F |
| 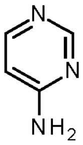 | **19A** | 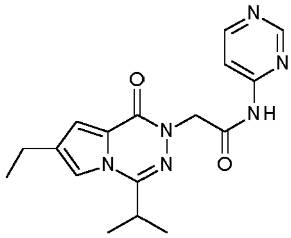 23F |
| 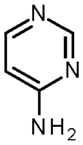 | **8A** | 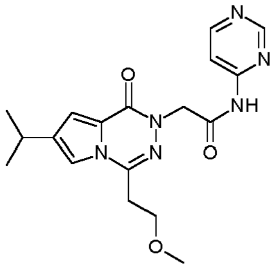 24F |
| 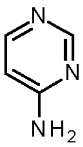 | **13A** | 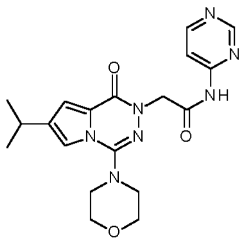 25F |
| 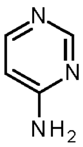 | **14A** | 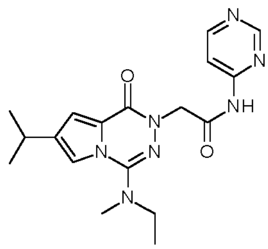 26F |
| 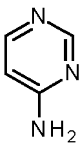 | **15A** | 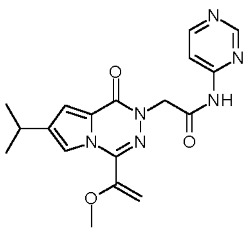 27F |

(continued)

| Reagent | Intermediate XX | Final compound |
|---|---|---|
| 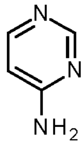 | **17A** | 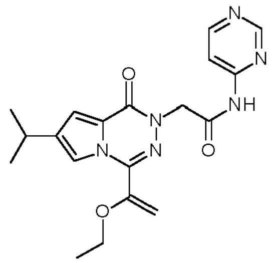 **28F** |
| 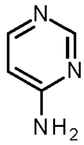 | **18A** | 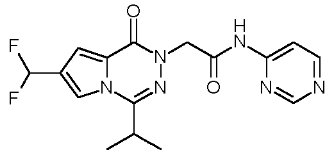 **29F** |
| 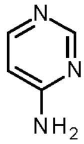 | **20A** | 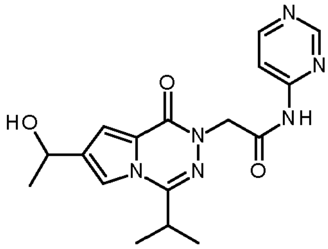 **38F** |
| 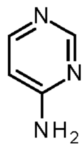 | **22A** | 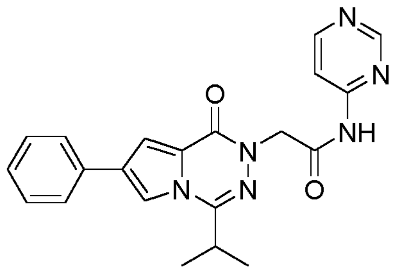 **47F** |
| 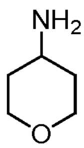 | **22A** | 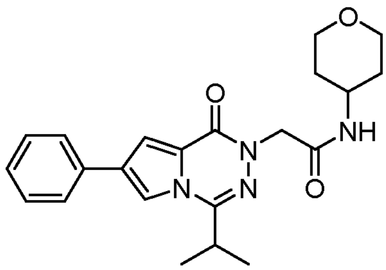 **48F** |

(continued)

| Reagent | Intermediate XX | Final compound |
|---|---|---|
| 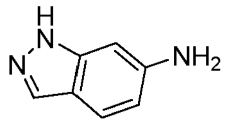 | **22A** | 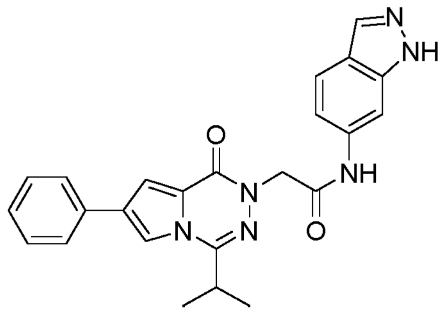 **49F** |
| 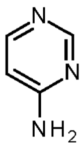 | **7A** | 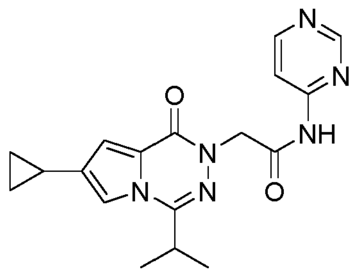 **50F** |
| 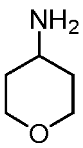 | **11A** | 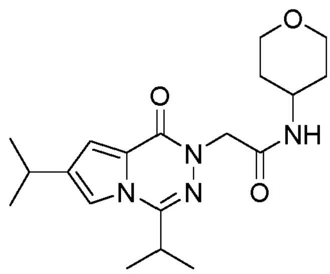 **51F** |
| 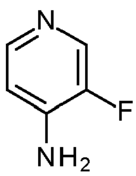 | **3A** | 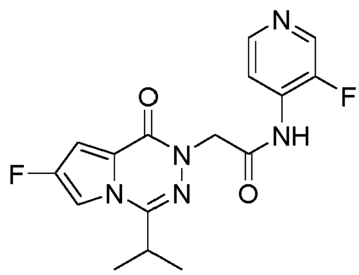 **52F** |
| 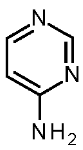 | **23A** | 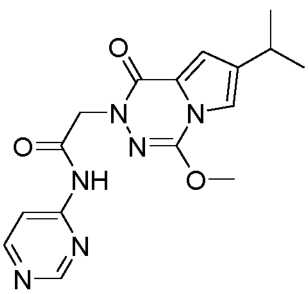 **53F** |

(continued)

| Reagent | Intermediate XX | Final compound |
|---|---|---|
| 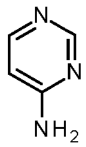 | **23A** | 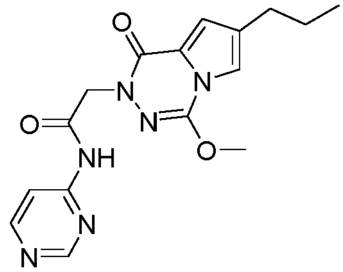 **54F** |
| 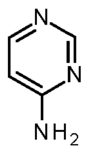 | **26A** | 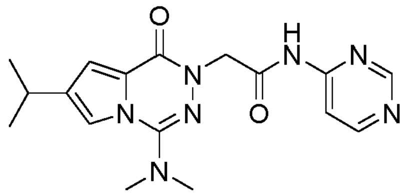 **55F** |
| 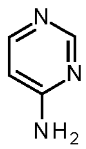 | **27A** | 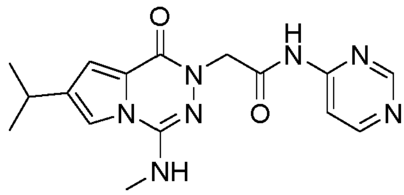 **56F** |
| 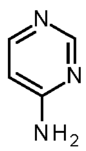 | **28A** | 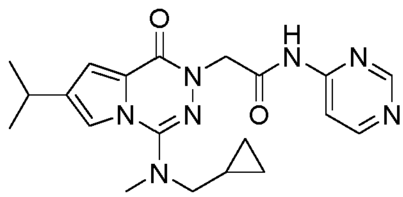 **57F** |
| 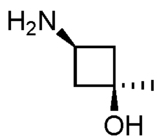 | **25A** | 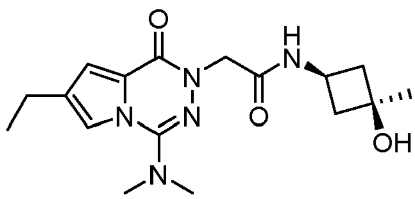 **58F** |
| 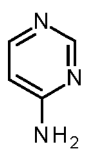 | **29A** | 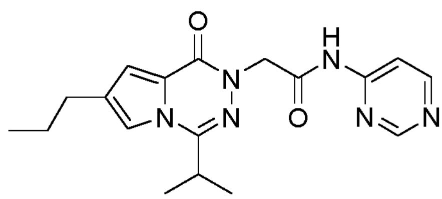 **59F** |
| 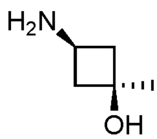 | **30A** | 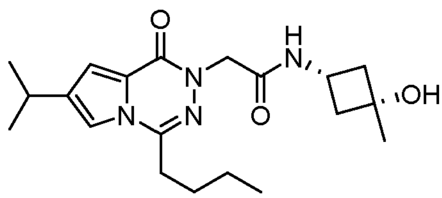 **60F** |

(continued)

| Reagent | Intermediate XX | Final compound |
|---|---|---|
| 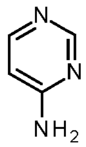 | **30A** | 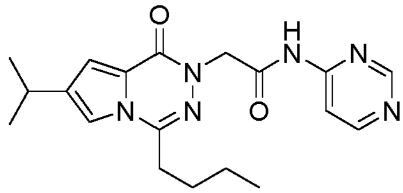 **61F** |
| 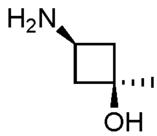 | **31A** | 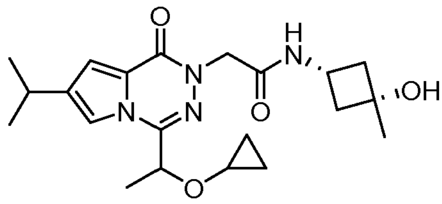 **62F** |
| 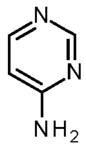 | **32A** | 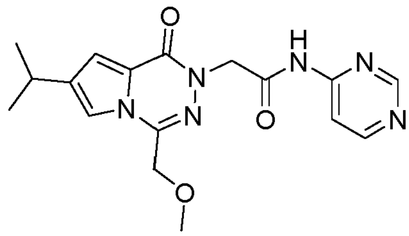 **63F** |
| 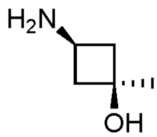 | **33A** | 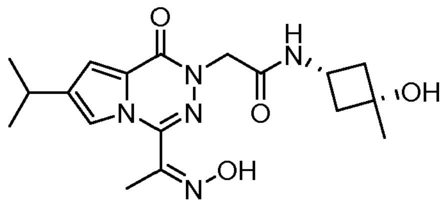 **64F** |
| 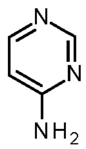 | **34A** | 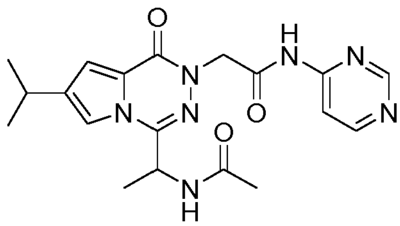 **65F** |
| 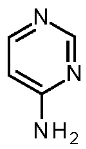 | **35A** | 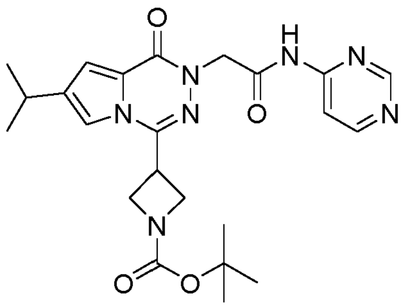 **56E** |

(continued)

| Reagent | Intermediate XX | Final compound |
|---|---|---|
| 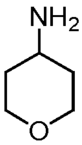 | **16A** | 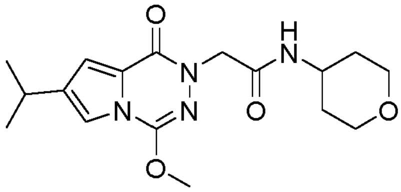 66F |
| 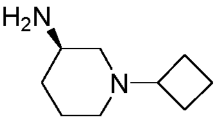 5D | **11A** | 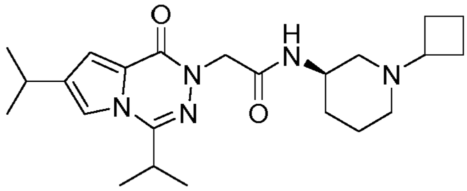 67F |
| 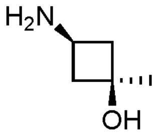 | **11A** | 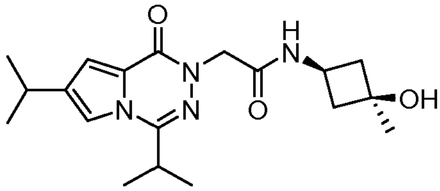 68F |
| 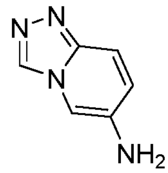 | **11A** | 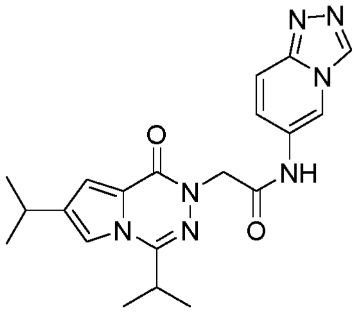 69F |

Synthesis of N-((4-isopropyl-1-oxo-2-(2-oxo-2-(pyrimidin-4-ylamino)ethyl)-1,2-dihydropyrrolo[1,2-d][1,2,4]triazin-7-yl)methyl)-N-methylacetamide **70F**

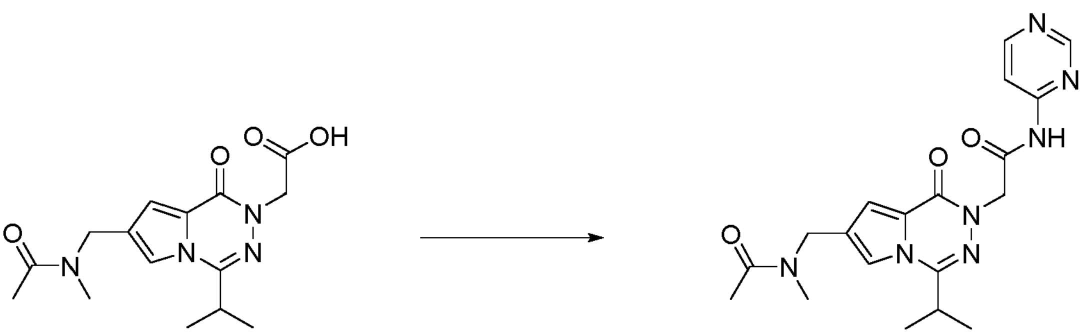

Pyrimidin-4-amine [591-54-8] (32 mg, 0.34 mmol), followed by HATU [148893-10-1] (120 mg, 0.32 mmol) and Et3N [121-44-8] (86 μL, 0.62 mmol) were added to a stirred solution of 2-(4-isopropyl-7-((N-methylacetamido)methyl)-1-oxopyrrolo[1,2-d][1,2,4]triazin-2(1H)-yl)acetic acid **24A** (65 mg, 0.2 mmol) in DMF (1 mL) in a tube and under N2. The mixture was stirred at rt for 3 h. The mixture was treated with 10% Na2CO3 and extracted with EtOAc. The organic layer

was separated, washed with 20% NH4Cl and brine, dried (MgSO4), filtered and the solvents evaporated in vacuo. The crude product was purified by RP flash chromatography (C18, ACN in NH4HCO3 0.25% solution in Water 10/90 to 100/0) and by RP HPLC (Stationary phase: C18 XBridge 30 x 100 mm 5 μm), Mobile phase: Gradient from 85% NH4HCO3 0.25% solution in Water, 15% CH3CN to 55% NH4HCO3 0.25% solution in Water, 45% CH3CN). The desired fractions were collected and extracted with DCM. The organic layer was separated, dried (MgSO4), filtered and the solvents evaporated in vacuo to yield N-((4-isopropyl-1-oxo-2-(2-oxo-2-(pyrimidin-4-ylamino)ethyl)-1,2-dihydropyrrolo[1,2-d][1,2,4]triazin-7-yl)methyl)-N-methylacetamide **70F** (18 mg, yield 22%) as a white solid.

Additional analogs were synthesized according to the above procedure substituting the reagents as appropriate (where reagent represents the amine, and intermediate XX represents **5A** or analogous intermediate).

| Reagent | Intermediate XX | Final compound |
|---|---|---|
| 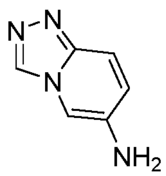 | 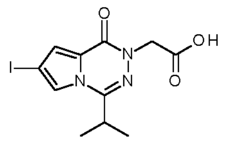 5A | 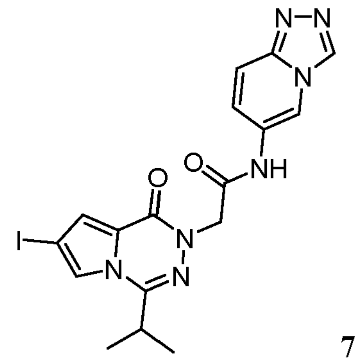 71F |
| 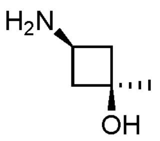 | 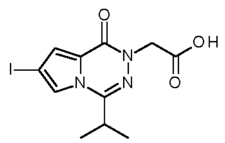 5A | 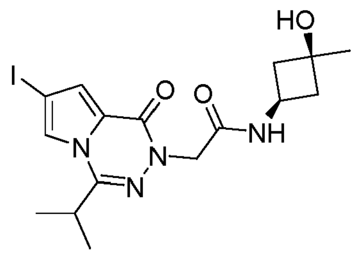 72F |

Synthesis of 2-(4-(dimethylamino)-7-ethyl-1-oxopyrrolo[1,2-d][1,2,4]triazin-2(1H)-yl)-N-(pyrimidin-4-yl)acetamide **73F**

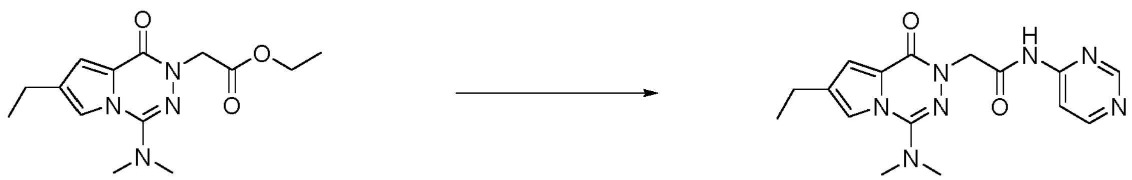

Isopropyl magnesium chloride (2M in THF) [1068-55-9] (0.22 mL, 0.44 mmol) was added dropwise to a stirred solution of ethyl 2-(4-(dimethylamino)-7-ethyl-1-oxopyrrolo[1,2-d][1,2,4]triazin-2(1H)-yl)acetate **40E** (54 mg, 0.18 mmol) and 4-aminopyrimidine [591-54-8] (23 mg, 0.24 mmol) in THF anhydrous (4 mL) under nitrogen at 0°C. The mixture was stirred at rt for 18h. The mixture was diluted with water at 0°C and extracted with AcOEt. The organic layer was separated, dried (MgSO4), filtered and the solvents evaporated in vacuo. The crude product was purified by flash column chromatography (silica, 12g; DCM:MeOH (9:1) in DCM 0/100 to 20/80). The desired fractions were collected and concentrated in vacuo to yield of 2-(4-(dimethylamino)-7-ethyl-1-oxopyrrolo[1,2-d][1,2,4]triazin-2(1H)-yl)-N-(pyrimidin-4-yl)acetamide **73F** (46 mg, 69% yield) as a white solid.

Additional analogs were synthesized according to the above procedure substituting the reagents as appropriate.

| Reagent | Final compound |
|---|---|
| 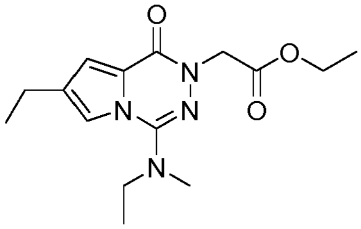 41E | 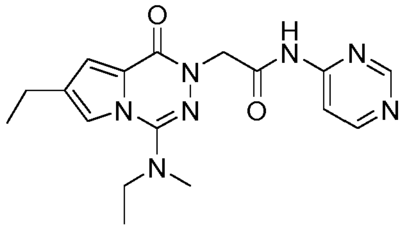 74F |

Synthesis of 2-(7-cyclobutyl-4-isopropyl-1-oxo-pyrrolo[1,2-d][1,2,4]triazin-2-yl)-N-pyrimidin-4-yl-acetamide **30F.**

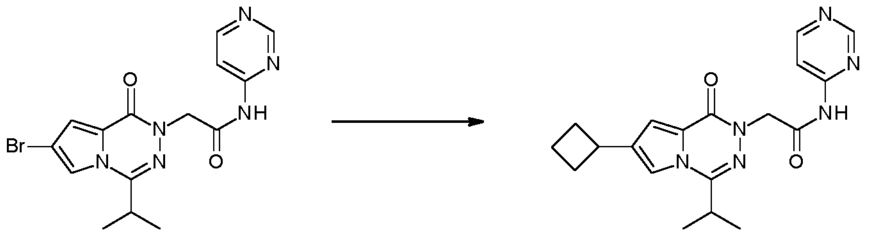

To a solution of 2-(7-bromo-4-isopropyl-1-oxo-pyrrolo[1,2-d][1,2,4]triazin-2-yl)-N-pyrimidin-4-yl-acetamide **1F** (150 mg, 0.383 mmol) in THF (3.9 mL) was added cyclobutyl-zinc bromide (2.9 mL, 1.15 mmol, 0.39 M) and bis[tris(tert-butyl)phospine] palladium (19.5 mg, 0.038 mmol). The reaction mixture was heated under microwave irradiation at 80°C for 15 min. The reaction mixture was quenched by the addition of a few drops of water. The crude reaction mixture was filtered through a pad of celite and rinsed with DCM and MeOH, then concentrated in vacuo. The residue was purified RP chromatography (Stationary phase: YMC 40 g, 25 μm, Mobile Phase: ACN in NH4HCO3 0.25% solution in Water 10/90 to 80/20). The desired fractions were collected and the solvent evaporated in vacuo to yield 2-(7-cyclobutyl-4-isopropyl-1-oxo-pyrrolo[1,2-d][1,2,4]triazin-2-yl)-N-pyrimidin-4-yl-acetamide **30F** as a white solid. (65mg, 46% yield, 0.177 mmol).

Additional analogs were synthesized according to the above procedure substituting the reagents as appropriate.

| Reagent | Intermediate | Final compound |
|---|---|---|
| 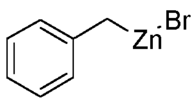 | **1F** | 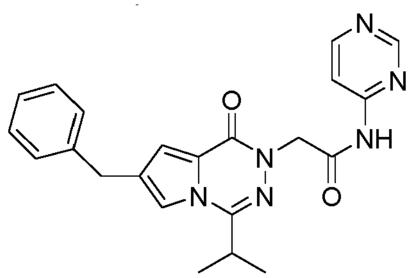 75F |
| 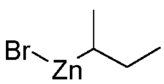 | **1E** | 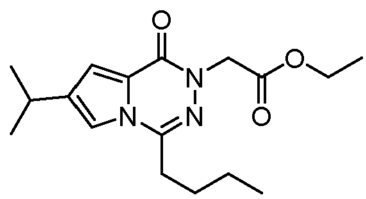 54E |

(continued)

| Reagent | Intermediate | Final compound |
|---|---|---|
| 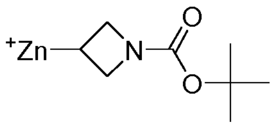 3D | 17E | 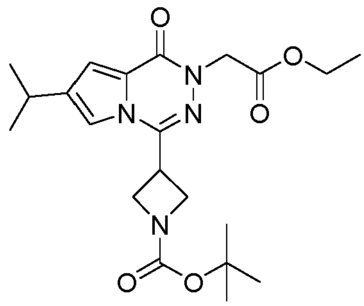 55E |

Synthesis of 4-isopropyl-N-methyl-1-oxo-2-(2-oxo-2-(pyrimidin-4-ylamino)ethyl)-1,2-dihydropyrrolo[1,2-d][1,2,4]triazine-7-carboxamide **76F**

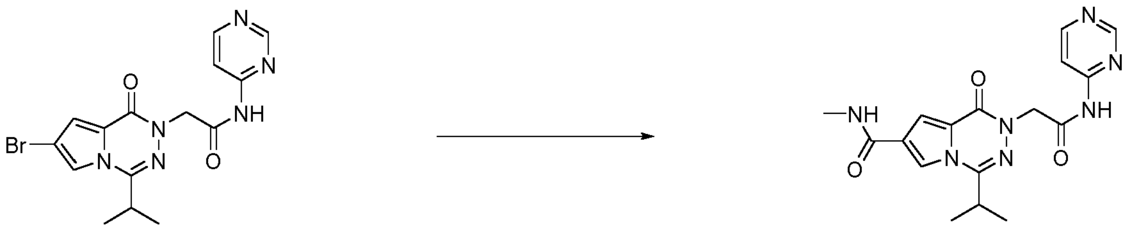

In a microwave vial charged with 2-(7-bromo-4-isopropyl-1-oxo-pyrrolo[1,2-d][1,2,4]triazin-2-yl)-N-pyrimidin-4-yl-acetamide **1F** (200 mg, 0.49 mmol), trans-bis(acetato)bis[o-(di-o-tolylphosphino)benzyl]dipalladium(II) [172418-32-5] (46 mg, 0.049 mmol), tri-tert-butylphosphonium tetrafluoroborate [131274-22-1] (14 mg, 0.049 mmol), and hexacarbonylmolybdenum(0) [13939-06-5] (256 mg, 0.97 mmol), THF (5 mL) was added. Then, 1,8-diazabicyclo[5.4.0]undec-7-ene [6674-22-2] (222 mg, 1.46 mmol) and methylamine [74-89-5] (0.49 mL, 0.97 mmol) were added. The mixture was stirred at 150°C for 20 minutes under microwave irradiation. The crude was filtered over a pad of celite, and the volatiles were removed under vacuo. The residue was purified by RP Flash (Stationary phase: YMC 40 g, 25 μm, Mobile Phase: ACN in NH4HCO3 0.25% solution in Water 0/100 to 50/50), and then by RP HPLC (Stationary phase: C18 XBridge 30 × 100 mm 5 μm), Mobile phase: Gradient from 98% NH4HCO3 0.25% solution in Water, 2% CH3CN to 70% NH4HCO3 0.25% solution in Water, 30% CH3CN). The desired fractions were collected and concentrated to yield 4-isopropyl-N-methyl-1-oxo-2-(2-oxo-2-(pyrimidin-4-ylamino)ethyl)-1,2-dihydropyrrolo[1,2-d][1,2,4]triazine-7-carboxamide **76F** (15 mg, 8% yield) as a white solid.

Additional analogs were synthesized according to the above procedure substituting the reagents as appropriate.

| Reagent | Final compound |
|---|---|
| 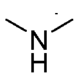 | 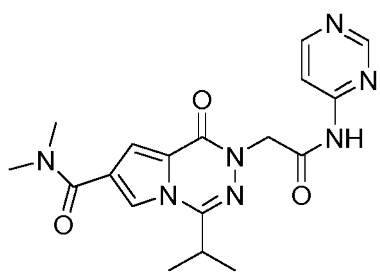 77F |

Synthesis of 2-[7-isopropyl-4-(1-methoxyethyl)-1-oxo-pyrrolo[1,2-d][1,2,4]triazin-2-yl]-N-pyrimidin-4-yl-acetamide, **31F**

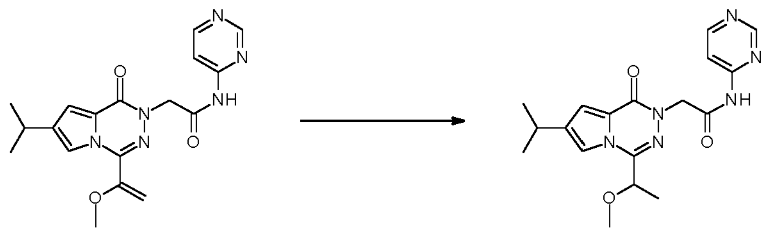

To a solution of 2-[7-isopropyl-4-(1-methoxyvinyl)-1-oxo-pyrrolo[1,2-d][1,2,4]triazin-2-yl]-N-pyrimidin-4-yl-acetamide **27F** (130 mg, 0.35 mmol) in MeOH ([67-56-1] (89.97 mL, 0.79 g/mL, 2218.14 mmol) was added Pd/C (10%), the resulting solution was stirred under and atmosphere of hydrogen for 2 hours. The reaction mixture was filtered through celite and concentrated in vacuo. The residue was purified on RP HPLC chromatography, (eluent: NH4HCO3/ **ACN**). The pure fractions were evaporated yielding 2-[7-isopropyl-4-(1-methoxyethyl)-1-oxo-pyrrolo[1,2-d][1,2,4]triazin-2-yl]-N-pyrimidin-4-yl-acetamide **31F** (90 mg, yield 68.85%, 0.24 mmol) as a white solid.

Additional analogs were synthesized according to the above procedure substituting the reagents as appropriate.

| | |
|---|---|
| 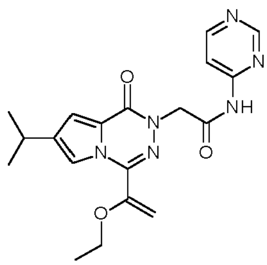 28F | 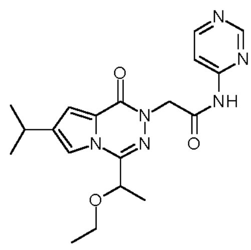 40F |

Synthesis of N-(3-fluoro-4-pyridyl)-2-[4-isopropyl-1-oxo-7-(trifluoromethyl)pyrrolo[1,2-d][1,2,4]triazin-2-yl]acetamide **32F**

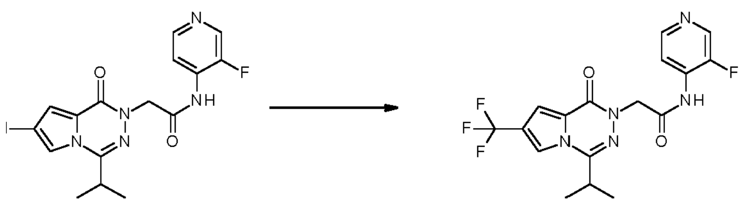

To a solution of N-(3-fluoro-4-pyridyl)-2-(7-iodo-4-isopropyl-1-oxo-pyrrolo[1,2-d][1,2,4]triazin-2-yl)acetamide **4F** (300 mg, 0.736 mmol) in DMF (5 mL) was added copper (I) iodine [7681-65-4] (700.989 mg, 3.681 mmol) and methyl-2,2-difluoro-2-(fluorosulfonyl)acetate [680-15-9] (468.602 μL, 1.509 g/mL, 3.681 mmol) the resulting reaction mixture was stirred vigorously and heated at 100 °C for 18h. The crude mixture was partitioned between 25% $NH_3$ aqueous solution (80 mL) and EtOAc (50 mL). The layers were separated, the aqueous layer re-extracted with EtOAc (2 × 30 mL). The combined organic layers were dried over sodium sulfate, filtered and concentrated in vacuo. The resulting yellow oil was purified by column chromatography eluting with gradient elution of Hept/EtOAc from 93:7 to 7:3. To give the N-(3-fluoro-4-pyridyl)-2-[4-isopropyl-1-oxo-7-(trifluoromethyl)pyrrolo[1,2-d][1,2,4]triazin-2-yl]acetamide **32F** as a colorless oil (188 mg, 77%, 2.84 mmol).

Synthesis of 2-[4-isopropyl-1-oxo-7-(3,3,3-trifluoropropyl)pyrrolo[1,2-d][1,2,4]triazin-2-yl]-N-pyrimidin-4-yl-acetamide **33F**

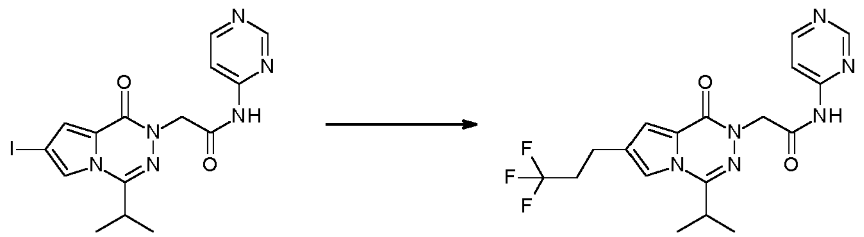

A solution of 1,1,1-trifluoro-3-iodopropane (205 mg, 0.92 mmol) in THF (2.7 mL) and a solution of 2-(7-iodo-4-isopropyl-1-oxo-pyrrolo[1,2-d][1,2,4]triazin-2-yl)-N-pyrimidin-4-yl-acetamide **5F** (20 mg, 0.0468 mmol), 1,1'-bis(diphenylphosphino)-ferrocine-palladium(II)dichloride dichloromethane complex (3.78 mg, 0.0047 mmol) in DMF (1mL) were pumped at 0.25 mL/min into a vaportec (reactor1: 40°C and reactor 2: 80°C residence time was 20 min. After which the reaction mixture was concentrated in vacuo and the residue was dissolved in DCM/NH4Cl (20% aqueous solution) (1:1) and filtered through HM-N cartridge after which the filtrate was evaporated in vacuo. The residue was purified by reverse phase HPLC (stationary phase C18 Xbridge 190 × 100 5 μm), Mobile phase: Gradient from 80% NH4HCO3 0.25% solution in water, 20% CH3CN to 0% NH4HCO3 0.25% water in CH3CN) to afford 2-[4-isopropyl-1-oxo-7-(3,3,3-trifluoropropyl)pyrrolo[1,2-d][1,2,4]triazin-2-yl]-N-pyrimidin-4-yl-acetamide **33F** (0.9 mg, 0.0022 mmol).

Synthesis of 2-(7-(3-fluoropropyl)-4-isopropyl-1-oxopyrrolo[1,2-d][1,2,4]triazin-2(1H)-yl)-N-(pyrimidin-4-yl)acetamide **78F**

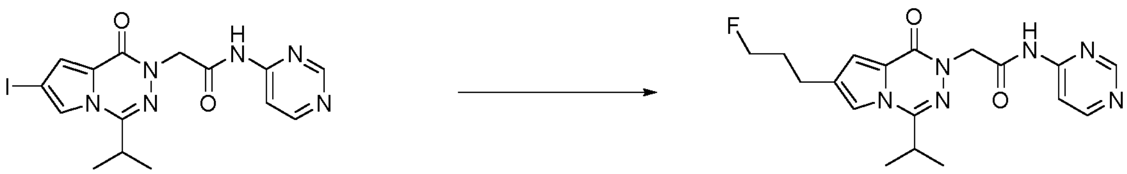

In the automatize Vapourtec were placed two solutions: 1-fluoro-3-iodopropane [462-40-8] (0.94 mmol) in THF (2 mL) and 2-(7-iodo-4-isopropyl-1-oxo-pyrrolo[1,2-d][1,2,4]triazin-2-yl)-N-pyrimidin-4-yl-acetamide **5F** (20.5 mg, 0.047 mmol) and 1,1'-bis(diphenylphosphino)ferrocene-palladium(II)dichloride dichloromethane complex (4 mg, 0.0047 mmol) in DMF (1mL) and the solution were pumped at 0.25mL (reactor1: 40°C, reactor2: 100°C (Rt:10min) Pressure >5 bar; 5 ml coil not irradiated). Reaction mixture solvent was evaporated and the crude redissolved in DCM/NH4Cl 20%, 2mL (1:1) and filtered using an isolute HM-N cartridge and the solvent evaporated in vacuo. The crude was submitted to HTP and purified by RP HPLC (Stationary phase: C18 XBridge 190 x 100 mm 5 μm), Mobile phase: Gradient from 80% NH4HCO3 0.25% solution in Water, 20% CH3CN to 0% NH4HCO3 0.25% solution in Water, 100% CH3CN), yielding 2-(7-(3-fluoropropyl)-4-isopropyl-1-oxopyrrolo[1,2-d][1,2,4]triazin-2(1H)-yl)-N-(pyrimidin-4-yl)acetamide **78F** (3 mg, 17% yield).

Additional analogs were synthesized according to the above procedure substituting the reagents as appropriate.

| Reagent | Final product |
|---|---|
| 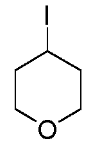 | 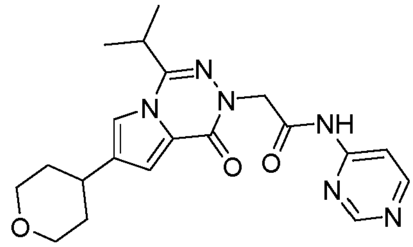 **79F** |

Synthesis of 2-(4-isopropyl-1-oxo-7-((tetrahydro-2H-pyran-3-yl)methyl)pyrrolo[1,2-d][1,2,4]triazin-2(1H)-yl)-N-(pyrimidin-4-yl)acetamide **80F**

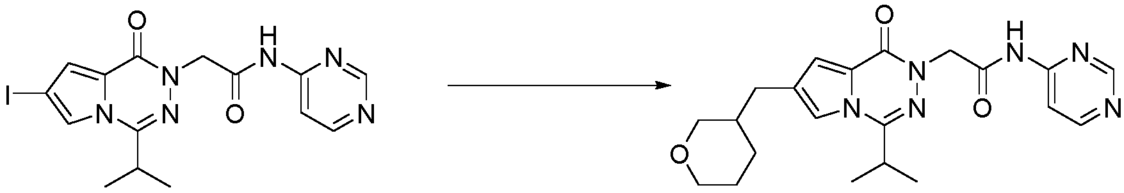

In the automatize Vapourtec were placed two solutions: 3-(iodomethyl)tetrahydro-2H-pyran [32730-60-2] (226 mg, 1 mmol) in THF (2 mL) and 2-(7-iodo-4-isopropyl-1-oxo-pyrrolo[1,2-d][1,2,4]triazin-2-yl)-N-pyrimidin-4-yl-acetamide **5F** (33 mg, 0.075 mmol), bis(dibenzylideneacetone)palladium [32005-36-0] (2 mg, 0.037 mg) and 2-dicyclohexylphosphino-2',6'-bis(n,n-dimethylamino)biphenyl [1160556-64-8] (3 mg, 0.0075 mmol) in DMF (1mL) and the solution were pumped at 0.25mL (reactor1: 40°C, reactor2: 100°C (Rt:10min) Pressure >5 bar; 5 ml coil not irradiated). Reaction mixture solvent was evaporated and the crude redissolved in DCM/NH4Cl 20%, 2mL (1:1) and filtered using an isolute HM-N cartridge and the solvent evaporated in vacuo. The crude was purified by RP HPLC (Stationary phase: C18 XBridge 190 x 100 mm 5 μm), Mobile phase: Gradient from 80% NH4HCO3 0.25% solution in Water, 20% CH3CN to 0% NH4HCO3 0.25% solution in Water, 100% CH3CN), yielding 2-(4-isopropyl-1-oxo-7-((tetrahydro-2H-pyran-3-yl)methyl)pyrrolo[1,2-d][1,2,4]triazin-2(1H)-yl)-N-(pyrimidin-4-yl)acetamide **80F** (1.4 mg, 5% yield).

Additional analogs were synthesized according to the above procedure substituting the reagents as appropriate.

| Reagent | Final product |
|---|---|
| 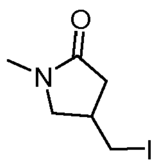 | 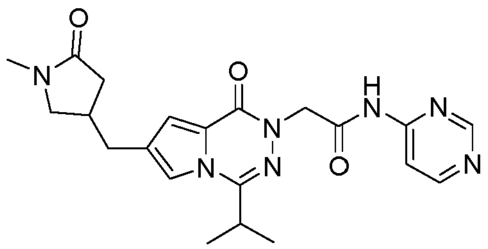 **81F** |

Synthesis of 2-(4-isopropyl-1-oxo-7-(piperidin-4-ylmethyl)pyrrolo[1,2-d][1,2,4]triazin-2(1H)-yl)-N-(pyrimidin-4-yl)acetamide **82F**

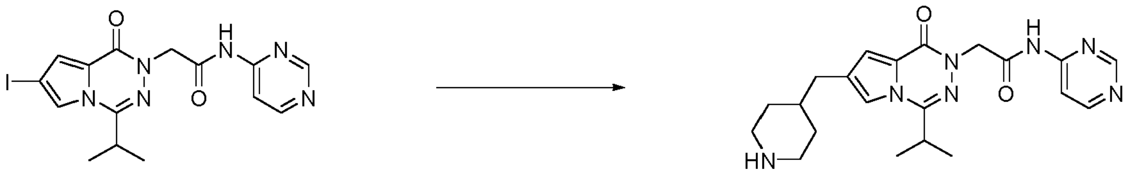

In the automatize Vapourtec were placed two solutions: tert-butyl 4-(iodomethyl)piperidine-1-carboxylate [145508-94-7] (0.94 mmol) in THF (2mL) and 2-(7-iodo-4-isopropyl-1-oxo-pyrrolo[1,2-d][1,2,4]triazin-2-yl)-N-pyrimidin-4-yl-acetamide **5F** (22 mg, 0.05 mmol) and 1,1'-bis(diphenylphosphino)ferrocene-palladium(II)dichloride dichloromethane complex (4 mg, 0.0047 mmol) in DMF (1mL) and the solution were pumped at 0.25mL (reactor1: 40°C, reactor2: 100°C (Rt:10min) Pressure >5 bar; 5 ml coil not irradiated). Reaction mixture solvent was evaporated and the crude redissolved in DCM/NH4Cl 20%, 2mL (1:1) and filtered using an isolute HM-N cartridge and the solvent evaporated in vacuo. The crude was redissolved in 1 mL HCl in dioxane to deprotect the BOC group. The reaction was followed with LCMS and when completed the solvent was evaporated. The crude was purified by RP HPLC (Stationary phase: C18 XBridge 190 × 100 mm 5 μm), Mobile phase: Gradient from 80% NH4HCO3 0.25% solution in Water, 20% CH3CN to 0% NH4HCO3 0.25% solution in Water, 100% CH3CN), yielding 2-(4-isopropyl-1-oxo-7-(piperidin-4-ylmethyl)pyrrolo[1,2-d][1,2,4]triazin-2(1H)-yl)-N-(pyrimidin-4-yl)acetamide **82F** (1.2 mg, 6% yield).

Synthesis of 2-(7-(azetidin-3-yl)-4-isopropyl-1-oxopyrrolo[1,2-d][1,2,4]triazin-2(1H)-yl)-N-(pyrimidin-4-yl)acetamide **83F**

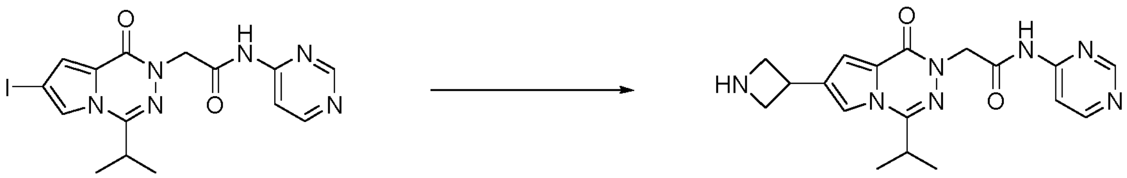

In the automatize Vapourtec were placed two solutions: 1-boc-3-iodoazetidine [254454-54-1] (283 mg, 1mmol) in THF (2 mL) and 2-(7-iodo-4-isopropyl-1-oxo-pyrrolo[1,2-d][1,2,4]triazin-2-yl)-N-pyrimidin-4-yl-acetamide **5F** (33 mg, 0.075 mmol), bis(dibenzylideneacetone)palladium [32005-36-0] (2 mg, 0.0037 mmol) and 2-dicyclohexylphosphino-2',6'-bis(n,n-dimethylamino)biphenyl [1160556-64-8] (3 mg, 0.0075 mmol) in THF (0.5 mL) and DMF (0.5 mL) and the solutions were pumped at 0.125mL (reactor1: 40°C, reactor2: 100°C (Rt:10min) Pressure >5 bar;5 ml coil not irradiated). Reaction mixture solvent was evaporated and the crude redissolved in DCM/NH4Cl 20%, 2mL (1:1) and filtered using an isolute HM-N cartridge and the solvent evaporated in vacuo. The crude was redissolved in DCM and 0.5 ml of TFA was added and the reaction was monitored until full deprotection. The solvent was evaporated and the crude was purified by RP HPLC (Stationary phase: C18 XBridge 190 × 100 mm 5 μm), Mobile phase: Gradient from 80% NH4HCO3 0.25% solution in Water, 20% CH3CN to 0% NH4HCO3 0.25% solution in Water, 100% CH3CN), yielding of 2-(7-(azetidin-3 -yl)-4-isopropyl-1-oxopyrrolo[1,2-d][1,2,4]triazin-2(1H)-yl)-N-(pyrimidin-4-yl)acetamide **83F** (0.9 mg, 3% yield).

Synthesis of 2-(7-isobutyl-4-isopropyl-1-oxo-pyrrolo[1,2-d][1,2,4]triazin-2-yl)-N-pyrimidin-4-yl-acetamide **34F**

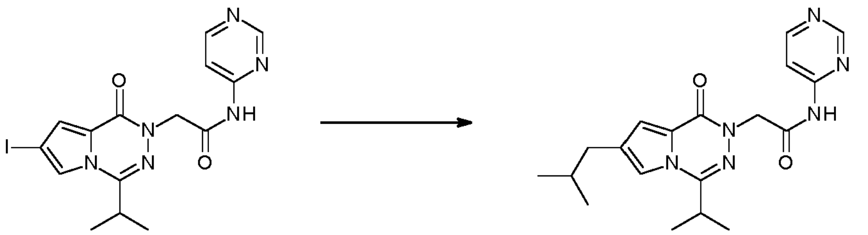

To a solution of 2-(7-iodo-4-isopropyl-1-oxo-pyrrolo[1,2-d][1,2,4]triazin-2-yl)-N-pyrimidin-4-yl-acetamide **5F** (20 mg, 0.0468 mmol) in DMF (1 mL) was added isobutylzinc bromide (0.76 mL, 0.3 M, 0.228 mmol), bis(dibenzylideneacetone) palladium (2.6 mg, 0.0046 mmol) and 2-dicyclohexylphosphino-2,6-bis(N,N-dimethylamino-biphenyl. The reaction mixture was heated at 50°C for 16 hours, after which the reaction mixture was concentrated in vacuo. The residue was dissolved in DCM/NH4Cl (20% aqueous solution) (1:1) and filtered through HM-N cartridge and the solvent was evaporated. The residue was purified by reverse phase HPLC (stationary phase C18 Xbridge 190 x 100 5 μm), Mobile phase: Gradient from 80% NH4HCO3 0.25% solution in water, 20% CH3CN to 0% NH4HCO3 0.25% water in CH3CN) to afford 2-(7-isobutyl-4-isopropyl-1-oxo-pyrrolo[1,2-d][1,2,4]triazin-2-yl)-N-pyrimidin-4-yl-acetamide **34F** (5.2mg, 30% yield, 0.0174 mmol).

Additional analogs were synthesized according to the above procedure substituting the reagents as appropriate.

| Reagent | Final product |
|---|---|
| 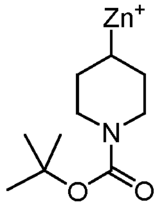 **2D** | 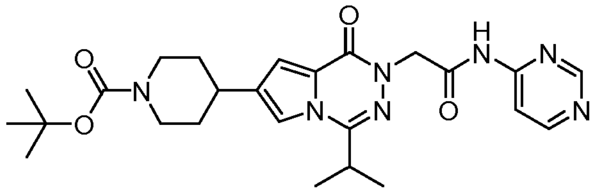 **57E** |

Synthesis of 2-(4-isopropyl-1-oxo-7-(piperidin-4-yl)pyrrolo[1,2-d][1,2,4]triazin-2(1H)-yl)-N-(pyrimidin-4-yl)acetamide **84F**

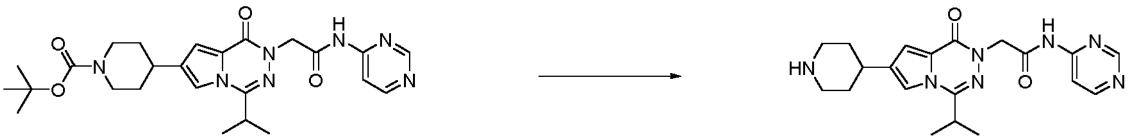

Tert-butyl 4-(4-isopropyl-1-oxo-2-(2-oxo-2-(pyrimidin-4-ylamino)ethyl)-1,2-dihydropyrrolo[1,2-d][1,2,4]triazin-7-yl)piperidine-1-carboxylate **57E** (30 mg, 0.031 mmol) in DCM (1 mL) and trifluoroacetic acid [76-05-1] (0.2 mL) was stirred overnight. The solvent was evaporated and the crude was purified by RP HPLC (Stationary phase: C18 XBridge 30 x 100 mm 5 μm), Mobile phase: Gradient from 70% NH4HCO3 0.25% solution in Water, 30% CH3CN to 35% NH4HCO3 0.25% solution in Water, 65% CH3CN). The desired fraction were collected and concentrated in vacuo to yield 2-(4-isopropyl-1-oxo-7-(piperidin-4-yl)pyrrolo[1,2-d][1,2,4]triazin-2(1H)-yl)-N-(pyrimidin-4-yl)acetamide **84F** (1 mg, 8% yield) as a white solid.

Additional analogs were synthesized according to the above procedure substituting the reagents as appropriate.

| Intermediate | Final product |
|---|---|
| 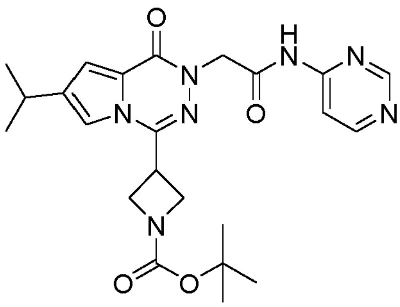 **56E** | 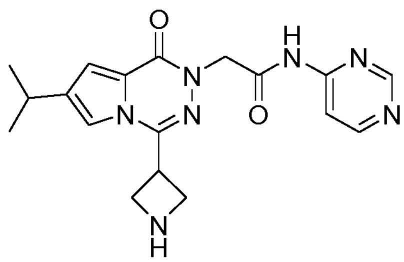 **45F** |

Synthesis of 2-(4-acetyl-7-isopropyl-1 -oxo-pyrrolo[1,2-d][1,2,4]triazin-2-yl)-N-pyrimidin-4-yl-acetamide **35F**

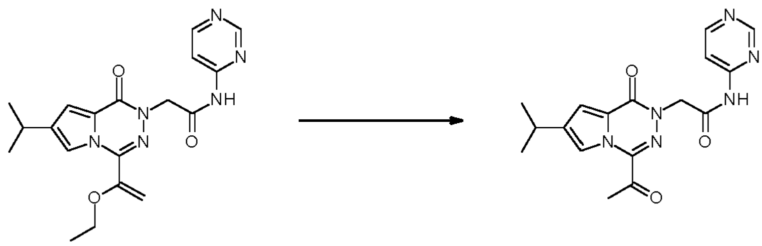

To a solution of 2-[7-isopropyl-4-(1-ethoxyvinyl)-1-oxo-pyrrolo[1,2-d][1,2,4]triazin-2-yl]-N-pyrimidin-4-yl-acetamide **28F** (570 mg, 1.5mmol) in THF (18.3) was added HCl (18.3 mL), the resulting solution was stirred at room temperature for 16 hours. The solids were collected by filtration washed with THF, then dissolved in water, the resulting solution was neutralized by the addition of NaHCO3 aqueous solution and extracted with DCM, the organic fraction was dried over MgSO4 and concentrated in vacuo yielding 2-(4-acetyl-7-isopropyl-1-oxo-pyrrolo[1,2-d] [1,2,4]triazin-2-yl)-N-pyrimidin-4-yl-acetamide **35F** (320 mg, 60% yield, 0.9 mmol) as a white solid.

Additional analogs were synthesized according to the above procedure substituting the reagents as appropriate.

| Intermediate | Intermediate |
|---|---|
| **20E** | 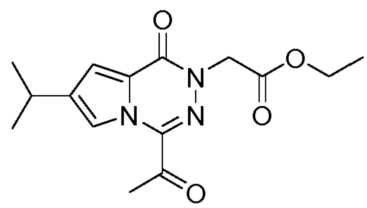 **44E** |

Synthesis of 2-[4-(1-hydroxyethyl)-7-isopropyl-1-oxo-pyrrolo[1,2-d][1,2,4]triazin-2-yl]-N-pyrimidin-4-yl-acetamide **36F**

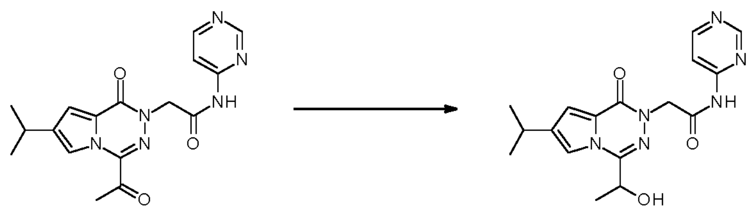

To a solution of 2-(4-acetyl-7-isopropyl-1-oxo-pyrrolo[1,2-d][1,2,4]triazin-2-yl)-N-pyrimidin-4-yl-acetamide **35F** (60 mg, 0.17 mmol) in MeOH (4 mL) was added NaBH4 (7.6 mg, 0.2 mmol), the resulting solution was stirred at room temperature for 1 hour. The reaction mixture was evaporated in vacuo and the residue dissolved in EtOAc and washed using an aqueous solution of NH4Cl. The organic fraction was dried over MgSO4 and concentrated in vacuo yielding 2-[4-(1-hydroxyethyl)-7-isopropyl-1-oxo-pyrrolo[1,2-d] [1,2,4]triazin-2-yl]-N-pyrimidin-4-yl-acetamide **36F** (14 mg, 23%yield, 0.03 mmol) as a white solid.

Synthesis of 2-[4-(1-hydroxy-1-methyl-ethyl)-7-isopropyl-1-oxo-pyrrolo[1,2-d][1,2,4]triazin-2-yl]-N-pyrimidin-4-yl-acetamide **37F**

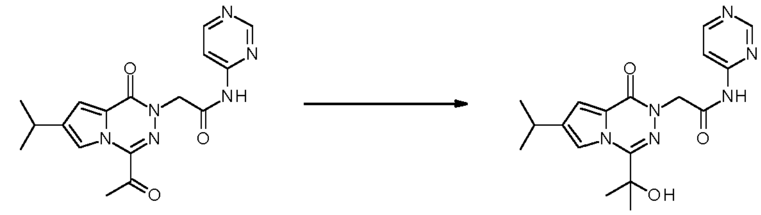

To a solution of 2-(4-acetyl-7-isopropyl-1-oxo-pyrrolo[1,2-d][1,2,4]triazin-2-yl)-N-pyrimidin-4-yl-acetamide **35F** (195 mg, 0.55 mmol) in THF (30 mL) was added MeMgBr (0.4 mL, 1.37 mmol, 3.4M), the resulting solution was stirred at 0°C for 1 hour and 1 hour at rt. The reaction mixture was quenched by the addition of an aqueous solution of NH4CI, and extracted with DCM, the organic fraction was dried over MgSO4 and concentrated in vacuo yielding 2-[4-(1-hydroxy-1-methyl-ethyl)-7-isopropyl-1-oxo-pyrrolo[1,2-d][1,2,4]triazin-2-yl]-N-pyrimidin-4-yl-acetamide **37F** (15 mg, 7%yield, 0.04 mmol) as a white solid.

Additional analogs were synthesized according to the above procedure substituting the reagents as appropriate.

| Intermediate | Final product |
|---|---|
| 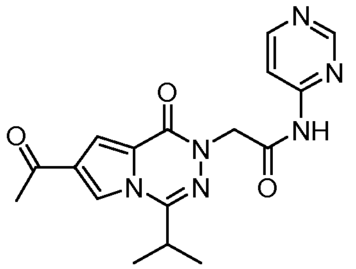 **85F** | 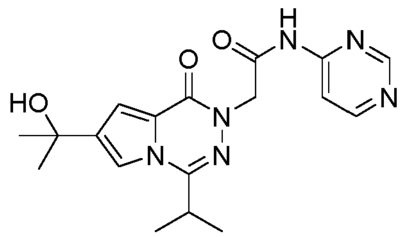 **86F** |

Synthesis of 2-(7-(2-fluoropropan-2-yl)-4-isopropyl-1-oxopyrrolo[1,2-d] [1,2,4]triazin-2(1H)-yl)-N-(pyrimidin-4-yl)acetamide **87F**

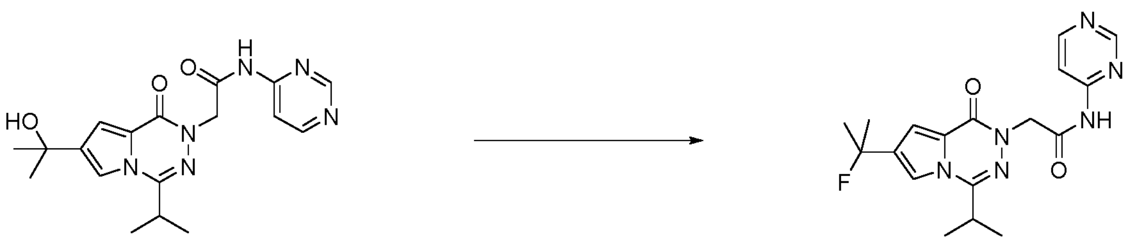

In a dry 2-5mL MW vial under nitrogen, 2-(7-(2-hydroxypropan-2-yl)-4-isopropyl-1-oxopyrrolo[1,2-d][1,2,4]triazin-2(1H)-yl)-N-(pyrimidin-4-yl)acetamide **86F** (34 mg, 0.0854 mmol) was dissolved in dry DCM [75-09-2] (0.847 mL) and the solution cooled to 0°C. Then neat diethylaminosulfur trifluoride [38078-09-0] (51 μL mL, 1.22 g/mL, 0.384 mmol) was added at 0°C. The mixture was stirred at 0 °C for 3h then allowed to warm to r.t. and stirred for further 13h. The mixture was cooled to 0 °C and diethylaminosulfur trifluoride [38078-09-0] (62 μL, 1.22 g/mL, 0.47 mmol) was added and the mixture stirred for further 2h at 0 °C. The mixture was quenched by addition of sat. aq. NaHCO3 (4 mL in total added) and the biphasic mixture extracted with DCM (3 × 5 mL). The volatiles were concentrated in vacuo and the crude residue purified by column chromatography (Silica 10g, 38 mL/min, Hept/EtOAc 65:35 to 0:1 over 30 CV) to give 2-(7-(2-fluoropropan-2-yl)-4-isopropyl-1-oxopyrrolo[1,2-d][1,2,4]triazin-2(1H)-yl)-N-(pyrimidin-4-yl)acetamide **87F** as a colorless solid (8.2 mg, 23%, ca. 90% purity).

Synthesis of 2-[7-(1-fluoroethyl)-4-isopropyl-1-oxo-pyrrolo[1,2-d] [1,2,4]triazin-2-yl]-N-pyrimidin-4-yl-acetamide, **39F**

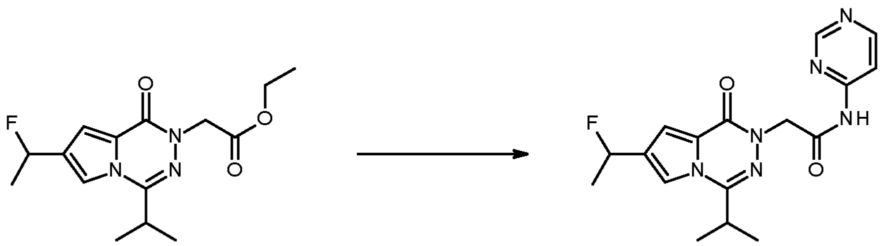

To a solution of ethyl 2-[7-(1-fluoroethyl)-4-isopropyl-1-oxo-pyrrolo[1,2-d][1,2,4]triazin-2-yl]acetate, **28E** (20 mg, 0.06mmol) in toluene (1.5 mL), was 4-amino-pyridimine (7.379 mg, 0.0776 mmol) and LiHMDS (1.0M in THF) [4039-32-1] (0.129 mL, 1 M, 0.129 mmol) the resulting mixture stirred for 2h at r.t. After 2h, the reaction was quenched by addition of NH4Claq. sat. (5 mL) and extracted with DCM (3 × 5 mL). The combined organic extracts were dried over Na2SO4 and concentrated. The obtained residue was purified by column chromatography eluting with Hept/EtOAc (65:35 to 0:1) to give a colorless solid 2-[7-(1-fluoroethyl)-4-isopropyl-1-oxo-pyrrolo[1,2-d][1,2,4]triazin-2-yl]-N-pyrimidin-4-yl-acetamide **39F** (13.8 mg, 60%).

Additional analogs were synthesized according to the above procedure substituting the reagents as appropriate.

| Intermediate | Final product |
|---|---|
| 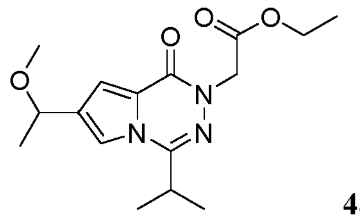 **48E** | 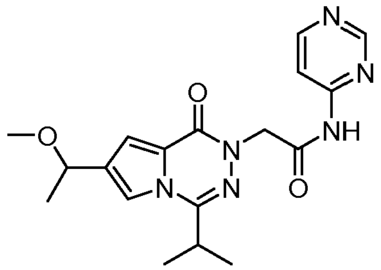 **88F** |

Synthesis of 2-(4-isopropyl-7-(1-methyl-1H-pyrazol-4-yl)-1-oxopyrrolo[1,2-d][1,2,4]triazin-2(1H)-yl)-N-(pyrimidin-4-yl)acetamide **89F**

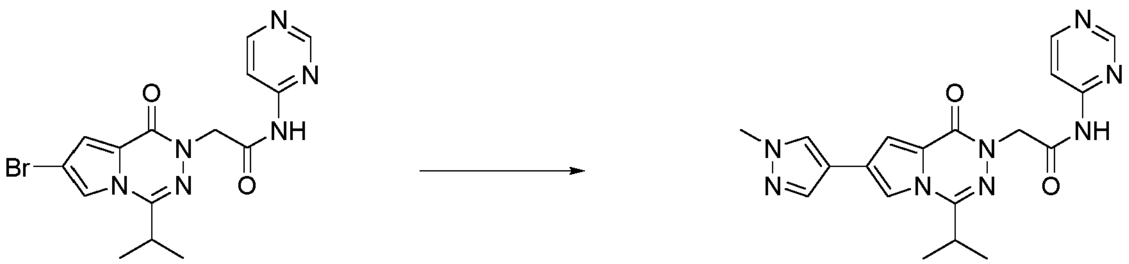

[1,1'-Bis(diphenylphosphino)ferrocene]dichloropalladium(II) [72287-26-4] (14 mg, 0.0192 mmol) was added to a degassed mixture of 2-(7-bromo-4-isopropyl-1-oxo-pyrrolo[1,2-d][1,2,4]triazin-2-yl)-N-pyrimidin-4-yl-acetamide **1F** (150 mg, 0.39 mmol), 1-methyl-4-(4,4,5,5-tetramethyl-1,3,2-dioxaborolan-2-yl)-1h-pyrazole [761446-44-0] (120 mg, 0.57 mmol), NaHCO3, sat. solution [144-55-8] (0.69 mL, 1.14 mmol) and 1,4-dioxane (6.5 mL) under nitrogen. The mixture was stirred at 110°C for 15 minutes under microwave irradiation. The mixture was treated with water and extracted with ethyl acetate. The organic layer was separated, dried (Na2SO4), filtered and evaporated in vacuo. The residue was purified by RP Flash (Stationary phase: YMC 40 g, 25 μm, Mobile Phase: ACN in NH4HCO3 0.25% solution in Water 10/90 to 90/10). The desired fractions were collected and the solvent evaporated in vacuo to yield 2-(4-isopropyl-7-(1-methyl-1H-pyrazol-4-yl)-1-oxopyrrolo[1,2-d][1,2,4]triazin-2(1H)-yl)-N-(pyrimidin-4-yl)acetamide **89F** (82 mg, 52% yield) as a white solid.

Additional analogs were synthesized according to the above procedure substituting the reagents as appropriate.

| Reagent | Final product |
|---|---|
| 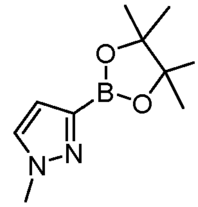 | 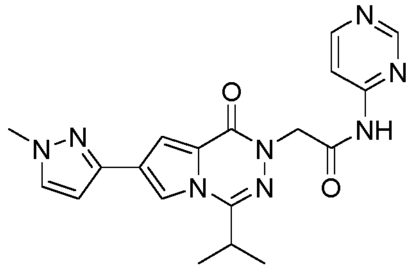 **90F** |
| 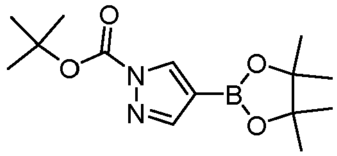 | 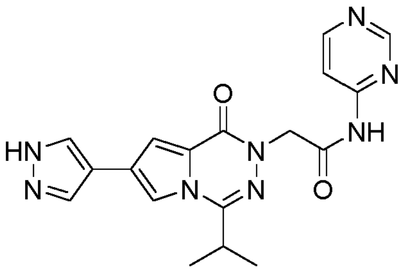 **91F** |
| 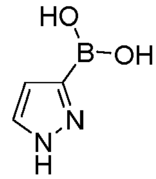 | 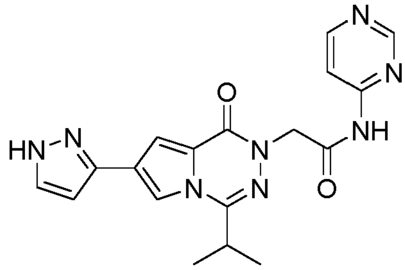 **92F** |

Synthesis of 2-(4-isopropyl-7-(1-methyl-1H-imidazol-4-yl)-1-oxopyrrolo[1,2-d][1,2,4]triazin-2(1H)-yl)-N-(pyrimidin-4-yl)acetamide **93F**

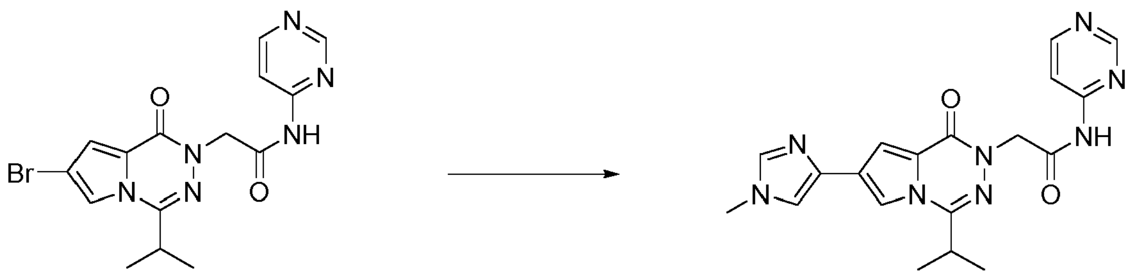

Tetrakis(triphenylphosphine)palladium(0) [14221-01-3] (44 mg, 0.038 mmol) was added to a degassed mixture of 2-(7-bromo-4-isopropyl-1-oxo-pyrrolo[1,2-d][1,2,4]triazin-2-yl)-N-pyrimidin-4-yl-acetamide **1F** (150 mg, 0.38 mmol), N-methyl-4-(tributylstannyl)imidazole [446285-73-0] (213 mg, 0.58 mmol) in toluene (4 mL) under nitrogen. The mixture was stirred at 150°C for 15 minutes under microwave irradiation. The reaction was quenched with some drops of DI water and the crude was filtered over a Pad of celite, and concentrated. The residue was purified by RP Flash (Stationary phase: YMC 40 g, 25 μm, Mobile Phase: ACN in NH4HCO3 0.25% solution in Water 10/90 to 75/25). The desired fractions were collected and the solvent evaporated in vacuo to yield 2-(4-isopropyl-7-(1-methyl-1H-imidazol-4-yl)-1-oxopyrrolo[1,2-d][1,2,4]triazin-2(1H)-yl)-N-(pyrimidin-4-yl)acetamide **93F** (81 mg, 54% yield) as a white solid.

Additional analogs were synthesized according to the above procedure substituting the reagents as appropriate.

| Reagent | Final product |
|---|---|
| 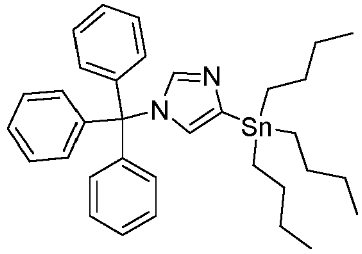 | 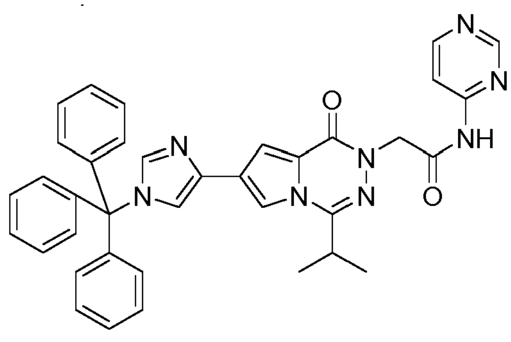 **58E** |

Synthesis of 2-(7-(1H-imidazol-4-yl)-4-isopropyl-1-oxopyrrolo[1,2-d][1,2,4]triazin-2(1H)-yl)-N-(pyrimidin-4-yl)acetamide **94F**

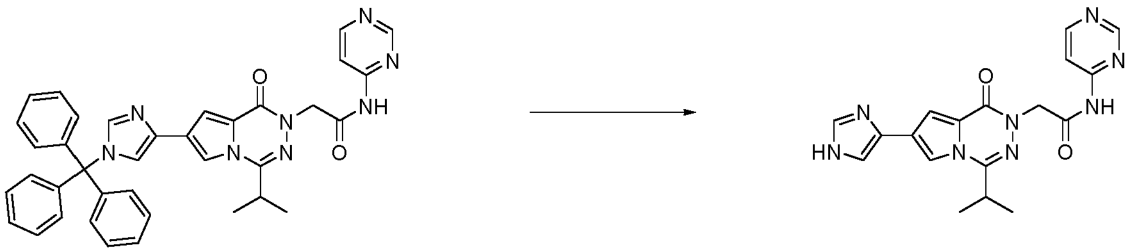

AcOH [64-19-7] (1 mL) was added to a solution of 2-(4-isopropyl-1-oxo-7-(1-trityl-1H-imidazol-4-yl)pyrrolo[1,2-d][1,2,4]triazin-2(1H)-yl)-N-(pyrimidin-4-yl)acetamide **58E** (278 mg, 0.35 mmol) in MeOH (7 mL). The reaction was stirred at reflux for 4 hours . AcOH [64-19-7] (1 mL) was added and the mixture was stirred for an additional 18 hours. The reaction was concentrated, and the residue was loaded on Celite and purified by RP Flash (Stationary phase: YMC 40 g, 25 μm, Mobile Phase: ACN in NH4HCO3 0.25% solution in Water 10/90 to 90/10, 20V). The desired fractions were collected and the solvent evaporated in vacuo to yield 2-(7-(1H-imidazol-4-yl)-4-isopropyl-1-oxopyrrolo[1,2-d][1,2,4]triazin-2(1H)-yl)-N-(pyrimidin-4-yl)acetamide **94F** (27 mg, 20% yield) as a white solid.

Synthesis of 2-(7-acetyl-4-isopropyl-1-oxopyrrolo[1,2-d][1,2,4]triazin-2(1H)-yl)-N-(pyrimidin-4-yl)acetamide **85F**

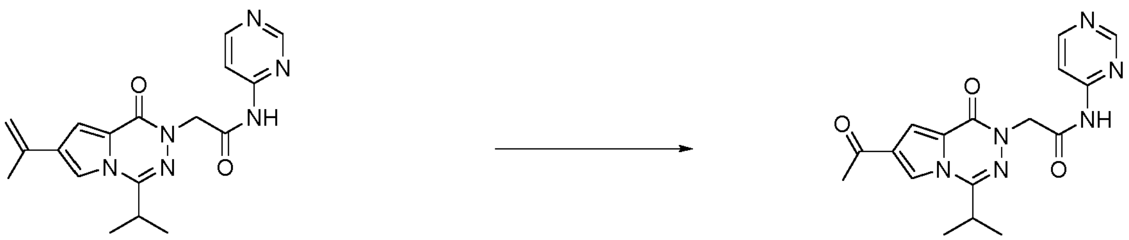

4-Methylmorpholine N-oxide [7529-22-8] (94.743 mg, 0.809 mmol), sodium periodate [7790-28-5] (230.646 mg, 1.078 mmol) and 2-(4-isopropyl-1-oxo-7-(prop-1-en-2-yl)pyrrolo[1,2-d][1,2,4]triazin-2(1H)-yl)-N-(pyrimidin-4-yl)acetamide **16F** (95 mg, 0.27 mmol) were placed in an 8-mL screw-cap tube equipped with a magnetic stir bar. These solids were suspended in 1,4-dioxane (1.77 mL) and osmium tetroxide [20816-12-0] (0.137 mL, 0.01 g/mL solution in

water, 0.00539 mmol) was added. The suspension was stirred vigorously at r.t. for 18h. The mixture was diluted with DI water (3 mL) and the suspension filtered. The obtained solid was washed twice with DI water (1 mL each time, vigorous trituration) and dried under vacuum at 50 °C for 24h to afford an off-white solid. This solid was purified by FCC on Biotage (Sfar 25g, 60 mL/min, Hept/EtOAc 1:1 to 0:1 over 30 CV then EtOAc for 10 CV) to give 2-(7-acetyl-4-isopropyl-1-oxopyrrolo[1,2-d][1,2,4]triazin-2(1H)-yl)-N-(pyrimidin-4-yl)acetamide **85F** as a colorless solid (8 mg, 8%). The insoluble solid remaining on top of the silica cartridge was collected and partitioned between water (50 mL) and DCM (100 mL). The aqueous layer was re-extracted with DCM (50 mL). The combined organic extracts were dried over Na2SO4, filtered and concentrated in vacuo to give 2-(7-acetyl-4-isopropyl-1-oxopyrrolo[1,2-d][1,2,4]triazin-2(1H)-yl)-N-(pyrimidin-4-yl)acetamide **85F** as an off-white solid (57 mg, 60%).

**Characterising Data - LC-MS and melting point**

| Final Cpd | LC-MS (Rt, area%, MW, $[M+H]^+$ / $[M-H]^-$, Method) | Melting Point (°C) |
|---|---|---|
| 1F | RT: 1.7; area 100%; MW:390; $[M+H]^+$ / $[M-H]^-$: 389/391 (Method G) | |
| 2F | RT: 1.78; area 100%; MW:407; $[M+H]^+$ / $[M-H]^-$: 406/408 (Method L) | |
| 3F | RT: 1.32; area 98.95%; MW:356.16; $[M-H]^-$: 355.2 (Method A) | |
| 4F | RT: 1.82; area 100%; MW:455; $[M+H]^+$ / $[M-H]^-$: 456/454 (Method L) | |
| 5F | RT: 1.75; area 100%; MW:438; $[M+H]^+$ / $[M-H]^-$: 439/437 (Method L) | |
| 6F | RT: 1.67; area 82.76%; MW:346; $[M+H]^+$ / $[M-H]^-$: 347/345 (Method G) | |
| 7F | RT: 1.55; area 97.5%; MW:330; $[M+H]^+$ / $[M-H]^-$: 331/329 (Method L) | 194.5 |
| 8F | RT: 1.68; area 77%; MW:389; $[M+H]^+$ / $[M-H]^-$: 390/388 (Method L) | |
| 9F | RT: 1.75; area 72%; MW:363; $[M+H]^+$ / $[M-H]^-$: 364/362 (Method L) | 211.91 |
| 10F | RT: 1.78; area 100%; MW:407; $[M+H]^+$ / $[M-H]^-$: 408/406 (Method L) | |
| 11F | RT: 1.49; area 95%; MW:312; $[M+H]^+$ / $[M-H]^-$: 313/311 (Method L) | |
| 12F | RT: 0.91; area 100%; MW:342.14; $[M-H]^-$: 341.1 (Method A) | |
| 13F | RT: 1.73; area 100%; MW:326.1; $[M+H]^+$ / $[M-H]^-$: 327/325 (Method K) | 167.68 |
| 14F | RT: 1.28; area 100%; MW:297; $[M+H]^+$ / $[M-H]^-$: 298/296 (Method K) | |
| 15F | RT: 1.71; area 99.12%; MW:260.1; $[M+H]^+$: 261 (Method H) | 227.68 |
| 16F | RT: 1.77; area 100%; MW:352; $[M+H]^+$ / $[M-H]^-$: 353/351 (Method L) | 185.03 |
| 17F | RT: 1.48 area 100%; MW:338; $[M+H]^+$ / $[M-H]^-$: 339/337 (Method L) | |
| 18F | RT: 1.92; area 100%; MW:371; $[M+H]^+$ / $[M-H]^-$: 372/370 (Method L) | |
| 19F | RT: 1.85; area 100%; MW:354; $[M+H]^+$ / $[M-H]^-$: 355/353 (Method L) | |
| 20F | RT: 1.63; area 100%; MW:338; $[M+H]^+$ / $[M-H]^-$: 339/337 (Method L) | |
| 21F | RT: 1.71; area 100%; MW:355; $[M+H]^+$ / $[M-H]^-$: 356/354 (Method L) | |
| 22F | RT: 1.8; area 100%; MW:357; $[M+H]^+$ / $[M-H]^-$: 358/356 (Method L) | 142.64 |
| 23F | RT: 1.74; area 97.08%; MW:340; $[M+H]^+$ / $[M-H]^-$: 341/339 (Method G) | |
| 24F | RT: 1.62; area 100%; MW:370.2; $[M+H]^+$: 371 (Method J) | 184.19 |
| 25F | RT: 1.65; area 100%; MW:397; $[M+H]^+$: 398 (Method J) | |
| 26F | RT: 1.85; area 100%; MW:369; $[M+H]^+$: 370 (Method L) | 150.14 |
| 27F | RT: 1.78; area 100%; MW:368; $[M+H]^+$ / $[M-H]^-$: 369/367 (Method G) | 201.01 |

(continued)

| Final Cpd | LC-MS (Rt, area%, MW, $[M+H]^+$ / $[M-H]^-$, Method) | Melting Point (°C) |
|---|---|---|
| 28F | RT: 1.99; area 92.51%; MW:382; $[M+H]^+$ / $[M-H]^-$: 383/381 (Method K) | 215.23 |
| 29F | RT: 1.61; area 100%; MW:362.1; $[M+H]^+$ / $[M-H]^-$: 363/361 (Method L) | 216.46 |
| 30F | RT: 2.1; area 99.25%; MW:366.18; $[M+H]^+$ / $[M-H]^-$: 367.2/365.2 (Method E) | |
| 31F | RT: 1.75; area 100%; MW:370.1; $[M+H]^+$: 371 (Method J) | |
| 32F | RT: 1.83; area 100%; MW:397; $[M+H]^+$ / $[M-H]^-$: 398/396 (Method L) | |
| 33F | RT: 1.04; area 88.7%; MW:408.2; $[M+H]^+$: 409.2 (Method F) | |
| 34F | RT: 1.38; area 96%; MW:368.4; $[M+H]^+$ / $[M-H]^-$: 369.3/367 (Method B) | |
| 35F | RT: 1.88; area 100%; MW:354; $[M+H]^+$ / $[M-H]^-$: 355/353 (Method K) | 284.78 |
| 36F | RT: 1.49; area 100%; MW:356.2; $[M+H]^+$: 357 (Method L) | 247.56 |
| 37F | RT: 1.61; area 100%; MW:370; $[M+H]^+$: 369/371 (Method L) | |
| 38F | RT: 1.58; area 100%; MW:370; $[M+H]^+$ / $[M-H]^-$: 371/369 (Method L) | 190.72 |
| 39F | RT: 1.36; area 100%; MW:356; $[M+H]^+$ / $[M-H]^-$: 357/355 (Method L) | |
| 40F | RT: 2.01; area 100%; MW:384.2; $[M+H]^+$ / $[M-H]^-$: 385/383 (Method K) | |
| 42F | RT: 1.49, Area %: 95.50, MW: 337.00, BPM 1/BPM2: 338/336 (Method: L) | |
| 43F | RT: 0.78, Area %: 98.04, MW: 369.19, BPM1: 370.2 (Method: A) | |
| 44F | RT: 0.65, Area %: 100.00, MW: 355.18, BPM1: 356.2 (Method: A) | |
| 45F | RT: 1.26, Area %: 98.63, MW: 367.00, BPM1/BPM2: 368/291 (Method: L) | |
| 46F | RT: 1.36, Area %: 100.00, MW: 409.00, BPM1BPM2: 410/408 (Method: N) | |
| 47F | RT: 1.87, MW: 388, BPM1BPM2: 389/387 (Method: L) | 209.70 |
| 48F | RT: 1.81, MW: 394, BPM1/BPM2: 395/393 (Method: L) | 217.95 |
| 50F | RT: 1.74, Area %: 94, MW: 352, BPM1/BPM2: 353/351 (Method: L) | 182.51 |
| 51F | RT: 1.80, Area %: 95.55, MW: 360.00, BPM1BPM2: 361/359 (Method: L) | 181.03 |
| 52F | RT: 1.65, Area %: 100.00, MW: 347.00, BPM1BPM2: 348/346 (Method: L) | |
| 53F | RT: 1.75, Area %: 99.24, MW: 342.1, BPM1/BPM2: 343.2/341.2 (Method: E) | |
| 54F | RT: 1.69, Area %: 97.96, MW: 342.14, BPM1/BPM2: 343/341.1 (Method: A) | |
| 55F | RT: 1.74, Area %: 100.00, MW: 355.20, BPM1/BPM2: 356/356 (Method: J) | 166.33 |
| 56F | RT: 1.50, Area %: 100.00, MW: 341.20, BPM1BPM2: 342/342 (Method: J) | |
| 57F | RT: 1.99, Area %: 100.00, MW: 395.00, BPM1BPM2: 396/815 (Method: L) | |
| 58F | RT: 2.349, Area %: 99, MW 347, BPM1: 348.1, (Method: M) | 171.4 |
| 59F | RT: 3.178, Area %: 98, MW 354, BPM1: 355.1, (Method: M) | 149.7 |
| 60F | RT: 1.02, Area %: 100.00, MW: 374.00, BPM1BPM2: 375/373 (Method: K) | |
| 61F | RT: 1.93, Area %: 100.00, MW: 368.00, BPM1/BPM2: 369/393 (Method: L) | 166.41 |
| 62F | RT: 1.76, Area %: 100.00, MW: 402.20, BPM1BPM2: 403/403 (Method: J) | |
| 63F | RT: 1.61, Area%: 100.00, MW: 356.20, BPM1BPM2: 262/355 (Method: N) | 201.37 |
| 64F | RT: 1.45, Area %: 100.00, MW: 375.00, BPM1/BPM2: 374/374 (Method: L) | 224.87 |
| 65F | RT: 1.44, Area %: 100.00, MW: 397.00, BPM1BPM2: 398/398 (Method: J) | 279.37 |

(continued)

| Final Cpd | LC-MS (Rt, area%, MW, $[M+H]^+$ / $[M-H]^-$, Method) | Melting Point (°C) |
|---|---|---|
| 66F | RT: 1.62, Area %: 100.00, MW: 348.2, BPM1/BPM2: 349.2/347.3 (Method: E) | |
| 67F | RT: 2.10, Area %: 81.95, MW: 413.00, BPM1/BPM2: 414/412 (Method: J) | 115.25 |
| 68F | RT: 1.73, Area %: 98.62, MW: 360.00, BPM1/BPM2: 361/359 (Method: J) | 196.59 |
| 69F | RT: 1.67, Area %: 100.00, MW: 393.00, BPM1/BPM2: 394.3/392.3 (Method: G) | |
| 70F | RT: 1.08, Area %: 96.97, MW: 397.20, BPM1: 398.2 (Method: A) | |
| 71F | RT: 1.49, Area %: 100.00, MW: 477.04, BPM1: 478.0 (Method: A) | |
| 72F | RT: 1.44, Area %: 100.00, MW: 444.07, BPM1: 445.1 (Method: A) | |
| 73F | RT: 2.609, Area %: 95, MW:341, BPM1: 342.1, (Method: M) | 178.1 |
| 74F | RT: 2.914, Area %: 99, MW:355, BPM1: 356.1, (Method: M) | 156.3 |
| 75F | RT: 2.17, Area %: 100.00, MW: 402.18, BPM1/BPM2: 403.2/401.2 (Method: E) | |
| 76F | RT: 0.91, Area %: 93.97, MW: 369.15, BPM1: 370.2 (Method: A) | |
| 77F | RT: 1.03, Area %: 99.41, MW: 383.17, BPM1: 382.2 (Method: A) | |
| 78F | RT: 1.11, Area %: 94, MW: 372.402, BPM1: 373.2444 (Method: B) | |
| 79F | RT: 0.80, Area %: 94.68, MW: 396.2000, BPM1/BPM2: 397.3/395.3 (Method: F) | |
| 80F | RT: 0.88, Area %: 1.76, MW: 410.21, BPM1/BPM2: 411.3/409.4 (Method: F) | |
| 81F | RT: 0.88, Area %: 100, MW: 423.475, BPM1/BPM2: 424.2218/422.3335 (Method: B) | |
| 82F | RT: 0.71, Area %: 90, MW: 409.608, BPM1/BPM2: 410.2077/408.2223 (Method: D) | |
| 83F | RT: 0.60, Area %: 93, MW: 367.41, BPM1/BPM2: 368.2213/366.2930 (Method: B) | |
| 84F | RT: 0.65, Area %: 93, MW: 395.464, BPM1/BPM2: 396.2729/394.1356 (Method: D) | |
| 85F | RT: 1.42, Area %: 70, MW: 354, BPM1: 355 (Method: J) | 262.30 |
| 86F | RT: 1.52, Area %: 81.52, MW: 370.00, BPM1BPM2: 371/369 (Method: L) | |
| 87F | decomposes on LCMS (RT: 1.73, Area %: 41.43, MW: 372.00, BPM1/BPM2: 373/371 (Method: L) | |
| 88F | RT: 1.58, Area %: 100.00, MW: 370.00, BPM1/BPM2: 371/369 (Method: L) | |
| 89F | RT: 1.32, Area %: 96.33, MW: 392.17, BPM1/BPM2: 393.2, BPM2: 391.2 (Method: E) | |
| 90F | RT: 1.43, Area %: 96.90, MW: 392.17, BPM1/BPM2: 393.2/391.2 (Method: E) | |
| 91F | RT: 1.13, Area %: 96.02, MW: 378.16, BPM1: 377.1 (Method: A) | |
| 92F | RT: 1.21, Area %: 100.00, MW: 378.16, BPM1/BPM2: 379.2/377.1479 (Method: A) | |
| 93F | RT: 1.22, Area %: 100.00, MW: 392.17, BPM1/BPM2: 393.2/391.2 (Method: E) | |
| 94F | RT: 0.99, Area %: 100.00, MW: 378.16, BPM1: 379.2 (Method: A) | |

**Characterising Data - NMR**

| Final Cpd | NMR |
|---|---|
| 1F | $^1$H NMR (400 MHz, DMSO-$d_6$) δ ppm 1.26 (d, *J*=6.7 Hz, 6 H), 3.39 (spt, *J*=6.8 Hz, 1 H), 4.87 (s, 2 H), 7.21 (d, *J*=1.5 Hz, 1 H), 7.98 (dd, *J*=5.8, 1.3 Hz, 1 H), 8.06 (d, *J*=1.6 Hz, 1 H), 8.66 (d, *J*=5.8 Hz, 1 H), 8.91 (d, *J*=1.3 Hz, 1 H), 11.23 (br s, 1 H) |
| 2F | $^1$H NMR (400 MHz, CHLOROFORM-*d*) δ ppm 1.41 (d, *J*=6.8 Hz, 6 H), 3.15 (spt, *J*=6.8 Hz, 1 H), 4.90 (s, 2 H), 7.27 (d, *J*=1.5 Hz, 1 H), 7.31 (d, *J*=1.5 Hz, 1 H), 8.29 - 8.34 (m, 2 H), 8.41 (d, *J*=2.4 Hz, 1 H), 8.90 (br s, 1 H) |
| 4F | $^1$H NMR (400 MHz, DMSO-$d_6$) δ ppm 1.25 (d, *J*=6.7 Hz, 6 H), 3.40 (spt, *J*=6.7 Hz, 1 H), 4.89 (s, 2 H), 7.22 (d, *J*=1.4 Hz, 1 H), 8.01 (d, *J*=1.5 Hz, 1 H), 8.11 (dd, *J*=6.9, 5.4 Hz, 1 H), 8.31 (d, *J*=5.4 Hz, 1 H), 8.55 (d, *J*=2.9 Hz, 1 H), 10.53 (br s, 1 H)<br>$^{13}$C NMR (101 MHz, DMSO-$d_6$) δ ppm 19.69, 28.46, 52.49, 70.76, 115.52, 117.34, 122.26, 124.93, 133.03 (d, *J*=9.2 Hz), 137.64 (d, *J*=20.8 Hz), 141.01, 146.53 (d, *J*=5.4 Hz), 149.33 (d, *J*=253.5 Hz), 151.86, 167.00<br>$^{19}$F NMR (377 MHz, DMSO-$d_6$) δ ppm -141.30 (dd, *J*=6.9, 3.0 Hz) |
| 5F | $^1$H NMR (400 MHz, DMSO-$d_6$) δ ppm 1.25 (d, *J*=6.8 Hz, 6 H), 3.39 (spt, *J*=6.8 Hz, 1 H), 4.85 (s, 2 H), 7.22 (d, *J*=1.4 Hz, 1 H), 7.97 (dd, *J*=5.8, 1.3 Hz, 1 H), 8.01 (d, *J*=1.5 Hz, 1 H), 8.66 (d, *J*=5.8 Hz, 1 H), 8.90 (d, *J*=1.3 Hz, 1H), 11.21 (s, 1 H) |
| 9F | $^1$H NMR (400 MHz, CHLOROFORM-*d*) δ ppm 1.41 (d, *J*=6.8 Hz, 6 H), 3.13 (d, *J*=6.8 Hz, 1 H), 4.91 (s, 2 H), 7.20 (d, *J*=1.6 Hz, 1 H), 7.26 (d, *J*=2.0 Hz, 1 H), 8.28 - 8.34 (m, 2 H), 8.40 (d, *J*=2.4 Hz, 1 H), 8.94 (br s, 1 H)<br>$^{13}$C NMR (101 MHz, CHLOROFORM-*d*) δ ppm 19.67, 29.86, 54.78, 112.16, 114.01, 114.73, 120.92, 123.16, 133.02 (d, *J*=8.4 Hz), 137.16 (d, *J*=21.4 Hz), 142.38, 146.91 (d, *J*=5.3 Hz), 149.04 (br d, *J*=252.5 Hz), 153.38, 166.22<br>$^{19}$F NMR (376 MHz, CHLOROFORM-*d*) δ ppm -145.77 (br s) |
| 10F | $^1$H NMR (400 MHz, CHLOROFORM-*d*) δ ppm 1.41 (d, *J*=6.8 Hz, 6 H), 3.15 (spt, *J*=6.8 Hz, 1 H), 4.90 (s, 2 H), 7.27 (d, *J*=1.5 Hz, 1 H), 7.31 (d, *J*=1.5 Hz, 1 H), 8.29 - 8.34 (m, 2 H), 8.41 (d, *J*=2.4 Hz, 1 H), 8.90 (br s, 1 H) |
| 18F | $^1$H NMR (400 MHz, CHLOROFORM-*d*) δ ppm 1.29 (d, *J*=6.9 Hz, 6 H), 1.40 (d, *J*=6.8 Hz, 6 H), 2.99 (spt, *J*=6.9 Hz, 1 H), 3.20 (spt, *J*=6.8 Hz, 1 H), 4.91 (s, 2 H), 7.08 (br s, 1 H), 7.18 (d, *J*=1.6 Hz, 1 H), 8.29 - 8.35 (m, 2 H), 8.38 (d, *J*=2.4 Hz, 1 H), 9.18 (br s, 1 H) |
| 19F | $^1$H NMR (400 MHz, CHLOROFORM-*d*) δ ppm 1.29 (d, *J*=6.9 Hz, 6 H), 1.40 (d, *J*=6.8 Hz, 6 H), 2.98 (spt, *J*=6.9 Hz, 1 H), 3.19 (spt, *J*=6.8 Hz, 1 H), 4.89 (s, 2 H), 7.05 - 7.08 (m, 1 H), 7.17 (d, *J*=1.6 Hz, 1 H), 8.14 (dd, *J*=5.8, 1.3 Hz, 1 H), 8.61 (d, *J*=5.7 Hz, 1 H), 8.84 (d, *J*=1.3 Hz, 1 H), 8.95 (br s, 1 H)<br>$^{13}$C NMR (101 MHz, CHLOROFORM-*d*) δ ppm 19.97, 23.80, 26.76, 29.91, 54.28, 110.57, 111.55, 113.05, 123.32, 139.26, 143.45, 154.57, 156.91, 158.56, 167.44 |
| 20F | $^1$H NMR (400 MHz, CHLOROFORM-*d*) δ ppm 0.63 - 0.67 (m, 2 H), 0.96 - 1.01 (m, 2 H), 1.39 (t, *J*=7.4 Hz, 3 H), 1.87 (tt, *J*=8.4, 5.0 Hz, 1 H), 2.84 (q, *J*=7.4 Hz, 2 H), 4.88 (s, 2 H), 6.96 (d, *J*=1.6 Hz, 1 H), 7.03 (d, *J*=1.6 Hz, 1 H), 8.14 (dd, *J*=5.8, 1.3 Hz, 1 H), 8.61 (d, *J*=5.8 Hz, 1 H), 8.84 (d, *J*=1.3 Hz, 1 H), 8.89 (br s, 1 H)<br>$^{13}$C NMR (101 MHz, CHLOROFORM-*d*) δ ppm 7.97, 8.84, 9.65, 23.97, 54.03, 110.27, 110.35, 113.26, 122.98, 134.82, 139.57, 154.20, 156.65, 158.31, 158.34, 167.12 |
| 21F | $^1$H NMR (400 MHz, CHLOROFORM-*d*) δ ppm 0.62 - 0.68 (m, 2 H), 0.91 - 1.06 (m, 2 H), 1.35 - 1.42 (m, 3 H), 1.81 - 1.93 (m, 1 H), 2.85 (q, *J*=7.4 Hz, 2 H), 4.91 (s, 2 H), 6.97 (d, *J*=1.5 Hz, 1 H), 7.04 (d, *J*=1.5 Hz, 1 H), 8.27 - 8.33 (m, 2 H), 8.38 (d, *J*=2.4 Hz, 1 H), 9.17 (br s, 1 H)<br>$^{13}$C NMR (101 MHz, CHLOROFORM-*d*) δ ppm 8.07, 8.96, 9.72, 24.06, 54.54, 110.19, 113.38, 114.74, 123.05, 133.17 (d, *J*=8.5 Hz), 134.99, 137.08 (d, *J*=20.8 Hz), 139.71, 146.80 (d, *J*=5.4 Hz), 149.06 (d, *J*=252.8 Hz), 154.41, 166.80 |

(continued)

| Final Cpd | NMR |
|---|---|
| 22F | $^1$H NMR (400 MHz, CHLOROFORM-*d*) δ ppm 1.27 (t, *J*=7.5 Hz, 3 H), 1.40 (d, *J*=6.8 Hz, 6 H), 2.66 (q, *J*=7.5 Hz, 2 H), 3.19 (spt, *J*=6.8 Hz, 1 H), 4.92 (s, 2 H), 7.09 (s, 1 H), 7.13 (d, *J*=1.5 Hz, 1 H), 8.25 - 8.33 (m, 2 H), 8.37 (d, *J*=2.4 Hz, 1 H), 9.24 (br s, 1 H)<br>$^{13}$C NMR (101 MHz, CHLOROFORM-*d*) δ ppm 14.67, 19.64, 20.07, 29.69, 54.43, 112.21, 113.87, 114.72, 123.10, 133.15 (d, *J*=7.7 Hz), 133.73, 136.98 (d, *J*=20.8 Hz), 143.15, 146.68 (d, *J*=5.4 Hz), 149.01 (d, *J*=252.8 Hz), 154.40, 166.84<br>$^{19}$F NMR (377 MHz, CHLOROFORM-*d*) δ ppm -145.35 - -145.24 (m) |
| 23F | $^1$H NMR (400 MHz, CHLOROFORM-*d*) δ ppm 1.27 (t, *J*=7.5 Hz, 3 H), 1.40 (d, *J*=6.8 Hz, 6 H), 2.66 (q, *J*=7.6 Hz, 2 H), 3.18 (spt, *J*=6.8 Hz, 1 H), 3.31 - 3.34 (m, 1 H), 4.90 (s, 2 H), 7.08 (s, 1 H), 7.13 (d, *J*=1.5 Hz, 1 H), 8.14 (dd, *J*=5.8, 1.4 Hz, 1 H), 8.61 (d, *J*=5.8 Hz, 1 H), 8.84 (d, *J*=1.3 Hz, 1 H), 9.04 (br s, 1 H)<br>$^{13}$C NMR (101 MHz, CHLOROFORM-*d*) δ ppm 14.76, 19.77, 20.12, 29.75, 54.07, 110.41, 112.43, 113.86, 123.19, 133.73, 143.14, 154.34, 156.78, 158.36, 167.29 |
| 30F | $^1$H NMR (400 MHz, CHLOROFORM-*d*) δ ppm 1.40 (d, *J*=6.8 Hz, 6 H), 1.87 - 2.16 (m, 4 H), 2.33 - 2.44 (m, 2 H), 3.18 (spt, *J*=6.8 Hz, 1 H), 3.49 - 3.58 (m, 1H), 4.89 (s, 2 H), 7.07 (d, *J*=1.6 Hz, 1 H), 7.18 (d, *J*=1.5 Hz, 1 H), 8.14 (dd, *J*=5.8, 1.3 Hz, 1 H), 8.62 (d, *J*=5.8 Hz, 1 H), 8.85 (d, *J*=1.3 Hz, 1 H), 8.96 (br s, 1 H) |
| 36F | $^1$H NMR (400 MHz, DMSO-$d_6$) δ ppm 1.25 (d, *J*=6.9 Hz, 6 H), 1.48 (d, *J*=6.6 Hz, 3 H), 2.96 (spt, *J*=6.9 Hz, 1 H), 4.82 (d, *J*=16.7 Hz, 1 H), 4.89 (d, *J*=16.7 Hz, 1 H), 4.95 (quin, *J*=6.3 Hz, 1 H), 5.92 (d, *J*=5.6 Hz, 1 H), 7.02 (d, *J*=1.6 Hz, 1 H), 7.60 - 7.64 (m, 1 H), 7.97 (dd, *J*=5.8, 1.3 Hz, 1 H), 8.66 (d, *J*=5.8 Hz, 1 H), 8.90 (d, *J*=1.3 Hz, 1 H), 11.21 (br s, 1 H) |
| 39F | $^1$H NMR (400 MHz, CHLOROFORM-d) 8.92 (br s, 1 H), 8.85 (d, J=1.1 Hz, 1 H), 8.62 (d, J=5.9 Hz, 1 H), 8.13 (dd, J=5.9, 1.3 Hz, 1 H), 7.31 (s, 1 H), 7.23 (t, J=1.1 Hz, 1 H), 5.76 (dq, J=48.2, 6.6 Hz, 1 H), 4.91 (s, 2 H), 3.20 (spt, J=6.8 Hz, 1 H), 1.70 (dd, J=23.7, 6.5 Hz, 3 H), 1.41 (d, J=6.6 Hz, 6 H) |
| 42F | 1H NMR (400 MHz, CHLOROFORM-d) 1.42 (d, J=6.8 Hz, 6 H), 3.18 (spt, J=6.7 Hz, 1 H), 4.91 (s, 2 H), 7.47 (d, J=1.3 Hz, 1 H), 7.70 (d, J=1.3 Hz, 1 H), 8.11 (br d, J=5.2 Hz, 1 H), 8.55 - 8.75 (m, 2 H), 8.88 (s, 1 H). |
| 47F | 1H NMR (400 MHz, CHLOROFORM-d) 1.44 (d, J=6.8 Hz, 6 H), 3.27 (spt, J=6.8 Hz, 1 H), 4.93 (s, 2 H), 7.31 - 7.37 (m, 1 H), 7.40 - 7.47 (m, 2 H), 7.52 (d, J=1.5 Hz, 1 H), 7.55 (d, J=1.6 Hz, 1 H), 7.59 - 7.65 (m, 2 H), 8.15 (dd, J=5.8, 1.2 Hz, 1 H), 8.62 (d, J=5.7 Hz, 1 H), 8.85 (d, J=1.1 Hz, 1 H), 9.01 (br s, 1 H). |
| 48F | 1H NMR (400 MHz, CHLOROFORM-d) 7.66 - 7.58 (m, 2 H), 7.50 (s, 2 H), 7.47 - 7.40 (m, 2 H), 7.37 - 7.31 (m, 1 H), 6.22 (br d, J=6.8 Hz, 1 H), 4.71 (s, 2 H), 4.10 - 3.97 (m, 1 H), 3.92 (dt, J=11.7, 3.5 Hz, 2 H), 3.46 (td, J=11.7, 2.2 Hz, 2 H), 3.33 - 3.17 (m, 1 H), 1.90 (br dd, J=12.5, 2.3 Hz, 2 H), 1.52 - 1.44 (m, 2 H), 1.42 (d, J=6.8 Hz, 6 H). |
| 50F | 1H NMR (400 MHz, CHLOROFORM-d) 9.17 (br s, 1 H), 8.83 (d, J=0.9 Hz, 1 H), 8.60 (d, J=5.7 Hz, 1 H), 8.13 (dd, J=5.7, 1.1 Hz, 1 H), 7.09 (d, J=1.4 Hz, 1 H), 6.96 (d, J=1.4 Hz, 1 H), 4.90 (s, 2 H), 3.16 (spt, J=6.8 Hz, 1 H), 1.89 - 1.82 (m, 1 H), 1.39 (d, J=6.8 Hz, 6 H), 1.02 - 0.94 (m, 2 H), 0.68 - 0.60 (m, 2 H). |
| 51F | 1H NMR (400 MHz, CHLOROFORM-d) 1.29 (d, J=6.9 Hz, 6 H), 1.38 (d, J=6.8 Hz, 6 H), 1.40 - 1.51 (m, 2 H), 1.88 (br dd, J=12.5, 2.2 Hz, 1 H), 1.84 - 1.92 (m, 1 H), 2.98 (spt, J=6.9 Hz, 1 H), 3.17 (spt, J=6.8 Hz, 1 H), 3.45 (td, J=11.6, 2.1 Hz, 2H), 3.91 (dt, J=11.6, 3.5 Hz, 2 H), 3.95 - 4.06 (m, 1 H), 4.68 (s, 2 H), 6.27 (br d, J=7.5 Hz, 1 H), 7.05 (s, 1 H), 7.12 (d, J=1.5 Hz, 1 H). |
| 52F | 1H NMR (400 MHz, CHLOROFORM-d) d ppm 1.40 (d, J=6.8 Hz, 6 H), 3.11 (spt, J=6.8 Hz, 1 H), 4.91 (s, 2 H), 7.02 (d, J=1.6 Hz, 1 H), 7.11 (dd, J=3.5, 1.7 Hz, 1 H), 8.26 - 8.35 (m, 2 H), 8.40 (d, J=2.3 Hz, 1 H), 9.00 (br s, 1 H). |
| 58F | 1H NMR (400 MHz, CDCl3) d 7.07 (dd, J = 13.0, 1.1 Hz, 2H), 6.62 (d, J = 7.3 Hz, 1H), 4.60 (s, 2H), 3.97 (h, J = 7.9 Hz, 1H), 2.85 (s, 6H), 2.64 (q, J = 7.6 Hz, 2H), 2.53 - 2.46 (m, 2H), 2.05 - 1.98 (m, 2H), 1.89 (s, 1H), 1.35 (s, 3H), 1.26 (t, J = 7.6 Hz, 3H). |

(continued)

| Final Cpd | NMR |
|---|---|
| 59F | 1H NMR (400 MHz, CDCl3) d 8.88 (s, 1H), 8.79 (d, J = 1.1 Hz, 1H), 8.56 (d, J = 5.8 Hz, 1H), 8.08 (dd, J = 5.8, 1.3 Hz, 1H), 7.05 (d, J = 1.4 Hz, 1H), 7.01 (s, 1H), 4.82 (s, 2H), 3.12 (hept, J = 6.8 Hz, 1H), 2.52 (q, J = 7.7 Hz, 2H), 1.65 - 1.55 (m, 2H), 1.34 (d, J = 6.8 Hz, 6H), 0.91 (t, J = 7.3 Hz, 3H). |
| 67F | 1H NMR (400 MHz, CHLOROFORM-d, at 298K) 1.28 (d, J=6.9 Hz, 6 H), 1.39 (d, J=6.8 Hz, 3 H), 1.40 (d, J=6.8 Hz, 3 H), 1.40 - 1.63 (m, 8 H), 1.77 - 2.01 (m, 3 H), 2.18 (br s, 2 H), 2.34 (br s, 1 H), 2.52 - 2.62 (m, 2 H), 2.97 (spt, J=6.9 Hz, 1 H), 3.18 (spt, J=6.8 Hz, 1 H), 3.98 - 4.08 (m, 1 H), 4.63 (d, J=15.7 Hz, 1 H), 4.75 (d, J=15.8 Hz, 1 H), 6.59 (br s, 1 H), 7.01 - 7.08 (m, 1 H), 7.13 (d, J=1.1 Hz, 1 H). |
| 68F | $^1$H NMR (400 MHz, CHLOROFORM-d) 1.28 (d, J=6.6 Hz, 6 H), 1.34 (s, 3 H), 1.37 (d, J=6.8 Hz, 6 H), 1.95 - 2.08 (m, 2 H), 2.42 - 2.53 (m, 2 H), 2.70 (s, 1 H), 2.97 (spt, J=6.9 Hz, 1 H), 3.16 (spt, J=6.8 Hz, 1 H), 3.98 (spt, J=7.9 Hz, 1 H), 4.66 (s, 2 H), 6.74 (br d, J=7.6 Hz, 1 H), 7.04 (s, 1 H), 7.09 (d, J=1.4 Hz, 1 H). |
| 69F | 1H NMR (400 MHz, CHLOROFORM-*d*) 1.29 (d, J=6.8 Hz, 6 H), 1.40 (d, J=6.8 Hz, 6 H), 2.99 (spt, J=6.8 Hz, 1 H), 3.19 (spt, J=6.8 Hz, 1 H), 4.90 (s, 2 H), 7.02 (dd, J=9.8, 1.8 Hz, 1 H), 7.10 (s, 1 H), 7.16 (d, J=1.3 Hz, 1 H), 7.66 (d, J=9.7 Hz, 1 H), 8.70 (d, J=0.7 Hz, 1 H), 9.11 - 9.19 (m, 1 H), 9.58 (s, 1 H). |
| 73F | 1H NMR (400 MHz, CDCl3) d 9.06 (s, 1H), 8.85 (d, J = 1.1 Hz, 1H), 8.61 (d, J = 5.8 Hz, 1H), 8.15 (dd, J = 5.8, 1.3 Hz, 1H), 7.11 (dd, J = 3.7, 1.1 Hz, 2H), 4.83 (s, 2H), 2.88 (s, 6H), 2.65 (q, J = 7.6 Hz, 2H), 1.26 (t, J = 7.6 Hz, 3H). |
| 74F | $^1$H NMR (400 MHz, CDCl3) d 9.11 (s, 1H), 8.85 (d, J = 1.0 Hz, 1H), 8.62 (d, J = 5.8 Hz, 1H), 8.17 (dd, J = 5.8, 1.2 Hz, 1H), 7.12 (d, J = 1.6 Hz, 1H), 7.08 - 7.05 (m, 1H), 4.83 (s, 2H), 3.25 - 3.16 (m, 2H), 2.85 (s, 3H), 2.65 (q, J = 7.5 Hz, 2H), 1.29 - 1.22 (m, 6H). |
| 85F | 1H NMR (400 MHz, CHLOROFORM-*d*) 1.42 (d, J=6.8 Hz, 6 H), 2.57 (s, 2 H), 2.53 - 2.60 (m, 1 H), 3.23 (quin, J=6.8 Hz, 2 H), 4.90 (s, 2 H), 7.59 (d, J=1.6 Hz, 1 H), 7.80 (d, J=1.5 Hz, 1 H), 8.13 (dd, J=5.7, 1.1 Hz, 1 H), 8.64 (d, J=5.7 Hz, 2 H), 8.80 - 8.94 (m, 1 H). |
| 86F | 1H NMR (400 MHz, CHLOROFORM-*d*) 1.39 (d, J=6.8 Hz, 6 H), 1.61 (s, 6 H), 2.26 (s, 1 H), 3.20 (spt, J=6.8 Hz, 1 H), 4.92 (s, 2 H), 7.21 (d, J=1.5 Hz, 1 H), 7.30 (d, J=1.5 Hz, 1 H), 8.13 (dd, J=5.7, 1.3 Hz, 1 H), 8.60 (d, J=5.9 Hz, 1 H), 8.83 (d, J=1.1 Hz, 1 H), 9.13 (s, 1 H). |
| 87F | 1H NMR (400 MHz, CHLOROFORM-d) 1.41 (d, J=6.8 Hz, 6 H), 1.73 (d, J=21.3 Hz, 6 H), 3.21 (spt, J=6.8 Hz, 1 H), 4.90 (s, 2 H), 7.17 (dd, J=1.4, 0.8 Hz, 1 H), 7.29 (d, J=1.5 Hz, 1 H), 8.14 (dd, J=5.9, 1.3 Hz, 1 H), 8.62 (d, J=5.7 Hz, 1 H), 8.85 (d, J=1.1 Hz, 1 H), 8.89 (br s, 1 H). |
| 88F | 1H NMR (400 MHz, CHLOROFORM-*d*) 1.41 (dd, J=6.8, 0.7 Hz, 7 H), 1.49 (d, J=6.4 Hz, 4 H), 3.20 (spt, J=6.8 Hz, 1 H), 3.32 (s, 3 H), 4.46 (q, J=6.5 Hz, 1 H), 4.91 (s, 2 H), 7.21 (d, J=1.3 Hz, 1 H), 7.25 (d, J=1.3 Hz, 1 H), 8.14 (dd, J=5.7, 1.3 Hz, 1 H), 8.62 (d, J=5.9 Hz, 1 H), 8.85 (d, J=1.1 Hz, 1 H), 8.97 (br s, 1 H). |

Example B - Pharmaceutical Compositions

A compound of the invention (for instance, a compound of the examples) is brought into association with a pharmaceutically acceptable carrier, thereby providing a pharmaceutical composition comprising such active compound. A therapeutically effective amount of a compound of the invention (e.g. a compound of the examples) is intimately mixed with a pharmaceutically acceptable carrier, in a process for preparing a pharmaceutical composition.

Example C - Biological Examples

The activity of a compound according to the present invention can be assessed by *in vitro* methods. A compound the invention exhibits valuable pharmacological properties, e.g. properties susceptible to inhibit NLRP3 activity, for instance as indicated the following test, and are therefore indicated for therapy related to NLRP3 inflammasome activity.

PBMC assay

Peripheral venous blood was collected from healthy individuals and human peripheral blood mononuclear cells (PBMCs) were isolated from blood by Ficoll-Histopaque (Sigma-Aldrich, A0561) density gradient centrifugation. After

isolation, PBMCs were stored in liquid nitrogen for later use. Upon thawing, PBMC cell viability was determined in growth medium (RPMI media supplemented with 10% fetal bovine serum, 1% Pen-Strep and 1% L-glutamine). Compounds were spotted in a 1:3 serial dilution in DMSO and diluted to the final concentration in 30 μl medium in 96 well plates (Falcon, 353072). PBMCs were added at a density of 7.5 × $10^4$ cells per well and incubated for 30 min in a 5% $CO_2$ incubator at 37 °C. LPS stimulation was performed by addition of 100 ng/ml LPS (final concentration, Invivogen, tlrl-smlps) for 6 hrs followed by collection of cellular supernatant and the analysis of IL-1β (μM) and TNF cytokines levels (μM) via MSD technology according to manufacturers' guidelines (MSD, K151A0H).

The $IC_{50}$ values (for IL-1β) and $EC_{50}$ values (TNF) were obtained on compounds of the invention/examples, and are depicted in the following table:

| Number | Compound | IL1β $IC_{50}$ (μM) | TNF $EC_{50}$ (μM) |
|---|---|---|---|
| **1F** | 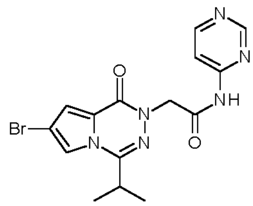 | 0.71 | >10 |
| **2F** | 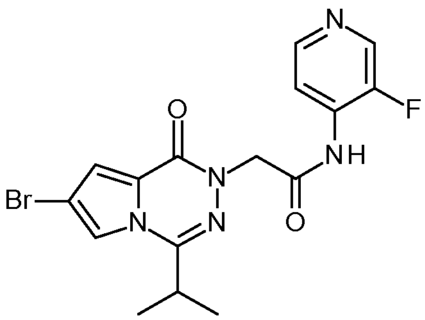 | 1.26 | >10 |
| **3F** | 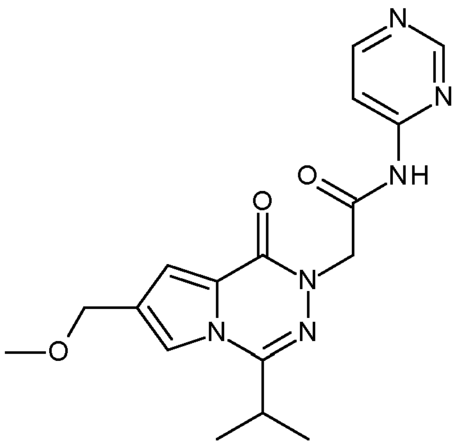 | 7.85 | >10 |
| **4F** | 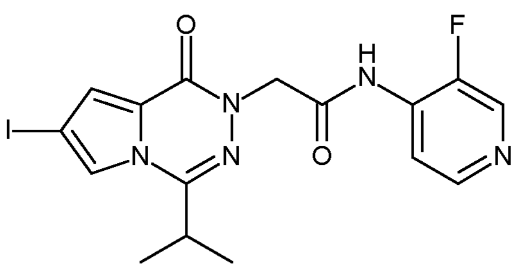 | 0.25 | >10 |

(continued)

| Number | Compound | IL1β $IC_{50}$ (μM) | TNF $EC_{50}$ (μM) |
|---|---|---|---|
| **5F** | 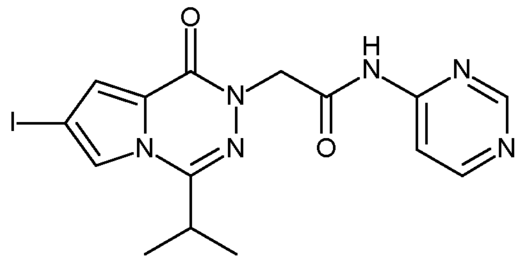 | 0.13 | >10 |
| **6F** | 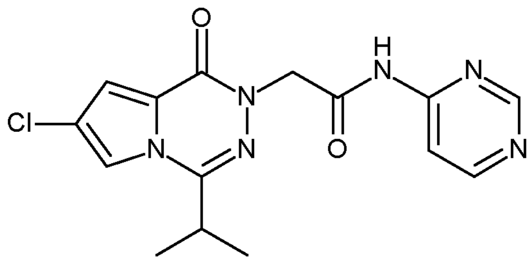 | 3.67 | >10 |
| **7F** | 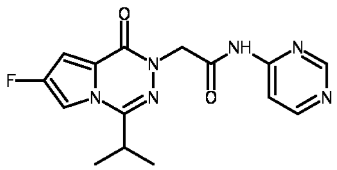 | >10 | >10 |
| **8F** | 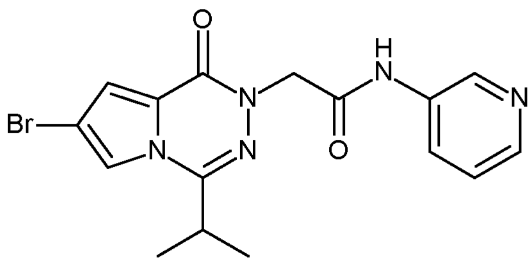 | 4.24 | >10 |
| **9F** | 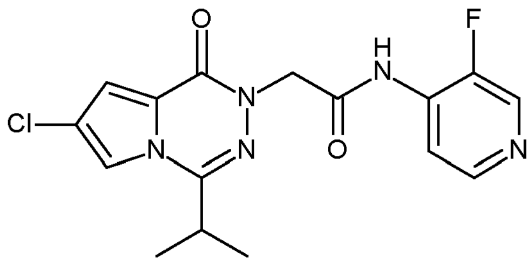 | 3.4 | >10 |
| **10F** | 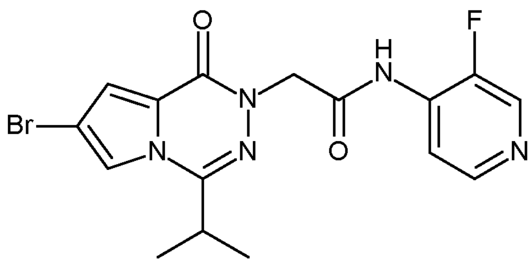 | 0.89 | >10 |

(continued)

| Number | Compound | IL1β $IC_{50}$ (μM) | TNF $EC_{50}$ (μM) |
|---|---|---|---|
| **11F** | 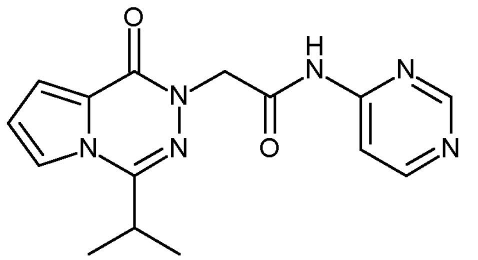 | >10 | >10 |
| **12F** | 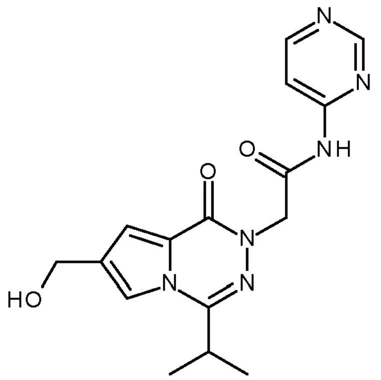 | 9.86 | >10 |
| **13F** | 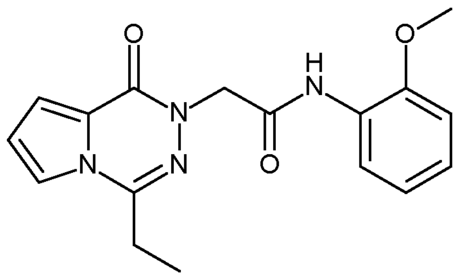 | >10 | >10 |
| **16F** | 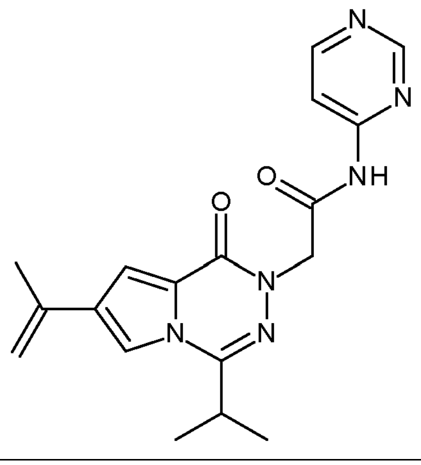 | 3.7 | >10 |

(continued)

| Number | Compound | IL1β $IC_{50}$ (μM) | TNF $EC_{50}$ (μM) |
|---|---|---|---|
| **17F** | 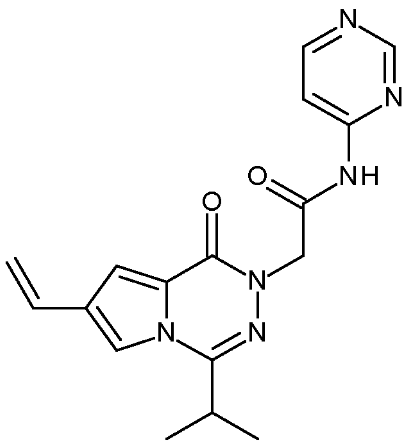 | 1.13 | >10 |
| **18F** | 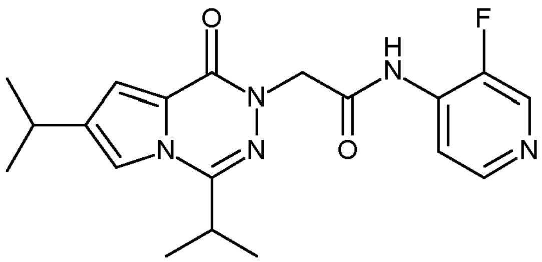 | 0.33 | >10 |
| **19F** | 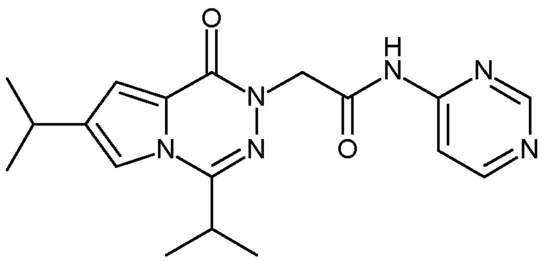 | 0.35 | >10 |
| **20F** | 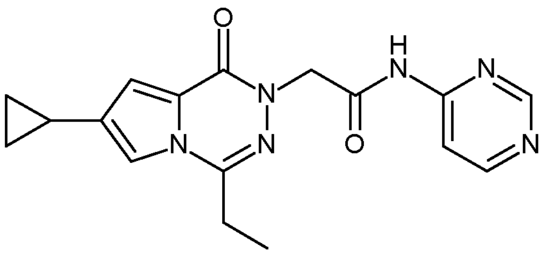 | 0.59 | >10 |
| **21F** | 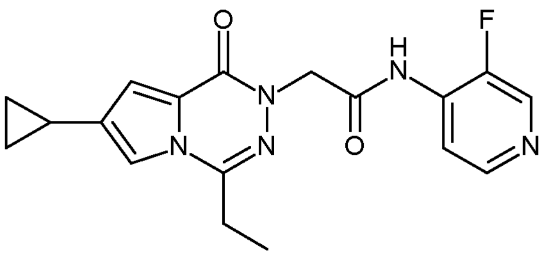 | 0.68 | >10 |

(continued)

| Number | Compound | IL1β $IC_{50}$ (μM) | TNF $EC_{50}$ (μM) |
|---|---|---|---|
| **22F** | 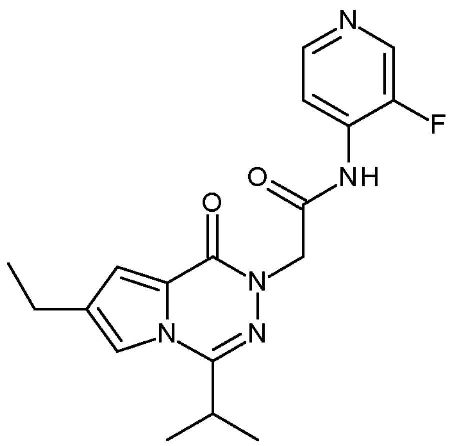 | 0.67 | >10 |
| **23F** | 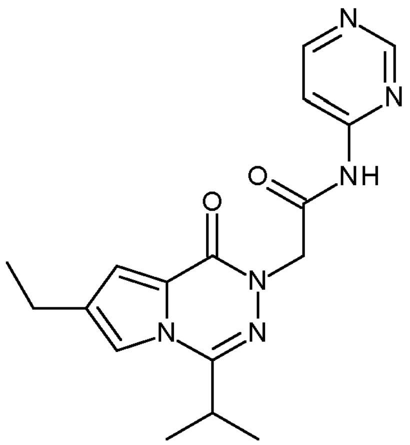 | 0.28 | >10 |
| **24F** | 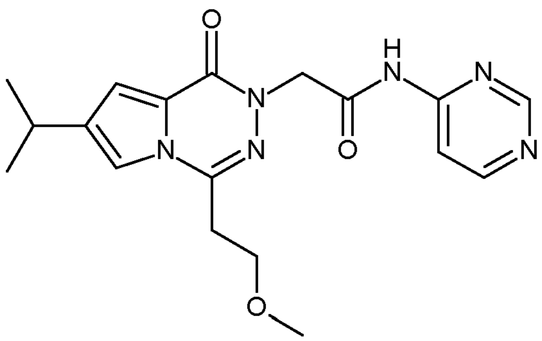 | 0.89 | >10 |

(continued)

| Number | Compound | IL1β $IC_{50}$ (μM) | TNF $EC_{50}$ (μM) |
|---|---|---|---|
| **25F** | 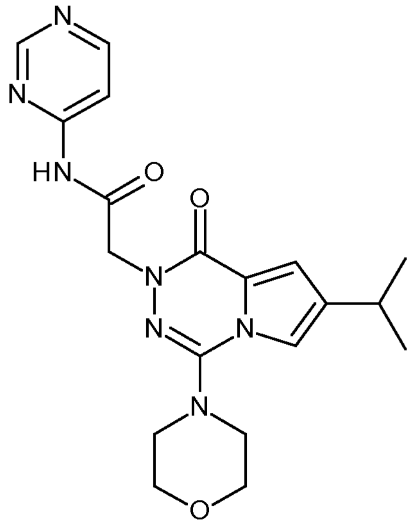 | >10 | >10 |
| **26F** | 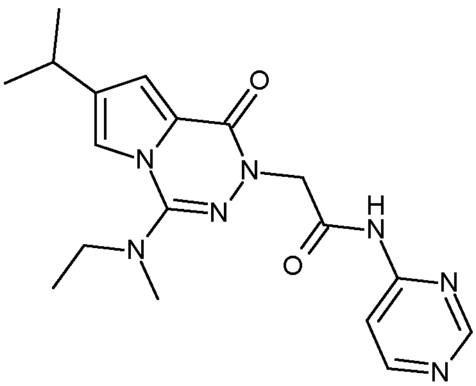 | 1.32 | >10 |
| **27F** | 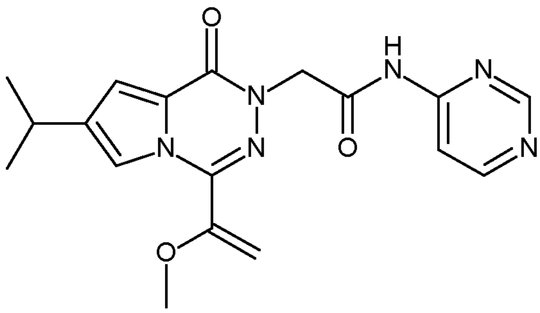 | 1.89 | >10 |
| **28F** | 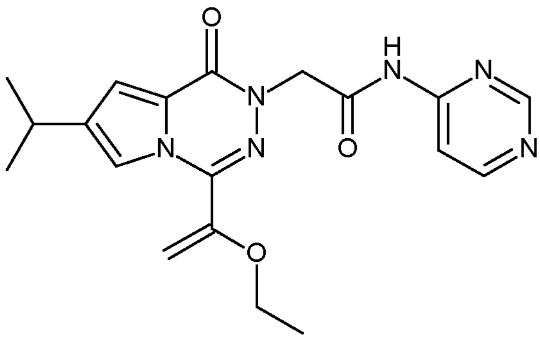 | 1.79 | >10 |

(continued)

| Number | Compound | IL1β $IC_{50}$ (μM) | TNF $EC_{50}$ (μM) |
|---|---|---|---|
| **29F** | 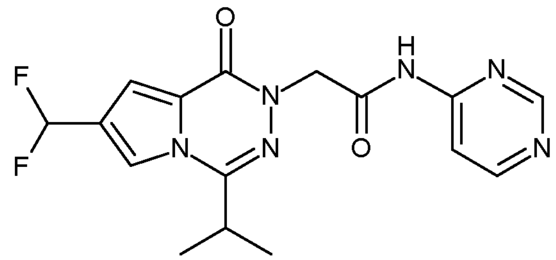 | 1.67 | >10 |
| **30F** | 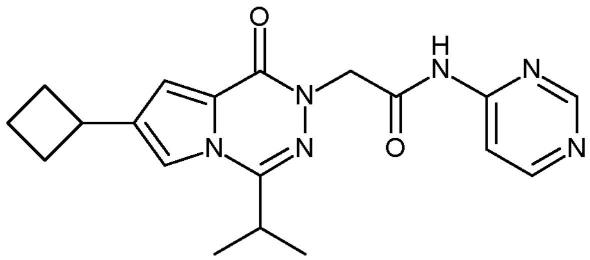 | 0.99 | >10 |
| **31F** | 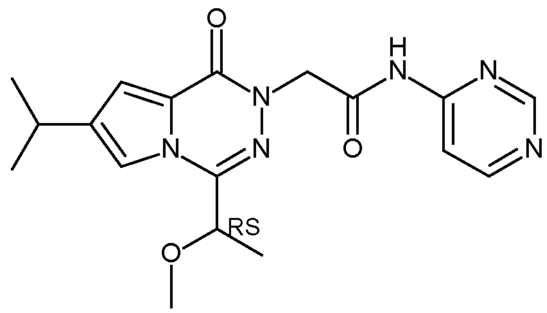 | 5.14 | >10 |
| **32F** | 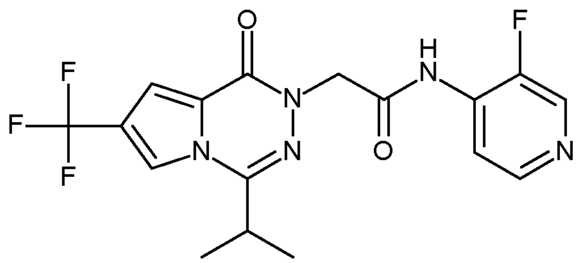 | 1.5 | >10 |
| **33F** | 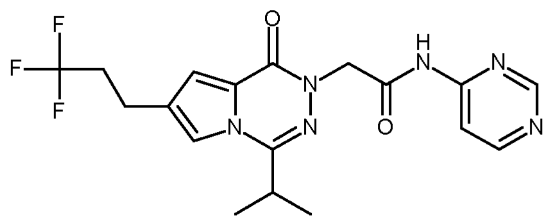 | 3.62 | >10 |

(continued)

| Number | Compound | IL1β $IC_{50}$ (μM) | TNF $EC_{50}$ (μM) |
|---|---|---|---|
| **34F** | 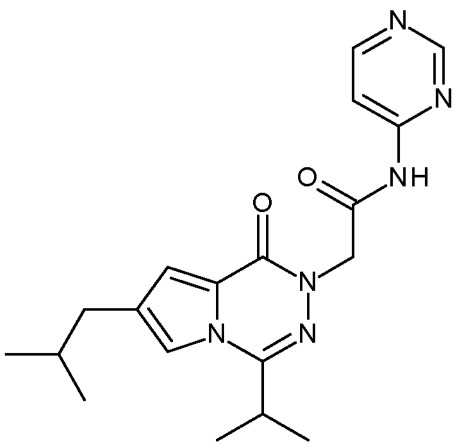 | 3.84 | >10 |
| **35F** | 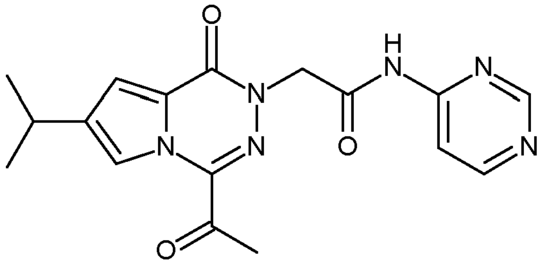 | >10 | >10 |
| **36F** | 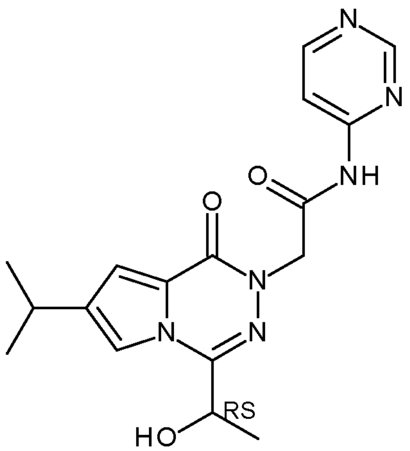 | 5.05 | >10 |
| **37F** | 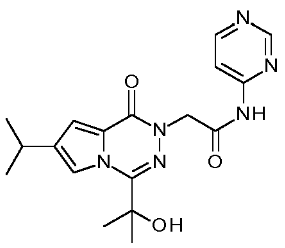 | 4.28 | >10 |

(continued)

| Number | Compound | IL1β $IC_{50}$ (μM) | TNF $EC_{50}$ (μM) |
|---|---|---|---|
| **38F** | 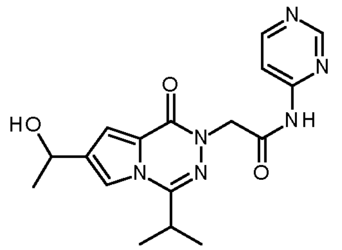 | 6.2 | >10 |
| **39F** | 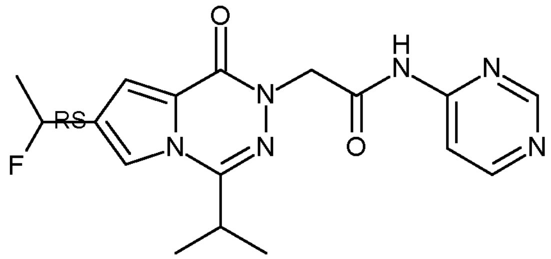 | 4.31 | >10 |
| **40F** | 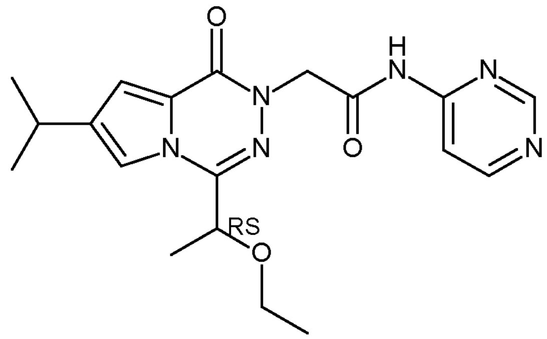 | 8.13 | >10 |
| **42F** | 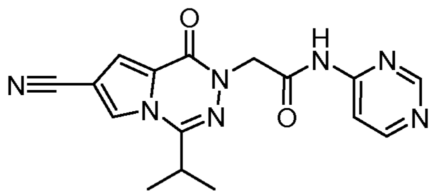 | >10 | >10 |
| **43F** | 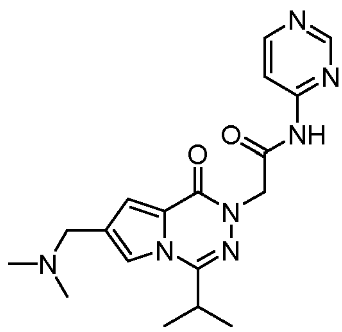 | >10 | >10 |

(continued)

| Number | Compound | IL1β $IC_{50}$ (μM) | TNF $EC_{50}$ (μM) |
|---|---|---|---|
| **44F** | 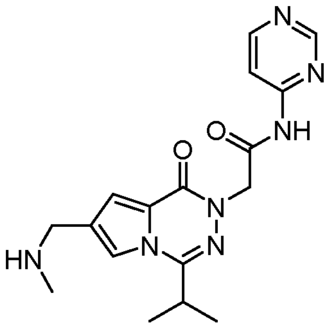 | >10 | >10 |
| **45F** | 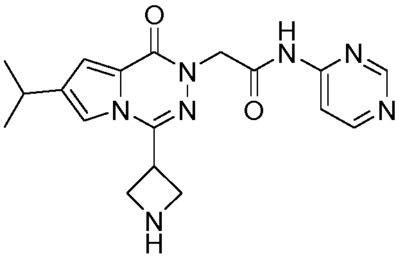 | >10 | >19.998 6<br>>10 |
| **46F** | 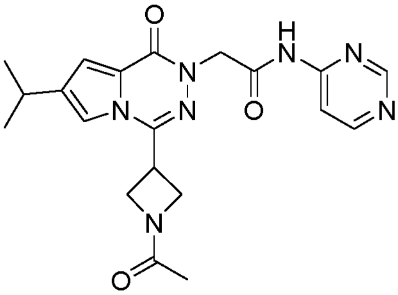 | >10 | >19.998 6<br>>10 |
| **47F** | 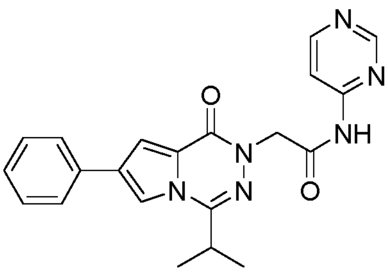 | >10 | >10 |
| **48F** | 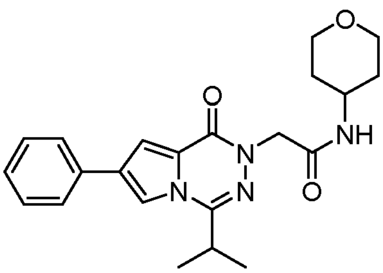 | >10 | >10 |

(continued)

| Number | Compound | IL1β $IC_{50}$ (μM) | TNF $EC_{50}$ (μM) |
|---|---|---|---|
| **49F** | 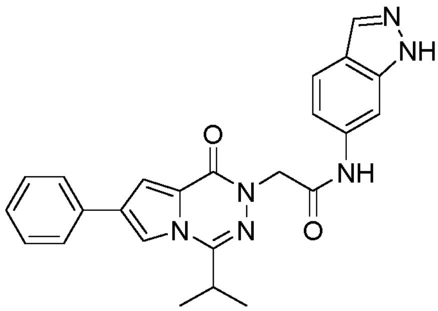 | | |
| **50F** | 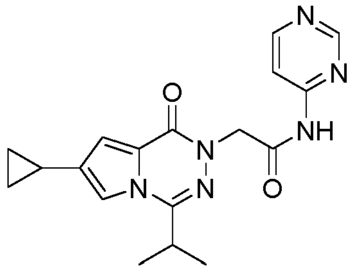 | -0.38 | >10 |
| **51F** | 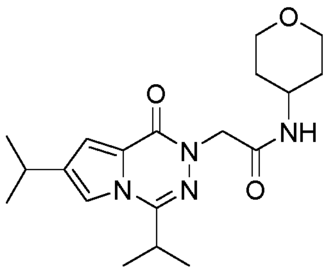 | -1.77 | >10 |
| **52F** | 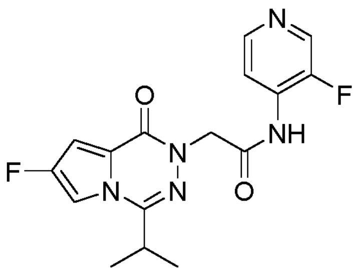 | >10 | >10 |
| **53F** | 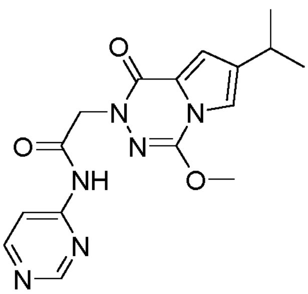 | 1.37<br>1.88 | >19.998 6<br>>10 |

(continued)

| Number | Compound | IL1β $IC_{50}$ (μM) | TNF $EC_{50}$ (μM) |
|---|---|---|---|
| **54F** | 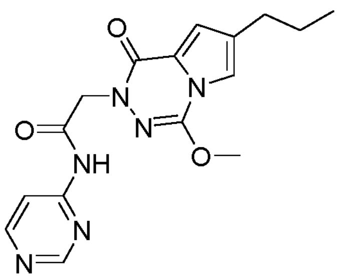 | 2.51 | >10 |
| **55F** | 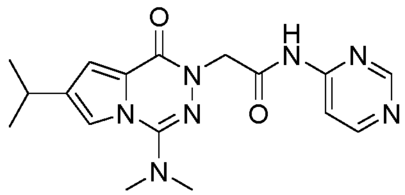 | 0.36 | >10 |
| **56F** | 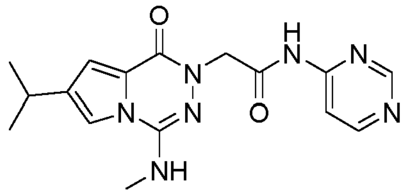 | 0.56 | >10 |
| **57F** | 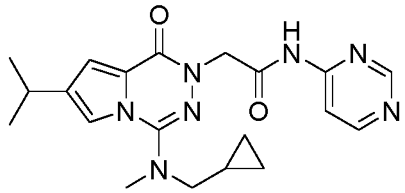 | 1.66 | >10 |
| **58F** | 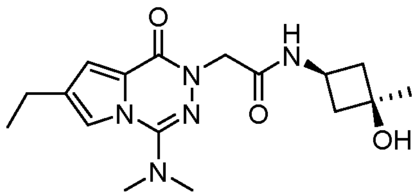 | 0.37 | >19.998 6 |
| **59F** | 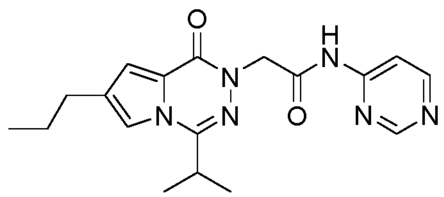 | 0.47 | >10 |
| **60F** | 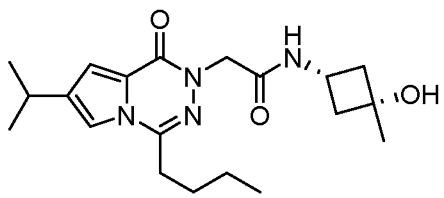 | 1.47 | >10 |

(continued)

| Number | Compound | IL1β $IC_{50}$ (μM) | TNF $EC_{50}$ (μM) |
|---|---|---|---|
| **61F** | 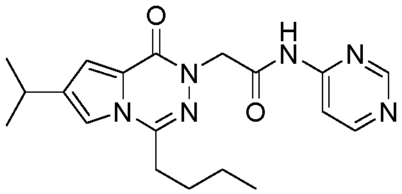 | 1.01 | >10 |
| **62F** | 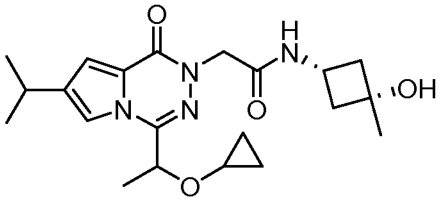 | 2.23 | >10 |
| **63F** | 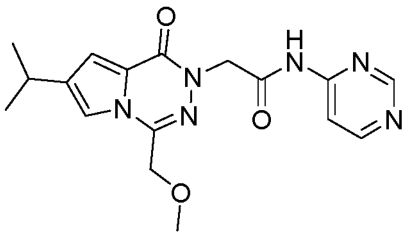 | 1.37 | >10 |
| **64F** | 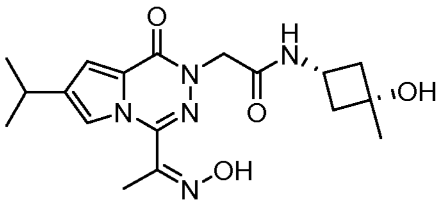 | 0.32 | >10 |
| **65F** | 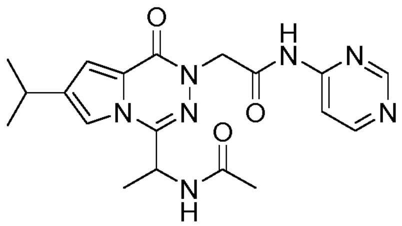 | 7.08<br>6.90 | >19.998 6<br>>10 |
| **66F** | 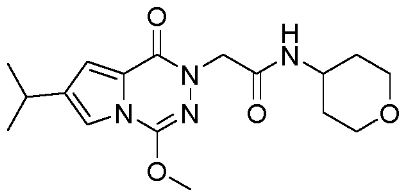 | 5.04 | >10<br>>10 |
| **67F** | 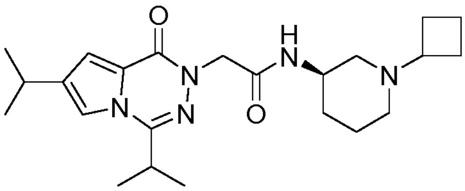 | 0.07 | >10 |

(continued)

| Number | Compound | IL1β $IC_{50}$ (μM) | TNF $EC_{50}$ (μM) |
|---|---|---|---|
| **68F** | 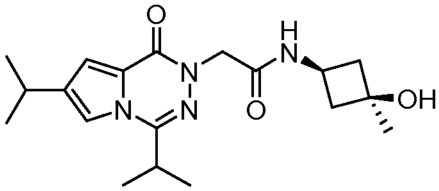 | 0.16 | >10 |
| **69F** | 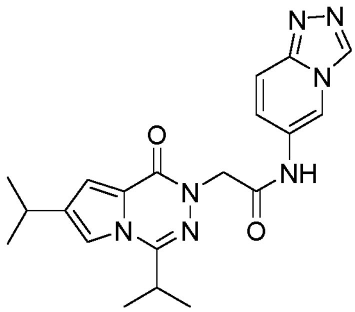 | 0.28 | >19.998 6 |
| **70F** | 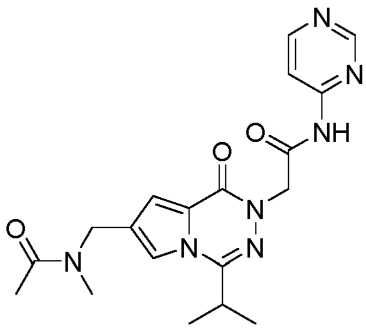 | >10 | >10 |
| **71F** | 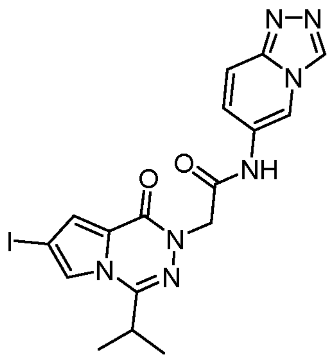 | 0.14<br>0.26 | 19.79<br>>10 |
| **72F** | 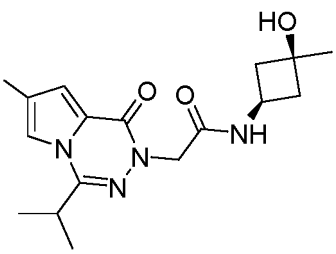 | 0.24 | >10 |

(continued)

| Number | Compound | IL1β $IC_{50}$ (μM) | TNF $EC_{50}$ (μM) |
|---|---|---|---|
| **73F** | 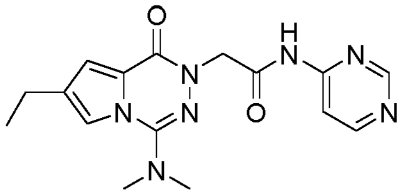 | 0.35 | >10 |
| **74F** | 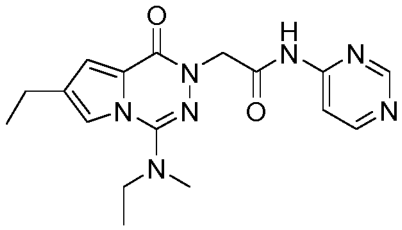 | 1.40 | >10 |
| **75F** | 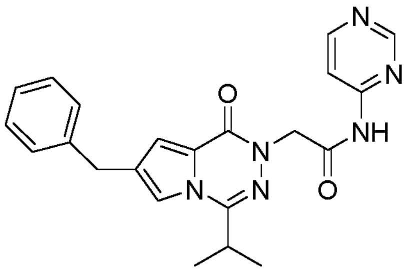 | >10 | >10 |
| **76F** | 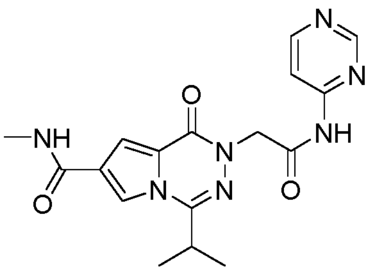 | >10 | >19.998 6<br>>10 |
| **77F** | 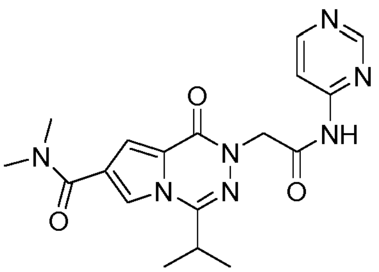 | >10 | >19.998 6<br>>10 |
| **78F** | 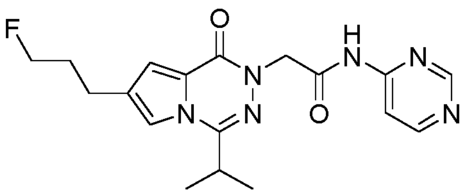 | 2.33 | >10 |

(continued)

| Number | Compound | IL1β $IC_{50}$ (μM) | TNF $EC_{50}$ (μM) |
|---|---|---|---|
| **79F** | 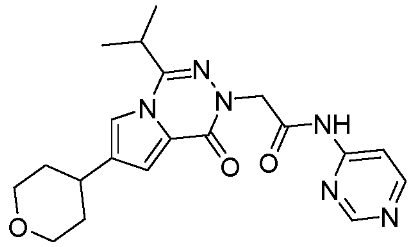 | >10 | >19.998 6<br>>10 |
| **80F** | 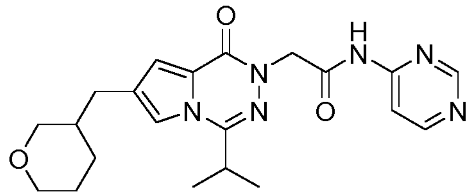 | >10 | >10 |
| **81F** | 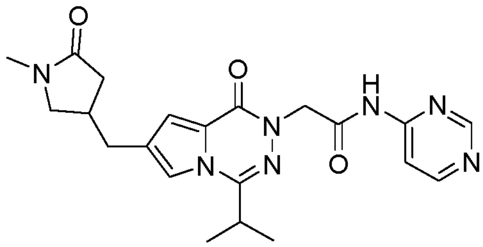 | >10 | >19.998 6 |
| **82F** | 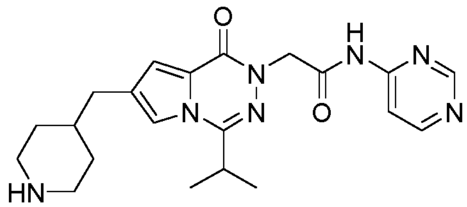 | >10 | >10 |
| **83F** | 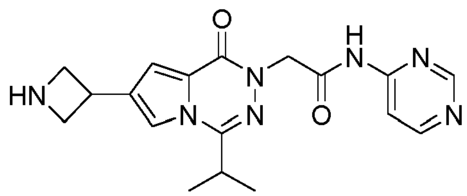 | >10 | >10 |
| **84F** | 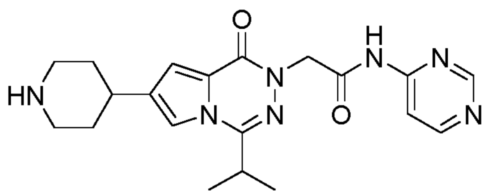 | >10 | >10 |
| **85F** | 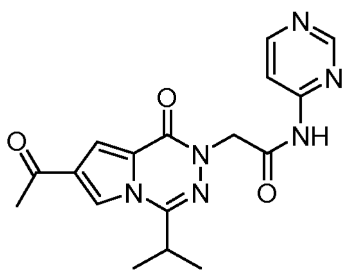 | >10 | >10 |

(continued)

| Number | Compound | IL1β $IC_{50}$ (μM) | TNF $EC_{50}$ (μM) |
|---|---|---|---|
| **86F** | 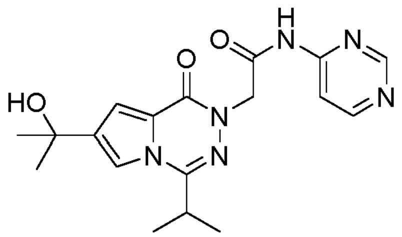 | >10 | >10 |
| **87F** | 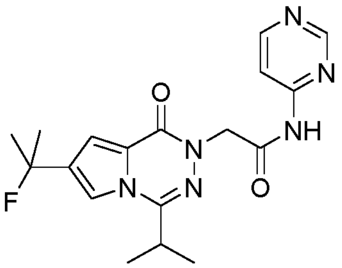 | 8.26 | >10 |
| **88F** | 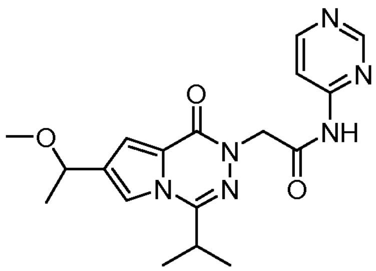 | 7.08<br><br>9.06 | >19.998 6<br><br>>10 |
| **89F** | 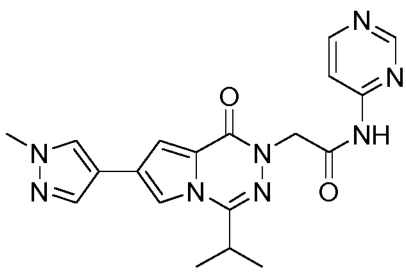 | >10 | >10 |
| **90F** | 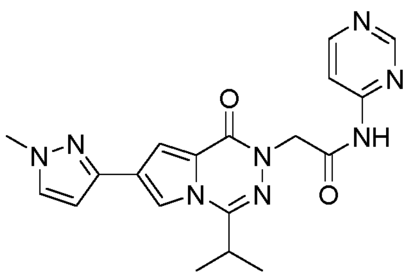 | >10 | >10 |

(continued)

| Number | Compound | IL1β $IC_{50}$ (μM) | TNF $EC_{50}$ (μM) |
|---|---|---|---|
| **91F** | 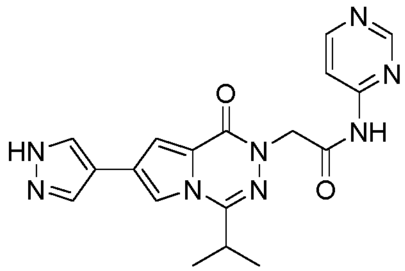 | >10 | >10 |
| **92F** | 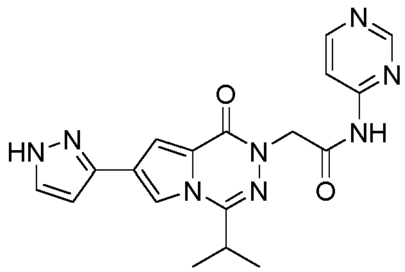 | >10 | >10 |
| **93F** | 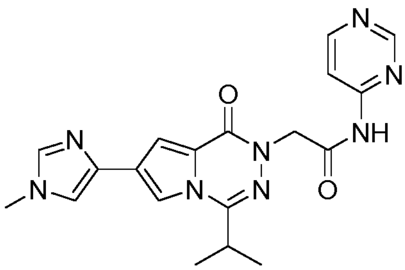 | >10 | >10 |
| **94F** | 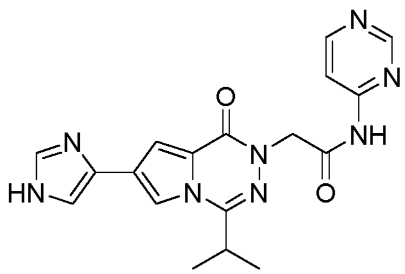 | >10 | >19.998 6<br>>10 |

Example D - Further Testing

One or more compound(s) of the invention (including compounds of the final examples) is/are tested in a number of other methods to evaluate, amongst other properties, permeability, stability (including metabolic stability and blood stability) and solubility.

**Permeability test**

The *in vitro* passive permeability and the ability to be a transported substrate of P-glycoprotein (P-gp) is tested using MDCKcells stably transduced with MDR1 (this may be performed at a commercial organisaiton offering ADME, PK services, e.g. Cyprotex). Permeability experiments are conducted in duplicate at a single concentration (5 μM) in a transwell system with an incubation of 120 min. The apical to basolateral (AtoB) transport in the presence and absence of the P-gp inhibitor GF120918 and the basolateral to apical (BtoA) transport in the absence of the P-gp inhibitor is measured and permeation rates (Apparent Permeability) of the test compounds ($P_{app}$ $\times 10^{-6}$ cm/sec) are calculated.

**Metabolic stability test in liver microsomes**

The metabolic stability of a test compound is tested (this may be performed at a commercial organisaiton offering ADME, PK services, e.g. Cyprotex) by using liver microsomes (0.5 mg/ml protein) from human and preclinical species incubated up to 60 minutes at 37°C with 1 μM test compound.

The *in vitro* metabolic half-life ($t_{1/2}$ ) is calculated using the slope of the log-linear regression from the percentage parent compound remaining versus time relationship (κ),

$$t_{1/2} = - \ln(2)/ \kappa.$$

The *in vitro* intrinsic clearance ($Cl_{int}$) (ml/min/mg microsomal protein) is calculated using the following formula:

$$Cl_{int} = \frac{0.693}{t_{1/2}} \times \frac{V_{inc}}{W_{mic\,prot,inc}}$$

Where:

$V_{inc}$ = incubation volume,
Wmic prot,inc = weight of microsomal protein in the incubation.

**Metabolic stability test in liver hepatocytes**

The metabolic stability of a test compound is tested using liver hepatocytes (1 milj cells) from human and preclinical species incubated up to 120 minutes at 37°C with 1 μM test compound.

The in vitro metabolic half-life ($t_{1/2}$) is calculated using the slope of the log-linear regression from the percentage parent compound remaining versus time relationship (κ), $t_{1/2} = - \ln(2)/ \kappa$.

The *in vitro* intrinsic clearance ($Cl_{int}$) (μl/min/million cells) is calculated using the following formula:

$$Cl_{int} = \frac{0.693}{t_{1/2}} \times \frac{V_{inc}}{\#\, cells_{inc}}\; x\; 1000$$

Where:

$V_{inc}$ = incubation volume,
# $cells_{inc}$ = number of cells ($\times 10^6$) in the incubation

**Solubility test**

The test/assay is run in triplicate and is semi-automated using the Tecan Fluent for all liquid handling with the following general steps:

- 20μl of 10mM stock solution is dispensed in a 500μl 96 well plate
- DMSO is evaporated (Genevac)
- a stir bar and 400μl of buffer/biorelevant media is added
- the solution is stirred for 72h (pH2 and pH7) or 24h (FaSSIF and FeSSIF)
- the solution is filtered
- the filtrate is quantified by UPLC/UV using a three-points calibration curve

The LC conditions are:
- Waters Acquity UPLC

  - Mobile phase A: 0.1% formic acid in H2O, B: 0.1% formic acid in CH3CN
  - Column: Waters HSS T3 1.8μm 2.1x50mm
  - Column temp.: 55°C

(continued)

- Inj.vol.: 2μl
- Flow: 0.6ml/min
- Wavelength UV: 250_350nm
- Gradient: 0min: 0%B, 0.3min: 5%B, 1.8min: 95%B, 2.6min: 95%B

**Blood Stability assay**

The compound of the invention/examples is spiked at a certain concentration in plasma or blood from the agreed preclinical species; then after incubating to predetermined times and conditions (37°C, 0°C (ice) or room temperature) the concentration of the test compound in the blood or plasma matrix with LCMS/MS can then be determined.

## Claims

**1.** A compound of Formula (I)

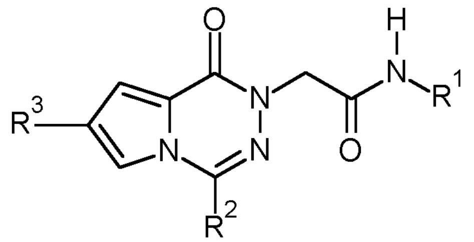

(I)

or a pharmaceutically acceptable salt thereof, wherein:

$R^1$ represents:

(i) $C_{3-6}$ cycloalkyl optionally substituted with one or more substituents independently selected from -OH and $-C_{1-3}$ alkyl;
(ii) aryl or heteroaryl, each of which is optionally substituted with 1 to 3 substituents independently selected from halo, -OH, $-O-C_{1-3}$ alkyl, $-C_{1-3}$ alkyl, halo$C_{1-3}$alkyl, hydroxy$C_{1-3}$ alkyl, halo$C_{1-3}$alkoxy; or
(iii) heterocyclyl, optionally substituted with 1 to 3 substituents independently selected from $C_{1-3}$ alkyl and $C_{3-6}$ cycloalkyl;

$R^2$ represents:

(i) $C_{1-6}$ alkyl optionally substituted with one or more substituents independently selected from halo, -OH, $-OC_{1-3}$alkyl, $-OC_{3-6}$ cycloalkyl, $-N(H)C(O)C_{1-3}$alkyl and oxo;
(ii) $C_{3-6}$ cycloalkyl;
(iii) $C_{2-4}$alkenyl optionally substituted with $-O-C_{1-3}$alkyl;
(iv) $-O-C_{1-3}$alkyl;
(v) $-C(O)C_{1-3}$alkyl;
(vi)$-N(H)C_{1-3}$alkyl or $-N-(C_{1-3}alkyl)_2$, where each alkyl may be optionally substituted with $-OC_{1-3}$ alkyl or $C_{3-6}$ cycloalkyl; or
(vii) heterocyclyl (optionally substituted by one or more substituents selected from halo, $C_{1-3}$ alkyl, $-C(O)C_{1-3}$alkyl and $-C(O)OC_{1-4}$alkyl);

$R^3$ represents:

(i) hydrogen;
(ii) halo or -CN;
(iii) $C_{1-4}$ alkyl optionally substituted with one or more substituents independently selected from halo, -OH, oxo, $-O-C_{1-3}$alkyl, -C(O)OH, -C(O)N(H)heteroaryl, -C(O)N(H)aryl, $-C(O)N(H)C_{1-3}$ alkyl, $-C(O)N(C_{1-3}alkyl)_2$,

-N($C_{1-3}$ alkyl)-C(O)-$C_{1-3}$alkyl, -N(H)$C_{1-3}$ alkyl, heterocyclyl, aryl and heteroaryl, which latter three groups may themselves be optionally substituted by one or more substituents selected from halo, $C_{1-3}$ alkyl and, where applicable, =O;
(iv) $C_{2-4}$ alkenyl;
(v) $C_{3-6}$ cycloalkyl;
(vi) -$OC_{1-4}$ alkyl;
(vii) -C(O)H or -C(O)$C_{1-3}$ alkyl;
(viii) -N(H)$C_{1-3}$alkyl or - N($C_{1-3}$alkyl)$_2$;
(ix) -C(O)N(H)$C_{1-3}$alkyl or -C(O)N($C_{1-3}$alkyl)$_2$;
(x) aryl or heteroaryl, which groups may themselves be optionally substituted by one or more substituents selected from halo, $C_{1-3}$ alkyl, -C(O)$OC_{1-4}$alkyl and, where applicable, =O; or
(xi) Heterocyclyl, optionally substituted by one or more substituents selected from halo, $C_{1-3}$ alkyl, -C(O)$OC_{1-4}$alkyl and, where applicable, =O,

provided that:

(i) when $R^2$ represents -$CH_3$, $R^3$ represents H, then $R^1$ does not represent cyclohexyl, 2,3-dimethylcyclohexyl, 2-methoxyphenyl, 2,3,5,6-tetrafluorophenyl, 2-bromo-4,6-difluorophenyl, 4-methylphenyl, 4-(diethylamino)-2-methylphenyl, 5-chloro-2-methoxyphenyl, 2,5-difluorophenyl, 2,3-dihydro-1,4-benzodioxin-6-yl, 4-methylcyclohexyl, 1,2,3,4-tetrahydro-1-naphthalenyl, 3-ethylphenyl, 4-isopropylphenyl, 2-ethyl-6-methylphenyl, 4-bromo-3-methylphenyl, 3,5-dimethylphenyl, 2,4,6-trimethylphenyl, 3,4-dimethylphenyl, 2-chloro-4-methylphenyl, 2,4-dimethylphenyl, 2,3,4,5,6-pentafluorophenyl, 2,5-dimethylphenyl, 3-chloro-2-methylphenyl, 2-methylphenyl, 4-bromo-2-fluorophenyl, 4-fluorophenyl, 2-fluorophenyl, 4-ethylphenyl, 2-ethylphenyl, 2,6-dimethylphenyl, 2,3-dimethylphenyl, 2,4-difluorophenyl, cyclopentyl, cycloheptyl, 5-chloro-2-methylphenyl, 5-chloro-2,4-dimethoxyphenyl, 4-chlorophenyl, 2-chlorophenyl, 2-bromophenyl, 3-methoxyphenyl, 3,4-dimethoxyphenyl, 3-(trifluoromethyl)phenyl or 2-(trifluoromethyl)phenyl;
(ii) when $R^2$ represents -$CH_2CH_3$, $R^3$ represents H, then $R^1$ does not represent cyclopentyl or cycloheptyl.

**2.** The compound of claim 1 wherein:

$R^1$ represents:

(i) $C_{3-6}$ cycloalkyl optionally substituted with one or more substituents independently selected from -OH and -$C_{1-3}$ alkyl;
(ii) aryl or heteroaryl, each of which is optionally substituted with 1 to 3 substituents independently selected from halo, -OH, -O-$C_{1-3}$ alkyl, -$C_{1-3}$ alkyl, halo$C_{1-3}$alkyl, hydroxy$C_{1-3}$ alkyl, halo$C_{1-3}$alkoxy; or
(iii) heterocyclyl, optionally substituted with 1 to 3 substituents independently selected from $C_{1-3}$ alkyl and $C_{3-6}$ cycloalkyl;

$R^2$ represents:

(i) $C_{1-3}$ alkyl optionally substituted with one or more substituents independently selected from halo, -OH, -$OC_{1-3}$alkyl and oxo;
(ii) $C_{3-6}$cycloalkyl;
(iii) $C_{2-4}$alkenyl optionally substituted with -O-$C_{1-3}$alkyl;
(iv) -O-$C_{1-3}$alkyl;
(v) -N(H)alkyl or -N-($C_{1-3}$alkyl)$_2$, where each alkyl may be optionally substituted with -$OC_{1-3}$ alkyl; or
(vi) heterocyclyl;

$R^3$ represents:

(i) hydrogen;
(ii) halo;
(iii) $C_{1-4}$ alkyl optionally substituted with one or more substituents independently selected from halo, -OH, oxo, -O-$C_{1-3}$alkyl, -C(O)OH, -C(O)N(H)heteroaryl, -C(O)N(H)aryl, -C(O)N(H)$C_{1-3}$ alkyl and -C(O)N($C_{1-3}$alkyl)$_2$;
(iv) $C_{2-4}$ alkenyl;

(v) $C_{3-6}$ cycloalkyl;
(vi) $-OC_{1-4}$ alkyl;
(vii) $-N(H)C_{1-3}$alkyl or - $N(C_{1-3}alkyl)_2$;
(viii) $-C(O)N(H)C_{1-3}$alkyl or $-C(O)N(C_{1-3}alkyl)_2$;
(ix) aryl or heteroaryl; or
(x) heterocyclyl.

provided that:

(i) when $R^2$ represents $-CH_3$, $R^3$ represents H, then $R^1$ does not represent cyclohexyl, 2,3-dimethylcyclohexyl, 2-methoxyphenyl, 2,3,5,6-tetrafluorophenyl, 2-bromo-4,6-difluorophenyl, 4-methylphenyl, 4-(diethylamino)-2-methylphenyl, 5-chloro-2-methoxyphenyl, 2,5-difluorophenyl, 2,3-dihydro-1,4-benzodioxin-6-yl, 4-methylcyclohexyl, 1,2,3,4-tetrahydro-1-naphthalenyl, 3-ethylphenyl, 4-isopropylphenyl, 2-ethyl-6-methylphenyl, 4-bromo-3-methylphenyl, 3,5-dimethylphenyl, 2,4,6-trimethylphenyl, 3,4-dimethylphenyl, 2-chloro-4-methylphenyl, 2,4-dimethylphenyl, 2,3,4,5,6-pentafluorophenyl, 2,5-dimethylphenyl, 3-chloro-2-methylphenyl, 2-methylphenyl, 4-bromo-2-fluorophenyl, 4-fluorophenyl, 2-fluorophenyl, 4-ethylphenyl, 2-ethylphenyl, 2,6-dimethylphenyl, 2,3-dimethylphenyl, 2,4-difluorophenyl, cyclopentyl, cycloheptyl, 5-chloro-2-methylphenyl, 5-chloro-2,4-dimethoxyphenyl, 4-chlorophenyl, 2-chlorophenyl, 2-bromophenyl, 3-methoxyphenyl, 3,4-dimethoxyphenyl, 3-(trifluoromethyl)phenyl, 2-(trifluoromethyl)phenyl or 2,4-di($-C(O)OCH_3$)phenyl (or 2,4-(dicarboxylic acid methyl ester)-phenyl);
(ii) when $R^2$ represents $-CH_2CH_3$, $R^3$ represents H, then $R^1$ does not represent cyclopentyl or cycloheptyl.

**3.** The compound of claim 1 or claim 2, wherein $R^1$ represents $C_{3-6}$ cycloalkyl optionally substituted by one or two substituents selected from $C_{1-3}$ alkyl and -OH.

**4.** The compound of claim 1 or claim 2, wherein $R^1$ represents a mono-cyclic 5- or 6-membered heterocyclyl group containing at least one nitrogen heteroatom, and which is optionally substituted by one substituent selected from $C_{1-3}$ alkyl and $C_{3-6}$ cycloalkyl.

**5.** The compound of claim 1 or claim 2, wherein $R^1$ represents: (i) phenyl; (ii) a 5- or 6-membered mono-cyclic heteroaryl group; or (iii) a 9- or 10-membered bicyclic heteroaryl group, all of which are optionally substituted with one or two substituent(s) selected from halo, -OH and $-OC_{1-3}$ alkyl.

**6.** The compound of claim 1 or claim 2, wherein $R^1$ represents cyclopropyl, as defined in claim 4 or claim 5,

**7.** The compound of any one of claims 1 to 6, wherein $R^2$ represents (i) $C_{1-3}$ alkyl optionally substituted by one or two substituent(s) selected from -OH, methoxy, ethoxy and oxo ; (ii) $C_{2-4}$alkenyl optionally substituted with methoxy or ethoxy; (iii) $-N-(C_{1-3}alkyl)z$, where the alkyl moiety is unsubstituted; (iv) a 6-membered heterocyclyl group in which there is at least one nitrogen heteroatom and optionally an oxygen heteroatom.

**8.** The compound of claim 7, wherein $R^2$ represents unsubstituted $C_{1-3}$ alkyl.

**9.** The compound of any of claims 1 to 8, wherein $R^3$ represents: (i) hydrogen; (ii) halo; (iii) $C_{1-4}$ alkyl optionally substituted by one or more substituent selected from fluoro, - OH and $-O-C_{1-2}$ alkyl; (iv) $C_{2-4}$ alkenyl; or (v) $C_{3-4}$ cycloalkyl.

**10.** A pharmaceutical composition comprising a therapeutically effective amount of a compound as defined in any one of claims 1 to 9, and a pharmaceutically acceptable carrier.

**11.** A process for preparing a pharmaceutical composition as defined in claim 10, **characterized in that** a pharmaceutically acceptable carrier is intimately mixed with a therapeutically effective amount of a compound as defined in any one of claims 1 to 9 .

**12.** A compound as claimed in any one of claims 1 to 9, for use as a pharmaceutical or medicament.

**13.** The compound, composition or combination for use according to claim 12, wherein the disease or disorder associated with inhibition of NLRP3 inflammasome activity is selected from inflammasome related diseases and disorders, immune diseases, inflammatory diseases, auto-immune diseases, auto-inflammatory fever syndromes, cryopyrin-associated periodic syndrome, chronic liver disease, viral hepatitis, nonalcoholic steatohepatitis, alcoholic steatohepatitis, alcoholic liver disease, inflammatory arthritis related disorders, gout, chondrocalcinosis, osteoarthritis, rheumatoid arthritis, chronic arthropathy, acute arthropathy, kidney related disease, hyperoxaluria, lupus nephritis, Type I and Type II diabetes, nephropathy, retinopathy, hypertensive nephropathy, hemodialysis related inflammation, neuroinflammation-related diseases, multiple sclerosis, brain infection, acute injury, neurodegenerative diseases, Alzheimer's disease, cardiovascular diseases, metabolic diseases, cardiovascular risk reduction, hypertension, atherosclerosis, peripheral artery disease, acute heart failure, inflammatory skin diseases, acne, wound healing and scar formation, asthma, sarcoidosis, age-related macular degeneration, colon cancer, lung cancer, myeloproliferative neoplasms, leukemias, myelodysplastic syndromes and myelofibrosis.

**14.** A process for the preparation of a compound of formula (I) as claimed in any of claims 1 to 9, which comprises:

(i) reaction of a compound of formula (II),

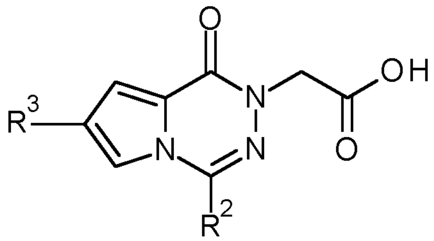

(II)

or a derivative thereof, wherein $R^2$ and $R^3$ are as defined in claim 1, with a compound of formula (III),

$H_2N\text{-}R^1$ (III)

or a derivative thereof, wherein $R^1$ is as defined in claim 1, under amideforming reaction conditions;

(ii) reaction of a compound of formula (IV),

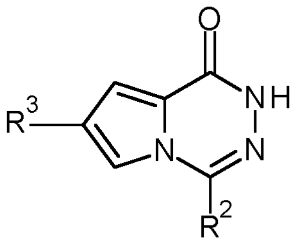

(IV)

wherein $R^2$ and $R^3$ are as defined in claim 1, with a compound of formula (V),

$LG^a\text{-}CH_2\text{-}C(O)\text{-}N(H)R^1$ (V)

wherein $LG^a$ represents a suitable leaving group and $R^1$ is as defined in claim 1;
(iii) by transformation of a certain compound of formula (I) into another.

**Patentansprüche**

**1.** Verbindung von Formel (I)

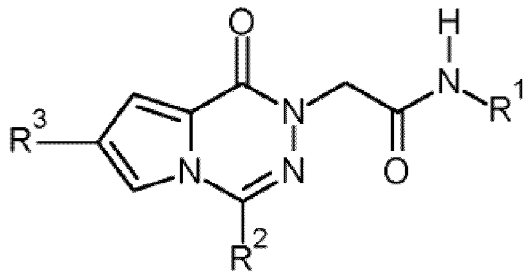

(I)

oder ein pharmazeutisch verträgliches Salz davon, worin:

$R^1$ steht für:

(i) $C_{3-6}$-Cycloalkyl, optional substituiert mit einem oder mehreren Substituenten, unabhängig ausgewählt aus -OH und -$C_{1-3}$-Alkyl;
(ii) Aryl oder Heteroaryl, jeweils optional substituiert mit 1 bis 3 Substituenten, unabhängig ausgewählt aus Halogen, -OH, -O-$C_{1-3}$-Alkyl, -$C_{1-3}$-Alkyl, Halogen-$C_{1-3}$-Alkyl, Hydroxy-$C_{1-3}$-Alkyl, Halogen-$C_{1-3}$-Alkoxy; oder
(iii) Heterocyclyl, optional substituiert mit 1 bis 3 Substituenten, unabhängig ausgewählt aus $C_{1-3}$-Alkyl und $C_{3-6}$-Cycloalkyl;

$R^2$ steht für:

(i) $C_{1-6}$-Alkyl, optional substituiert mit einem oder mehreren Substituenten, unabhängig ausgewählt aus Halogen, -OH, -O$C_{1-3}$-Alkyl, -O$C_{3-6}$-Cycloalkyl, -N(H)C(O)$C_{1-3}$-Alkyl und Oxo;
(ii) $C_{3-6}$-Cycloalkyl;
(iii) $C_{2-4}$-Alkenyl optional substituiert mit -O-$C_{1-3}$-Alkyl;
(iv) -O-$C_{1-3}$-Alkyl;
(v) -C(O)$C_{1-3}$-Alkyl;
(vi) -N(H)$C_{1-3}$-Alkyl oder -N-($C_{1-3}$-Alkyl)$_2$, wobei jedes Alkyl optional durch -O$C_{1-3}$-Alkyl oder $C_{3-6}$-Cycloalkyl substituiert sein kann; oder
(vii) Heterocyclyl (optional substituiert durch einen oder mehrere Substituenten ausgewählt aus Halogen, $C_{1-3}$-Alkyl, -C(O)$C_{1-3}$-Alkyl und -C(O)O$C_{1-4}$-Alkyl);

$R^3$ steht für:

(i) Wasserstoff;
(ii) Halogen oder -CN;
(iii) $C_{1-4}$-Alkyl, optional substituiert mit einem oder mehreren Substituenten, unabhängig ausgewählt aus Halogen, -OH, Oxo, -O-$C_{1-3}$-Alkyl, -C(O)OH, -C(O)N(H)Heteroaryl, -C(O)N(H)Aryl, -C(O)N(H)$C_{1-3}$-Alkyl, -C(O)N($C_{1-3}$-Alkyl)$_2$, -N($C_{1-3}$-Alkyl)-C(O)-$C_{1-3}$ Alkyl, -N(H)$C_{1-3}$-Alkyl, Heterocyclyl, Aryl und Heteroaryl, wobei die letzten drei Gruppen selbst optional durch einen oder mehrere Substituenten, ausgewählt aus Halogen, $C_{1-3}$-Alkyl und optional =O, substituiert sein können;
(iv) $C_{2-4}$-Alkenyl;
(v) $C_{3-6}$-Cycloalkyl;
(vi) -O$C_{1-4}$-Alkyl;
(vii) -C(O)H oder -C(O)$C_{1-3}$-Alkyl;
(viii) -N(H)$C_{1-3}$-Alkyl oder -N($C_{1-3}$-Alkyl)$_2$;
(ix) -C(O)N(H)$C_{1-3}$-Alkyl oder -C(O)N($C_{1-3}$-Alkyl)$_2$;
(x) Aryl oder Heteroaryl, wobei diese Gruppen selbst gegebenenfalls durch einen oder mehrere Substituenten substituiert sein können, ausgewählt aus Halogen, $C_{1-3}$-Alkyl, -C(O)O$C_{1-4}$-Alkyl und gegebenenfalls =0; oder
(xi) Heterocyclyl, optional substituiert durch einen oder mehrere Substituenten ausgewählt aus Halogen, $C_{1-3}$-Alkyl, -C(O)O$C_{1-4}$-Alkyl und gegebenenfalls =O,

unter der Voraussetzung, dass:

(i) wenn $R^2$ für $-CH_3$ steht, $R^3$ für H steht, dann steht $R^1$ nicht für Cyclohexyl, 2,3-Dimethylcyclohexyl, 2-Methoxyphenyl, 2,3,5,6-Tetrafluorphenyl, 2-Brom-4,6-difluorphenyl, 4-Methylphenyl, 4-(Diethylamino)-2-methylphenyl, 5-Chlor-2-methoxyphenyl, 2,5-Difluorphenyl, 2,3-Dihydro-1 ,4-benzodioxin-6-yl, 4-Methylcyclohexyl, 1,2,3,4-Tetrahydro-1-naphthalenyl, 3-Ethylphenyl, 4-Isopropylphenyl, 2-Ethyl-6-methylphenyl, 4-Brom-3-methylphenyl, 3,5-Dimethylphenyl, 2,4,6-Trimethylphenyl, 3,4-Dimethylphenyl, 2-Chlor-4-methylphenyl, 2,4-Dimethylphenyl, 2,3,4,5,6-Pentafluorphenyl, 2,5-Dimethylphenyl, 3-Chlor-2-methylphenyl, 2-Methylphenyl, 4-Brom-2-fluorphenyl, 4-Fluorphenyl, 2-Fluorphenyl, 4-Ethylphenyl, 2-Ethylphenyl, 2,6-Dimethylphenyl, 2,3-Dimethylphenyl, 2,4-Difluorphenyl, Cyclopentyl, Cycloheptyl, 5-Chlor-2-methylphenyl, 5-Chlor-2,4-dimethoxyphenyl, 4-Chlorphenyl, 2-Chlorphenyl, 2-Bromphenyl, 3-Methoxyphenyl, 3,4-Dimethoxyphenyl, 3-(Trifluormethyl)phenyl oder 2-(Trifluormethyl)phenyl;
(ii) wenn $R^2$ für $-CH_2CH_3$ steht, $R^3$ für H steht, dann steht $R^1$ nicht für Cyclopentyl oder Cycloheptyl.

**2.** Verbindung nach Anspruch 1
worin:

$R^1$ steht für:

(i) $C_{3-6}$-Cycloalkyl, optional substituiert mit einem oder mehreren Substituenten, unabhängig ausgewählt aus -OH und $-C_{1-3}$-Alkyl;
(ii) Aryl oder Heteroaryl, jeweils optional substituiert mit 1 bis 3 Substituenten, unabhängig ausgewählt aus Halogen, -OH, $-O-C_{1-3}$-Alkyl, $-C_{1-3}$-Alkyl, Halogen-$C_{1-3}$-Alkyl, Hydroxy-$C_{1-3}$-Alkyl, Halogen-$C_{1-3}$-Alkoxy; oder
(iii) Heterocyclyl, optional substituiert mit 1 bis 3 Substituenten, unabhängig ausgewählt aus $C_{1-3}$-Alkyl und $C_{3-6}$-Cycloalkyl;

$R^2$ steht für:

(i) $C_{1-3}$-Alkyl, optional substituiert mit einem oder mehreren Substituenten, unabhängig ausgewählt aus Halogen, -OH, $-OC_{1-3}$-Alkyl und Oxo;
(ii) $C_{3-6}$-Cycloalkyl;
(iii) $C_{2-4}$-Alkenyl optional substituiert mit $-O-C_{1-3}$-Alkyl;
(iv) $-O-C_{1-3}$-Alkyl;
(v) -N(H)Alkyl oder $-N-(C_{1-3}\text{-Alkyl})_2$, wobei jedes Alkyl optional durch $-OC_{1-3}$-Alkyl substituiert sein kann; oder
(vi) Heterocyclyl;

$R^3$ steht für:

(i) Wasserstoff;
(ii) Halogen;
(iii) $C_{1-4}$-Alkyl, optional substituiert mit einem oder mehreren Substituenten, unabhängig ausgewählt aus Halogen, -OH, Oxo, $-OC_{1-3}$-Alkyl, -C(O)OH,
-C(O)N(H)Heteroaryl, -C(O)N(H)Aryl, $-C(O)N(H)C_{1-3}$-Alkyl und $-C(O)N(C_{1-3}\text{-Alkyl})_2$;
(iv) $C_{2-4}$-Alkenyl;
(v) $C_{3-6}$-Cycloalkyl;
(vi) $-OC_{1-4}$-Alkyl;
(vii) $-N(H)C_{1-3}$-Alkyl oder $-N(C_{1-3}\text{-Alkyl})_2$;
(viii) $-C(O)N(H)C_{1-3}$-Alkyl oder $-C(O)N(C_{1-3}\text{-Alkyl})_2$;
(ix) Aryl oder Heteroaryl; oder
(x) Heterocyclyl.

unter der Voraussetzung, dass:

(i) wenn $R^2$ für $-CH_3$ steht, $R^3$ für H steht, dann steht $R^1$ nicht für Cyclohexyl, 2,3-Dimethylcyclohexyl, 2-Methoxyphenyl, 2,3,5,6-Tetrafluorphenyl, 2-Brom-4,6-difluorphenyl, 4-Methylphenyl, 4-(Diethylamino)-2-methylphenyl, 5-Chlor-2-methoxyphenyl, 2,5-Difluorphenyl, 2,3-Dihydro-1,4-benzodioxin-6-yl, 4-Methylcyclohexyl, 1,2,3,4-Tetrahydro-1-naphthalenyl, 3-Ethylphenyl, 4-Isopropylphenyl, 2-Ethyl-6-methylphenyl, 4-Brom-3-methylphenyl, 3,5-Dimethylphenyl, 2,4,6-Trimethylphenyl, 3,4-Dimethylphenyl, 2-Chlor-4-methylphenyl, 2,4-Dimethylphenyl, 2,3,4,5,6-Pentafluorphenyl, 2,5-Dimethylphenyl, 3-Chlor-2-methylphenyl, 2-Methylphenyl, 4-Brom-2-fluorphenyl, 4-Fluorphenyl, 2-Fluorphenyl, 4-Ethylphenyl, 2-Ethylphenyl, 2,6-Dimethylphenyl, 2,3-Dimethylphenyl, 2,4-Difluorphenyl, Cyclopentyl, Cycloheptyl, 5-Chlor-2-methylphenyl, 5-Chlor-2,4-dimethoxyphenyl, 4-Chlorphenyl, 2-Chlorphenyl, 2-Bromphenyl, 3-Methoxyphenyl, 3,4-Dimethoxyphenyl, 3-(Trifluormethyl)phenyl, 2-(Trifluormethyl)phenyl oder 2,4-Di(-C(O)$OCH_3$)phenyl (oder 2,4-(Dicarbonsäuremethylester)-phenyl);
(ii) wenn $R^2$ für -$CH_2CH_3$ steht, $R^3$ für H steht, dann steht $R^1$ nicht für Cyclopentyl oder Cycloheptyl.

**3.** Verbindung nach Anspruch 1 oder Anspruch 2, wobei $R^1$ für $C_{3-6}$-Cycloalkyl steht, optional substituiert durch einen oder zwei Substituenten ausgewählt aus $C_{1-3}$-Alkyl und -OH.

**4.** Verbindung nach Anspruch 1 oder Anspruch 2, wobei $R^1$ für eine monocyclische 5- oder 6-gliedrige Heterocyclylgruppe steht, die mindestens ein Stickstoff-Heteroatom enthält und gegebenenfalls durch einen Substituenten substituiert ist, ausgewählt aus $C_{1-3}$-Alkyl und $C_{3-6}$-Cycloalkyl.

**5.** Verbindung nach Anspruch 1 oder Anspruch 2, wobei $R^1$ steht für: (i) Phenyl; (ii) eine 5- oder 6-gliedrige monozyklische Heteroarylgruppe; oder (iii) eine 9- oder 10-gliedrige bicyclische Heteroarylgruppe, die alle optional mit einem oder zwei Substituenten ausgewählt aus Halogen, -OH und -$OC_{1-3}$-Alkyl substituiert sind.

**6.** Verbindung nach Anspruch 1 oder 2, wobei $R^1$ für Cyclopropyl steht, wie in Anspruch 4 oder Anspruch 5 definiert,

**7.** Verbindung nach einem der Ansprüche 1 bis 6, wobei $R^2$ steht für (i) $C_{1-3}$-Alkyl, optional substituiert durch einen oder zwei Substituenten, ausgewählt aus -OH, Methoxy, Ethoxy und Oxo; (ii) $C_{2-4}$-Alkenyl, optional substituiert mit Methoxy oder Ethoxy; (iii) -N-($C_{1-3}$-Alkyl)$_2$, wobei der Alkylrest unsubstituiert ist; (iv) eine 6-gliedrige Heterocyclylgruppe, in der mindestens ein Stickstoff-Heteroatom und optional ein Sauerstoff-Heteroatom vorhanden sind.

**8.** Verbindung nach Anspruch 7, wobei $R^2$ für unsubstituiertes $C_{1-3}$-Alkyl steht.

**9.** Verfahren nach einem der Ansprüche 1 bis 8, wobei $R^3$ steht für: (i) Wasserstoff; (ii) Halogen; (iii) $C_{1-4}$-Alkyl optional durch einen oder mehrere Substituenten substituiert ist, die ausgewählt sind aus Fluor, -OH und -O-$C_{1-2}$-Alkyl; (iv) $C_{2-4}$-Alkenyl; oder (v) $C_{3-4}$-Cycloalkyl.

**10.** Pharmazeutische Zusammensetzung, die eine therapeutisch wirksame Menge einer Verbindung wie in einem der Ansprüche 1 bis 9 definiert und einen pharmazeutisch verträglichen Träger umfasst.

**11.** Verfahren zum Herstellen einer pharmazeutischen Zusammensetzung wie in Anspruch 10 definiert, **dadurch gekennzeichnet, dass** ein pharmazeutisch verträglicher Träger mit einer therapeutisch wirksamen Menge einer Verbindung nach einem der Ansprüche 1 bis 9 innig vermischt wird.

**12.** Verbindung nach einem der Ansprüche 1 bis 9, zur Verwendung als ein Pharmazeutikum oder Medikament.

**13.** Verbindung, Zusammensetzung oder Kombination zur Verwendung nach Anspruch 12, wobei die Krankheit oder Störung, die mit der Hemmung der NLRP3-Inflammasomaktivität verbunden ist, ausgewählt ist aus Inflammasombezogenen Krankheiten und Störungen, Immunkrankheiten, entzündlichen Krankheiten, Autoimmunkrankheiten, autoinflammatorischen Fiebersyndromen, Cryopyrin-assoziiertem periodischem Syndrom, chronischer Leberkrankheit, viraler Hepatitis, nichtalkoholischer Steatohepatitis, alkoholischer Steatohepatitis, alkoholischer Leberkrankheit, entzündlichen Arthritis-bezogenen Störungen, Gicht, Chondrokalzinose, Osteoarthritis, rheumatoider Arthritis, chronischer Arthropathie, akuter Arthropathie, Nieren-bezogenen Krankheiten, Hyperoxalurie, Lupusnephritis, Typ-I- und Typ-II-Diabetes, Nephropathie, Retinopathie, hypertensiver Nephropathie, Hämodialyse-bezogenen Entzündungen, Neuroinflammation-bezogenen Krankheiten, Multipler Sklerose, Gehirninfektion, akuter Verletzung, neurodegenerativen Krankheiten, Alzheimer-Krankheit, Herz-Kreislauf-Krankheiten, Stoffwechselkrankheiten, Herz-Kreislauf-Risikominderung, Bluthochdruck, Arteriosklerose, periphere arterielle Verschlusskrankheit, akute Herzinsuffizienz, entzündliche Hauterkrankungen, Akne, Wundheilung und Narbenbildung, Asthma, Sarkoidose, altersbedingte Makuladegeneration, Dickdarmkrebs, Lungenkrebs, myeloproliferative Neoplasien, Leukämien, myelodysplastische Syndrome und Myelofibrose.

**14.** Verfahren zur Herstellung einer Verbindung der Formel (I) nach einem der Ansprüche 1 bis 9, welches umfasst:

(i) Umsetzen einer Verbindung der Formel (II),

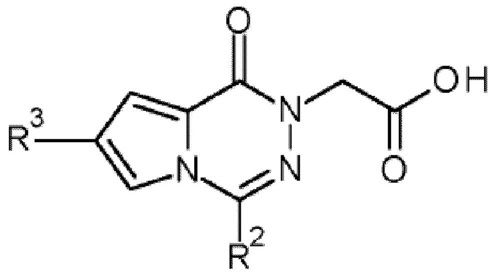

(II)

oder eines Derivats davon, worin $R^2$ und $R^3$ wie in Anspruch 1 definiert sind, mit einer Verbindung der Formel (III),

$H_2N-R^1$ (III)

oder einem Derivat davon, wobei $R^1$ wie in Anspruch 1 definiert ist, unter amidbildenden Umsatzbedingungen;

(ii) Umsetzen einer Verbindung der Formel (IV),

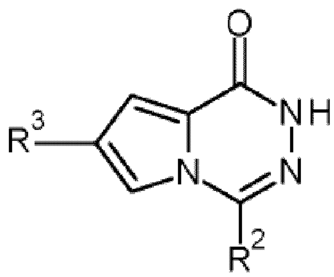

(IV)

worin $R^2$ und $R^3$ wie in Anspruch 1 definiert sind, mit einer Verbindung der Formel (V),

$LG^a-CH_2-C(O)-N(H)R^1$ (V)

worin $LG^a$ eine geeignete Abgangsgruppe darstellt und $R^1$ wie in Anspruch 1 definiert ist;

(iii) durch Umwandlung einer bestimmten Verbindung der Formel (I) in eine andere.

## Revendications

**1.** Composé de Formule (I)

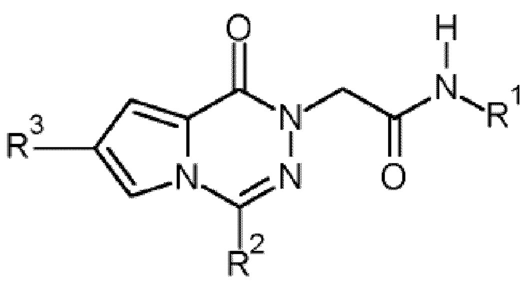

(I)

ou un sel pharmaceutiquement acceptable de celui-ci, où :

$R^1$ représente :

(i) $C_{3-6}$ cycloalkyle éventuellement substitué par un ou plusieurs substituants choisis indépendamment parmi -OH et -$C_{1-3}$ alkyle ;
(ii) aryle ou hétéroaryle, chacun étant éventuellement substitué par 1 à 3 substituants choisis indépendamment parmi halo, -OH, -O-$C_{1-3}$ alkyle, -$C_{1-3}$ alkyle, halo$C_{1-3}$alkyle, hydroxy$C_{1-3}$ alkyle, halo$C_{1-3}$alkoxy ; ou
(iii) hétérocyclyle, éventuellement substitué par 1 à 3 substituants choisis indépendamment parmi $C_{1-3}$ alkyle and $C_{3-6}$ cycloalkyle ;

$R^2$ représente :

(i) $C_{1-6}$ alkyle éventuellement substitué par un ou plusieurs substituants choisis indépendamment parmi halo, -OH, -O$C_{1-3}$alkyle, -O$C_{3-6}$ cycloalkyle, - N(H)C(O)$C_{1-3}$ alkyle et oxo ;
(ii) $C_{3-6}$ cycloalkyle ;
(iii) $C_{2-4}$alkényle éventuellement substitué par -O-$C_{1-3}$alkyle ;
(iv) -O-$C_{1-3}$alkyle ;
(v) -C(O)$C_{1-3}$alkyle ;
(vi) -N(H)$C_{1-3}$alkyle ou -N-($C_{1-3}$alkyle)$_2$, où chaque alkyle peut être éventuellement substitué par -O$C_{1-3}$ alkyle or $C_{3-6}$ cycloalkyle ; ou
(vii) hétérocyclyle (éventuellement substitué par un ou plusieurs substituants choisis parmi halo, $C_{1-3}$ alkyle, -C(O)$C_{1-3}$alkyle et -C(O)O$C_{1-4}$alkyle) ;

$R^3$ représente :

(i) hydrogène ;
(ii) halo ou -CN ;
(iii) $C_{1-4}$ alkyle éventuellement substitué par un ou plusieurs substituants choisis indépendamment parmi halo, -OH, oxo, -O-$C_{1-3}$alkyle, -C(O)OH,
-C(O)N(H)hétéroaryle, -C(O)N(H)aryle, -C(O)N(H)$C_{1-3}$ alkyle, -C(O)N($C_{1-3}$-alkyle)$_2$, -N($C_{1-3}$ alkyl)-C(O)-$C_{1-3}$alkyle, -N(H)$C_{1-3}$ alkyle, hétérocyclyle, aryle et hétéroaryle, lesquels trois derniers groupes peuvent eux-mêmes être éventuellement substitués par un ou plusieurs substituants choisis parmi halo, $C_{1-3}$ alkyle et, le cas échéant, =0 ;
(iv) $C_{2-4}$ alkényle ;
(v) $C_{3-6}$ cycloalkyle ;
(vi) -O$C_{1-4}$ alkyle ;
(vii) -C(O)H ou -C(O)$C_{1-3}$ alkyle ;
(viii) -N(H)$C_{1-3}$alkyle ou - N($C_{1-3}$alkyle)$_2$ ;
(ix) -C(O)N(H)$C_{1-3}$alkyle ou -C(O)N($C_{1-3}$alkyle)$_2$ ;
(x) aryle ou hétéroaryle, lesquels groupes peuvent eux-mêmes être éventuellement substitués par un ou plusieurs substituants choisis parmi halo, $C_{1-3}$ alkyle, -C(O)O$C_{1-4}$alkyle et, le cas échéant, =0 ; ou
(xi) Hétérocyclyle, éventuellement substitué par un ou plusieurs substituants choisis parmi halo, $C_{1-3}$ alkyle, -C(O)O$C_{1-4}$alkyle et, le cas échéant, =0,

à condition que :

(i) lorsque $R^2$ représente -$CH_3$, $R^3$ représente H, alors $R^1$ ne représente pas cyclohexyle, 2,3-diméthylcyclohexyle, 2-méthoxyphényle, 2,3,5,6-tétrafluorophényle, 2-bromo-4,6-difluorophényle, 4-méthylphényle, 4-(diéthylamino)-2-méthylphényle, 5-chloro-2-méthoxyphényle, 2,5-difluorophényle, 2,3-dihydro-1,4-benzodioxin-6-yl, 4-méthylcyclohexyle, 1,2,3,4-tétrahydro-1-naphtalényle, 3-éthylphényle, 4-isopropylphényle, 2-éthyl-6-méthylphényle, 4-bromo-3-méthylphényle, 3,5-diméthylphényle, 2,4,6-triméthylphényle, 3,4-diméthylphényle, 2-chloro-4-méthylphényle, 2,4-diméthylphényle, 2,3,4,5,6-pentafluorophényle, 2,5-diméthylphényle, 3-chloro-2-méthylphényle, 2-méthylphényle, 4-bromo-2-fluorophényle, 4-fluorophényle, 2-fluorophényle, 4-éthylphényle, 2-éthylphényle, 2,6-diméthylphényle, 2,3-diméthylphényle, 2,4-difluorophényle, cyclopentyle, cycloheptyle, 5-chloro-2-méthylphényle, 5-chloro-2,4-diméthoxyphényle, 4-chlorophényle, 2-chlorophényle, 2-bromophényle, 3-méthoxyphényle, 3,4-diméthoxyphényle, 3-(trifluorométhyl)phényle ou 2-(trifluorométhyl)phényle ;

(ii) lorsque $R^2$ représente $-CH_2CH_3$, $R^3$ représente H, alors $R^1$ ne représente pas cyclopentyle ou cycloheptyle.

**2.** Composé selon la revendication 1, dans lequel :

$R^1$ représente :

(i) $C_{3-6}$ cycloalkyle éventuellement substitué par un ou plusieurs substituants choisis indépendamment parmi -OH et $-C_{1-3}$ alkyle ;
(ii) aryle ou hétéroaryle, chacun étant éventuellement substitué par 1 à 3 substituants choisis indépendamment parmi halo, -OH, $-O-C_{1-3}$ alkyle, $-C_{1-3}$ alkyle, halo$C_{1-3}$alkyle, hydroxy$C_{1-3}$ alkyle, halo$C_{1-3}$alkoxy ; ou
(iii) hétérocyclyle, éventuellement substitué par 1 à 3 substituants choisis indépendamment parmi $C_{1-3}$ alkyle and $C_{3-6}$ cycloalkyle ;

$R^2$ représente :

(i) $C_{1-3}$ alkyle éventuellement substitué par un ou plusieurs substituants choisis indépendamment parmi halo, -OH, $-OC_{1-3}$alkyle et oxo ;
(ii) $C_{3-6}$cycloalkyle ;
(iii) $C_{2-4}$alkényle éventuellement substitué par $-O-C_{1-3}$ alkyle ;
(iv) $-O-C_{1-3}$alkyle ;
(v) -N(H)alkyle ou $-N-(C_{1-3}alkyle)_2$, où chaque alkyle peut être éventuellement substitué par $-OC_{1-3}$ alkyle ; ou
(vi) hétérocyclyle ;

$R^3$ représente :

(i) hydrogène ;
(ii) halo ;
(iii) $C_{1-4}$ alkyle éventuellement substitué par un ou plusieurs substituants choisis indépendamment parmi halo, -OH, oxo, $-O-C_{1-3}$alkyle, -C(O)OH,
-C(O)N(H)hétéroaryle, -C(O)N(H)aryle, $-C(O)N(H)C_{1-3}$ alkyle et $-C(O)N(C_{1-3}alkyle)_2$ ;
(iv) $C_{2-4}$ alkényle ;
(v) $C_{3-6}$ cycloalkyle ;
(vi) $-OC_{1-4}$ alkyle ;
(vii) $-N(H)C_{1-3}$alkyle ou - $N(C_{1-3}alkyle)_2$ ;
(viii) $-C(O)N(H)C_{1-3}$alkyle ou $-C(O)N(C_{1-3}alkyle)_2$ ;
(ix) aryle ou hétéroaryle ; ou
(x) hétérocyclyle.

à condition que :

(i) lorsque $R^2$ représente $-CH_3$, $R^3$ représente H, alors $R^1$ ne représente pas cyclohexyle, 2,3-diméthylcyclohexyle, 2-méthoxyphényle, 2,3,5,6-tétrafluorophényle, 2-bromo-4,6-difluorophényle, 4-méthylphényle, 4-(diéthylamino)-2-méthylphényle, 5-chloro-2-méthoxyphényle, 2,5-difluorophényle, 2,3-dihydro-1,4-benzodioxin-6-yl, 4-méthylcyclohexyle, 1,2,3,4-tétrahydro-1-naphtalényle, 3-éthylphényle, 4-isopropylphényle, 2-éthyl-6-méthylphényle, 4-bromo-3-méthylphényle, 3,5-diméthylphényle, 2,4,6-triméthylphényle, 3,4-diméthylphényle, 2-chloro-4-méthylphényle, 2,4-diméthylphényle, 2,3,4,5,6-pentafluorophényle, 2,5-diméthylphényle, 3-chloro-2-méthylphényle, 2-méthylphényle, 4-bromo-2-fluorophényle, 4-fluorophényle, 2-fluorophényle, 4-éthylphényle, 2-éthylphényle, 2,6-diméthylphényle, 2,3-diméthylphényle, 2,4-difluorophényle, cyclopentyle, cycloheptyle, 5-chloro-2-méthylphényle, 5-chloro-2,4-diméthoxyphényle, 4-chlorophényle, 2-chlorophényle, 2-bromophényle, 3-méthoxyphényle, 3,4-diméthoxyphényle, 3-(trifluorométhyl)phényle, 2-(trifluorométhyl)phényle ou 2,4-di($-C(O)OCH_3$)phényle (ou 2,4-(ester méthylique d'acide dicarboxylique)-phényle) ;
(ii) lorsque $R^2$ représente $-CH_2CH_3$, $R^3$ représente H, alors $R^1$ ne représente pas cyclopentyle ou cycloheptyle.

3. Composé selon la revendication 1 ou la revendication 2, dans lequel $R^1$ représente $C_{3-6}$ cycloalkyle éventuellement substitué par un ou deux substituants choisis parmi $C_{1-3}$ alkyle et -OH.

4. Composé selon la revendication 1 ou la revendication 2, dans lequel $R^1$ représente un groupe hétérocyclyle mono-cyclique à 5 ou 6 chaînons contenant au moins un hétéroatome d'azote, et qui est éventuellement substitué par un substituant choisi parmi $C_{1-3}$ alkyle et $C_{3-6}$ cycloalkyle.

5. Composé selon la revendication 1 ou la revendication 2, dans lequel $R^1$ représente : (i) phényle ; (ii) un groupe hétéroaryle mono-cyclique à 5 ou 6 chaînons ; ou (iii) un groupe hétéroaryle bicyclique à 9 ou 10 chaînons, tous éventuellement substitués par un ou deux substituants choisis parmi halo, -OH et - $OC_{1-3}$ alkyle.

6. Composé selon la revendication 1 ou la revendication 2, dans lequel $R^1$ représente cyclopropyle, selon la revendication 4 ou la revendication 5,

7. Composé selon l'une quelconque des revendications 1 à 6, dans lequel $R^2$ représente (i) un $C_{1-3}$ alkyle éventuellement substitué par un ou deux substituants choisis parmi -OH, méthoxy, éthoxy et oxo ; (ii) $C_{2-4}$alkényle éventuellement substitué par méthoxy ou éthoxy ; (iii) -N-($C_{1-3}$alkyle)$_2$, où la fraction alkyle est non substituée ; (iv) un groupe hétérocyclyle à 6 chaînons dans lequel il y a au moins un hétéroatome d'azote et éventuellement un hétéroatome d'oxygène.

8. Composé selon la revendication 7, dans lequel $R^2$ représente $C_{1-3}$ alkyle non substitué.

9. Procédé selon l'une quelconque des revendications 1 à 8, dans lequel $R^3$ représente : (i) hydrogène ; (ii) halo ; (iii) $C_{1-4}$ alkyle éventuellement substitué par un ou plusieurs substituants choisis parmi fluoro, -OH et -O-$C_{1-2}$ alkyle ; (iv) $C_{2-4}$alkényle ; ou (v) $C_{3-4}$ cycloalkyle.

10. Composition pharmaceutique comprenant une quantité thérapeutiquement efficace d'un composé selon l'une quelconque des revendications 1 à 9 et un support pharmaceutiquement acceptable.

11. Procédé pour la préparation d'une composition pharmaceutique selon la revendication 10, **caractérisé en ce qu'**un support pharmaceutiquement acceptable est intimement mélangé avec une quantité thérapeutiquement efficace d'un composé selon l'une quelconque des revendications 1 à 9.

12. Composé selon l'une quelconque des revendications 1 à 9, pour une utilisation en tant que produit pharmaceutique ou médicament.

13. Composé, composition ou combinaison pour une utilisation selon la revendication 12, dans lequel la maladie ou le trouble associé à une inhibition de l'activité de l'inflammasome NLRP3 est choisi parmi maladies et troubles liés à l'inflammasome, maladies immunitaires, maladies inflammatoires, maladies autoimmunes, syndromes de fièvre auto-inflammatoire, syndrome périodique associé à la cryopyrine, maladie chronique du foie, hépatite virale, stéatohépatite non alcoolique, stéatohépatite alcoolique, maladie alcoolique du foie, troubles inflammatoires liés à l'arthrite, goutte, chondrocalcinose, ostéoarthrite, arthrite rhumatoïde, arthropathie chronique, arthropathie aiguë, maladie rénale, hyperoxalurie, néphrite lupique, diabète de type I et de type II, néphropathie, rétinopathie, néphropathie hypertensive, inflammation liée à l'hémodialyse, maladies liées à la neuroinflammation, sclérose en plaques, infection cérébrale, lésion aiguë, maladies neurodégénératives, maladie d'Alzheimer, maladies cardiovasculaires, maladies métaboliques, réduction du risque cardiovasculaire, hypertension, athérosclérose, artériopathie périphérique, insuffisance cardiaque aiguë, maladies inflammatoires de la peau, acné, cicatrisation et formation de cicatrices, asthme, sarcoïdose, dégénérescence maculaire liée à l'âge, cancer du côlon, cancer du poumon, néoplasmes myéloprolifératifs, leucémies, syndromes myélodysplasiques et myélofibrose.

14. Procédé pour la préparation d'une composition de formule (I) selon l'une quelconque des revendications 1 à 9, qui comprend :

(i) une réaction d'un composé de formule (II),

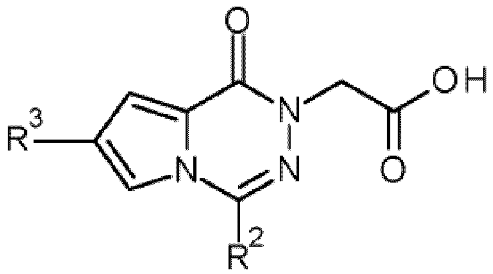

(II)

ou un dérivé de celui-ci, où $R^2$ et $R^3$ sont tels que définis dans la revendication 1, avec un composé de formule (III),

$H_2N-R^1$ (III)

ou un dérivé de celui-ci, dans lequel $R^1$ est tel que défini dans la revendication 1, dans des conditions de réaction de formation d'amide ;

(ii) une réaction d'un composé de formule (IV),

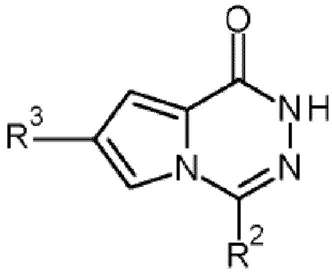

(IV)

où $R^2$ et $R^3$ sont tels que définis dans la revendication 1, avec un composé de formule (V),

$LG^a-CH_2-C(O)-N(H)R^1$ (V)

où $LG^a$ représente un groupe partant approprié et $R^1$ est tel que défini dans la revendication 1 ;

(iii) par transformation d'un certain composé de formule (I) en un autre.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020018975 A
- WO 2020037116 A
- WO 2020021447 A
- WO 2020010143 A
- WO 2019079119 A
- WO 20190166621 A
- WO 2019121691 A


### Non-patent literature cited in the description


- **VAN OPDENBOSCH N ; LAMKANFI M.** *Immunity,* 18 June 2019, vol. 50 (6), 1352-1364
- **TARTEY S ; KANNEGANTI TD.** *Immunology,* April 2019, vol. 156 (4), 329-338
- **KELLEY et al.** *Int J Mol Sci,* 06 July 2019, vol. 20 (13
- **FUNG et al.** *Emerg Microbes Infect,* 14 March 2020, vol. 9 (1), 558-570
- **YANG Y et al.** *Cell Death Dis,* 12 February 2019, vol. 10 (2), 128
- **SHARIF et al.** *Nature,* June 2019, vol. 570 (7761), 338-343
- **MIYAMAE T.** *Paediatr Drugs,* 01 April 2012, vol. 14 (2), 109-17
- **SZABO G ; PETRASEK J.** *Nat Rev Gastroenterol Hepatol,* July 2015, vol. 12 (7), 387-400
- **ZHEN Y ; ZHANG H.** *Front Immunol,* 28 February 2019, vol. 10, 276
- **VANDE WALLE L et al.** *Nature,* 07 August 2014, vol. 512 (7512), 69-73
- **KNAUF et al.** *Kidney Int,* November 2013, vol. 84 (5), 895-901
- **KRISHNAN et al.** *Br J Pharmacol,* February 2016, vol. 173 (4), 752-65
- **SHAHZAD et al.** *Kidney Int,* January 2015, vol. 87 (1), 74-84
- **SARKAR et al.** *NPJ Parkinsons Dis,* 17 October 2017, vol. 3, 30
- **RIDKER et al.** *CANTOS Trial Group. N Engl J Med,* 21 September 2017, vol. 377 (12), 1119-1131
- **TOLDO S ; ABBATE A.** *Nat Rev Cardiol,* April 2018, vol. 15 (4), 203-214
- **KELLY et al.** *Br J Dermatol,* December 2015, vol. 173 (6
- **NIETO-TORRES et al.** *Virology,* November 2015, vol. 485, 330-9
- **DOYLE et al.** *Nat Med,* May 2012, vol. 18 (5), 791-8
- **RIDKER et al.** *Lancet,* 21 October 2017, vol. 390 (10105), 1833-1842
- **DERANGERE et al.** *Cell Death Differ.,* December 2014, vol. 21 (12), 1914-24
- **BASIORKA et al.** *Lancet Haematol,* September 2018, vol. 5 (9), e393-e402
- **ZHANG et al.** *Hum Immunol,* January 2018, vol. 79 (1), 57-62
- **BUNDEGAARD, H.** Design of Prodrugs. Elesevier, 1985, 1-92
- **T.W. GREENE ; P.G.M. WUTZ.** Protective Groups in Organic Synthesis. Wiley-Interscience, 1999
- **MENU et al.** *Clinical and Experimental Immunology,* 2011, vol. 166, 1-15
- **STROWIG et al.** *Nature,* 2012, vol. 481, 278-286
- **OZAKI et al.** *J. Inflammation Research,* 2015, vol. 8, 15-27
- **SCHRODER et al.** *Cell,* 2010, vol. 140, 821-832
- **COOK et al.** *Eur. J. Immunol.,* 2010, vol. 40, 595-653
- **BRADDOCK et al.** *Nat. Rev. Drug Disc.,* 2004, vol. 3, 1-10
- **INOUE et al.** *Immunology,* 2013, vol. 139, 11-18
- **COLL et al.** *Nat. Med.,* 2015, vol. 21 (3), 248-55
- **SCOTT et al.** *Clin. Exp. Rheumatol.,* 2016, vol. 34 (1), 88-93
- **LU et al.** *J. Immunol.,* 2017, vol. 198 (3), 1119-29
- **ARTLETT et al.** *Arthritis Rheum.,* 2011, vol. 63 (11), 3563-74
- **DE NARDO et al.** *Am. J. Pathol.,* 2014, vol. 184, 42-54
- **KIM et al.** *Am. J. Respir. Crit. Care Med,* 2017, vol. 196 (3), 283-97
- **WALSH et al.** *Nature Reviews,* 2014, vol. 15, 84-97
- **DEMPSEY et al.** *Brain. Behav. Immun.,* 2017, vol. 61, 306-16

- **ZHANG et al.** *J. Stroke and Cerebrovascular Dis.,* 2015, vol. 24 (5), 972-9
- **ISMAEL et al.** *J. Neurotrauma,* 2018, vol. 35 (11), 1294-1303
- **WEN et al.** *Nature Immunology,* 2012, vol. 13, 352-357
- **DUEWELL et al.** *Nature,* 2010, vol. 464, 1357-1361
- **STROWIG et al.** *Nature,* 2014, vol. 481, 278-286
- **MRIDHA et al.** *J. Hepatol.,* 2017, vol. 66 (5), 1037-46
- **VAN HOUT et al.** *Eur. Heart J.,* 2017, vol. 38 (11), 828-36
- **SANO et al.** *J. Am. Coll. Cardiol.,* 2018, vol. 71 (8), 875-66
- **WU et al.** *Arteriosc/er. Thromb. Vase. Biol.,* 2017, vol. 37 (4), 694-706
- **RIDKER et al.** *N. Engl. J. Med.,* 2017, vol. 377 (12), 1119-31
- **DOYLE et al.** *Nature Medicine,* 2012, vol. 18, 791-798
- **TARALLO et al.** *Cell,* 2012, vol. 149 (4), 847-59
- **LOUKOVAARA et al.** *Acta Ophthalmol.,* 2017, vol. 95 (8), 803-8
- **PUYANG et al.** *Sci. Rep.,* 2016, vol. 6, 20998
- **HENAO-MEIJA et al.** *Nature,* 2012, vol. 482, 179-185
- **PRIMIANO et al.** *J. Immunol.,* 2016, vol. 197 (6), 2421-33
- **FANG et al.** *J Dermatol Sci.,* 2016, vol. 83 (2), 116-23
- **NIEBUHR et al.** *Allergy,* 2014, vol. 69 (8), 1058-67
- **ALIKHAN et al.** *J. Am. Acad. Dermatol.,* 2009, vol. 60 (4), 539-61
- **JAGER et al.** *Am. J. Respir. Crit. Care Med.,* 2015, vol. 191, A5816
- **BRADDOCK et al.** *Nat. Rev. Drug Disc,* 2004, vol. 3, 1-10
- **GUGLIANDOLO et al.** *Int. J. Mol. Sci.,* 2018, vol. 19 (7), E1992
- **LANNITTI et al.** *Nat. Commun.,* 2016, vol. 7, 10791
- **GRANATA et al.** *PLoS One,* 2015, vol. 10 (3), eoi22272
- **NEUDECKER.** *J. Exp. Med.,* 2017, vol. 214 (6), 1737-52
- **LAZARIDIS et al.** *Dig. Dis. Sci.,* 2017, vol. 62 (9), 2348-56
- **DOLUNAY et al.** *Inflammation,* 2017, vol. 40, 366-86
- **TATE et al.** *Sci Rep.,* 2016, vol. 10 (6), 27912-20
- **NOVIAS et al.** *PLOS Pathogens,* 2017, vol. 13 (2), e1006196
- **RIDKER et al.** *Lancet,* 2017, vol. 390 (10105), 1833-42
- **WANG et al.** *Onco/Rep.,* 2016, vol. 35 (4), 2053-64
- **BASIORKA et al.** *Blood,* 2016, vol. 128 (25), 2960-75
- **LI et al.** *Am. J. Cancer Res.,* 2015, vol. 5 (1), 442-9
- **ALLEN et al.** *J. Exp. Med.,* 2010, vol. 207 (5), 1045-56
- **HU et al.** *PNAS,* 2010, vol. 107 (50), 21635-40
- **LI et al.** *Hematology,* 2016, vol. 21 (3), 144-51
- **HUANG et al.** *J. Exp. Clin. Cancer Res.,* 2017, vol. 36 (1), 116
- **FENG et al.** *J. Exp. Clin. Cancer Res.,* 2017, vol. 36 (1), 81
- **JIA et al.** *Mol. Pain.,* 2017, vol. 13, 1-11
- **YAN-GANG et al.** *Cell Death and Disease,* 2017, vol. 8 (2), 2579
- **ALEXANDER et al.** *Hepatology,* 2014, vol. 59 (3), 898-910
- **BALDWIN et al.** *J. Med. Chem.,* 2016, vol. 59 (5), 1691-1710
- **OZAKI et al.** *J. Inflammation Research,* 2015, vol. 8, 15-27
- **ZHEN et al.** *Neuroimmunology Neuroinflammation,* 2014, vol. 1 (2), 60-65
- **MATTIA et al.** *J. Med. Chem.,* 2014, vol. 57 (24), 10366-82
- **SATOH et al.** *Cell Death and Disease,* 2013, vol. 4, 644
- Remington The Science and Practice of Pharmacy. Pharmaceutical Press, 2013, 1049-1070